(19) Europäisches Patentamt
European Patent Office
Office européen des brevets

(11) EP 2 323 980 B1

# (12) EUROPÄISCHE PATENTSCHRIFT

(45) Veröffentlichungstag und Bekanntmachung des Hinweises auf die Patenterteilung:
**29.02.2012 Patentblatt 2012/09**

(21) Anmeldenummer: **09782239.9**

(22) Anmeldetag: **26.08.2009**

(51) Int Cl.:
***C07D 209/42*** (2006.01) ***C07D 401/12*** (2006.01)
***C07D 403/12*** (2006.01) ***C07D 405/12*** (2006.01)
***C07D 405/14*** (2006.01) ***A61K 31/404*** (2006.01)
***A61P 11/00*** (2006.01)

(86) Internationale Anmeldenummer:
**PCT/EP2009/061025**

(87) Internationale Veröffentlichungsnummer:
**WO 2010/026096 (11.03.2010 Gazette 2010/10)**

(54) **7-(PIPERAZIN-1-YLMETHYL)-1H-INDOL-2-CARBONSÄURE (PHENYL) -AMIDDERIVATE UND VERWANDTE VERBINDUNGEN ALS P38 MAP-KINASE-INHIBITOREN ZUR BEHANDLUNG VON ATEMWEGSERKRANKUNGEN**

7-(PIPERAZINE-1-YMETHYL)-1H-INDOLE-2-CARBOXYLIC ACID (PHENYL)-AMIDE DERIVATIVES AND ALLIED COMPOUNDS AS P38 MAP KINASE INHIBITORS FOR THE TREATMENT OF RESPIRATORY DISEASES

DÉRIVÉS 7-(PIPÉRAZIN-1-YLMÉTHYL)-1H-INDOL-2-ACIDE CARBOXYLIQUE (PHÉNYL) -AMIDES ET COMPOSÉS ANALOGUES UTILISÉS COMME INHIBITEURS DE LA P38 MAP-KINASE POUR LE TRAITEMENT DES MALADIES DES VOIES RESPIRATOIRES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **02.09.2008 EP 08163526**

(43) Veröffentlichungstag der Anmeldung:
**25.05.2011 Patentblatt 2011/21**

(73) Patentinhaber: **Boehringer Ingelheim International GmbH
55216 Ingelheim am Rhein (DE)**

(72) Erfinder:
- **WAGNER, Holger
55216 Ingelheim am Rhein (DE)**
- **JUNG, Birgit
55216 Ingelheim am Rhein (DE)**
- **HIMMELSBACH, Frank
55216 Ingelheim am Rhein (DE)**
- **GOEGGEL, Rolf
55216 Ingelheim am Rhein (DE)**
- **DAHMANN, Georg
55216 Ingelheim am Rhein (DE)**

(74) Vertreter: **Hammann, Heinz et al
Boehringer Ingelheim
Pharma GmbH & Co. KG
CD Patents
Binger Strasse 173
55216 Ingelheim Am Rhein (DE)**

(56) Entgegenhaltungen:
**WO-A-2006/026235 WO-A-2008/057775**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 323 980 B1

## Beschreibung

Gegenstand der vorliegenden Erfindung sind neue substituierte Heteroarylcarboxamid-Derivate der allgemeinen Formel

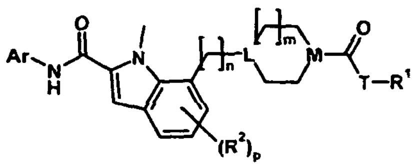

**I**

wobei die Reste Ar, L, M, T, m, n, p, $R^1$, und $R^2$ wie nachfolgend definiert sind, einschließlich deren Tautomere, deren Stereoisomere, deren Gemische und deren Salze. Ein weiterer Gegenstand dieser Erfindung betrifft Arzneimittel enthaltend eine erfindungsgemäße Verbindung der Formel **I** sowie die Verwendung einer erfindungsgemäßen Verbindung zur Herstellung eines Arzneimittels zur Behandlung von Atemwegserkrankungen. Darüber hinaus sind Verfahren zur Herstellung eines Arzneimittels Gegenstand dieser Erfindung.

In der Literatur werden Verbindungen, die eine Hemmwirkung auf das Enzym p38 Mitogenaktivierte Protein (MAP)-Kinase (auch p38 MAP-Kinase beziehungsweise p38-Kinase) besitzen, u.a. zur Behandlung von Atemwegserkrankungen, insbesondere von COPD und Asthma, vorgeschlagen (siehe P. J. Bames, Journal of Allergy and Clinical Immunology 2007, 119 (5), 1055-1062 und M. F. Fitzgerlad, J. C. Fox, Drug Discovery Today 2007, 12 (11/12), 479-486, sowie darin zitierte Literatur).

Aus den internationalen Offenlegungsschriften WO 2003/087085; WO 2005/016918; WO 2005/108387 und WO 2006/026235 sind substituierte Heteroarylcarboxamide sowie deren Herstellung und deren mögliche Aktivität als p38 MAP-Kinase-Inhibitoren bekannt.

## Aufgabe der Erfindung

Der vorliegenden Erfindung liegt die Aufgabe zugrunde, neue substituierte Heteroarylcarboxamide aufzuzeigen, insbesondere solche, die eine Aktivität auf das Enzym p38 MAP-Kinase besitzen. Eine weitere Aufgabe der vorliegenden Erfindung besteht im Aufzeigen von substituierten Heteroarylcarboxamiden, die *in vitro* und/oder *in vivo* eine Hemmwirkung auf das Enzym p38 MAP-Kinase besitzen und geeignete pharmakologische und/oder pharmakokinetische und/oder physikochemische Eigenschaften aufweisen, um diese als Arzneimittel verwenden zu können.

Ferner ist es eine Aufgabe der vorliegenden Erfindung, neue Arzneimittel bereit zu stellen, welche zur Prophylaxe und/oder Behandlung von Atemwegserkrankungen, insbesondere von COPD und Asthma geeignet sind.

Weitere Aufgaben der vorliegenden Erfindung ergeben sich für den Fachmann unmittelbar aus den vorhergehenden und nachfolgenden Ausführungen.

## Gegenstand der Erfindung

Ein erster Gegenstand der vorliegenden Erfindung sind Heteroarylcarboxamide der allgemeinen Formel **I**

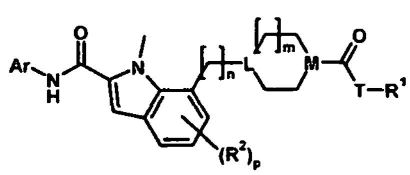

**I**

in der

Ar einen Substituenten der Formel (**II**), (**III**) oder (**IV**)

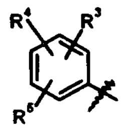 (II), 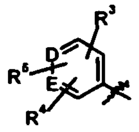 (III), 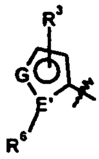 (IV)

bedeutet
worin $R^3$ $C_{1-6}$-Alkyl, $C_{3-6}$-Cycloalkyl, $C_{1-4}$-Alkoxy, $C_{1-2}$-Perfluoroalkyl, 3-Methyl-oxetan-3-yl, $C_{1-2}$-Perfluoroalkoxy, Morpholinyl bedeutet,
worin der Cycloalkyl-Rest gegebenenfalls mit $C_{1-3}$-Alkyl substituiert sein kann, worin $R^4$ H, $C_{1-4}$-Alkyl, $C_{1-4}$-Alkoxy bedeutet,
worin $R^5$ H, $C_{1-5}$-Alkyl-sulfonyl-amino, $C_{3-6}$-Cycloalkyl-sulfonyl-amino, ($C_{1-5}$-Alkyl-sulfonyl)-(methyl)-amino, ($C_{3-6}$-Cycloalkyl-sulfonyl)-(methyl)-amino, $C_{1-5}$-Alkyl-carbonyl-amino, $C_{3-6}$-Cycloalkyl-carbonyl-amino, ($C_{1-5}$-Alkyl-carbonyl)-(methyl)-amino, ($C_{3-6}$-Cycloalkyl-carbonyl)-(methyl)-amino, Aminocarbonyl, $C_{1-5}$-Alkyl-amino-carbonyl, $C_{3-6}$-Cycloalkyl-amino-carbonyl, Di-($C_{1-3}$-Alkyl)-amino-carbonyl, Di-($C_{1-3}$-Alkyl)-amino-$C_{1-2}$-alkyl, Di-($C_{1-3}$-Alkyl)-amino-pyrrolidin-1-yl-$C_{1-2}$-alkyl, 4-$C_{1-5}$-Alkyl-piperazin-1-yl-$C_{1-2}$-alkyl, 4-Di-($C_{1-3}$-Alkyl)-amino-piperidin-1-yl-$C_{1-2}$-alkyl, 3-Di-($C_{1-3}$-Alkyl)-amino-piperidin-1-yl-$C_{1-2}$-alkyl, $C_{1-5}$-Alkyl-sulfinyl, $C_{3-6}$-Cycloalkyl-sulfinyl, $C_{1-5}$-Alkyl-sulfonyl, $C_{3-6}$-Cycloalkyl-sulfonyl, Hydroxy-$C_{1-2}$-alkyl, $C_{1-5}$-Alkyl-sulfinyl-methyl, $C_{1-5}$-Alkyl-sulfonyl-methyl bedeutet,
worin $R^6$ $C_{1-3}$-Alkyl oder Phenyl bedeutet,
wobei der $C_{1-3}$-Alkyl-Rest gegebenenfalls mit Hydroxy oder Di-($C_{1-3}$-Alkyl)-amino substituiert sein kann und wobei der Phenyl-Rest gegebenenfalls mit Fluor oder $C_{1-3}$-Alkyl substituiert sein kann.
worin D oder E Stickstoff bedeuten,
worin G und E' unabhängig voneinander Stickstoff oder Sauerstoff bedeuten,
worin $R^6$ nur dann an E' gebunden ist, wenn E' Stickstoff bedeutet,

$R^1$ ein $C_{4-6}$-Cycloalkyl-System bedeutet, welches 1 bis 2-Stickstoffatome enthält, welches gegebenenfalls 1- bis 2-mal mit $R^7$ substituiert sein kann,
worin $R^7$ $C_{1-3}$-Alkyl, Hydroxy, $C_{1-3}$-Alkoxy, Amino, $C_{1-3}$-Alkyl-amino, Di-($C_{1-3}$-Alkyl)-amino sein kann.

$R^2$ Wasserstoff, Halogen oder $C_{1-4}$-Alkyl bedeutet,

L -C(H)< oder -N< bedeutet,

M -C(H)< oder -N< bedeutet,

T eine Bindung oder $C_{1-4}$-Alkylen bedeutet, wobei der $C_{1-4}$-Alkylen Rest mit $C_{1-2}$-Alkyl substituiert sein kann,

m 0, 1, 2 oder 3 bedeutet,

n 1, 2 oder 3 bedeutet,

p 0, 1, 2 oder 3 bedeutet,

wobei, soweit nichts anderes erwähnt wurde, die vorstehend erwähnten Alkylgruppen geradkettig oder verzweigt sein können,
deren Tautomere, deren Stereoisomere, deren Gemische und deren Salze, insbesondere deren physiologisch verträglichen Salze.

In einer bevorzugten Ausführungsform betrifft die vorliegende Erfindung Verbindungen der allgemeinen Formel (I),
worin
Ar ein Substituent nach der allgemeinen Formel (II') ist

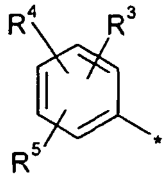

(II')

worin $R^3$ $-C_1$-$C_6$-Alkyl, $-C_3$-$C_6$-Cycloalkyl, $-CF_3$, $-CF_2CF_3$ oder

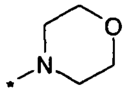

bedeutet,

wobei der $-C_3$-$C_6$-Cycloalkyl-Rest mit $-C_1$-$C_3$-Alkyl substituiert sein kann, worin $R^4$ -H, $-C_1$-$C_4$-Alkyl oder $-O-C_1$-$C_4$-Alkyl bedeutet,
worin $R^5$ ausgewählt ist aus
$-NH-S(O)_2-C_1$-$C_4$-Alkyl,
$-NH-S(O)_2-C_3$-$C_5$-Cycloalkyl;
$-N(CH_3)-S(O)_2-C_1$-$C_4$-Alkyl;
$-N(CH_3)-S(O)_2-C_3$-$C_5$-Cycloalkyl;
$-NH-C(O)-C_1$-$C_4$-Alkyl;
$-NH-C(O)-C_3$-$C_5$-Cycloalkyl;
$-N(CH_3)-C(O)-C_1$-$C_4$-Alkyl;
$-N(CH_3)-C(O)-C_3$-$C_5$-Cycloalkyl;
$-C(O)-NH_2$;
$-C(O)-NH-C_1$-$C_4$-Alkyl;
$-C(O)-N$(Di-$C_1$-$C_4$-Alkyl);
$-C(O)-NH-C_3$-$C_5$-Cycloalkyl;
$-C(O)-N(C_1$-$C_4$-Alkyl)($C_3$-$C_5$-Cycloalkyl);
$-S(O)-C_1$-$C_4$-Alkyl;
$-S(O)-C_3$-$C_5$-Cycloalkyl;
$-S(O)_2-C_1$-$C_4$-Alkyl;
$-S(O)_2-C_3$-$C_5$-Cycloalkyl;
$-C_1$-$C_4$-Alkyl-OH
$-CH_2-S(O)-C_1$-$C_4$-Alkyl;
$-CH_2-S(O)-C_3$-$C_5$-Cycloalkyl;
$-CH_2-S(O)_2-C_1$-$C_4$-Alkyl;
$-CH_2-S(O)_2-C_3$-$C_5$-Cycloalkyl;
$-C_1$-$C_4$-Alkylen-$N(R^8,R^{8'})$;
worin $R^1$ $-C_4$-$C_6$-Cycloalkyl bedeutet, welches 1 bis 2 Stickstoffatome im Ring enthält,
worin $R^1$ gegebenenfalls substituiert ist mit einer Gruppe ausgewählt aus $-C_1$-$C_4$-Alkyl, -OH, $-O-C_1$-$C_4$-Alkyl, und $-N(R^9, R^{9'})$,
worin $R^2$ ausgewählt ist aus -H, -Halogen und $C_1$-$C_4$-Alkyl,
worin jeweils $R^8$, $R^{8'}$, $R^9$ und $R^{9'}$ unabhängig voneinander ausgewählt sind aus -H und $-C_1$-$C_5$-Alkyl,
worin die beiden Reste $R^8$ und $R^{8'}$ zusammen mit dem Stickstoff, an den sie gebunden sind, ein 4 bis 6-gliedriges Ringsystem bilden können, welches mit -OH oder $-N(R^{10},R^{10'})$ substituiert sein kann,
worin jeweils $R^{10}$ und $R^{10'}$ unabhängig voneinander ausgewählt sind aus -H und $-C_1$-$C_5$-Alkyl,
worin L und M unabhängig voneinander ausgewählt sind aus -CH< und -N<,
worin T ausgewählt ist aus einer Bindung und $-C_1$-$C_3$-Alkylen,
wobei der $C_{1-3}$-Alkylen Rest mit Methyl substituiert sein kann,
worin m 0-3 sein kann,
worin n 1-3 sein kann,
worin p 0-3 sein kann,
gegebenenfalls in Form ihrer individuellen optischen Isomere, Mischungen dieser Isomere, Racemate, gegebenenfalls

in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin

Ar ein Substituent nach der allgemeinen Formel (II") ist

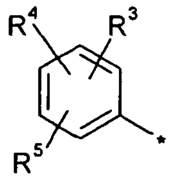

(II")

worin $R^3$ ausgewählt ist aus $-CH_3$, $-C_2H_5$, $-n-C_3H_7$, $-i-C_3H_7$, $-C(CH_3)_3$, $-n-C_4H_9$, $-CH_2-i-C_3H_7$, $-CH(CH_3)(C_2H_5)$, $-n-C_5H_{11}$, $-CH_2-CH_2-i-C_3H_7$, $-CH_2-C(CH_3)_3$, $-CF_3$, $-CF_2CF_3$ oder

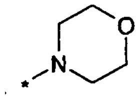

,

worin $R^4$ ausgewählt ist aus H, $-O-CH_3$, $-O-C_2H_5$,

worin $R^5$ ausgewählt ist aus H, $-NH-S(O)_2-CH_3$, $-NH-S(O)_2-C_2H_5$, $-NH-S(O)_2-n-C_3H_7$, $-NH-S(O)_2-i-C_3H_7$, $NH-S(O)_2-c-C_3H_5$, $-NH-S(O)_2-n-C_4H_9$, $-NH-S(O)_2-CH_2-i-C_3H_7$, $-NH-S(O)_2-C(CH_3)_3$, $-NH-S(O)_2-c-C_4H_7$, $-NH-S(O)_2-n-C_5H_{11}$, $-NH-S(O)_2-(CH_2)_2-i-C_3H_7$, $-NH-S(O)_2-CH_2-C(CH_3)_3$, $-NH-S(O)_2-c-C_5H_9$, $-NH-C(O)-CH_3$, $-NH-C(O)-C_2H_5$, $-NH-C(O)-n-C_3H_7$, $-NH-C(O)-i-C_3H_7$, $-NH-C(O)-c-C_3H_5$, $-NH-C(O)-n-C_4H_9$, $-NH-C(O)-CH_2-i-C_3H_7$, $-NH-C(O)-C(CH_3)_3$, $-NH-C(O)-c-C_4H_7$, $-NH-C(O)-n-C_5H_{11}$, $-NH-C(O)-(CH_2)_2-i-C_3H_7$, $-NH-C(O)-CH_2-C(CH_3)_3$, $-NH-C(O)-c-C_5H_9$, $-C(O)-NH_2$, $-C(O)-NH-CH_3$, $-C(O)-N(CH_3)_2$, $-C(O)-NH-C_2H_5$, $-C(O)-N(C_2H_5)_2$, $-C(O)-NH-n-C_3H_7$, $-C(O)-N(C_3H_7)_2$, $-C(O)-NH-i-C_3H_7$, $-C(O)-N(i-C_3H_7)_2$, $-C(O)-NH-c-C_3H_5$, $-C(O)-NH-n-C_4H_9$, $-C(O)-N(n-C_4H_9)_2$, $-C(O)-NH-CH_2-i-C_3H_7$, $-C(O)-N(CH_2-i-C_3H_7)_2$, $-C(O)-NH-c-C_4H_7$, $-CH_2-N(CH_3)_2$, $-CH_2-N(C_2H_5)_2$, $-CH_2-N(n-C_3H_7)_2$, $-CH_2-N(i-C_3H_7)_2$, $-CH_2-N(n-C_4H_9)_2$, $-CH_2-N(CH_2-i-C_3H_7)_2$,

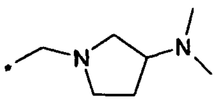 , 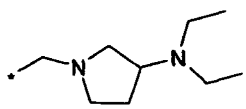 , 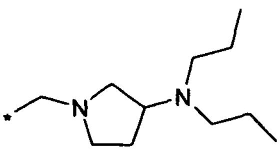 ,

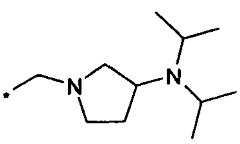 , 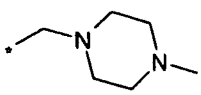 , 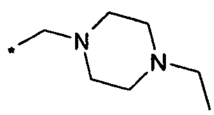 , 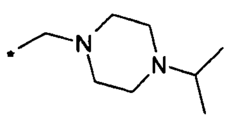 ,

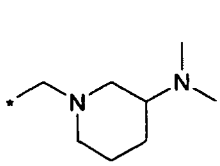 , 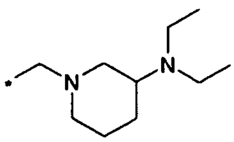 , 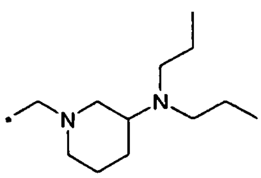 , 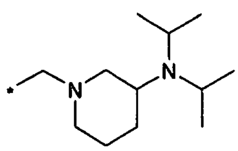 ,

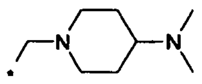, 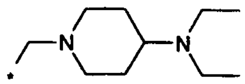, 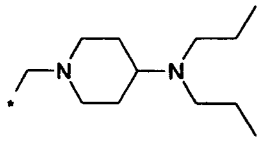, 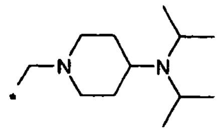,

$-S(O)-CH_3$, $-S(O)_2-CH_3$, $-S(O)-C_2H_5$, $-S(O)_2-C_2H_5$, $-CH_2-OH$, $-CH_2-S(O)-CH_3$, $-CH_2-S(O)_2-CH_3$, $-CH_2-S(O)-C_2H_5$ oder $-CH_2-S(O)_2-C_2H_5$,
worin $R^1$ ausgewählt ist aus

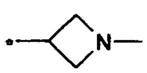, 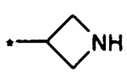, 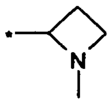, 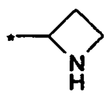, 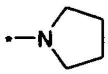, 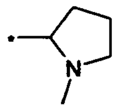,

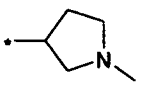, 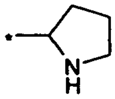, 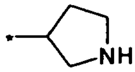, 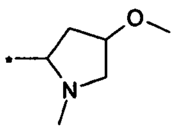, 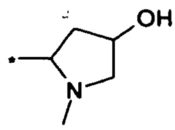, 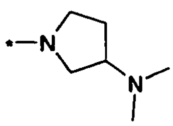,

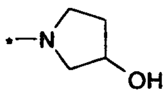, 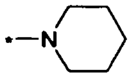, 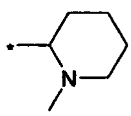, 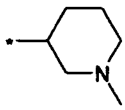, 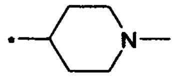, 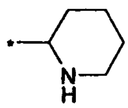, 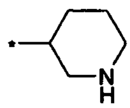,

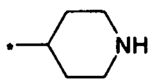, 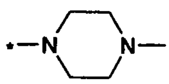, 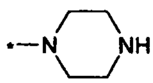, 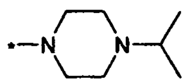,

worin $R^2$ H bedeutet,
worin L ausgewählt ist aus -CH< oder -N<,
worin M -N< oder -CH< bedeutet,
worin T ausgewählt ist aus einer Bindung oder $-CH_2-$,
wobei der $-CH_2-$ Rest mit Methyl substituiert sein kann,
worin m 1 oder 2 sein kann
worin n 1 oder 2 sein kann,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I),
worin
Ar ein Substituent nach der allgemeinen Formel (II''') ist

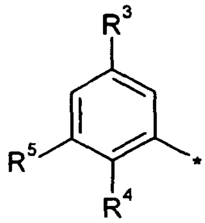

(II''')

worin $R^3$ $-C(CH_3)_3$, $-CH(CH_3)(C_2H_5)$, $-i-C_3H_7$, $-CH_2-i-C_3H_7$, $-CF_3$ oder $-CF_2CF_3$ bedeutet,
worin $R^4$ $-O-CH_3$ oder $-O-C_2H_5$ bedeutet,
worin $R^5$ ausgewählt ist aus $-NH-S(O)_2-CH_3$, $-NH-S(O)_2-n-C_3H_7$, $-NH-S(O)_2-c-C_3H_5$, $-NH-C(O)-CH_3$, $-NH-C(O)-C_2H_5$, $-NH-C(O)-n-C_3H_7$, $-NH-C(O)-i-C_3H_7$, $-NH-C(O)-c-C_3H_5$, $-C(O)-NH_2$, $-C(O)-NH-CH_3$, $-C(O)-NH-C_2H_5$, $-C(O)-NH-i-C_3H_7$, $-C(O)-NH-c-C_3H_5$,- $C(O)-N(CH_3)_2$,$-CH_2-N(CH_3)_2$, $-CH_2-N(C_2H_5)_2$, ,$-CH_2-N(i-C_3H_7)_2$,

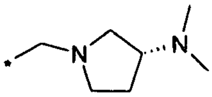 ,

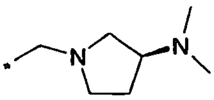 , 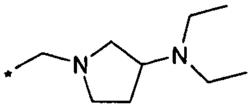 , 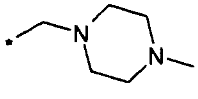 , 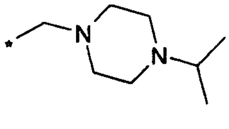 ,

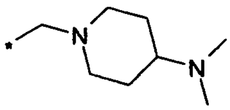 , 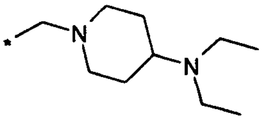 , 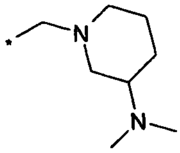 ,

$-S(O)-CH_3$, $-S(O)_2-CH_3$, $-CH_2-OH$, $-CH_2-S(O)-CH_3$ oder $-CH_2-S(O)_2-CH_3$,
worin $R^1$ ausgewählt ist aus,

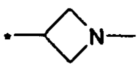 , 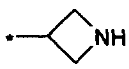 , 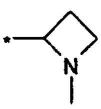 , 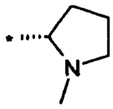 , 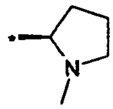 ,

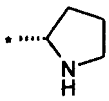 , 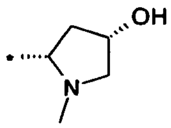 , 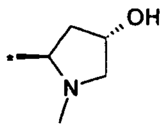 , 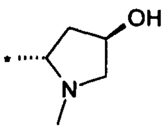 , 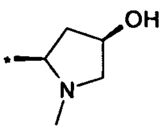 , 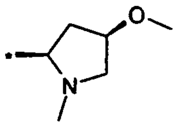 ,

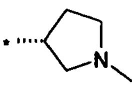 , 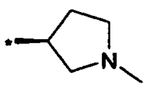 , 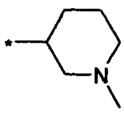 , 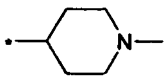 , 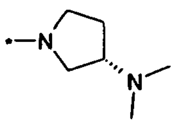 , 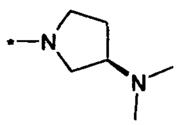 ,

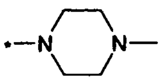 , 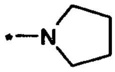 , 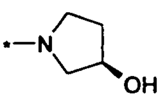 , 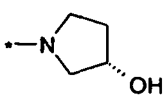 , 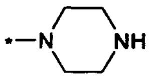 , 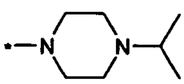 ,

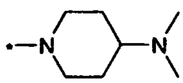 ,

worin $R^2$ -H bedeutet,
worin L ausgewählt ist aus -CH< und -N<,
worin M -N< bedeutet,

worin T eine Bindung bedeutet,
worin m 1 bedeutet,
worin n 1 bedeutet,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (**I**), worin
Ar ein Substituent nach einer der allgemeinen Formeln (**IIa**), (**IIIa**) oder (**IVa**) ist,

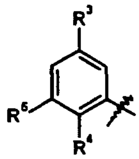 (**IIa**), 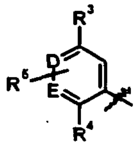 (**IIIa**), 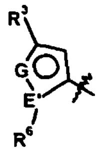 (**IVa**)

worin $R^3$ $C_{1-6}$-Alkyl, $C_{3-6}$-Cycloalkyl, $C_{1-4}$-Alkoxy, $C_{1-2}$-Perfluoroalkyl, 3-Methyl-oxetan-3-yl, $C_{1-2}$-Perfluoroalkoxy, Morpholinyl bedeutet,
worin der Cycloalkyl-Rest gegebenenfalls mit $C_{1-3}$-Alkyl substituiert sein kann,
worin $R^4$ H, $C_{1-4}$-Alkyl, $C_{1-4}$-Alkoxy bedeutet,
worin $R^5$ H, $C_{1-5}$-Alkyl-sulfonyl-amino, $C_{3-6}$-Cycloalkyl-suffonyl-amino, ($C_{1-5}$-Alkyl-sulfonyl)-(methyl)-amino, ($C_{3-6}$-Cycloalkyl-sulfonyl)-(methyl)-amino, $C_{1-5}$-Alkyl-carbonyl-amino, $C_{3-6}$-Cycloalkyl-carbonyl-amino, ($C_{1-5}$-Alkyl-carbonyl)-(methyl)-amino, ($C_{3-6}$-Cycloalkyl-carbonyl)-(methyl)-amino, Aminocarbonyl, $C_{1-5}$-Alkyl-amino-carbonyl, $C_{3-6}$-Cycloalkyl-amino-carbonyl, Di-($C_{1-3}$-Alkyl)-amino-carbonyl, Di-($C_{1-3}$-Alkyl)-amino-$C_{1-2}$-alkyl, Di-($C_{1-3}$-Alkyl)-amino-pyrrolidin-1-yl-$C_{1-2}$-alkyl, 4-$C_{1-5}$-Alkyl-piperazin-1-yl-$C_{1-2}$-alkyl, 4-Di-($C_{1-3}$-Alkyl)-amino-piperidin-1-yl-$C_{1-2}$-alkyl, 3-Di-($C_{1-3}$-Alkyl)-amino-piperidin-1-yl-$C_{1-2}$-alkyl, $C_{1-5}$-Alkyl-sulfinyl, $C_{3-6}$-Cycloalkyl-sulfinyl, $C_{1-5}$-Alkyl-sulfonyl, $C_{3-6}$-Cycloalkyl-sulfonyl, Hydroxy-$C_{1-2}$-alkyl, $C_{1-5}$-Alkyl-sulfinyl-methyl, $C_{1-5}$-Alkyl-sulfonyl-methyl bedeutet,
worin $R^6$ $C_{1-3}$-Alkyl oder Phenyl bedeutet,
wobei der $C_{1-3}$-Alkyl-Rest gegebenenfalls mit Hydroxy oder Di-($C_{1-3}$-Alkyl)-amino substituiert sein kann und
wobei der Phenyl-Rest gegebenenfalls mit Fluor oder $C_{1-3}$-Alkyl substituiert sein kann.
worin D oder E Stickstoff bedeuten,
worin G und E' unabhängig voneinander Stickstoff oder Sauerstoff bedeuten,
worin $R^6$ nur dann an E' gebunden ist, wenn E' Stickstoff bedeutet,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (**I**), worin
Ar einen Substituenten der Formel (**IIa**) oder (**IIIa**) bedeutet,

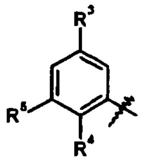 (**IIa**), 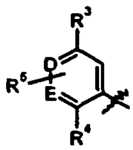 (**IIIa**),

worin $R^3$ Methyl, Ethyl, n-Propyl, i-Propyl, n-Butyl, i-Butyl, sec.-Butyl, tert-Butyl, n-Pentyl, i-Pentyl, neo-Pentyl, 1-Methylcyclopropyl, Methoxy, Trifluormethyl, Pentafluorethyl, 3-Methyl-oxetan-3-yl, Trifluormethoxy, Morpholin-4-yl bedeutet,
worin $R^4$ H, Methyl, Ethyl, n-Propyl, Methoxy, Ethoxy, n-Propyloxy, i-Propyloxy bedeutet,
worin $R^5$ H, Methyl-sulfonyl-amino, Ethyl-sulfonyl-amino, n-Propyl-sulfonyl-amino, i-Propyl-sulfonyl-amino, n-Butyl-sulfonyl-amino, i-Butyl-sulfonyl-amino, sec-Butyl-sulfonyl-amino, tert.-Butyl-sulfonyl-amino, n-Pentyl-sulfonyl-amino, i-Pentyl-sulfonyl-amino, neo-Pentyl-sulfonyl-amino, Cyclopropyl-sulfonyl-amino, Cyclobutyl-sulfonyl-amino, Cyclopentyl-sulfonyl-amino, Acetyl-amino, Ethyl-carbonyl-amino, n-Propyl-carbonyl-amino, i-Propyl-carbonyl-amino, Cyclopropyl-carbonyl-amino, n-Butyl-carbonyl-amino, i-Butyl-carbonyl-amino, sec-Butyl-carbonyl-amino, tert.-Butyl-carbonyl-amino, Cyclobutyl-carbonyl-amino, n-Pentyl-carbonyl-amino, i-Pentyl-carbonyl-amino, neo-Pentyl-carbonyl-amino, Cyclopentyl-carbonyl-amino, Amino-carbonyl, Methylamino-carbonyl, Ethylamino-carbonyl, n-Propylamino-carbonyl, i-Propylamino-carbonyl, n-Butylamino-carbonyl, i-Butylamino-carbonyl, sec-Butylamino-carbonyl, tert.-Butylamino-carbonyl, n-Pentylamino-carbonyl, i-Pentylamino-carbonyl, neo-Pentylamino-carbonyl, Cyclopropylamino-carbonyl, Cyclobutylamino-carbonyl, Cyclopentylamino-carbonyl, Dimethylamino-carbonyl, Diethylamino-carbonyl, Di-n-propylamino-carbonyl, Di-i-propylamino-carbonyl, Dimethylamino-methyl, Diethylamino-methyl, Di-n-Propylamino-methyl, Di-i-Propylamino-methyl, 3-Dimethylamino-pyrrolidin-1-yl-methyl, 3-Diethylamino-pyrrolidin-1-yl-methyl, 3-Di-n-propylamino-pyrrolidin-1-yl-methyl, 3-Di-i-propylamino-pyrrolidin-1-yl-methyl, 4-Methyl-piperazin-1-yl-methyl, 4-Ethyl-piperazin-1-yl-methyl, 4-n-Propyl-piperazin-1-yl-methyl, 4-i-Propyl-piperazin-1-yl-methyl, 4-n-Butyl-piperazin-1-yl-methyl, 4-sec-But-yl-piperazin-1-yl-methyl, 4-i-Butyl-piperazin-1-yl-methyl, 4-tert-Butyl-piperazin-1-yl-methyl, 4-Dimethylamino-piperidin-1-yl-methyl, 4-Diethylamino-piperidin-1-yl-methyl, 4-Di-n-Propylamino-piperidin-1-yl-methyl, 4-Di-i-Propylamino-piperidin-1-yl-methyl, 3-Dimethylamino-piperidin-1-yl-methyl, 3-Diethylamino-piperidin-1-yl-methyl, 3-Di-n-Propylamino-piperidin-1-yl-methyl, 3-Di-i-Propylamino-piperidin-1-yl-methyl, Methyl-sulfinyl, Ethyl-sulfinyl, n-Propyl-sulfinyl, i-Propyl-sulfinyl, Cyclopropyl-sulfinyl, Methyl-sulfonyl, Ethyl-sulfonyl, n-Propyl-sulfonyl, i-Propyl-sulfonyl, Cyclopropyl-sulfonyl, Hydroxy-methyl, Methyl-sulfinyl-methyl, Methyl-sulfonyl-methyl bedeutet.
worin D oder E Stickstoff bedeuten,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (**I**), worin
Ar einen Substituenten der Formel (**IIa**) bedeutet,

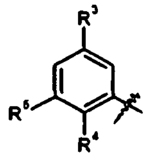
**(IIa)**,

worin $R^3$ i-Propyl, i-Butyl, sec.-Butyl, tert.-Butyl, 1-Methyl-cyclopropyl, Methoxy, Trifluormethyl, Pentafluorethyl, Trifluormethoxy bedeutet,
worin $R^4$ Methoxy, Ethoxy bedeutet,
worin $R^5$ Methyl-sulfonyl-amino, Ethyl-sulfonyl-amino, n-Propyl-sulfonyl-amino, i-Propyl-sulfonyl-amino, Cyclopropyl-sulfonyl-amino, Acetyl-amino, Ethyl-carbonyl-amino, n-Propyl-carbonyl-amino, i-Propyl-carbonyl-amino, Cyclopropyl-carbonyl-amino, Amino-carbonyl, Methylamino-carbonyl, Ethylamino-carbonyl, i-Propylamino-carbonyl, Cyclopropylamino-carbonyl, Dimethylamino-carbonyl, Diethylamino-carbonyl, Di-n-propylamino-carbonyl, Di-i-propylamino-carbonyl, Dimethylamino-methyl, Diethylamino-methyl, Di-n-propylamino-methyl, Di-i-propylamino-methyl, 3-Dimethylamino-pyrrolidin-1-yl-methyl, 3-Diethylamino-pyrrolidin-1-yl-methyl, 3-Di-n-propylamino-pyrrolidin-1-yl-methyl, 3-Di-i-propylamino-pyrrolidin-1-yl-methyl, 4-Methyl-piperazin-1-yl-methyl, 4-Ethyl-piperazin-1-yl-methyl, 4-n-Propyl-piperazin-1-yl-methyl, 4-i-Propyl-piperazin-1-yl-methyl, 4-Dimethylamino-piperidin-1-yl-methyl, 4-Diethylamino-piperidin-1-yl-methyl, 4-Di-n-propylamino-piperidin-1-yl-methyl, 4-Di-i-propylamino-piperidin-1-yl-methyl, 3-Dimethylamino-piperidin-1-yl-methyl, 3-Diethylamino-piperidin-1-yl-methyl, 3-Di-n-propylamino-piperidin-1-yl-methyl, 3-Di-i-propylamino-piperidin-1-yl-methyl, Methyl-sulfinyl, Ethyl-sulfinyl, n-Propyl-sulfinyl, i-Propyl-sulfinyl, Cyclopropyl-sulfinyl, Methyl-sulfonyl, Ethyl-sulfonyl, n-Propyl-sulfonyl, i-Propyl-sulfonyl, Cyclopropyl-sulfonyl, Hydroxy-methyl, Methyl-sulfinyl-methyl, Methyl-sulfonyl-methyl bedeutet.
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (**I**), worin
Ar einen Substituenten der Formel (**IIa**) bedeutet,

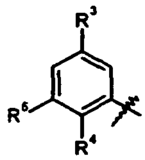
**(IIa)**,

worin $R^3$ tert.-Butyl, sec-Butyl, i-Propyl, i-Butyl, Trifluormethyl, Pentafluorethyl bedeutet,
worin $R^4$ Methoxy, Ethoxy bedeutet,
worin $R^5$ Methyl-sulfonyl-amino, n-Propyl-sulfonyl-amino, Cyclopropyl-sulfonyl-amino, Acetyl-amino, Ethyl-carbonyl-amino, n-Propyl-carbonyl-amino, i-Propyl-carbonyl-amino, Cyclopropyl-carbonyl-amino, Amino-carbonyl, Methylamino-carbonyl, Ethylamino-carbonyl, i-Propylamino-carbonyl, Cyclopropylamino-carbonyl, Dimethylamino-carbonyl, Dimethylamino-methyl, Diethylamino-methyl, Di-i-propylamino-methyl, (R)-3-Dimethylamino-pyrrolidin-1-yl-methyl, (S)-3-Dimethylamino-pyrrolidin-1-yl-methyl, 3-Diethylamino-pyrrolidin-1-yl-methyl, 4-Methyl-piperazin-1-yl-methyl, 4-i-Propyl-piperazin-1-yl-methyl, 4-Dimethylamino-piperidin-1-yl-methyl, 4-Diethylamino-piperidin-1-yl-methyl, 3-Dimethylamino-piperidin-1-yl-methyl, Methyl-sulfinyl, Methyl-sulfonyl, Hydroxy-methyl, Methyl-sulfinyl-methyl, Methyl-sulfonyl-methyl bedeutet.

gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin

Ar ein Substituent nach der allgemeinen Formel (II') ist

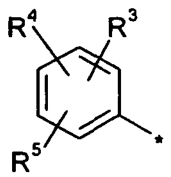

(II')

worin $R^3$ $-C_1-C_6$-Alkyl, $-C_3-C_6$-Cycloalkyl, $-CF_3$, $-CF_2CF_3$ oder

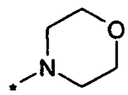

bedeutet,

wobei der $-C_3-C_6$-Cycloalkyl-Rest mit $-C_1-C_3$-Alkyl substituiert sein kann,

worin $R^4$ -H, $-C_1-C_4$-Alkyl oder $-O-C_1-C_4$-Alkyl bedeutet,

worin $R^5$ ausgewählt ist aus

$-NH-S(O)_2-C_1-C_4$-Alkyl;

$-NH-S(O)_2-C_3-C_5$-Cycloalkyl;

$-N(CH_3)-S(O)_2-C_1-C_4$-Alkyl;

$-N(CH_3)-S(O)_2-C_3-C_5$-Cycloalkyl;

$-NH-C(O)-C_1-C_4$-Alkyl;

$-NH-C(O)-C_3-C_5$-Cycloalkyl;

$-N(CH_3)-C(O)-C_1-C_4$-Alkyl;

$-N(CH_3)-C(O)-C_3-C_5$-Cycloalkyl;

$-C(O)-NH_2$;

$-C(O)-NH-C_1-C_4$-Alkyl;

$-C(O)-N(Di-C_1-C_4$-Alkyl);

$-C(O)-NH-C_3-C_5$-Cycloalkyl;

$-C(O)-N(C_1-C_4$-Alkyl)($C_3-C_5$-Cycloalkyl);

$-S(O)-C_1-C_4$-Alkyl;

$-S(O)-C_3-C_5$-Cycloalkyl;

$-S(O)_2-C_1-C_4$-Alkyl;

$-S(O)_2-C_3-C_5$-Cycloalkyl;

$-C_1-C_4$-Alkyl-OH

$-CH_2-S(O)-C_1-C_4$-Alkyl;

$-CH_2-S(O)-C_3-C_5$-Cycloalkyl;

$-CH_2-S(O)_2-C_1-C_4$-Alkyl;

$-CH_2-S(O)_2-C_3-C_5$-Cycloalkyl;

$-C_1-C_4$-Alkylen-$N(R^8,R^{8'})$;

worin jeweils $R^8$ und $R^{8'}$ unabhängig voneinander ausgewählt sind aus -H und $-C_1-C_5$-Alkyl,

worin die beiden Reste $R^8$ und $R^{8'}$ zusammen mit dem Stickstoff, an den sie gebunden sind, ein 4 bis 6-gliedriges Ringsystem bilden können, welches mit -OH oder $-N(R^{10},R^{10'})$ substituiert sein kann,

worin jeweils $R^{10}$ und $R^{10'}$ unabhängig voneinander ausgewählt sind aus -H und $-C_1-C_5$-Alkyl,

gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin

Ar ein Substituent nach der allgemeinen Formel (II") ist

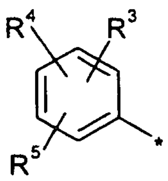

(II")

worin $R^3$ ausgewählt ist aus $-CH_3$, $-C_2H_5$, $-n-C_3H_7$, $-i-C_3H_7$, $-C(CH_3)_3$, $-n-C_4H_9$, $-CH_2-i-C_3H_7$, $-CH(CH_3)(C_2H_5)$,, $-n-C_5H_{11}$, $-CH_2-CH_2-i-C_3H_7$, $-CH_2-C(CH_3)_3$, $-CF_3$, $-CF_2CF_3$ oder

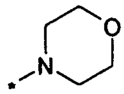

,

worin $R^4$ ausgewählt ist aus H, $-O-CH_3$ oder $-O-C_2H_5$,

worin $R^5$ ausgewählt ist aus H, $-NH-S(O)_2-CH_3$, $-NH-S(O)_2-C_2H_5$, $-NH-S(O)_2-n-C_3H_7$, $-NH-S(O)_2-i-C_3H_7$, $NH-S(O)_2-c-C_3H_5$, $-NH-S(O)_2-n-C_4H_9$, $-NH-S(O)_2-CH_2-i-C_3H_7$, $-NH-S(O)_2-C(CH_3)_3$,, $-NH-S(O)_2-c-C_4H_7$, $-NH-S(O)_2-n-C_5H_{11}$, $-NH-S(O)_2-(CH_2)_2-i-C_3H_7$, $-NH-S(O)_2-CH_2-C(CH_3)_3$, $-NH-S(O)_2-C-C_5H_9$, $-NH-C(O)-CH_3$, $-NH-C(O)-C_2H_5$, $-NH-C(O)-n-C_3H_7$, $-NH-C(O)-i-C_3H_7$, $-NH-C(O)-c-C_3H_5$, $-NH-C(O)-n-C_4H_9$, $-NH-C(O)-CH_2-i-C_3H_7$, $-NH-C(O)-C(CH_3)_3$, $-NH-C(O)-c-C_4H_7$, $-NH-C(O)-n-C_5H_{11}$, $-NH-C(O)-(CH_2)_2-i-C_3H_7$, $-NH-C(O)-CH_2-C(CH_3)_3$, $-NH-C(O)-c-C_5H_9$, $-C(O)-NH_2$, $-C(O)-NH-CH_3$, $-C(O)-N(CH_3)_2$, $-C(O)-NH-C_2H_5$, $-C(O)-N(C_2H_5)_2$, $-C(O)-NH-n-C_3H_7$, $-C(O)-N(C_3H_7)_2$, $-C(O)-NH-i-C_3H_7$, $-C(O)-N(i-C_3H_7)_2$, $-C(O)-NH-c-C_3H_5$, $-C(O)-NH-n-C_4H_9$, $-C(O)-N(n-C_4H_9)_2$, $-C(O)-NH-CH_2-i-C_3H_7$, $-C(O)-N(CH_2-i-C_3H_7)_2$, $-C(O)-NH-c-C_4H_7$, $-CH_2-N(CH_3)_2$, $-CH_2-N(C_2H_5)_2$, $-CH_2-N(n-C_3H_7)_2$, $-CH_2-N(i-C_3H_7)_2$, $-CH_2-N(n-C_4H_9)_2$, $-CH_2-N(CH_2-i-C_3H_7)_2$,

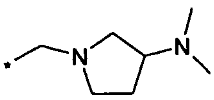 , 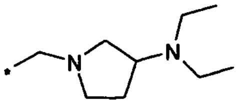 , 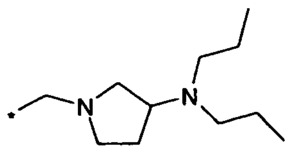 ,

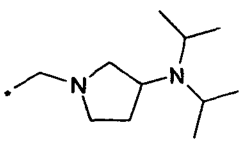 , 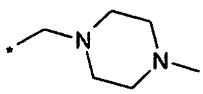 , 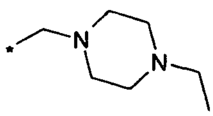 , 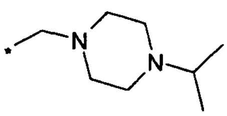 ,

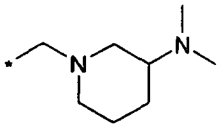 , 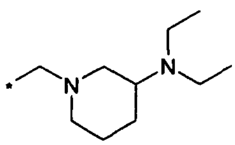 , 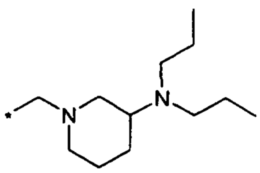 , 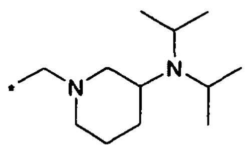 .

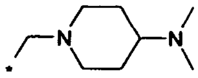, 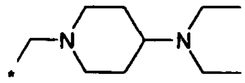, 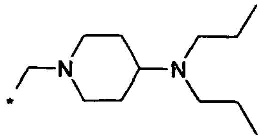, 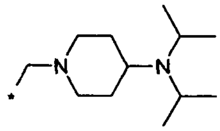,

$-S(O)-CH_3$, $-S(O)_2-CH_3$, $-S(O)-C_2H_5$, $-S(O)_2-C_2H_5$, $-CH_2-OH$, $-CH_2-S(O)-CH_3$, $-CH_2-S(O)_2-CH_3$, $-CH_2-S(O)-C_2H_5$ oder $-CH_2-S(O)_2-C_2H_5$,

gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin

Ar ein Substituent nach der allgemeinen Formel (II''') ist

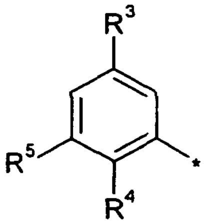 (II''')

worin $R^3$ $-C(CH_3)_3$, $-CH(CH_3)(C_2H_5)$, $-i-C_3H_7$, $-CH_2-i-C_3H_7$, $-CF_3$ oder $-CF_2CF_3$ bedeutet,

worin $R^4$ $-O-CH_3$ oder $-O-C_2H_5$ bedeutet,

worin $R^5$ ausgewählt ist aus $-NH-S(O)_2-CH_3$, $-NH-S(O)_2-n-C_3H_7$, $-NH-S(O)_2-c-C_3H_5$, $-NH-C(O)-CH_3$, $-NH-C(O)-C_2H_5$, $-NH-C(O)-n-C_3H_7$, $-NH-C(O)-i-C_3H_7$, $-NH-C(O)-c-C_3H_5$,- $C(O)-NH_2$, $-C(O)-NH-CH_3$, $-C(O)-NH-C_2H_5$, $-C(O)-NH-i-C_3H_7$, $-C(O)-NH-c-C_3H_5$, $-C(O)-N(CH_3)_2$,$-CH_2-N(CH_3)_2$, ,$-CH_2-N(C_2H_5)_2$, ,$-CH_2-N(i-C_3H_7)_2$,

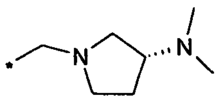,

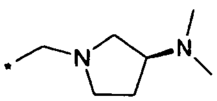, 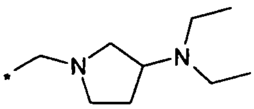, 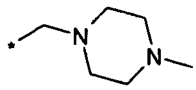, 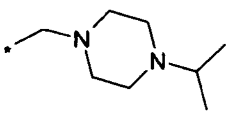,

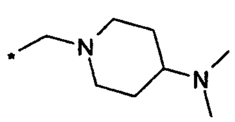, 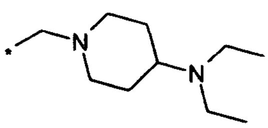, 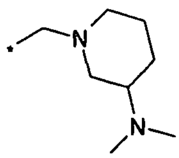,

$-S(O)-CH_3$, $-S(O)_2-CH_3$, $-CH_2-OH$, $-CH_2-S(O)-CH_3$ oder $-CH_2-S(O)_2-CH_3$ bedeutet,

gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin $R^1$

ein $C_{4-6}$-Cycloalkyl-System bedeutet, welches 1 bis 2-Stickstoffatome enthält, welches gegebenenfalls 1- bis 2-mal mit $R^7$ substituiert sein kann,

worin $R^7$ $C_{1-3}$-Alkyl, Hydroxy, $C_{1-3}$-Alkoxy, Amino, $C_{1-3}$-Alkyl-amino, Di-($C_{1-3}$-Alkyl)-amino sein kann.

gegebenenfalls in Form ihrer individuellen optischen Isomere, Mischungen dieser Isomere, Racemate, gegebenenfalls

in Form ihrer Säureadditionssalze mit pharmakologisch akzeptablen Säuren.

In einer weiteren bevorzugten Ausführungsform betrifft die vorliegende Erfindung Verbindungen der allgemeinen Formel (**I**), worin $R^1$

Azetidin-2-yl, Azetidin-3-yl, 1-Methyl-azetidin-2-yl, 1-Methyl-azetidin-3-yl, 1-Ethyl-azetidin-2-yl, 1-Ethyl-azetidin-3-yl, Pyrrolidin-1-yl, Pyrrolidin-2-yl, Pyrrolidin-3-yl, 3-Hydroxy-pyrrolidin-1-yl, 3-Amino-pyrrolidin-1-yl, 3-Methylamino-pyrrolidin-1-yl, 3-Dimethylamino-pyrrolidin-1-yl, 3-Ethylamino-pyrrolidin-1-yl, 3-Diethylamino-pyrrolidin-1-yl, 1-Methyl-pyrrolidin-2-yl, 1-Methyl-pyrrolidin-3-yl, 1-Ethyl-pyrrolidin-2-yl, 1-Ethyl-pyrrolidin-3-yl, 1-Methyl-4-hydroxy-pyrrolidin-2-yl, 1-Methyl-4-methoxy-pyrrolidin-2-yl, Piperidin-1-yl, Piperidin-2-yl, Piperidin-3-yl, Piperidin-4-yl, 4-Amino-piperidin-1-yl, 4-Methylamino-piperidin-1-yl, 4-Dimethylamino-piperidin-1-yl, 4-Ethylamino-piperidin-1-yl, 4-Diethylamino-piperidin-1-yl, 1-Methyl-piperidin-2-yl, 1-Methyl-piperidin-3-yl, 1-Methyl-piperidin-4-yl, 1-Ethyl-piperidin-2-yl, 1-Ethyl-piperidin-3-yl, 1-Ethyl-piperidin-4-yl, Piperazin-1-yl, 4-Methyl-piperazin-1-yl, 4-Ethyl-piperazin-1-yl, 4-Isopropyl-piperazin-1-yl bedeutet, gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin $R^1$

1-Methyl-azetidin-3-yl, Azetidin-3-yl, (2RS)-1-Methyl-azetidin-2-yl,
(2S)-1-Methyl-pyrrolidin-2-yl, (2R)-1-Methyl-pyrrolidin-2-yl, (2S)-Pyrrolidin-2-yl,
(2S,4S)-1-Methyl-4-hydroxy-pyrrolidin-2-yl, (2R,4S)-1-Methyl-4-hydroxy-pyrrolidin-2-yl,
(2S,4R)-1-Methyl-4-hydroxy-pyrrolidin-2-yl, (2R,4R)-1-Methyl-4-hydroxy-pyrrolidin-2-yl,
(2R,4R)-1-Methyl-4-methoxy-pyrrolidin-2-yl,
(3S)-1-Methyl-pyrrolidin-3-yl, (3R)-1-Methyl-pyrrolidin-3-yl, 1-Methyl-piperidin-3-yl,
1-Methyl-piperidin-4-yl, (3S)-3-Dimethylamino-pyrrolidin-1-yl,
(3R)-3-Dimethylamino-pyrrolidin-1-yl, 4-Methyl-piperazin-1-yl, Pyrrolidin-1-yl,
(3R)-3-Hydroxy-pyrrolidin-1-yl, (3S)-3-Hydroxy-pyrrolidin-1-yl, Piperazin-1-yl,
4-Isopropyl-piperazin-1-yl, 4-Dimethylamino-piperidin-1-yl darstellt,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin

$R^1$ ausgewählt ist aus $-C_4-C_6$-Cycloalkyl, welches 1 bis 2 Stickstoffatome im Ring enthält, worin $R^1$ gegebenenfalls substituiert ist mit einer Gruppe ausgewählt aus $-C_1-C_4$-Alkyl, -OH, $-O-C_1-C_4$-Alkyl, und $-N(R^9,R^{9'})$,
wobei $R^9,R^{9'}$ die vorstehend genannten Bedeutungen haben,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin

$R^1$ ausgewählt ist aus

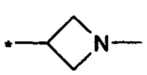 , 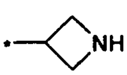 , 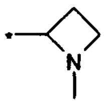 , 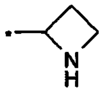 , 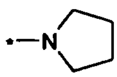 , 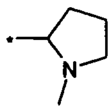 ,

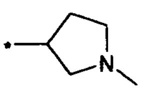 , 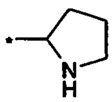 , 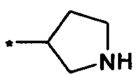 , 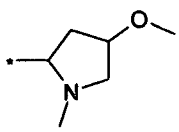 , 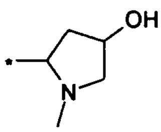 , 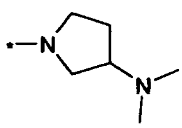 ,

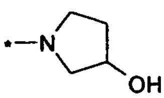 , 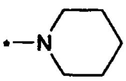 , 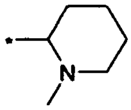 , 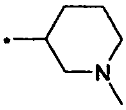 , 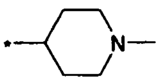 , 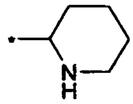 , 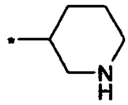 ,

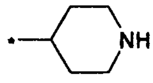, 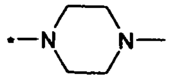, 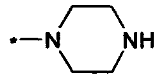, 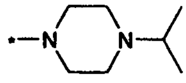,

gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin
$R^1$ ausgewählt ist aus

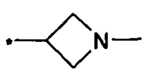, 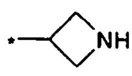, 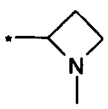, 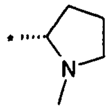, 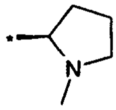, 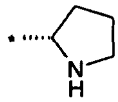,

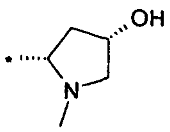, 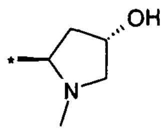, 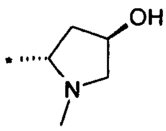, 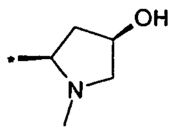, 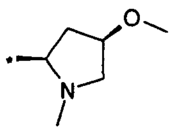, 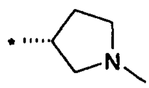,

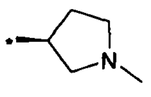, 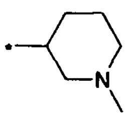, 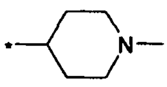, 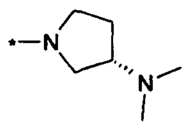, 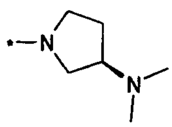, 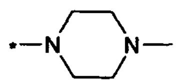,

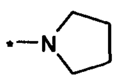, 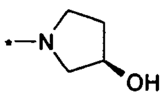, 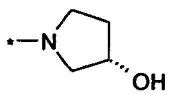, 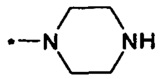, 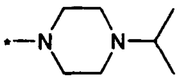, 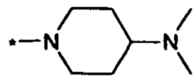,

gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin
$R^2$ ausgewählt ist aus -H, -F, -Cl und $-CH_3$,
und worin
p ausgewählt ist aus 0, 1, 2 oder 3
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin
$R^2$ H ist,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin
L und M unabhängig voneinander ausgewählt sind aus -CH< and -N<,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin
L ausgewählt ist aus -CH< oder -N<,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin
M -N< bedeutet,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin
T ausgewählt ist aus einer Bindung und $-C_1-C_3$-Alkylen,
wobei der $-C_1-C_3$-Alkylen Rest mit Methyl substituiert sein kann,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin
T ausgewählt ist aus einer Bindung,und $-CH_2-$,
wobei der $-CH_2-$ Rest mit Methyl substituiert sein kann,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

In einer weiteren bevorzugten Ausführungsform betrifft die vorliegende Erfindung Verbindungen der allgemeinen Formel (I), worin
T eine Bindung bedeutet,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

Eine weitere bevorzugte Ausführungsform betrifft die vorstehend beschriebenen Verbindungen nach der allgemeinen Formel (I) zur Behandlung von Erkrankungen ausgewählt aus Asthma oder COPD.

Eine weitere bevorzugte Ausführungsform betrifft die Verwendung der vorstehend beschriebenen Verbindungen nach der allgemeinen Formel (I) zur Herstellung eines Medikamentes zur Behandlung von Erkrankungen ausgewählt aus Asthma oder COPD.

Die erfindungsgemäßen Verbindungen der allgemeinen Formel I und ihre physiologisch verträglichen Salze weisen wertvolle pharmakologische Eigenschaften auf, insbesondere eine Hemmwirkung auf das Enzym p38 MAP-Kinase.

Gegenstand der vorliegenden Erfindung sind auch die physiologisch verträglichen Salze der erfindungsgemäßen Verbindungen mit anorganischen oder organischen Säuren. Gegenstand der Erfindung sind die jeweiligen Verbindungen der Formel I in Form ihrer pharmakologisch verträglichen Salze. Diese pharmakologisch verträglichen Salze der Verbindungen der Formel I können ebenfalls in Form ihrer jeweiligen Hydrate (z.B. Monohydrate, Dihydrate etc.) sowie in Form Ihrer jeweiligen Solvate vorliegen.

Unter einem Hydrat der Verbindung nach Formel I versteht man im Rahmen der Erfindung ein kristallines, kristallwasserhaltiges Salz der Verbindung nach Formel I. Unter einem Solvat der Verbindung nach Formel I versteht man im Rahmen der Erfindung ein kristallines Salz der Verbindung nach Formel I, welches Lösungsmittelmoleküle-Moleküle (z.B. Ethanol, Methanol etc) im Kristallgitter enthalten. Dem Fachmann sind Standardverfahren zur Gewinnung von Hydraten und Solvaten (z.B. das Umkristallisieren aus dem entsprechenden Lösungsmittel bzw. aus Wasser) bekannt.

Daher ist die Verwendung der erfindungsgemäßen Verbindungen, einschließlich der physiologisch verträglichen Salze, als Arzneimittel ebenfalls ein Gegenstand dieser Erfindung.

Ein weiterer Gegenstand dieser Erfindung sind Arzneimittel, enthaltend mindestens eine erfindungsgemäße Verbindung oder ein erfindungsgemäßes physiologisch verträgliches Salz neben gegebenenfalls einem oder mehreren inerten Trägerstoffen und/oder Verdünnungsmitteln.

Ebenfalls ein Gegenstand dieser Erfindung ist die Verwendung mindestens einer erfindungsgemäßen Verbindung oder eines physiologisch verträglichen Salzes solch einer Verbindung zur Herstellung eines Arzneimittels, das zur Behandlung oder Prophylaxe von Erkrankungen oder Zuständen geeignet ist, die durch Inhibierung des Enzyms p38 MAP-Kinase beeinflussbar sind.

Ein weiterer Gegenstand dieser Erfindung ist die Verwendung mindestens einer erfindungsgemäßen Verbindung zur Herstellung eines Arzneimittels zur Inhibition des Enzyms p38 MAP-Kinase.

Ferner ist ein Verfahren zur Herstellung eines erfindungsgemäßen Arzneimittels Gegenstand dieser Erfindung, dadurch gekennzeichnet, dass auf nicht-chemischem Wege eine erfindungsgemäße Verbindung in einen oder mehrere inerte Trägerstoffe und/oder Verdünnungsmittel eingearbeitet wird.

Explizit nicht Teil der vorliegenden Erfindung sind die folgenden Verbindungen **Va**, **Vb** und **Vc**:

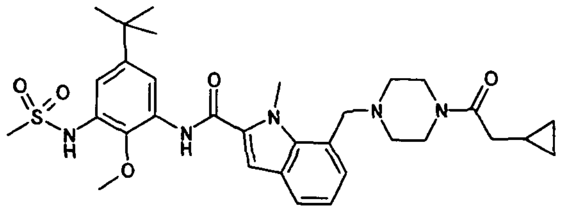

**Va**

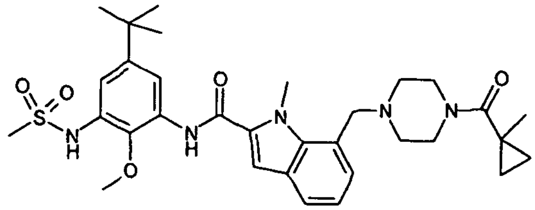

**Vb**

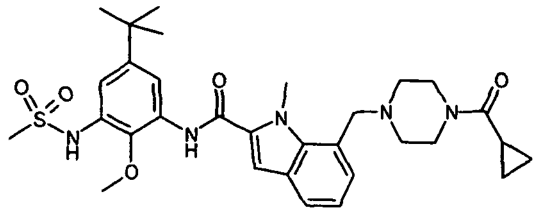

**Vc**

## Detaillierte Beschreibung der Erfindung

Sofern nicht anders angegeben besitzen die Gruppen, Reste und Substituenten, insbesondere Ar, $R^1$ bis $R^7$, L, M, T, m, n und p die zuvor und nachfolgend angegebenen Bedeutungen.

Kommen Reste, Substituenten oder Gruppen in einer Verbindung mehrfach vor, so können diese gleiche oder verschiedene Bedeutungen aufweisen.

Bevorzugte Ausführungsformen der Erfindung sind durch die folgenden Definitionen wiedergegeben:

***a)*** Die Definitionen ***($a^i$)*** für Ar gemäß ihrer zunehmenden Präferenz, beginnend mit bevorzugt ***($a^1$)*** über besonders bevorzugt ***($a^2$)***, ganz besonders bevorzugt ***($a^3$)*** zu überaus bevorzugt ***($a^4$)*** sind wie folgt:

***($a^1$):*** Bevorzugt bedeutet Ar einen Substituenten der Formel (**IIa**), (**IIIa**) oder (**IVa**),

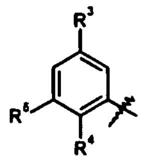 **(IIa),** 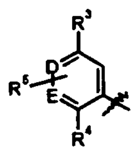 **(IIIa),** 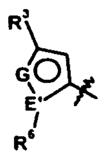 **(IVa)**

worin $R^3$ $C_{1-6}$-Alkyl, $C_{3-6}$-Cycloalkyl, $C_{1-4}$-Alkoxy, $C_{1-2}$-Perfluoroalkyl, 3-Methyl-oxetan-3-yl, $C_{1-2}$-Perfluoroalkoxy, Morpholinyl bedeutet,
worin der Cycloalkyl-Rest gegebenenfalls mit $C_{1-3}$-Alkyl substituiert sein kann,
worin $R^4$ H, $C_{1-4}$-Alkyl, $C_{1-4}$-Alkoxy bedeutet,
worin $R^5$ H, $C_{1-5}$-Alkyl-sulfonyl-amino, $C_{3-6}$-Cycloalkyl-sulfonyl-amino, ($C_{1-5}$-Alkyl-sulfonyl)-(methyl)-amino, ($C_{3-6}$-Cycloalkyl-sulfonyl)-(methyl)-amino, $C_{1-5}$-Alkyl-carbonyl-amino, $C_{3-6}$-Cycloalkyl-carbonyl-amino, ($C_{1-5}$-Alkyl-carbonyl)-(methyl)-amino, ($C_{3-6}$-Cycloalkyl-carbonyl)-(methyl)-amino, Aminocarbonyl, $C_{1-5}$-Alkyl-amino-carbonyl, $C_{3-6}$-Cycloalkyl-amino-carbonyl, Di-($C_{1-3}$-Alkyl)-amino-carbonyl, Di-($C_{1-3}$-Alkyl)-amino-$C_{1-2}$-alkyl, Di-($C_{1-3}$-Alkyl)-amino-pyrrolidin-1-yl-$C_{1-2}$-alkyl, 4-$C_{1-5}$-Alkyl-piperazin-1-yl-$C_{1-2}$-alkyl. 4-Di-($C_{1-3}$-Alkyl)-amino-piperidin-1-yl-$C_{1-2}$-alkyl, 3-Di-($C_{1-3}$-Alkyl)-amino-piperidin-1-yl-$C_{1-2}$-alkyl, $C_{1-5}$-Alkyl-sulfinyl, $C_{3-6}$-Cycloalkyl-sulfinyl, $C_{1-5}$-Alkyl-sulfonyl, $C_{3-6}$-Cycloalkyl-sulfonyl, Hydroxy-$C_{1-2}$-alkyl, $C_{1-5}$-Alkyl-sulfinyl-methyl, $C_{1-5}$-Alkyl-sulfonyl-methyl bedeu-

tet,
worin $R^6$ $C_{1-3}$-Alkyl oder Phenyl bedeutet,
wobei der $C_{1-3}$-Alkyl-Rest gegebenenfalls mit Hydroxy oder Di-($C_{1-3}$-Alkyl)-amino substituiert sein kann und
wobei der Phenyl-Rest gegebenenfalls mit Fluor oder $C_{1-3}$-Alkyl substituiert sein kann.
worin D oder E Stickstoff bedeuten,
worin G und E' unabhängig voneinander Stickstoff oder Sauerstoff bedeuten,
worin $R^6$ nur dann an E' gebunden ist, wenn E' Stickstoff bedeutet,

***($a^2$):*** Besonders bevorzugt bedeutet Ar einen Substituenten der Formel (**IIa**) oder (**IIIa**)

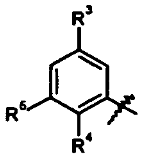
(**IIa**),
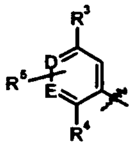
(**IIIa**),

worin $R^3$ Methyl, Ethyl, n-Propyl, i-Propyl, n-Butyl, i-Butyl, sec.-Butyl, tert-Butyl, n-Pentyl, i-Pentyl, neo-Pentyl, 1-Methyl-cyclopropyl, Methoxy, Trifluormethyl, Pentafluorethyl, 3-Methyl-oxetan-3-yl, Trifluormethoxy, Morpholin-4-yl bedeutet,
worin $R^4$ H, Methyl, Ethyl, n-Propyl, Methoxy, Ethoxy, n-Propyloxy, i-Propyloxy bedeutet,
worin $R^5$ H, Methyl-sulfonyl-amino, Ethyl-sulfonyl-amino, n-Propyl-suffonyl-amino, i-Propyl-sulfonyl-amino, n-Butyl-sulfonyl-amino, i-Butyl-sulfonyl-amino, sec-Butyl-sulfonyl-amino, tert.-Butyl-sulfonyl-amino, n-Pentyl-sulfonyl-amino, i-Pentyl-sulfonyl-amino, neo-Pentyl-sulfonyl-amino, Cyclopropyl-sulfonyl-amino, Cyclobutyl-sulfonyl-amino, Cyclopentyl-sulfonyl-amino, Acetyl-amino, Ethyl-carbonyl-amino, n-Propyl-carbonyl-amino, i-Propyl-carbonyl-amino, Cyclopropyl-carbonyl-amino, n-Butyl-carbonyl-amino, i-Butyl-carbonyl-amino, sec-Butyl-carbonyl-amino, tert.-Butyl-carbonyl-amino, Cyclobutyl-carbonyl-amino, n-Pentyl-carbonyl-amino, i-Pentyl-carbonyl-amino, neo-Pentyl-carbonyl-amino, Cyclopentyl-carbonyl-amino, Amino-carbonyl, Methylamino-carbonyl, Ethylamino-carbonyl, n-Propylamino-carbonyl, i-Propylamino-carbonyl, n-Butylamino-carbonyl, i-Butylamino-carbonyl, sec-Butylamino-carbonyl, tert.-Butylamino-carbonyl, n-Pentylamino-carbonyl, i-Pentylamino-carbonyl, neo-Pentylamino-carbonyl, Cyclopropylamino-carbonyl, Cyclobutylamino-carbonyl, Cyclopentylamino-carbonyl, Dimethylamino-carbonyl, Diethylamino-carbonyl, Di-n-propylamino-carbonyl, Di-i-propylamino-carbonyl, Dimethylamino-methyl, Diethylamino-methyl, Di-n-Propylamino-methyl, Di-i-Propylamino-methyl, 3-Dimethylamino-pyrrolidin-1-yl-methyl, 3-Diethylamino-pyrrolidin-1-yl-methyl, 3-Di-n-propylamino-pyrrolidin-1-yl-methyl, 3-Di-i-propylamino-pyrrolidin-1-yl-methyl, 4-Methyl-piperazin-1-yl-methyl, 4-Ethyl-piperazin-1-yl-methyl, 4-n-Propyl-piperazin-1-yl-methyl, 4-i-Propyl-piperazin-1-yl-methyl, 4-n-Butyl-piperazin-1-yl-methyl, 4-sec-But-yl-piperazin-1-yl-methyl, 4-i-Butyl-piperazin-1-yl-methyl, 4-tert.-Butyl-piperazin-1-yl-methyl, 4-Dimethylamino-piperidin-1-yl-methyl, 4-Diethylamino-piperidin-1-yl-methyl, 4-Di-n-Propylamino-piperidin-1-yl-methyl, 4-Di-i-Propylamino-piperidin-1-yl-methyl, 3-Dimethylamino-piperidin-1-yl-methyl, 3-Diethylamino-piperidin-1-yl-methyl, 3-Di-n-Propylamino-piperidin-1-yl-methyl, 3-Di-i-Propylamino-piperidin-1-yl-methyl, Methyl-sulfinyl, Ethyl-sulfinyl, n-Propyl-sulfinyl, i-Propyl-sulfinyl, Cyclopropyl-sulfinyl, Methyl-sulfonyl, Ethyl-sulfonyl, n-Propyl-sulfonyl, i-Propyl-sulfonyl, Cyclopropyl-sulfonyl, Hydroxy-methyl, Methyl-sulfinyl-methyl, Methyl-sulfonyl-methyl bedeutet.
worin D oder E Stickstoff bedeuten,

***($a^3$):*** Ganz besonders bevorzugt bedeutet Ar einen Substituenten der Formel (**IIa**),

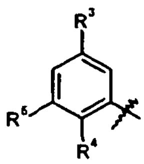
(**IIa**),

worin $R^3$ i-Propyl, i-Butyl, sec.-Butyl, tert.-Butyl, 1-Methyl-cyclopropyl, Methoxy, Trifluormethyl, Pentafluorethyl, Trifluormethoxy bedeutet,
worin $R^4$ Methoxy, Ethoxy bedeutet,
worin $R^5$ Methyl-sulfonyl-amino, Ethyl-sulfonyl-amino, n-Propyl-sulfonyl-amino, i-Propyl-sulfonyl-amino, Cyclopropyl-sulfonyl-amino, Acetyl-amino, Ethyl-carbonyl-amino, n-Propyl-carbonyl-amino, i-Propyl-carbonyl-amino, Cyclopropyl-carbonyl-amino, Amino-carbonyl, Methylamino-carbonyl, Ethylamino-carbonyl, i-Propylamino-carbonyl, Cyclopropylamino-carbonyl, Dimethylamino-carbonyl, Diethylamino-carbonyl, Di-n-propylamino-carbonyl, Di-i-propylamino-carbonyl, Dimethylamino-methyl, Diethylamino-methyl, Di-n-propylamino-methyl, Di-i-propylamino-methyl, 3-Dimethylamino-pyrrolidin-1-yl-methyl, 3-Diethylamino-pyrrolidin-1-yl-methyl, 3-Di-n-propylamino-pyrrolidin-1-yl-methyl, 3-Di-i-propylamino-pyrrolidin-1-yl-methyl, 4-Methyt-piperazin-1-yl-methyl, 4-Ethyl-piperazin-1-yl-methyl, 4-n-Propyl-piperazin-1-yl-methyl, 4-i-Propyl-piperazin-1-yl-methyl, 4-Dimethylamino-piperidin-1-yl-methyl, 4-Diethylamino-piperidin-1-yl-methyl, 4-Di-n-propylamino-piperidin-1-yl-methyl, 4-Di-1-propylamino-piperidin-1-yl-methyl, 3-Dimethylamino-piperidin-1-yl-methyl, 3-Diethylamino-piperidin-1-yl-methyl, 3-Di-n-propylamino-piperidin-1-yl-methyl, 3-Di-i-propylamino-piperidin-1-yl-methyl, Methyl-sulfinyl, Ethyl-sulfinyl, n-Propyl-sulfinyl, i-Propyl-sulfinyl, Cyclopropyl-sulfinyl, Methyl-sulfonyl, Ethyl-sulfonyl, n-Propyl-sulfonyl, i-Propyl-sulfonyl, Cyclopropyl-sulfonyl, Hydroxymethyl, Methyl-sulfinyl-methyl, Methyl-sulfonyl-methyl bedeutet.

***(a⁴):*** Überaus bevorzugt bedeutet Ar einen Substituenten der Formel (**IIa**),

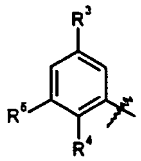

**(IIa)**,

worin $R^3$ tert.-Butyl, sec-Butyl, i-Propyl, i-Butyl, Trifluormethyl, Pentafluorethyl bedeutet,
worin $R^4$ Methoxy, Ethoxy bedeutet,
worin $R^5$ Methyl-sulfonyl-amino, n-Propyl-sulfonyl-amino, Cyclopropyl-sulfonyl-amino, Acetyl-amino, Ethyl-carbonyl-amino, n-Propyl-carbonyl-amino, i-Propyl-carbonyl-amino, Cyclopropyl-carbonyl-amino, Amino-carbonyl, Methylamino-carbonyl, Ethylamino-carbonyl, i-Propylamino-carbonyl, Cyclopropylamino-carbonyl, Dimethylamino-carbonyl, Dimethylamino-methyl, Diethylamino-methyl, Di-i-propylamino-methyl, (R)-3-Dimethylamino-pyrrolidin-1-yl-methyl, (S)-3-Dimethylamino-pyrrolidin-1-yl-methyl, 3-Diethylamino-pyrrolidin-1-yl-methyl, 4-Methyl-piperazin-1-yl-methyl, 4-i-Propyl-piperazin-1-yl-methyl, 4-Dimethylamino-piperidin-1-yl-methyl, 4-Diethylamino-piperidin-1-yl-methyl, 3-Dimethylamino-piperidin-1-yl-methyl, Methyl-sulfinyl, Methyl-sulfonyl, Hydroxy-methyl, Methyl-sulfinyl-methyl, Methyl-sulfonyl-methyl bedeutet.

***b)*** Die Definitionen ***($b^i$)*** für $R^1$ gemäß ihrer zunehmenden Präferenz, beginnend mit bevorzugt ***($b^1$)*** über besonders bevorzugt ***($b^2$)*** zu ganz besonders bevorzugt ***($b^3$)***, sind wie folgt:

***($b^1$):*** Bevorzugt bedeutet $R^1$ ein $C_{4-6}$-Cycloalkyl-System bedeutet, welches 1 bis 2-Stickstoffatome enthält, welches gegebenenfalls 1- bis 2-mal mit $R^7$ substituiert sein kann,
worin $R^7$ $C_{1-3}$-Alkyl, Hydroxy, $C_{1-3}$-Alkoxy, Amino, $C_{1-3}$-Alkyl-amino, Di-($C_{1-3}$-Alkyl)-amino sein kann.

***($b^2$):*** Besonders bevorzugt bedeutet $R^1$ Azetidin-2-yl, Azetidin-3-yl, 1-Methyl-azetidin-2-yl, 1-Methyl-azetidin-3-yl, 1-Ethyl-azetidin-2-yl, 1-Ethyl-azetidin-3-yl, Pyrrolidin-1-yl, Pyrrolidin-2-yl, Pyrrolidin-3-yl, 3-Hydroxy-pyrrolidin-1-yl, 3-Amino-pyrrolidin-1-yl, 3-Methylamino-pyrrolidin-1-yl, 3-Dimethylamino-pyrrolidin-1-yl, 3-Ethylamino-pyrrolidin-1-yl, 3-Diethylamino-pyrrolidin-1-yl, 1-Methyl-pyrrolidin-2-yl, 1-Methyl-pyrrolidin-3-yl, 1-Ethyl-pyrrolidin-2-yl, 1-Ethyl-pyrrolidin-3-yl, 1-Methyl-4-hydroxy-pyrrolidin-2-yl, 1-Methyl-4-methoxy-pyrrolidin-2-yl, Piperidin-1-yl, Piperidin-2-yl, Piperidin-3-yl, Piperidin-4-yl, 4-Amino-piperidin-1-yl, 4-Methylamino-piperidin-1-yl, 4-Dimethylamino-piperidin-1-yl, 4-Ethylamino-piperidin-1-yl, 4-Diethylamino-piperidin-1-yl, 1-Methyl-piperidin-2-yl, 1-Methyl-piperidin-3-yl, 1-Methyl-piperidin-4-yl, 1-Ethyl-piperidin-2-yl, 1-Ethyl-piperidin-3-yl, 1-Ethyl-piperidin-4-yl, Piperazin-1-yl, 4-Methyl-piperazin-1-yl, 4-Ethyl-piperazin-1-yl, 4-Isopropyl-piperazin-1-yl.

***($b^3$)***: Ganz besonders bevorzugt ist $R^1$ 1-Methyl-azetidin-3-yl, Azetidin-3-yl, (2RS)-1-Methyl-azetidin-2-yl, (2S)-1-Methyl-pyrrolidin-2-yl, (2R)-1-Methyl-pyrrolidin-2-yl, (2S)-Pyrrolidin-2-yl, (2S,4S)-1-Methyl-4-hydroxy-pyrrolidin-2-yl, (2R,4S)-1-Methyl-4-hydroxy-pyrrolidin-2-yl, (2S,4R)-1-Methyl-4-hydroxy-pyrrolidin-2-yl, (2R,4R)-1-Methyl-4-hydroxy-pyrrolidin-2-yl, (2R,4R)-1-Methyl-4-methoxy-pyrrolidin-2-yl, (3S)-1-Methyl-pyrrolidin-3-yl, (3R)-1-Methyl-pyrrolidin-3-yl, 1-Methyl-piperidin-3-yl, 1-Methyl-piperidin-4-yl, (3S)-3-Dimethylamino-pyrrolidin-1-yl, (3R)-3-Dimethylamino-pyrrolidin-1-yl, 4-Methyl-piperazin-1-yl, Pyrrolidin-1-yl, (3R)-3-Hydroxy-pyrrolidin-1-yl, (3S)-3-Hydroxy-pyrrolidin-1-yl, Piperazin-1-yl, 4-Isopropyl-piperazin-1-yl, 4-Dimethylamino-piperidin-1-yl.

***c)*** Die Definitionen ***($c^i$)*** für $R^2$ gemäß ihrer zunehmenden Präferenz, beginnend mit bevorzugt ***($c^1$)*** zu besonders

bevorzugt ***($c^2$)***, sind wie folgt:

***($c^1$):*** Bevorzugt bedeutet $R^2$ Wasserstoff, Fluor, Chlor oder Methyl.

***($c^2$):*** Besonders bevorzugt bedeutet $R^2$ Wasserstoff.

***d)*** Die Definitionen ***(d)*** für M gemäß ihrer zunehmenden Präferenz, beginnend mit bevorzugt ***($d^1$)***, zu besonders bevorzugt ***($d^2$)***, sind wie folgt:

***($d^1$):*** Bevorzugt ist M -N< oder -CH<.

***($d^2$):*** Bevorzugt ist M -N<.

***e)*** Die Definitionen ***($e^i$)*** für L gemäß ihrer zunehmenden Präferenz, beginnend mit bevorzugt ***($e^1$)***, zu besonders bevorzugt ***($e^2$)***, sind wie folgt:

***($e^1$):*** Bevorzugt ist L -N< oder -CH<.

***($e^2$):*** Bevorzugt ist L -N<.

***f)*** Die Definitionen ***($f^i$)*** für T gemäß ihrer zunehmenden Präferenz, beginnend mit bevorzugt ***($f^1$)***, über besonders bevorzugt ***($f^2$)*** zu besonders bevorzugt ***($f^3$)***, sind wie folgt:

***($f^1$):*** Bevorzugt bedeutet T eine Bindung oder $C_{1-2}$-Alkylen.

***($f^2$):*** Besonders bevorzugt bedeutet T eine Bindung oder Methylen.

***($f^3$):*** Ganz besonders bevorzugt bedeutet T eine Bindung.

***g)*** Die Definitionen ***($g^i$)*** für m gemäß ihrer zunehmenden Präferenz, beginnend mit bevorzugt ***($g^1$)*** zu besonders bevorzugt ***($g^2$)***, sind wie folgt:

***($g^1$):*** Bevorzugt bedeutet m 0, 1 oder 2.

***($g^2$):*** Besonders bevorzugt bedeutet m 1 oder 2.

***($h^1$):*** Bevorzugt ist n 1.

***i)*** Die Definitionen ***($i^i$)*** für p entsprechend ihrer zunehmenden Präferenz, beginnend mit bevorzugt ***($i^1$)***, über besonders bevorzugt ***($i^2$)*** zu ganz besonders bevorzugt ***($i^3$)***, lauten wie folgt:

***($i^1$):*** Bevorzugt ist p 0, 1 oder 2.

***($i^2$):*** Besonders bevorzugt ist p 0 oder 1.

***($i^3$):*** Ganz besonders bevorzugt ist p 0.

Jedes $\mathbf{a^i}$, $\mathbf{b^i}$, $\mathbf{c^i}$, $\mathbf{d^i}$, $\mathbf{e^i}$, $\mathbf{f^i}$, $\mathbf{g^i}$, $\mathbf{h^i}$, $\mathbf{i^i}$ repräsentiert eine definierte, individuelle Ausführungsform der jeweiligen Gruppe so wie oben dargestellt. Gemäß der oben dargestellten Definitionen ist jede einzelne bevorzugte Ausführungsform durch den Ausdruck ($\mathbf{a^ib^ic^id^ie^if^ig^ih^i}$) vollständig charakterisiert, wobei der Index i jeweils für eine individuelle Ausführungsform steht und die einzelnen Indices i unabhängig voneinander einstellbar sind. Alle individuellen Ausführungsformen, die von dem Ausdruck in Klammem umfasst sind, wobei die Indices i gemäß der oben dargestellten Definitionen beliebig einstell- und kombinierbar sind, sollen von der vorliegenden Erfindung umfasst sein.

In der nachfolgenden Tabelle 1 sind exemplarisch, mit aufsteigender Präferenz von der ersten zur letzten Reihe, die bevorzugten Ausführungsformen E-1 bis E-14 aufgelistet. Demgemäß hat die Ausführungsform E-14, dargestellt in der letzten Reihe von Tabelle 1, die höchste Präferenz.

Tabelle 1.

| | Ar | R¹ | R² | M | L | T | m | n | p |
|---|---|---|---|---|---|---|---|---|---|
| **E-1** | $1^1$ | $b^1$ | $c^1$ | $d^1$ | $e^1$ | $f^1$ | $g^1$ | $h^1$ | $i^1$ |
| **E-2** | $a^2$ | $b^1$ | $c^1$ | $d^1$ | $e^1$ | $f^2$ | $g^1$ | $h^1$ | $i^2$ |
| **E-3** | $a^1$ | $b^1$ | $c^1$ | $d^1$ | $e^1$ | $f^1$ | $g^1$ | $h^1$ | $i^1$ |
| **E-4** | $a^2$ | $b^2$ | $c^2$ | $c^1$ | $e^1$ | $f^2$ | $g^2$ | $h^1$ | $i^3$ |
| **E-5** | $a^2$ | $b^2$ | $c^2$ | $d^1$ | $e^1$ | $f^2$ | $g^2$ | $h^1$ | $i^3$ |
| **E-6** | $a^2$ | $b^3$ | $c^2$ | $c^2$ | $e^1$ | $f^3$ | $g^2$ | $h^1$ | $i^3$ |
| **E-7** | $a^3$ | $b^2$ | $c^2$ | $d^2$ | $e^1$ | $f^3$ | $g^2$ | $h^1$ | $i^3$ |
| **E-9** | $a^2$ | $b^3$ | $c^2$ | $d^1$ | $e^1$ | $f^2$ | $g^2$ | $h^1$ | $i^3$ |
| **E-10** | $a^3$ | $b^2$ | $c^2$ | $d^1$ | $e^1$ | $f^2$ | $g^2$ | $h^1$ | $i^3$ |
| **E-11** | $a^3$ | $b^3$ | $c^2$ | $d^2$ | $e^1$ | $f^2$ | $g^2$ | $h^1$ | $i^3$ |
| **E-12** | $a^3$ | $b^3$ | $c^2$ | $d^2$ | $e^2$ | $f^3$ | $g^2$ | $h^1$ | $i^3$ |
| **E-13** | $a^4$ | $b^3$ | $c^2$ | $d^2$ | $e^1$ | $f^2$ | $g^2$ | $h^1$ | $i^3$ |
| **E-14** | $a^4$ | $b^3$ | $c^2$ | $d^2$ | $e^2$ | $f^3$ | $g^2$ | $h^1$ | $i^3$ |

einschließlich deren Tautomere, deren Stereoisomere und deren Gemische.

Die in der vorliegenden Anmeldung beschriebenen und beanspruchten Verbindungen nach Formel (I) haben sich als in zweifacher Hinsicht besonders vorteilhaft erwiesen. Einerseits sind sie vergleichsweise gut löslich. Andererseits inhibieren sie das Cytochrom P450 2C9-Isoenzym in vergleichsweise geringem Umfang, was allgemein als Indiz akzeptiert dafür ist, daß die hier beschriebenen und beanspruchten Verbindungen nur in geringem Maße oder überhaupt nicht zu Drug-Drug-Interaktionen, welche auf dem Cytochrom P450 2C9-Isoenzym beruhen, neigen. Beispielsweise seien folgende Verbindungen als bevorzugte Verbindungen erwähnt:

- 1-Methyl-7-[4-(1-methyl-piperidin-4-carbonyl)-piperazin-1-ylmethyl]-1H-indol-2-carbonsäure (5-tert-butyl-3-methansulfonylamino-2-methoxy-phenyl)-amid

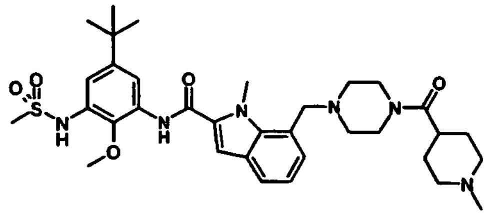

- (S)-1-Methyl-7-[4-(1-methyl-pyrrolidin-2-carbonyl)-piperazin-1-ylmethyl]-1H-indol-2-carbonsäure (5-tert-butyl-3-methansulfonylamino-2-methoxy-phenyl)-amid

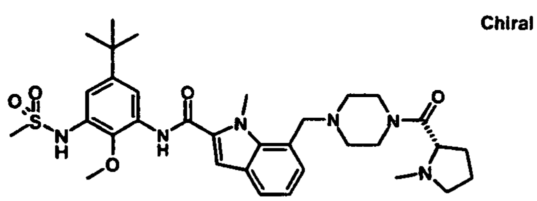


- 1-Methyl-7-[4-(1-methyl-azetidin-3-carbonyl)-piperazin-1-ylmethyl]-1H-indol-2-carbonsäure (5-tert-butyl-3-methansulfonylamino-2-methoxy-phenyl)-amid

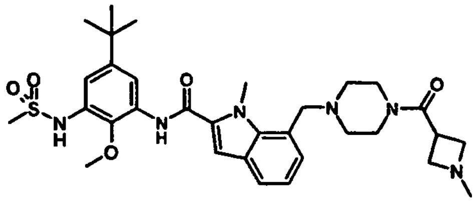

- (R)-1-Methyl-7-[4-(1-methyl-pyrrolidin-2-carbonyl)-piperazin-1-ylmethyl]-1H-indol-2-carbonsäure (5-tert-butyl-3-methansulfonylamino-2-methoxy-phenyl)-amid

Chiral

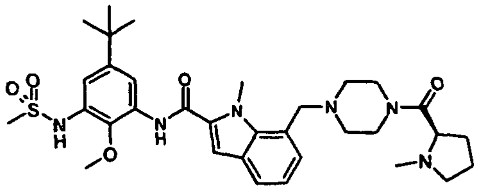

- (S)-1-Methyl-7-[4-(pyrrolidin-2-carbonyl)-piperazin-1-ylmethyl]-1H-indol-2-carbonsäure (5-tert-butyl-3-methansulfonylamino-2-methoxy-phenyl)-amid

Chiral

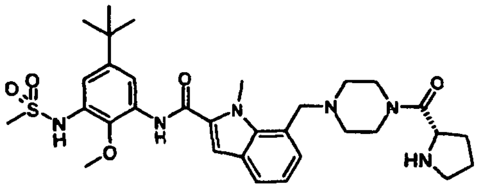

- 1-Methyl-7-[4-(1-methyl-azetidin-2-carbonyl)-piperazin-1-ylmethyl]-1H-indol-2-carbonsäure (5-tert-butyl-3-methansulfonylamino-2-methoxy-phenyl)-amid

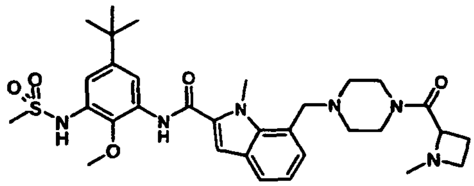

- (R)-1-Methyl-7-[1-(1-methyl-pyrrolidin-2-carbonyl)-piperidin-4-ylmethyl]-1H-indol-2-carbonsäure (5-tert-butyl-3-methansulfonylamino-2-methoxy-phenyl)-amid

Chiral

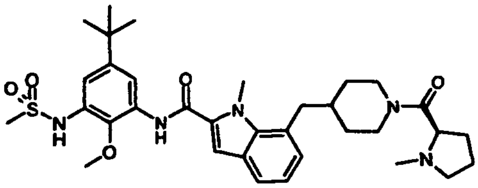

- (S)-1-Methyl-7-[1-(1-methyl-pyrrolidin-2-carbonyl)-piperidin-4-ylmethyl]-1H-indol-2-carbonsäure (5-tert-butyl-3-methansulfonylamino-2-methoxy-phenyl)-amid

Chiral

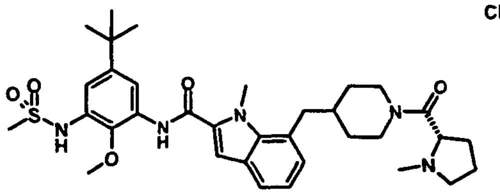

- 7-[4-((25,4R)-4-Hydroxy-1-methyl-pyrrolidin-2-carbonyl)-piperazin-1-ylmethyl]-1-methyl-1H-indol-2-carbonsäure (5-tert-butyl-3-methansulfonylamino-2-methoxy-phenyl)-amid

Chiral

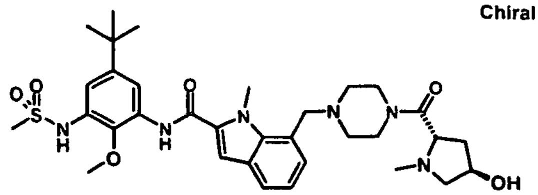

- 7-[4-((2R,4R)-4-Methoxy-1-methyl-pyrrolidin-2-carbonyl)-piperazin-1-ylmethyl]-1-methyl-1H-indol-2-carbonsäure (5-tert-butyl-3-methansulfonylamino-2-methoxy-phenyl)-amid

Chiral

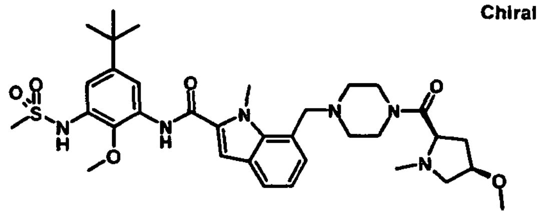

- 7-[4-((2R,4R)-4-Hydroxy-1-methyl-pyrrolidin-2-carbonyl)-piperazin-1-ylmethyl]-1-methyl-1H-indol-2-carbonsäure (5-tert-butyl-3-methansulfonylamino-2-methoxy-phenyl)-amid

Chiral

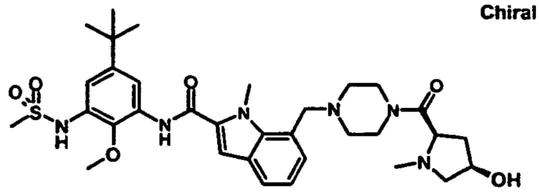

- 7-[4-((2R,4S)-4-Hydroxy-1-methyl-pyrrolidin-2-carbonyl)-piperazin-1-ylmethyl]-1-methyl-1H-indol-2-carbonsäure (5-tert-butyl-3-methansulfonylamino-2-methoxy-phenyl)-amid

Chiral

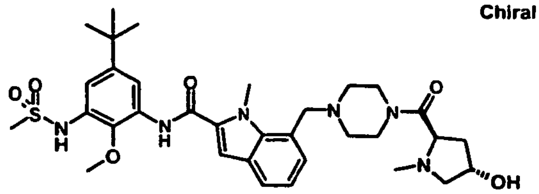

- 7-[4-((2S,4S)-4-Hydroxy-1-methyl-pyrrolidin-2-carbonyl)-piperazin-1-ylmethyl]-1-methyl-1H-indol-2-carbonsäure (5-tert-butyl-3-methansulfonylamino-2-methoxy-phenyl)-amid

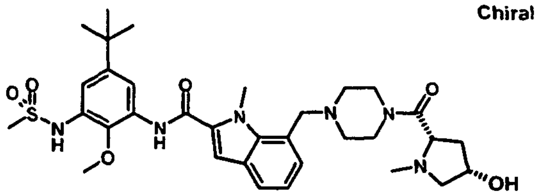

- 1-Methyl-7-[4-(2-pyrrolidin-1-yl-propionyl)-piperazin-1-ylmethyl]-1H-indol-2-carbonsäure-(5-tert-butyl-3-methansulfonylamino-2-methoxy-phenyl)-amid

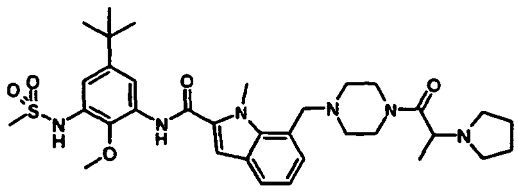

- 7-{4-[2-(4-Isopropyl-piperazin-1-yl)-acetyl]-piperazin-1-ylmethyl}-1-methyl-1H-indol-2-carbonsäure-(5-tert-butyl-3-methansulfonylamino-2-methoxy-phenyl)-amid

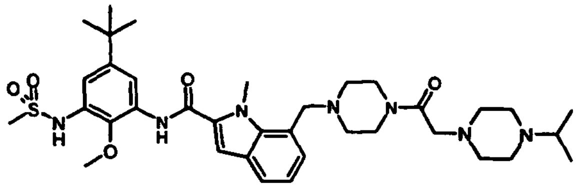

- (S)-7-{4-[2-(3-Hydroxy-pyrrolidin-1-yl)-acetyl]-piperazin-1-ylmethyl}-1-methyl-1H-indol-2-carbonsäure-(5-tert-butyl-3-methansulfonylamino-2-methoxy-phenyl)-amid

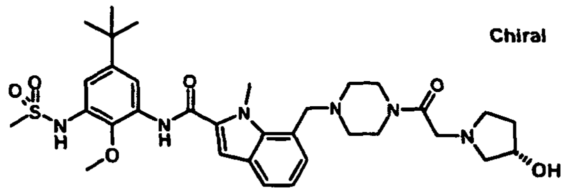

- 1-Methyl-7-[4-(2-pyrrolidin-1-yl-acetyl)-piperazin-1-ylmethyl]-1H-indol-2-carbonsäure-(5-tert-butyl-3-methansulfonylamino-2-methoxy-phenyl)-amid

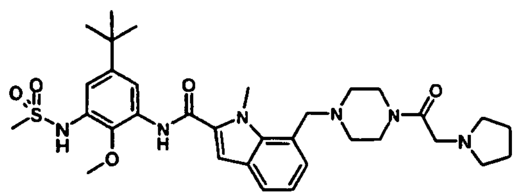

- 1-Methyl-7-{4-[2-(4-methyl-piperazin-1-yl)-acetyl]-piperazin-1-ylmethyl}-1H-indol-2-carbonsäure-(5-tert-butyl-3-methansulfonylamino-2-methoxy-phenyl)-amid

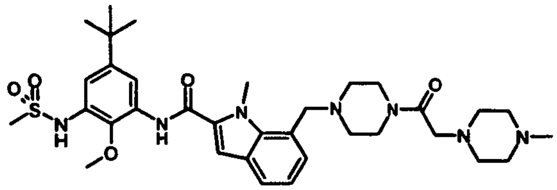

- 1-Methyl-7-[1-((2S)-1-methyl-pyrrolidin-2-carbonyl)-azepan-4-ylmethyl]-1H-indol-2-carbonsäure-(5-tert-butyl-3-methansulfonylamino-2-methoxy-phenyl)-amid

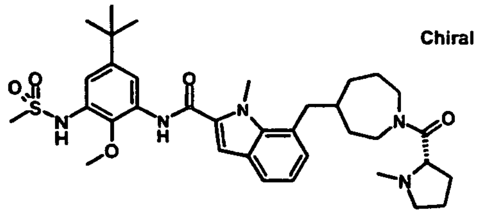

- 1-Methyl-7-[1-(1-methyl-piperidin-4-carbonyl)-azepan-4-ylmethyl]-1H-indol-2-carbonsäure-(5-tert-butyl-3-methansulfonylamino-2-methoxy-phenyl)-amid

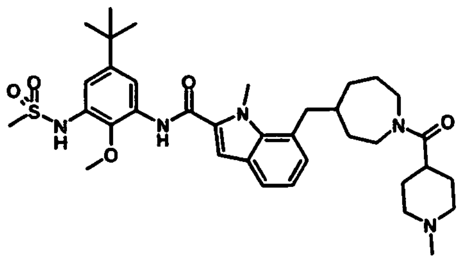

- 1-Methyl-7-[1-((2R)-1-methyl-pyrrolidin-2-carbonyl)-azepan-4-ylmethyl]-1H-indol-2-carbonsäure-(5-tert-butyl-3-methansulfonylamino-2-methoxy-phenyl)-amid

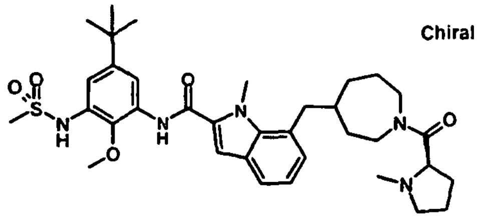

- 1-Methyl-7-[4-((2R)-1-methyl-pyrrolidin-2-carbonyl)-[1,4]diazepan-1-ylmethyl]-1H-indol-2-carbonsäure-(5-tert-butyl-3-methansulfonylamino-2-methoxy-phenyl)-amid

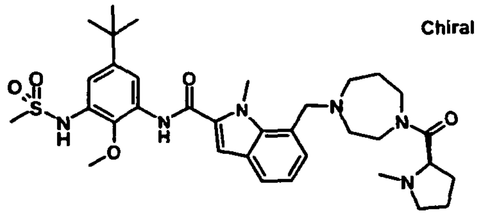

- 1-Methyl-7-[4-(4-methyl-piperazin-1-carbonyl)-piperidin-1-ylmethyl]-1H-indol-2-carbonsäure-(5-tert-butyl-3-methansulfonylamino-2-methoxy-phenyl)-amid

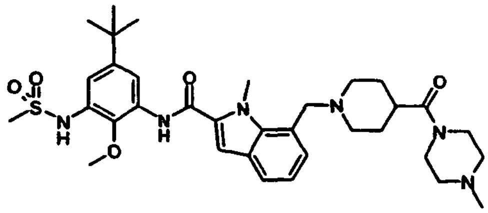

- (S)-7-[4-(3-Dimethylamino-pyrrolidin-1-carbonyl)-piperidin-1-ylmethyl]-1-methyl-1H-indol-2-carbonsäure-(5-tert-butyl-3-methansulfonylamino-2-methoxy-phenyl)-amid

Chiral

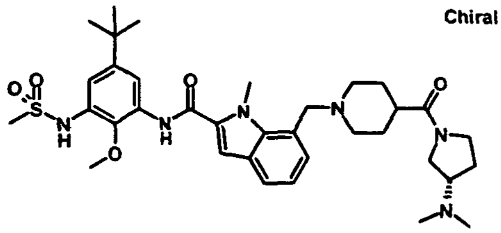

- (R)-7-[4-(3-Dimethylamino-pyrrolidin-1-carbonyl)-piperidin-1-ylmethyl]-1-methyl-1H-indol-2-carbonsäure-(5-tert-butyl-3-methansulfonylamino-2-methoxy-phenyl)-amid

Chiral

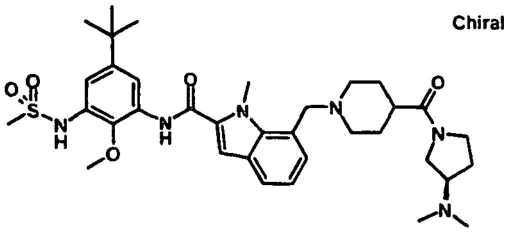

- (S)-1-Methyl-7-[4-(1-methyl-pyrrolidin-2-carbonyl)-piperazin-1-ylmethyl]-1H-indol-2-carbonsäure-(3-morpholin-4-yl-phenyl)-amid

Chiral

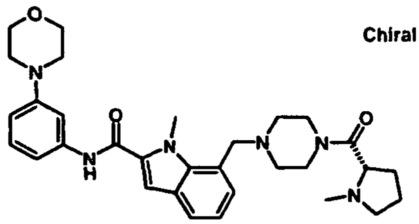

- (R)-7-[4-(3-Dimethylamino-pyrrolidin-1-carbonyl)-piperazin-1-ylmethyl]-1-methyl-1H-indol-2-carbonsäure (5-tert-butyl-3-methansulfonylamino-2-methoxy-phenyl)-amid

Chiral

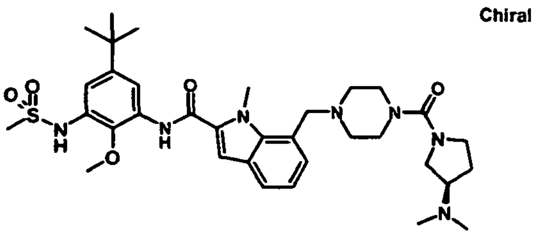

- (S)-7-[4-(3-Dimethylamino-pyrrolidin-1-carbonyl)-piperazin-1-ylmethyl]-1-methyl-1H-indol-2-carbonsäure (5-tert-butyl-3-methansulfonylamino-2-methoxy-phenyl)-amid

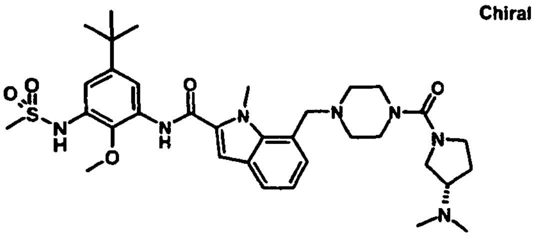

- 7-[4-(Azetidin-3-carbonyl)-piperazin-1-ylmethyl]-1-methyl-1H-indol-2-carbonsäure (5-tert-butyl-3-methansulfonyl-amino-2-methoxy-phenyl)-amid

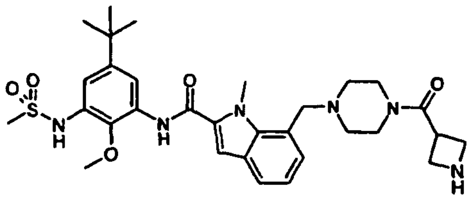

- 1-Methyl-7-[4-(1-methyl-piperidin-3-carbonyl)-piperazin-1-ylmethyl]-1H-indol-2-carbonsäure (5-tert-butyl-3-methansulfonylamino-2-methoxy-phenyl)-amid

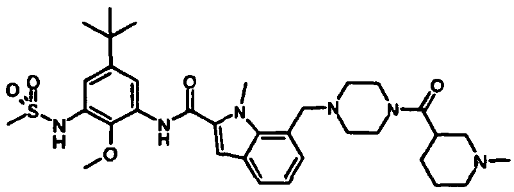

- (S)-1-Methyl-7-[4-(1-methyl-pyrrolidin-3-carbonyl)-piperazin-1-ylmethyl]-1H-indol-2-carbonsäure (5-tert-butyl-3-methansulfonylamino-2-methoxy-phenyl)-amid

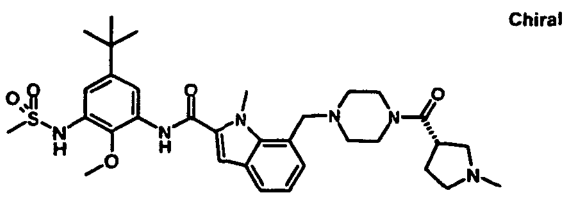

- (S)-1-Methyl-7-[4-(1-methyl-pyrrolidin-3-carbonyl)-[1,4]diazepan-1-ylmethyl]-1H-indol-2-carbonsäure-(5-tert-butyl-3-methansulfonylamino-2-methoxy-phenyl)-amid

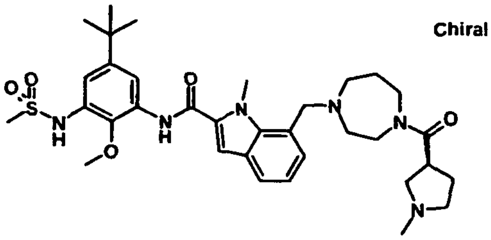

- (R)-1-Methyl-7-[4-(1-methyl-pyrrolidin-3-carbonyl)-[1,4]diazepan-1-ylmethyl]-1H-indol-2-carbonsäure-(5-tert-butyl-3-methansulfonylamino-2-methoxy-phenyl)-amid

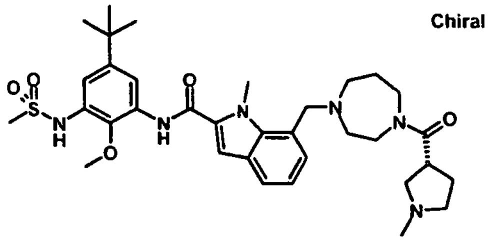


- 7-{4-[2-(4-Dimethylamino-piperidin-1-yl)-acetyl]-piperazin-1-ylmethyl}-1-methyl-1H-indol-2-carbonsäure-(5-tert-butyl-3-methansulfonylamino-2-methoxy-phenyl)-amid

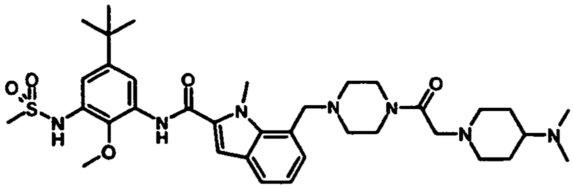

- (R)-7-{4-[2-(3-Hydroxy-pyrrolidin-1-yl)-acetyl]-piperazin-1-ylmethyl}-1-methyl-1H-indol-2-carbonsäure-(5-tert-butyl-3-methansulfonylamino-2-methoxy-phenyl)-amid

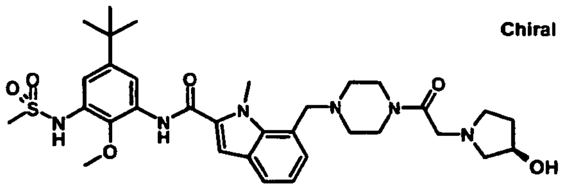


- (R)-7-{4-[2-(3-Dimethylamino-pyrrolidin-1-yl)-acetyl]-piperazin-1-ylmethyl}-1-methyl-1H-indol-2-carbonsäure-(5-tert-butyl-3-methansulfonylamino-2-methoxy-phenyl)-amid

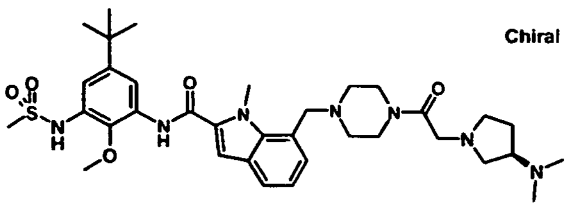


- (S)-7-{4-[2-(3-Dimethylamino-pyrrolidin-1-yl)-acetyl]-piperazin-1-ylmethyl}-1-methyl-1H-indol-2-carbonsäure-(5-tert-butyl-3-methansulfonylamino-2-methoxy-phenyl)-amid

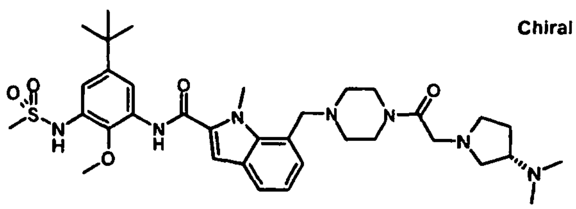


- 1-Methyl-7-[4-(2-piperazin-1-yl-acetyl)-piperazin-1-ylmethyl]-1H-indol-2-carbonsäure-(5-tert-butyl-3-methansulfonylamino-2-methoxy-phenyl)-amid

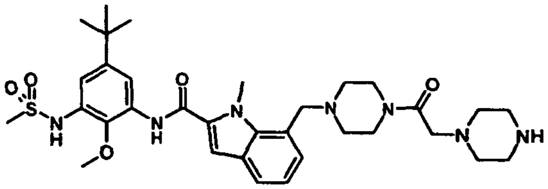

- 1-Methyl-7-[4-((2S)-1-methyl-pyrrolidin-2-carbonyl)-[1,4]diazepan-1-ylmethyl]-1H-indol-2-carbonsäure-(5-tert-butyl-3-methansulfonylamino-2-methoxy-phenyl)-amid

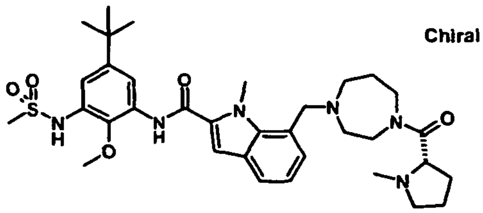


- 1-Methyl-7-[4-(1-methyl-piperidin-4-carbonyl)-[1,4]diazepan-1-ylmethyl]-1H-indol-2-carbonsäure-(5-tert-butyl-3-methansulfonylamino-2-methoxy-phenyl)-amid

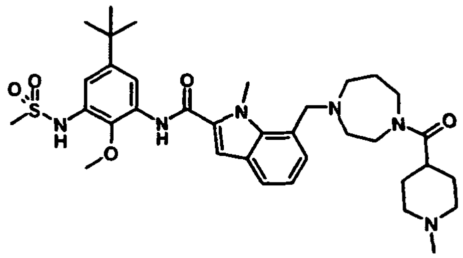

- (S)-1-Methyl-7-[4-(1-methyl-pyrrolidin-2-carbonyl)-piperazin-1-ylmethyl]-1H-indol-2-carbonsäure-(5-isobutyl-3-methansulfonylamino-2-methoxy-phenyl)-amid

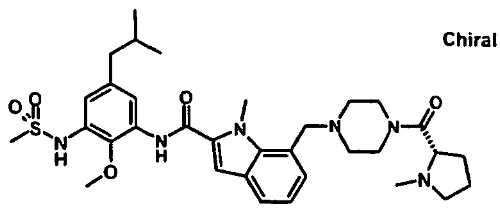


- (S)-1-Methyl-7-[4-(1-methyl-pyrrolidin-2-carbonyl)-piperazin-1-ylmethyl]-1H-indol-2-carbonsäure-[5-tert-butyl-2-methoxy-3-(4-methyl-piperazin-1-ylmethyl)-phenyl]-amid

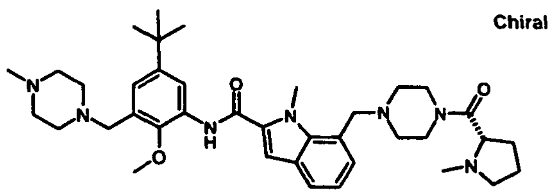


- (S)-1-Methyl-7-[4-(1-methyl-pyrrolidin-2-carbonyl)-piperazin-1-ylmethyl]-1H-indol-2-carbonsäure-{5-tert-butyl-3-[(diisopropylamino)-methyl]-2-methoxyphenyl}-amid

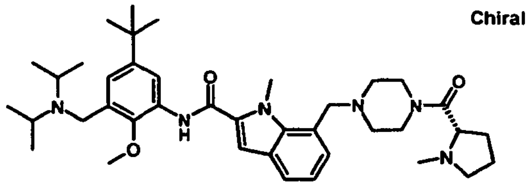

Chiral

- (S)-1-Methyl-7-[4-(1-methyl-pyrrolidin-2-carbonyl)-piperazin-1-ylmethyl]-1H-indol-2-carbonsäure-(5-tert-butyl-3-diethylaminomethyl-2-methoxy-phenyl)-amid

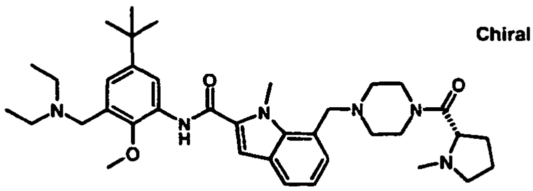

Chiral

- 1-Methyl-7-[4-((2S)-1-methyl-pyrrolidin-2-carbonyl)-piperazin-1-ylmethyl]-1H-indol-2-carbonsäure-[5-tert-butyl-3-(3-dimethylamino-piperidin-1-ylmethyl)-2-methoxy-phenyl]-amid

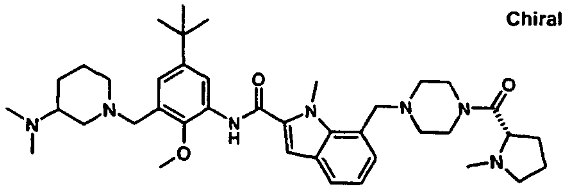

Chiral

- 1-Methyl-7-[4-((2S)-1-methyl-pyrrolidin-2-carbonyl)-piperazin-1-ylmethyl]-1H-indol-2-carbonsäure-[5-tert-butyl-3-(3-diethylamino-pyrrolidin-1-ylmethyl)-2-methoxy-phenyl]-amid

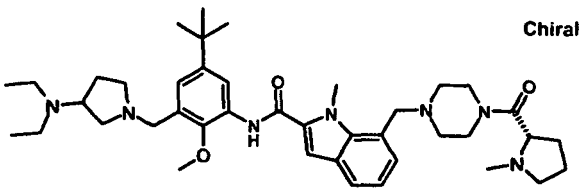

Chiral

- (S)-1-Methyl-7-[4-(1-methyl-pyrrolidin-2-carbonyl)-piperazin-1-ylmethyl]-1H-indol-2-carbonsäure-[5-tert-butyl-3-(4-dimethylamino-piperidin-1-ylmethyl)-2-methoxy-phenyl]-amid

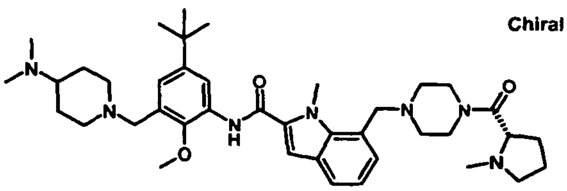

Chiral

- (S)-1-Methyl-7-[4-(1-methyl-pyrrolidin-2-carbonyl)-piperazin-1-ylmethyl]-1H-indol-2-carbonsäure-[5-tert-butyl-3-(4-diethylamino-piperidin-1-ylmethyl)-2-methoxy-phenyl]-amid

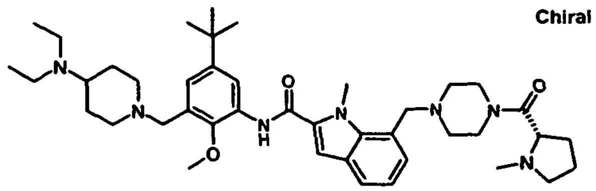


- (S)-1-Methyl-7-[4-(1-methyl-pyrrolidin-2-carbonyl)-piperazin-1-ylmethyl]-1H-indol-2-carbonsäure-[5-tert-butyl-3-(4-isopropyl-piperazin-1 -ylmethyl)-2-methoxy-phenyl]-amid

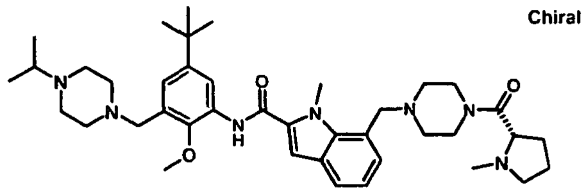


- (S)-1-Methyl-7-[4-(1-methyl-pyrrolidin-2-carbonyl)-piperazin-1-ylmethyl]-1H-indol-2-carbonsäure-(5-tert-butyl-3-cyclopropylcarbamoyl-2-methoxy-phenyl)-amid

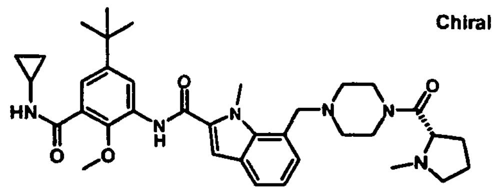


- (S)-1-Methyl-7-[4-(1-methyl-pyrrolidin-2-carbonyl)-piperazin-1-ylmethyl]-1H-indol-2-carbonsäure-(5-tert-butyl-3-isopropylcarbamoyl-2-methoxy-phenyl)-amid

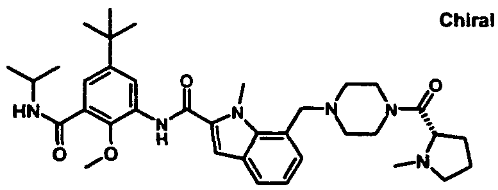


- (S)-1-Methyl-7-[4-(1-methyl-pyrrolidin-2-carbonyl)-piperazin-1-ylmethyl]-1H-indol-2-carbonsäure-(5-tert-butyl-3-ethylcarbamoyl-2-methoxy-phenyl)-amid

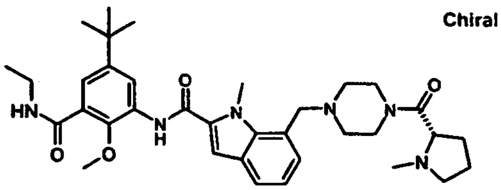


- (S)-1-Methyl-7-[4-(1-methyl-pyrrolidin-2-carbonyl)-piperazin-1-ylmethyl]-1H-indol-2-carbonsäure-(5-tert-butyl-2-methoxy-3-methylcarbamoyl-phenyl)-amid

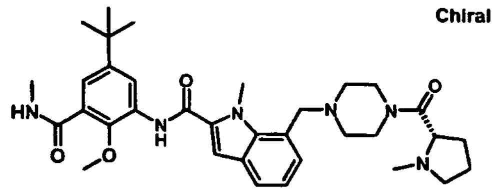

- (S)-1-Methyl-7-[4-(1-methyl-pyrrolidin-2-carbonyl)-piperazin-1-ylmethyl]-1H-indol-2-carbonsäure-(5-tert-butyl-3-carbamoyl-2-methoxy-phenyl)-amid

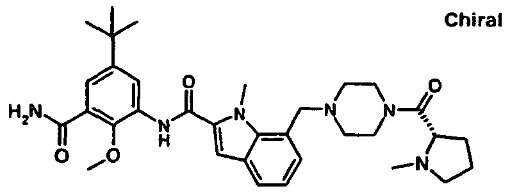

- (S)-1-Methyl-7-[4-(1-methyl-pyrrolidin-2-carbonyl)-piperazin-1-ylmethyl]-1H-indol-2-carbonsäure-(5-tert-butyl-3-dimethylcarbamoyl-2-methoxy-phenyl)-amid

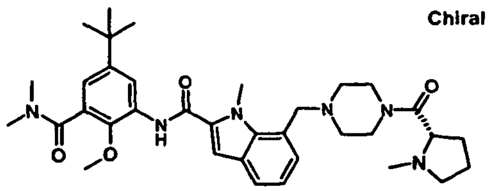

- 1-Methyl-7-[4-((2S)-1-methyl-pyrrolidin-2-carbonyl)-piperazin-1-ylmethyl]-1H-indol-2-carbonsäure-(5-sec-butyl-3-methansulfonylamino-2-methoxy-phenyl)-amid

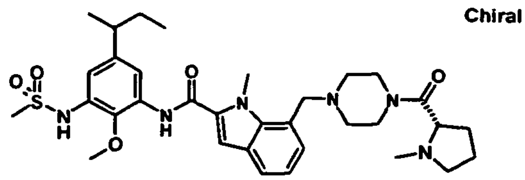

- (S)-1-Methyl-7-[4-(1-methyl-pyrrolidin-2-carbonyl)-piperazin-1-ylmethyl]-1H-indol-2-carbonsäure-(5-isopropyl-3-methansulfonylamino-2-methoxy-phenyl)-amid

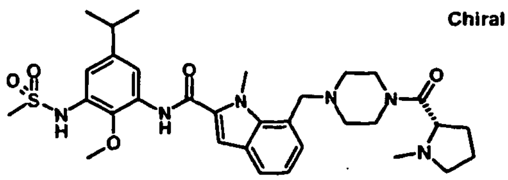

- 1-Methyl-7-[4-((2S)-1-methyl-pyrrolidin-2-carbonyl)-piperazin-1-ylmethyl]-1H-indol-2-carbonsäure-(5-tert-butyl-3-methansulfinyl-2-methoxy-phenyl)-amid

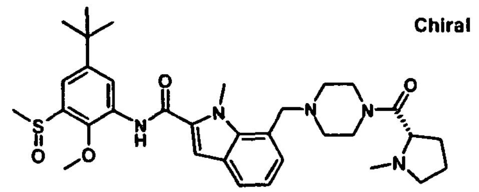


- (S)-1-Methyl-7-[4-(1-methyl-pyrrolidin-2-carbonyl)-piperazin-1-ylmethyl]-1H-indol-2-carbonsäure-(5-tert-butyl-3-methansulfonyl-2-methoxy-phenyl)-amid

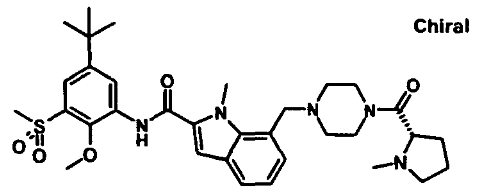


- (S)-1-Methyl-7-[4-(1-methyl-pyrrolidin-2-carbonyl)-piperazin-1-ylmethyl]-1H-indol-2-carbonsäure-(5-tert-butyl-3-methansulfonylmethyl-2-methoxy-phenyl)-amid

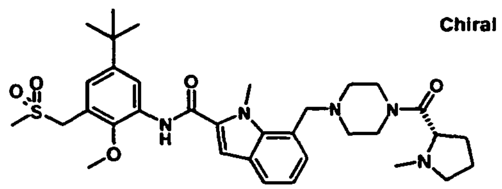


- 1-Methyl-7-[4-((2S)-1-methyl-pyrrolidin-2-carbonyl)-piperazin-1-ylmethyl]-1H-indol-2-carbonsäure-(5-tert-butyl-3-methansulfinylmethyl-2-methoxy-phenyl)-amid

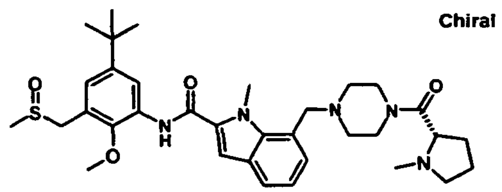


- (S)-1-Methyl-7-[4-(1-methyl-pyrrolidin-2-carbonyl)-piperazin-1-ylmethyl]-1H-indol-2-carbonsäure-(5-tert-butyl-3-methansulfonylamino-2-ethoxy-phenyl)-amid

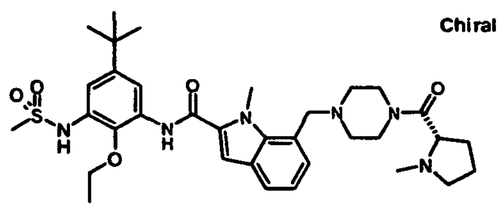


- (S)-1-Methyl-7-[4-(1-methyl-pyrrolidin-2-carbonyl)-piperazin-1-ylmethyl]-1H-indol-2-carbonsäure-(5-tert-butyl-2-methoxy-3-propionylamino-phenyl)-amid

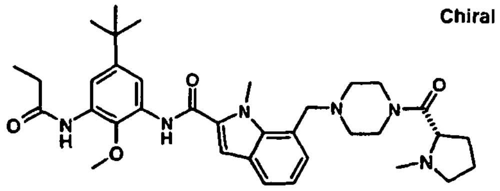

- (S)-1-Methyl-7-(4-(1-methyl-pyrrolidin-2-carbonyl)-piperazin-1-ylmethyl]-1H-indol-2-carbonsäure-(5-tert-butyl-3-butyrylamino-2-methoxy-phenyl)-amid

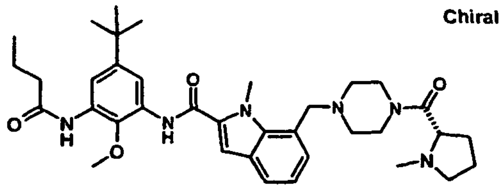

- (S)-1-Methyl-7-[4-(1-methyl-pyrrolidin-2-carbonyl)-piperazin-1-ylmethyl]-1H-indol-2-carbonsäure-(3-acetylamino-5-tert-butyl-2-methoxy-phenyl)-amid

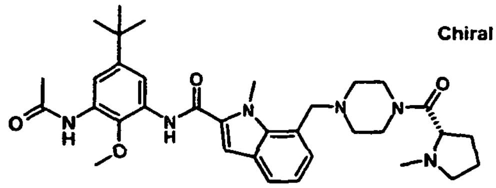

- (S)-1-Methyl-7-[4-(1-methyl-pyrrolidin-2-carbonyl)-piperazin-1-ylmethyl]-1H-indol-2-carbonsäure-(5-tert-butyl-3-isobutyrylamino-2-methoxy-phenyl)-amid

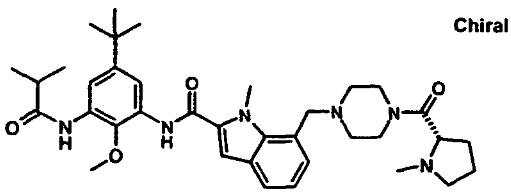

- (S)-1-Methyl-7-[4-(1-methyl-pyrrolidin-2-carbonyl)-piperazin-1-ylmethyl]-1H-indol-2-carbonsäure-[5-tert-butyl-3-(cyclopropancarbonyl-amino)-2-methoxyphenyl]-amid

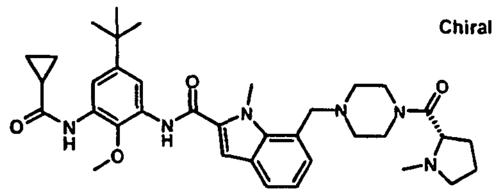

- (S)-1-Methyl-7-[4-(1-methyl-pyrrolidin-2-carbonyl)-piperazin-1-ylmethyl]-1H-indol-2-carbonsäure-[5-tert-butyl-2-methoxy-3-(propan-1-sulfonylamino)-phenyl]-amid

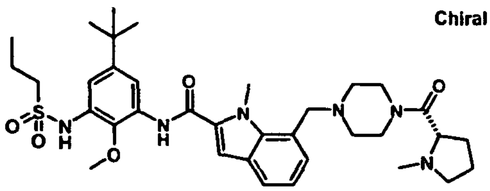

- (S)-1-Methyl-7-[4-(1-methyl-pyrrolidin-2-carbonyl)-piperazin-1-ylmethyl]-1H-indol-2-carbonsäure-(5-tert-butyl-3-cyclopropansulfonylamino-2-methoxyphenyl)-amid

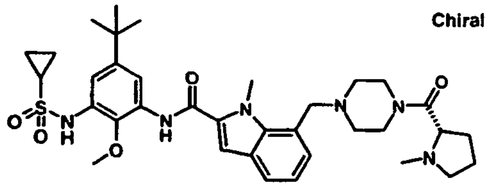

- (S)-1-Methyl-7-[4-(1-methyl-pyrrolidin-2-carbonyl)-piperazin-1-ylmethyl]-1H-indol-2-carbonsäure-(3-methansulfonyfamino-2-methoxy-5-pentafluoroethyl-phenyl)-amid

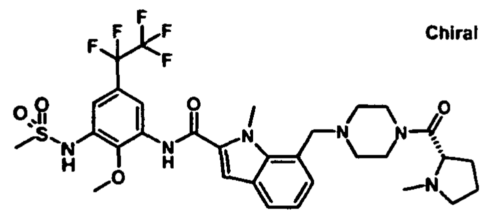

- (S)-1-Methyl-7-[4-(1-methyl-pyrrolidin-2-carbonyl)-piperazin-1-ylmethyl]-1H-indol-2-carbonsäure-(3-methansulfonylamino-2-methoxy-5-trifluoromethyl-phenyl)-amid

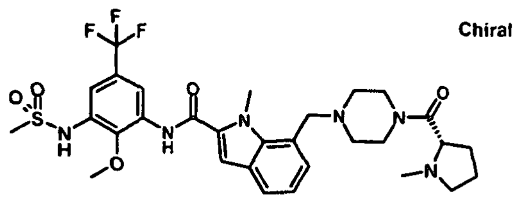

- (S)-1-Methyl-7-[4-(1-methyl-pyrrolidin-2-carbonyl)-piperazin-1-ylmethyl]-1H-indol-2-carbonsäure-(5-tert-butyl-3-hydroxymethyl-2-methoxy-phenyl)-amid

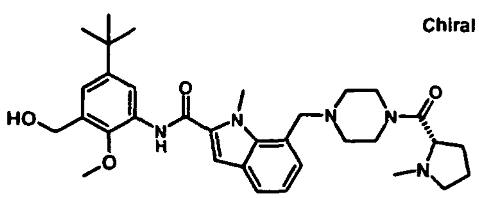

einschließlich deren Tautomere, deren Stereoisomere und deren Gemische.

Im folgenden werden Begriffe, die zuvor und nachfolgend zur Beschreibung der erfindungsgemäßen Verbindungen verwendet werden, näher definiert.

Der Begriff "substituiert", wie er hierin verwendet wird, bedeutet, dass ein oder mehrere Wasserstoffatom(e) an einem bestimmten Atom (einer Gruppe/eines Restes) durch ein Atom (eine Gruppe/ein Rest) ausgewählt aus einer bestimmten Gruppe ersetzt ist, vorausgesetzt die mögliche Valenzzahl des entsprechenden Atoms wird nicht überschritten und die Substitution führt zu einer stabilen Verbindung.

Die Bezeichnung Halogen bezeichnet ein Atom ausgewählt aus der Gruppe bestehend aus F, Cl, Br und I.

Die Bezeichnung Heteroatom bezeichnet ein Atom ausgewählt aus N, O, S oder P. Äquivalent berzeichnen z.B. Heteroalkyl, Heteroaryl usw. Alkyl- oder Arylstrukturen, in denen Kohlenstoffatome durch Heteroatome ersetzt wurden.

Die Bezeichnung $C_{1-n}$-Alkyl, wobei n einen wie zuvor angegebenen Wert besitzen kann, bedeutet eine gesättigte, verzweigte oder unverzweigte Kohlenwasserstoffgruppe mit 1 bis n C-Atomen. Beispiele solcher Gruppen umfassen Methyl, Ethyl, n-Propyl, i-Propyl, Butyl, i-Butyl, sec.-Butyl, tert.-Butyl, n-Pentyl, i-Pentyl, neo-Pentyl, tert.-Pentyl, n-Hexyl, i-Hexyl, etc..

Der Begriff $C_{1-n}$-Alkoxy oder $C_{1-n}$-Alkyloxy bezeichnet eine $C_{1-n}$-Alkyl-O-Gruppe, worin $C_{1-n}$-Alkyl wie oben definiert ist. Beispiele solcher Gruppen umfassen Methoxy, Ethoxy, n-Propoxy, i-Propoxy, n-Butoxy, i-Butoxy, sec.-Butoxy, tert.-Butoxy, n-Pentoxy, i-Pentoxy, neo-Pentoxy, tert.-Pentoxy, n-Hexoxy, i-Hexoxy etc..

Der Begriff $C_{1-n}$-Alkylcarbonyl beziehungsweise $C_{1-n}$-Alkyl-carbonyl bezeichnet eine $C_{1-n}$-Alkyl-C(=O)-Gruppe, worin $C_{1-n}$-Alkyl wie oben definiert ist. Beispiele solcher Gruppen umfassen Methylcarbonyl, Ethylcarbonyl, n-Propylcarbonyl, i-Propylcarbonyl, n-Butylcarbonyl, i-Butylcarbonyl, sec.-Butylcarbonyl, tert.-Butylcarbonyl, n-Pentylcarbonyl, i-Pentylcarbonyl, neo-Pentylcarbonyl, tert.-Pentylcarbonyl, n-Hexylcarbonyl, i-Hexylcarbonyl, etc..

Der Begriff $C_{3-n}$-Cycloalkyl bezeichnet, wobei n Werte wie zuvor angegeben annehmen kann, eine gesättigte mono-, bi-, tri- oder spirocarbocyclische Gruppe mit 3 bis n C-Atomen. Beispiele solcher Gruppen umfassen Cyclopropyl, Cyclobutyl, Cyclopentyl, Cyclohexyl, Cycloheptyl, Cyclooctyl, Cyclononyl, Cyclododecyl, Bicyclo[3.2.1.]octyl, Spiro[4.5]decyl, Norpinyl, Norbornyl, Norcaryl, Adamantyl, etc.. Vorzugsweise umfasst der Begriff $C_{3-7}$-Cycloalkyl gesättigte monocyclische Gruppen. Ebenso für cyclische Reste steht der Suffix "c-". Zum Beispiel bedeutet "c-Hexyl" Cyclohexyl oder "c-$C_{3-n}$-Alkyl" einen cyclischen Alkylrest mit 3-n Ringatomen.

Der Begriff $C_{3-n}$-Cycloalkylcarbonyl beziehungsweise $C_{3-n}$-Cycloalkyl-carbonyl bezeichnet eine $C_{3-n}$-Cycloalkyl-C(=O)-Gruppe, worin $C_{3-n}$-Cycloalkyl wie oben definiert ist.

Der Begriff $C_{3-n}$-Cycloalkylsulfonyl beziehungsweise $C_{3-n}$-Cycloalkyl-sulfonyl bezeichnet eine $C_{3-n}$-Cycloalkyl-$S(=O)_2$-Gruppe, worin $C_{3-n}$-Cycloalkyl wie oben definiert ist.

Der Begriff Di-($C_{1-3}$-alkyl)amino umfasst Amino-Gruppen, die gleiche oder zwei verschiedene Alkylgruppen aufweisen.

Falls in Gruppen, beispielsweise in $R^2$, $R^3$, $R^4$, $R^5$, $R^6$, oder $R^7$, vorkommende Alkyl-Reste substituiert, beispielsweise fluoriert, sein können, so umfasst dies nicht nur Alkyl-Reste in den Gruppen die unmittelbar Alkyl bedeuten, sondern auch in anderen, Alkyl-Reste aufweisenden Bedeutungen, wie beispielsweise Alkyloxy, Alkylcarbonyl, Alkoxyalkyl, etc.. So umfasst beispielsweise $R^2$, $R^3$, $R^4$, $R^5$, $R^6$ oder $R^7$ in der Bedeutung Alkyloxy beziehungsweise Alkoxy, wobei Alkylreste teilweise oder vollständig fluoriert sein können, auch Difluormethoxy und Trifluormethoxy.

Die vorstehend und nachfolgend verwendete Schreibweise, bei der in einer Phenylgruppe beziehungsweise einer Heteroarylgruppe eine Bindung eines Substituenten zur Mitte des Phenylrings beziehungsweise des Heteroarylrings hin dargestellt ist, bedeutet, sofern nicht anders angegeben, dass dieser Substituent an jede freie, ein H-Atom tragende Position des Phenylrings beziehungsweise Heteroarylrings gebunden sein kann.

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf das Enzym p38 MAP-Kinase.

Die biologischen Eigenschaften der neuen Verbindungen können wie folgt geprüft werden:

Die Hemmung der p38 MAP-Kinase vermittelten Signafübertragung kann z.B. mittels eines enzymatischen Assays nachgewiesen werden. Dabei wird die Menge eines durch die Kinase phosphorylierten Substrats quantifiziert. Der Test wird wie folgt durchgeführt:

<u>Enzymatischer Assay:</u>

Die Kinasereaktion wird in Anwesenheit von HEPES (20 mM, pH 7), MgCl2 (10 mM), DTT (1 mM) und TWEEN20 (0.01%) durchgeführt. Zuerst werden in einem Reaktionsgefäß Dimethylsulfoxid oder Inhibitor (Endkonzentration: 1 $\mu$M) gelöst in Dimethylsulfoxid vorgelegt. Danach wird die aktivierte p38 MAP-Kinase (Endkonzentration 1 nM) zugegeben und das Gemisch für 4 h bei Raumtemperatur inkubiert. Danach wird ein Gemisch aus Substrat (GST-tagged ATF2) und ATP zugegeben und alles zusammen für eine weitere Stunde inkubiert (Endkonzentration ATP: 100 $\mu$M; Endkonzentration ATF2: 10 nM).

Die Konzentration von phosphoryliertem ATF2 wird durch Chemolumineszenz induzierte Lichtemission quantifiziert. Dazu wird zu dem Reaktionsgemisch ein Glutathion-Donor-Bead (Endkonzentration: 5 $\mu$g/ml), welcher an das Gluthathion am ATF2 bindet, und phospho-ATF2 Antikörper hinzugegeben (Endkonzentration: 3 nM; dieser bindet die durch die Kinasereaktion angefügte Phosphatgruppe) an welchen ein Protein A Acceptor Bead (Endkonzentration: 5 $\mu$g/ml) bindet. Diese Komponenten sind in einem Puffer gelöst, welcher 20 mM HEPES pH 7.0, 200 mM NaCL, 80 mM

EDTA sowie 0.3% BSA enthält. Dieses Reaktionsgemisch wird für 1 Stunde bei Raumtemperatur im Dunkeln inkubiert. Kommen diese beiden Beads in räumliche Nähe wird sichtbares Licht emittiert, welches in einem Photometer bei 520-620nm gemessen wird.

Auswertung:

Zur Bestimmung der inhibitorischen Aktivität der erfindungsgemäßen Verbindungen wird berechnet um wieviel Prozent die Kinaseaktivität bei einer festen Inhibitorkonzentration von 1 μM inhibiert ist. Die maximale Aktivität wird dabei durch die nicht inhibierte Kinase bestimmt. Die minimale Aktivität bzw. unspezifische Hintergrundaktivität wird durch einen Reaktionsansatz ohne Kinase ermittelt. Die erfindungsgemäßen Verbindungen der allgemeinen Formel I zeigen beispielsweise Inhibitionswerte von >50%, vorzugsweise von >90%.

In Tabelle 2 ist das Maß der Inhibition des Enzyms p38 MAP-Kinase -so wie oben beschrieben bestimmt-vermittelt durch die erfindungsgemäßen Verbindungen, die im Abschnitt "Herstellung der Endverbindungen" dargestellt sind, zusammengefasst.

Bestimmung der Löslichkeit:

Gesättigte Lösungen werden in geeigneten Well-Platten durch Addition von 0,75 ml eines McIlvaine-Puffers (pH 7,4) in das jeweilige Well, welches 0,75 mg Feststoff einer Wirksubstanz enthält, hergestellt. Die Wells werden 2 Stunden geschüttelt und dann wird wird durch eine geeignete Filtermembran (typischerweise Polytetrafluorethylen mit 0,45 μm Porengröße) filtriert. Filterabsorption wird durch Verwerfen der ersten Tropfen des Filtrats verhindert. Die Menge der gelösten Wirksubstanz wird UV-spektroskopisch bestimmt.

Bestimmung der Cyp2C9 Aktivität:

Es wird die Inhibition der Cytochrom P450 2C9-Isoenzym katalysierten O-demethylierung von [O-Methyl-$^{14}$C]-naproxen durch die Testverbindung bei 37°C mit humanem rekombinantem Cytochrom P450 2C9 gemessen.

Der Assay wird auf einem Robotersystem in 96-Wellplatten durchgeführt. Das finale Inkubationsvolumen beträgt 200 μl TRIS-Puffer (0,1 M), Magnesiumchlorid (5 mM), rekombinantes Protein (40 pmol/ml), $^{14}$C-markiertes Naproxen (80 μM) und die Testverbindung in einer von 4 Konzentrationen (50 μM, 10 μM, 2 μM und 0,4 μM) jeweils als Duplikat. Nach einer kurzen Präinkubationsperiode werden die Reaktionen durch Zugabe des Kofaktors (NADPH, 1mM) gestartet und nach 15 min durch Zugabe von 50 μl einer 10 %-igen wässrigen Trichloressigsäurelösung beendet. Ein Aliquot des Inkubats wird auf eine 96-well Festphasenextraktionsplatte transferiert und extrahiert. Dabei wird das bei der Reaktion entstandene $^{14}$C-Formaldehyd nicht auf der Festphasenextraktionsplatte zurückbehalten und somit von dem unmetabolisiertem Substrat durch Waschen der Festphasenextraktionsplatte mit Wasser getrennt. Ein Aliquot des Eluats wird in eine für Flüssigszintillisation geeignete Wellplatte transferiert. Die Rate der Bildung von $^{14}$C-Formaldehyd in diesen Inkubationen wird mit einer Kontrolle, welche keine Testverbindung enthält, verglichen.

Der $IC_{50}$ wird nach der Methode der kleinsten Fehlerquadrate anhand folgender Formal bestimmt:

$$\text{\% Kontrollaktivität} = (100\ \text{\% Kontrollaktivität} / (1+(I / IC_{50})S))-B$$

mit

I = Inhibitorkonzentration
S = Steigung
B = Hintergrundaktivität

Wenn die Inhibition der Reaktion bereits bei der kleinsten Konzentration der Testverbindung >50% beträgt, wird der $IC_{50}$ mit <0,4 μM angegeben.

Tabelle 2. Hemmwirkung von erfindungsgemäßen Verbindungen der allgemeinen Formel I auf das Enzym p38 MAP-Kinase bei einer Inhibitorkonzentration von 1 μM

Tabelle 2:

| Beispiel | p38 (% Inh @ 1 μM) | CYP 2C9 IC50 (μM) | Löslichkelt bei pH 7,4 (mg/ml) | Beispiel | p38 (% inh @ 1μM) | CYP 2C9 IC50 (μM) | Lösllchkeit bei pH 7,4 (mg/ml) |
|---|---|---|---|---|---|---|---|
| 1(1) | 99,6 | 2,13 | 0,340 | 13 | 99,1 | 0,50 | 0,017 |
| 2 | 99,1 | 1,29 | 0,010 | 15 | 99,4 | 2,54 | 0,230 |
| 2(1) | 99,0 | 0,88 | 0,028 | 10(4) | 99,1 | 0,44 | 0,370 |
| 3 | 99,0 | 1,26 | 0,085 | 10(3) | 99,3 | 1,60 | 0,590 |
| 1(2) | 98,5 | 1,34 | 0,059 | 10(2) | 99,4 | 0,46 | 0,390 |
| 1(3) | 99,1 | 1,04 | 0,160 | 10(1) | 99,1 | 1,29 | 0,380 |
| 1(4) | 99,2 | 0,89 | 0,100 | 10 | 99,1 | 1,32 | 0,420 |
| 1(5) | 99,0 | 1,63 | 0,670 | 9(2) | 98,2 | 7,76 | 0,860 |
| 1(6) | 99,3 | 1,43 | 0,560 | 9(1) | 95,8 | 6,70 | 0,570 |
| 1 | 98,9 | 2,42 | 0,360 | 11(2) | 18,0 | 10,40 | > 1,000 |
| 4 | 99,4 | 0,44 | 0,078 | 11(1) | 13,2 | 2,65 | > 1,000 |
| 1(7) | 98,9 | 0,78 | 0,170 | 9 | 98,8 | 10,98 | > 1,000 |
| 1(8) | 98,9 | 1,02 | 0,084 | 9(7) | 98,8 | 44,69 | 0,880 |
| 1(9) | 98,9 | 1,12 | 0,440 | 9(6) | 97,7 | 10,42 | 0,470 |
| 5 | 98,6 | 1,15 | 0,620 | 9(5) | 97,5 | 6,88 | 0,490 |
| 1(10) | 99,6 | 0,41 | 0,043 | 9(4) | 98,0 | 8,11 | 0,690 |
| 1(11) | 99,2 | 0,41 | 0,045 | 9(3) | 98,9 | 6,84 | 0,700 |
| 1(12) | 98,9 | 1,55 | 0,140 | 1(26) | 19,8 | 2,39 | 0,049 |
| 1(14) | 99,1 | 0,63 | 0,120 | 1(25) | 35,4 | 7,91 | 0,048 |
| 1(13) | 98,7 | 0,56 | 0,040 | 11(3) | 99,0 | 0,87 | 0,680 |
| 1(19) | 98,5 | 3,59 | 0,098 | 11(3) | 98,7 | 0,56 | > 1,000 |
| 1(18) | 98,0 | 0,84 | 0,080 | 1(24) | 54,2 | 2,47 | 0,036 |
| 1(17) | 99,1 | 0,99 | 0,046 | 14(1) | 97,9 | 5,27 | 0,097 |
| 6(3) | 98,5 | 2,93 | 0,910 | 14 | 98,8 | 1,43 | 0,027 |
| 7 | 98,7 | 5,32 | 0,110 | 11(6) | 99,6 | 3,18 | 0,680 |
| 1(16) | 98,4 | 20,42 | 0,620 | 11(5) | 99,6 | 2,34 | 0,053 |
| 6(2) | 98,9 | 0,70 | > 1,000 | 8(2) | 99,5 | 5,77 | 0,120 |
| 6(1) | 99,2 | 0,47 | 0,047 | 1(27) | 79,6 | 16,40 | 0,550 |
| 6 | 98,6 | 3,43 | > 1,000 | 8(1) | 98,1 | 6,48 | 0,120 |
| 1 (15) | 98,9 | 1,21 | 0,073 | 8 | 97,3 | 1,67 | 0,280 |
| 11(4) | 99,5 | 0,46 | 0,043 | 1(23) | 97,0 | 1,77 | 0,280 |
| 11 | 92,0 | 5,90 | 0,450 | 1(22) | 99,1 | 1,54 | 0,042 |
| 11(3) | 98,6 | 0,78 | 0,160 | 1(21) | 98,9 | 4,02 | 0,045 |
| 11(7) | 99,3 | 0,41 | 0,110 | 1(20) | 99,1 | 2,59 | 0,124 |
| 13(1) | 99,3 | 0,67 | 0,012 | 5(1) | 94,9 | 2,04 | 0,170 |
| 12(4) | 96,4 | 0,65 | 0,059 | 5(2) | 98,0 | 0,99 | 0,160 |

(fortgesetzt)

| Beispiel | p38 (% Inh @ 1 μM) | CYP 2C9 IC50 (μM) | Löslichkelt bei pH 7,4 (mg/ml) | Beispiel | p38 (% inh @ 1μM) | CYP 2C9 IC50 (μM) | Lösllchkeit bei pH 7,4 (mg/ml) |
|---|---|---|---|---|---|---|---|
| 12(3) | 97,3 | 2,15 | 0,091 | Vc | 99,1 | <0,4 (BC) | < 0,001 |
| 12(2) | 98,2 | 3,46 | 0,630 | Va | 98,8 | <0,4 (BC) | < 0,001 |
| 12(1) | 87,7 | 0,96 | 0,044 | Vb | 99,1 | <0,4 (BC) | < 0,001 |
| 12 | 97,9 | 0,50 | 0,660 | | | | |

Indikationen

Im Hinblick auf die Fähigkeit die p38 MAP-Kinase-Aktivität zu hemmen, sind die erfindungsgemäßen Verbindungen der allgemeinen Formel I und ihre entsprechenden pharmazeutisch akzeptablen Salze prinzipiell geeignet, alle diejenigen Zustände oder Krankheiten zu behandeln und/oder vorbeugend zu behandeln, die durch eine Hemmung der p38 MAP-Kinase-Aktivität beeinflusst werden. Die erfindungsgemäßen Verbindungen eignen sich z.B. zur Verbesserung eines durch p38 MAP-Kinase vermittelten abnormen Zytokinspiegels, im Besonderen zur Regulierung einer Überproduktion der Zytokine IL-1, IL-4, IL-8 und TNF-α. Daher sind die erfindungsgemäßen Verbindungen zur Prophylaxe oder Behandlung von Krankheiten, insbesondere bei Atemwegserkrankungen, gastrointestinalen Erkrankungen oder Beschwerden, entzündlichen Krankheiten (insbesondere der Atemwege, der Gelenke, der Haut oder der Augen), Autoimmunerkrankungen, destruktiven Störungen der Knochen, Proliferationsstörungen, Störungen der Angiogenese, Neurodegenerativen Erkrankungen, Infektionskrankheiten und Viruserkrankungen sowie Erkrankungen des peripheren oder zentralen Nervensystems anwendbar.

Bevorzugt genannt sei die Vorbeugung und Behandlung von Atemwegs- oder Lungenerkrankungen, welche mit einer erhöhten Schleimproduktion, Entzündungen und/oder obstruktiven Erkrankungen der Atemwege einhergehen wie z.B. akute, allergische oder chronische Bronchitis, chronisch obstruktive Bronchitis (COPD), Husten, Lungenemphysem, allergische oder nicht-allergische Rhinitis oder Sinusitis, chronische Rhinitis oder Sinusitis, Asthma, Alveolitis, Farmers Krankheit, hyperreaktive Atemwege, infektiöse Bronchitis oder Pneumonitis, pediatrisches Asthma, Bronchiektasien, Lungenfibrose, ARDS (akutes Atemnotsyndrom des Erwachsenen), Bronchialödem, Lungenödem, Bronchitis, Pneumonie oder interstitielle Pneumonie ausgelöst durch Ursachen wie Aspiration, Inhalation von toxischen Gasen oder Bronchitis, Pneumonie oder interstitielle Pneumonie ausgelöst durch Herzinsuffizienz, Bestrahlung, Chemotherapie, Zystische Fibrose oder Mukoviszidose und alpha1-Antitrypsin-Mangel.

Des Weiteren sei die Behandlung von entzündlichen Erkrankungen des Gastrointestinaltraktes wie z.B. akute oder chronische entzündliche Veränderungen bei Gallenblasenentzündung, Morbus Crohn, Colitis ulcerosa, entzündliche Pseudopolypen, juvenile Polypen, Colitis cystica profunda, Pneumatosis cystoides intestinales, Erkrankungen der Gallengänge und Gallenblase, z.B. Gallensteine und Konglomerate, entzündliche Erkrankungen der Gelenke wie rheumatoide Arthritis oder entzündliche Erkrankungen der Haut (z.B. Psoriasis) und der Augen bevorzugt genannt.

Außerdem sei die Vorbeugung und Behandlung von Erkrankungen des peripheren oder zentralen Nervensystems wie z.B. Alzheimersche Erkrankung, Parkinsonsche Erkrankung, akute und chronische Multiple Sklerose, akute und chronische Schmerzzustände sowie Verletzungen des Gehirns hervorgerufen durch Schlaganfall, Hypoxie oder Schädel-Hirn-Trauma bevorzugt genannt.

Ganz besonders sind die erfindungsgemäßen Verbindungen, einschließlich deren physiologisch verträglichen Salze, zur Prophylaxe oder Behandlung von Atemwegserkrankungen, insbesondere COPD und Asthma geeignet.

Kombinationen

Auf Grund ihrer biologischen Eigenschaften können die erfindungsgemäßen Verbindungen der allgemeinen Formel I allein oder in Kombination mit anderen erfindungsgemäßen Wirkstoffen der Formel I zur Anwendung gelangen. Gegebenenfalls können die Verbindungen der Formel I auch in Kombination mit einem oder mehreren weiteren pharmakologisch aktiven Wirkstoffen eingesetzt werden. Für die Behandlung von Atemwegserkrankungen können die erfindungsgemäßen Verbindungen der allgemeinen Formel I allein oder in Kombination mit anderen Atemwegstherapeutika, wie z.B. sekretolytisch (z.B. Ambroxol, N-acetylcystein, EGFR-Hemmern), broncholytisch (z.B. Tiotropium oder Ipratropium oder Fenoterol, Salmeterol, Salbutamol) und/oder entzündungshemmend [z.B. Theophylline oder Glucocorticoide (wie z.B., Prednisolon, Prednison, Butixocortpropionat, Beclomethason, Budesonid, Fluticason, Mometason, Ciclesonid, Dexamethason, Betamethason), Leukotrienrezeptorinhibitoren oder Leukotrienbiosyntheseinhibitoren, Antihistaminika, PDE4 Inhibitoren (wie z.B. Enprofyllin, Theophyllin, Roflumilast, Ariflo (Cilomilast), Tofimilast, Pumafentrin,

Lirimilast, Arofyllin, Atizoram)] wirksamen Substanzen angewendet werden. Außerdem können diese Verbindungen mit nicht-steroidalen antünfiammatorischen Substanzen ("NSAID"; wie z.B. Ibuprofen, Celecoxib und Rofecoxib), Dopamin-Agonisten, Statinen, antiviralen Wirkstoffen wie Abacavir, PI3-Kinase Inhibitoren, MRP4-Inhibitoren, PAF-Antagonisten und antiproliferativen Agentien (z.B. Methotrexat, Leflunomid, FK506 (Tacrolimus, Prograf)) kombiniert werden. Die Kombinationen, die eine oder mehrere der oben genannten Verbindungen enthalten, können zusammen oder nacheinander, zur simultanen, sequentiellen oder separaten Verabreichung eingesetzt werden. Die Anwendung dieser Verbindungen entweder alleine oder in Kombination mit anderen Wirkstoffen kann intravenös, subkutan, intramuskulär, intraperitoneal, intranasal, durch Inhalation oder transdermal oder oral erfolgen, wobei zur Inhalation insbesondere Aerosolformulierungen geeignet sind.

Zur Behandlung von Erkrankungen im Bereich des Magen-Darm-Traktes können die erfindungsgemäßen Verbindungen der allgemeinen Formel I ebenfalls alleine oder in Kombination mit Motilitäts- oder Sekretions-beeinflussenden Substanzen gegeben werden. Diese Kombinationen können entweder simultan oder sequentiell verabreicht werden.

Desweiteren können die erfindungsgemäßen Verbindungen in der Tumortherapie in Kombination mit Anti-Tumor Therapeutika, beispielsweise in Kombination mit Topoisomerase-Inhibitoren (z.B. Etoposide), Mitoseinhibitoren (z.B. Vinblastin), mit Nukleinsäuren interagierenden Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Antikörpern etc. angewandt werden. Diese Kombinationen können entweder simultan oder sequentiell verabreicht werden.

## Darreichungsformen

Die zur Erzielung einer entsprechenden Wirkung bei der Behandlung oder Prophylaxe erforderliche Dosierung hängt üblicherweise von der zu verabreichenden Verbindung, vom Patienten, von der Art und Schwere der Krankheit oder des Zustandes und der Art und Häufigkeit der Verabreichung ab und liegt im Ermessen des zu behandelnden Arztes. Zweckmäßigerweise kann die Dosierung bei inhalativer Gabe im Bereich von 0.01 bis 100 mg/kg, vorzugsweise 0.01 bis 10 mg/kg, und bei oraler Gabe im Bereich von 0.01 bis 100 mg/kg, vorzugsweise 0.01 bis 10 mg/kg, jeweils 1 bis 4 x täglich, liegen. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen, Konservierungs- und/oder Verdünnungsmitteln, z.B. mit Glucose, Arabinose, Lactose, Saccharose, Maltose, Dextrane, Maisstärke, Milchzucker, Rohrzucker, mikrokristalliner Zellulose, Sorbit, Mannit, Xylit, Magnesiumstearat, Polyvinylpyrrolidon, Zitronensäure, Weinsäure, Äpfelsäure, Ascorbinsäure, Maleinsäure, Bernsteinsäure, Fumarsäure, Essigsäure, Natriumchlorid, Calciumcarbonat, Wasser, Wasser/Ethanol, Wasser/Glycerin, Wasser/Sorbit, Wasser/Polyethylenglykol, Propylenglykol, Cetylstearylalkohol, Carboxymethylcellulose, fetthaltigen Substanzen wie Hartfett oder deren geeigneten Gemischen, Cetylpyridiniumchlorid, Benzalkoniumchlorid, Benzoesäure, Natriumbenzoat, oberflächenaktive Stoffe wie Sojalecithin, Ölsäure, Polysorbate oder Polyvinylpyrrolidon in üblichen galenischen Zubereitungen wie Tabletten, Dragées, Kapseln, Pulver, Lösungen, Suspensionen, Zäpfchen, Emulsionen, Inhalationspulver oder -aerosole einarbeiten. Zur Herstellung von treibgashaltigen Inhalationsaerosolen werden Treibgase bzw. Gemische von Treibgasen wie z.B. n-Propan, n-Butan, Isobutan, Halogenkohlenwasserstoffe wie fluorierte Derivate des Methans, Ethans [z.B. 1,1,1,2-Tetrafluorethan (TG134a)], Propans [z.B. 1,1,1,2,3,3,3-Heptafluorpropan (TG227)], Butans, Cyclopropans oder Cyclobutans verwendet.

Die Dosis für die zuvor angeführten Kombinationspartner beträgt hierbei zweckmäßigerweise 1/5 der üblicherweise empfohlenen niedrigsten Dosierung bis zu 1/1 der normalerweise empfohlenen Dosierung.

Daher betrifft ein weiterer Gegenstand dieser Erfindung die Verwendung einer erfindungsgemäßen Verbindung oder eines physiologisch verträglichen Salzes solch einer Verbindung in Kombination mit mindestens einem der zuvor als Kombinationspartner beschriebenen Wirkstoffe zur Herstellung eines Arzneimittels, das zur Behandlung oder Prophylaxe von Erkrankungen oder Zuständen geeignet ist, die durch Inhibierung des Enzyms p38 MAP-Kinase beeinflussbar sind. Hierbei handelt es sich vorzugsweise um eine Atemwegserkrankung, insbesondere eine der zuvor angeführten Erkrankungen oder Zustände, ganz besonders COPD oder Asthma.

Die Verwendung der erfindungsgemäßen Verbindung, oder eines physiologisch verträglichen Salzes hiervon, in Kombination mit einem weiteren Wirkstoff kann zeitgleich oder zeitlich versetzt, insbesondere aber zeitnah erfolgen. Bei einer zeitgleichen Verwendung werden beide Wirkstoffe dem Patienten zusammen verabreicht; bei einer zeitlich versetzten Verwendung werden beide Wirkstoffe dem Patienten in einem Zeitraum von kleiner gleich 12, insbesondere kleiner gleich 6 Stunden nacheinander verabreicht.

Folglich betrifft ein weiterer Gegenstand dieser Erfindung ein Arzneimittel, das eine erfindungsgemäße Verbindung oder ein physiologisch verträgliches Salz solch einer Verbindung sowie mindestens einen der zuvor als Kombinationspartner beschriebenen Wirkstoffe neben gegebenenfalls einem oder mehreren inerten Trägerstoffen, Konservierungs- und/oder Verdünnungsmitteln aufweist.

Die erfindungsgemäße Verbindung, oder eines physiologisch verträglichen Salzes, und der damit zu kombinierende weitere Wirkstoff können zusammen in einer Darreichungsform, beispielsweise einer Tablette, Kapsel, eines Inhalationspulvers oder -aerosols, oder getrennt in zwei gleichen oder verschiedenen Darreichungsformen, beispielsweise als sogenanntes kit-of-parts, vorliegen.

## Herstellungsverfahren

Die erfindungsgemäßen Verbindungen sind unter Anwendung im Prinzip bekannter Syntheseverfahren erhältlich. Bevorzugt werden die Verbindungen nach den nachfolgend näher erläuterten erfindungsgemäßen Herstellungsverfahren erhalten.

Die Herstellung von Verbindungen der allgemeinen Formel I kann gemäß dem in Schema 1, in dem Ar, m, T, $R^2$, p, und $R^1$ wie zuvor definiert sind, A Stickstoff bedeutet, $R^{13}$ Br oder I darstellt und Wasserstoff, Methyl oder Ethyl bedeutet, gezeigten erfindungsgemäßen Verfahren a) ausgehend von Verbindungen der allgemeinen Formel V erfolgen.

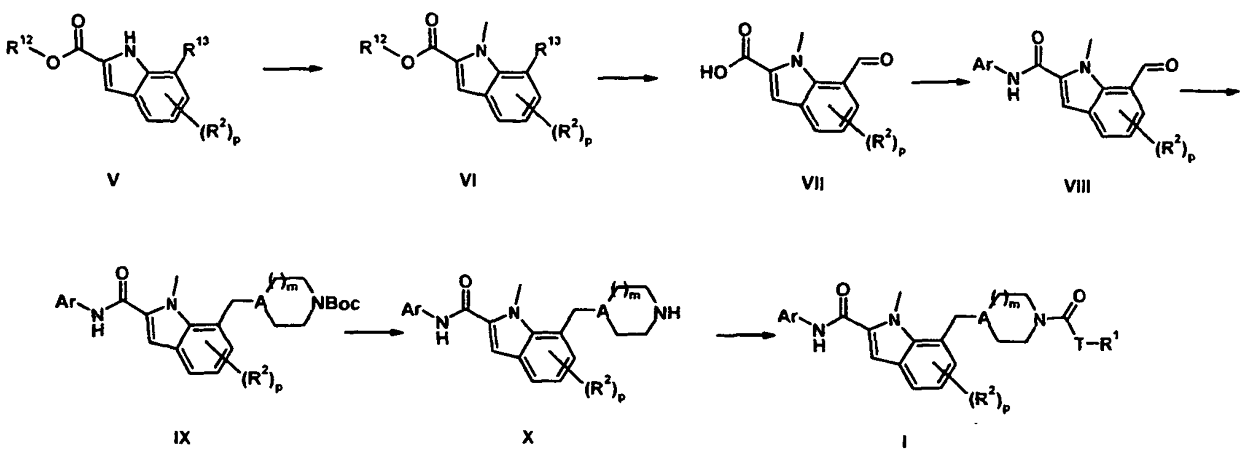

V VI VII VIII

IX X I

Schema 1 (Verfahren a))

Die Verbindung der allgemeinen Formel V in der $R^{13}$ Br darstellt und $R^{12}$ Ethyl bedeutet ist in J. Org. Chem. 2007, 72, 2978-2987 beschrieben. Verbindungen der allgemeinen Formel V können in Verbindungen der allgemeinen Formel VI überführt werden, indem mit einem Methylierungsmittel wie beispielsweise Iodmethan, Brommethan, Dimethylsulfat oder Dimethylcarbonat umgesetzt wird. Die Reaktion wird in Anwesenheit einer Base wie Natriumhydrid, Kaliumhydrid, Kalium-tert.-butanolat, Natrium-tert.-butanolat, 1,4-Diazabicyclo[2.2.2]octan, 1,8-Diazabicyclo[5.4.0]undec-7-en, Kaliumcarbonat oder Caesiumcarbonat in einem Lösungsmittel wie Tetrahydrofuran, 1,4-Dioxan, Acetonitril, N,N-Dimethylformamid oder N-Methyl-pyrrolidon bei Temperaturen zwischen -30°C und 150°C, bevorzugt jedoch zwischen 0°C und 100°C durchgeführt. Werden bei dieser Reaktion Verbindungen der allgemeinen Formel V, in der $R^{12}$ Wasserstoff bedeutet, eingesetzt, so entstehen hierbei Verbindungen der allgemeinen Formel VI, in der $R^{12}$ Methyl bedeutet. Verbindungen der allgemeinen Formel VI, in der $R^{12}$ Methyl oder Ethyl bedeutet, werden in Verbindungen der allgemeinen Formel VI, in der $R^{12}$ Wasserstoff bedeutet, überführt. Diese Umsetzung erfolgt hydrolytisch in einem wässrigen Lösungsmittel, z.B. in Wasser, Methanol/Wasser, Isopropanol/Wasser, Essigsäure/Wasser, Tetrahydrofuran/Wasser oder 1,4-Dioxan/Wasser, vorzugsweise jedoch in Methanol/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid, vorzugsweise jedoch Natriumhydroxid, oder aprotisch, z.B. in Gegenwart von Iodtrimethylsilan, bei Temperaturen zwischen 0 und 120°C, vorzugsweise bei Temperaturen zwischen 10 und 100°C. Verbindungen der allgemeinen Formel VI, in denen $R^{12}$ Wasserstoff bedeutet, werden dann in Verbindungen der allgemeinen Formel VII überführt. In einem Halogen-Metall-Austausch durch Umsetzung mit n-Butyllithium, sec.-Butyllithium, tert.-Butyllithium oder Isopropylmagnesiumchlorid-Lithiumchlorid-Komplex in einem inerten Lösungsmittel wie beispielsweise Diethylether, Tetrahydrofuran oder 1,4-Dioxan bei Temperaturen zwischen - 90°C und 0°C bevorzugt jedoch zwischen -78°C und -20°C gegebenenfalls nach einer vorherigen Zugabe einer Base wie Natriumhydrid oder Kaliumhydrid bei Temperaturen zwischen -50°C und 50°C wird zunächst eine Organometallspezies erzeugt. Diese wird dann mit einem Elektrophil wie N,N-Dimethylformamid, N-Formylpiperidin oder N-Formylpyrrolidin bei Temperaturen zwischen -78°C und -20°C zu Verbindungen der allgemeinen Formel VII umgesetzt.

Carbonsäuren der allgemeinen Formel VII werden dann mit einem Anilin zum Anilid der allgemeinen Formel VIII umgesetzt. Dazu wird die Carbonsäure in situ durch Zugabe von N,N'-Diisopropylcarbodiimid, N,N'-Dicyclohexylcarbodiimid, N-(3-Dimethylaminopropyl)-N'-ethyl-carbodiimid, O-(Benzotriazol-1-yl)-N,N,N',N'-tetramethyluronium-tetrafluoroborat (TBTU), O-(Benzotriazol-1-yl)-N,N,N',N'-tetramethyluronium-hexafluorophosphat (HBTU), O-(7-AzaBenzotriazol-1-yl)-N,N,N',N'-tetramethyluronium-hexafluorophosphat (HATU), (Benzotriazol-1-yloxy)-tris-(dimethylamino)-phosphonium-hexafluorophosphat (BOP), (Benzotriazol-1-yloxy)-tripyrrolidinophosphonium-hexafluorophosphat (PyBOP) oder n-Propylphosphonsäureanhydrid aktiviert und in einem dipolar aprotischen Lösungsmittel wie beispielsweise N,N-Dimethylformamid, Dimethylacetamid, Tetrahydrofuran, Acetonitril, N-Methylpyrrolidon oder Dimethylsulfoxid, vorzugsweise jedoch in N,N-Dimethylformamid oder N-Methylpyrrolidon mit einem Anilin in Anwesenheit einer Base wie Triethylamin, N,N-Diisopropyl-N-ethyl-amin und gegebenenfalls eines Katalysators wie 4-N,N-Dimethylaminopyridin bei Temperaturen zwischen -20°C und 80°C, bevorzugt jedoch zwischen 0°C und 50°C umgesetzt.

Alternativ wird die Carbonsäure zunächst in ein Säurechlorid überführt. Dazu wird die Carbonsäure mit Thionylchlorid, Phosphoroxychlorid oder Oxalylchlorid gegebenenfalls in einem Lösungsmittel wie Toluol, Benzol oder Dichlormethan bei Temperaturen zwischen 0°C und 120°C umgesetzt. Das so erhaltene Säurechlorid wird mit dem Anilin in einem Lösungsmittel wie Dichlormethan, Tetrahydrofuran, 1,4-Dioxan oder N,N-Dimethylformamid bei Temperaturen zwischen -30°C und 150°C, bevorzugt jedoch zwischen 0°C und 80°C und gegebenenfalls in Anwesenheit einer Base wie Triethylamin, N,N-Diisopropyl-N-ethyl-amin und gegebenenfalls in Anwesenheit eines Katalysators wie 4-N,N-Dimethylaminopyridin umgesetzt.

Desweiteren kann die Carbonsäure in ein Säureimidazolid überführt werden. Dazu wird sie mit Carbonyldiimidazol in einem Lösungsmittel wie Dichlormethan, Tetrahydrofuran oder 1,4-Dioxan bei Temperaturen zwischen 20°C und 100°C zur Reaktion gebracht. Das so erhaltene Säureimidazolid wird mit dem Anilin in einem Lösungsmittel wie Dichlormethan, Tetrahydrofuran, 1,4-Dioxan oder N,N-Dimethylformamid, bevorzugt jedoch Tetrahydrofuran, bei Temperaturen zwischen -30°C und 150°C, bevorzugt jedoch zwischen 0°C und 80°C, und gegebenenfalls in Anwesenheit einer Base wie Triethylamin, N,N-Diisopropyl-N-ethylamin und gegebenenfalls in Anwesenheit eines Katalysators wie 4-N,N-Dimethylaminopyridin umgesetzt.

Aldehyde der allgemeinen Formel VIII werden mit N-Boc-Piperazin in Anwesenheit eines Reduktionsmittels wie Natriumtriacetoxyborhydrid oder Natriumcyanoborhydrid sowie einer Säure wie beispielsweise Essigsäure oder Trifluoressigsäure in einem Lösungsmittel wie Dichlormethan, 1,2-Dichlorethan, Methanol oder Ethanol bei Temperaturen zwischen 0°C und 60°C zu Verbindungen der allgemeinen Formel IX umgesetzt.

Die tert.-Butyloxycarbonylgruppe in Verbindungen der allgemeinen Formel IX wird durch Behandlung mit einer Säure wie Trifluoressigsäure oder Salzsäure oder durch Behandlung mit Iodtrimethylsilan gegebenenfalls unter Verwendung eines Lösungsmittels wie Methylenchlorid, 1,4-Dioxan, Methanol oder Diethylether abgespalten.

Die dabei erhaltenen Amine der allgemeinen Formel X werden dann zu Verbindungen der allgemeinen Formel I acyliert. Dabei können Acylreste eingeführt werden durch Umsetzung einer Verbindung der allgemeinen Formel X mit einem Acylierungsreagens wie beispielsweise einem Säurechlorid oder Säureanhydrid. Die Umsetzung kann in Anwesenheit einer Base wie Natriumhydroxid, Natriumhydrid, Natriumcarbonat, Kaliumcarbonat, Cäsiumcarbonat, Triethylamin oder N,N-Diisopropyl-N-ethyl-amin sowie in einem Lösungsmittel wie Dichlormethan, Tetrahydrofuran, 1,4-Dioxan oder N,N-Dimethylformamid bei Temperaturen zwischen -30°C und 120°C, bevorzugt jedoch zwischen 0°C und 80°C erfolgen.

Alternativ kann die Umsetzung durch Acylierung mit einer Säure erfolgen. Dazu wird die Säure in situ durch Zugabe von N,N'-Diisopropylcarbodiimid, N,N'-Dicyclohexylcarbodiimid, N-(3-Dimethylaminopropyl)-N'-ethyl-carbodümid, O-(Benzotriazol-1-yl)-N,N,N',N'-tetramethyluronium-tetrafluoroborat (TBTU), O-(Benzotriazol-1-yl)-N,N,N',N'-tetramethyluronium-hexafluorophosphat (HBTU), O-(7-AzaBenzotriazol-1-yl)-N,N,N',N'-tetramethyluronium-hexafluorophosphat (HATU), (Benzoiriazol-1-yloxy)-tris-(dimethylamino)-phosphonium-hexafluorophosphat (BOP), (Benzotriazol-1-yloxy)-tripyrrolidinophosphonium-hexafluorophosphat (PyBOP) oder n-Propylphosphonsäureanhydrid aktiviert und in einem dipolar aprotischen Lösungsmittel wie beispielsweise N,N-Dimethylformamid, Dimethylacetamid, Tetrahydrofuran, Acetonitril, N-Methylpyrrolidon oder Dimethylsulfoxid, vorzugsweise jedoch in N,N-Dimethylformamid oder N-Methylpyrrolidon mit einer Verbindung der allgemeinen Formel X in Anwesenheit einer Base wie Triethylamin, N,N-Diisopropyl-N-ethyl-amin und gegebenenfalls eines Katalysators wie 4-N,N-Dimethylaminopyridin bei Temperaturen zwischen -20°C und 80°C, bevorzugt jedoch zwischen 0°C und 50°C umgesetzt.

Aminocarbonylreste können eingeführt werden durch Umsetzung mit einem Isocyanat, gegebenenfalls in Anwesenheit einer Base wie beispielsweise Natriumcarbonat, Kaliumcarbonat, Cäsiumcarbonat, Triethylamin oder N,N-Diisopropyl-N-ethyl-amin in einem Lösungsmittel wie Dichlormethan, Tetrahydrofuran, 1,4-Dioxan oder N,N-Dimethylformamid bei Temperaturen zwischen -30°C und 150°C, bevorzugt jedoch zwischen 0°C und 100°C. Alternativ werden Aminocarbonylreste durch Umsetzung einer Verbindung der allgemeinen Formal X mit Phosgen, Diphosgen oder Triphosgen in einem Lösungsmittel wie Dichlormethan, Tetrahydrofuran oder 1,4-Dioxan in Anwesenheit einer Base wie beispielsweise Natriumcarbonat, Kaliumcarbonat, Cäsiumcarbonat, Triethylamin oder N,N-Diisopropyl-N-ethyl-amin, bevorzugt jedoch N,N-Diisopropyl-N-ethyl-amin und nachfolgende Behandlung mit einem Amin bei Temperaturen zwischen -20°C und 100°C, bevorzugt jedoch zwischen 0°C und 50°C erhalten.

Die Herstellung von Verbindungen der allgemeinen Formel VII, in denen $R^2$ Wasserstoff bedeutet, kann gemäß

dem in Schema 2 gezeigten erfindungsgemäßen Verfahren b) ausgehend von 1H-Indol-7-carbaldehyd XI erfolgen.

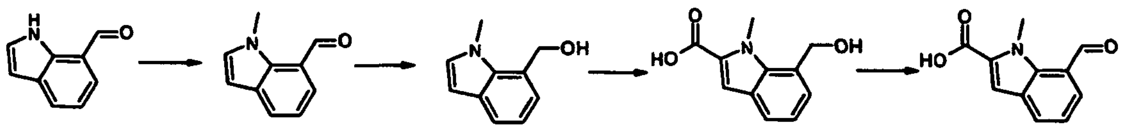

XI XII XIII XIV VII

Schema 2 (Verfahren b))

Dabei wird 1H-Indol-7-carbaldehyd XI am Indolstickstoff methyliert, wobei Verbindung XII erhalten wird. Diese Reaktion wird mit einem Methylierungsmittel wie beispielsweise Iodmethan, Brommethan, Dimethylsulfat oder Dimethylcarbonat in Anwesenheit einer Base wie Natriumhydrid, Kaliumhydrid, Kalium-tert.-butanolat, Natrium-tert.-butanolat, 1,4-Diazabicyclo[2.2.2]octan, 1,8-Diazabicyclo[5.4.0]undec-7-en, Kaliumcarbonat oder Caesiumcarbonat in einem Lösungsmittel wie Tetrahydrofuran, 1,4-Dioxan, Acetonitril, N,N-Dimethylformamid oder N-Methyl-pyrrolidon bei Temperaturen zwischen -30°C und 150°C bevorzugt jedoch zwischen 0°C und 100°C durchgeführt.

Anschließend wird die Aldehydgruppe zum Alkohol reduziert. Hierzu wird Verbindung XII mit einem Reduktionsmittel wie beispielsweise Natriumborhydrid, Lithiumborhydrid, Lithiumaluminiumhydrid oder Diisobutylaluminiumhydrid in einem Lösungsmittel wie Methanol, Ethanol, Diethylether, Tetrahydrofuran, 1,4-Dioxan oder Dichlormethan bei Temperaturen zwischen -78°C und 80°C, bevorzugt jedoch zwischen -40°C und 50°C, umgesetzt, wobei Verbindung XIII erhalten wird.

Zur Einführung der Carbonsäuregruppe wird Verbindung XIII mit einem Überschuss einer starken Base wie beispielsweise n-Butyllithium, sec.-Butyllithium, tert.-Butyllithium oder 2,2,6,6-Tetramethylpiperidin-1-yl-magnesiumchlorid-Lithiumchlorid-Komplex in einem inerten Lösungsmittel wie beispielsweise Diethylether, Tetrahydrofuran, 1,4-Dioxan oder 1,2-Dimethoxyethan bei Temperaturen zwischen -50°C und 80°C bevorzugt jedoch zwischen - 20°C und 40°C in 2-Position metalliert und anschließend mit Kohlendioxid zur Carbonsäure XIV umgesetzt.

Die Alkoholgruppe in XIV wird zum Aldehyd VII oxidiert. Diese Transformation kann mit Dess-Martin-Periodinan (J. Chem. Soc. 1983, 48, 4156), durch Swern-Oxidation (J. Org. Chem. 1976, 41, 957), durch Ley-Oxidation (Synthesis 1994, 639) oder durch eine TEMPO-katalysierte Oxidation (Tetrahedron Lett. 1992, 5029) durchgeführt werden.

Die so hergestellte Verbindung VII kann dann gemäß dem in Schema 1 gezeigten Verfahren a) in die Endverbindungen der allgemeinen Formel I, in denen $R^2$ Wasserstoff und A Stickstoff bedeuten, umgesetzt werden.

Die Herstellung von Verbindungen der allgemeinen Formel VIII kann außerdem gemäß dem in Schema 3, in dem Ar, $R^2$ und p wie zuvor definiert sind und $R^{14}$ $-OS(O)_2CF_3$, Br oder I bedeutet, gezeigten erfindungsgemäßen Verfahren c) ausgehend von Verbindungen der allgemeinen Formel XV erfolgen.

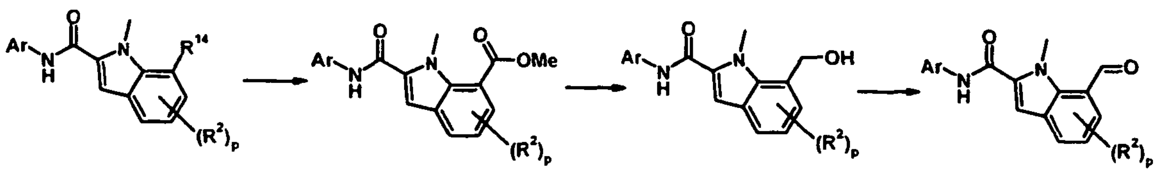

XV XVI XVII VIII

Schema 3 (Verfahren c))

Dabei werden die Verbindungen der allgemeinen Fromel XV durch Umsetzung mit Kohlenmonoxid und Methanol in Anwesenheit von katalytischen Mengen Palladium-IIacetats, katalytischen Mengen eines Liganden wie beispielsweise 1,3-Bis-(diphenylphosphino)-propan oder 1,1'-Bis-(diphenylphosphino)-ferrocen sowie einer Base wie Triethylamin oder N,N-Diisopropyl-N-ethyl-amin in die Methylester XVI überführt.

Diese werden durch Umsetzung mit einem Reduktionsmittel wie Lithiumaluminiumhydrid, Diisobutylaluminiumhydrid oder Lithiumborhydrid bevorzugt jedoch Lithiumaluminiumhydrid in einem inerten Lösungsmittel wie Diethylether, Tetrahydrofuran oder 1,4-Dioxan bei Temperaturen zwischen -30°C und 80°C zu den Alkoholen der allgemeinen Formel XVII überführt.

Oxidation der Verbindungen der allgemeinen Formel XVII zu den Aldehyden der allgemeinen Formel VIII kann dann mit Dess-Martin-Periodinan (J. Chem. Soc. 1983, 48, 4156), durch Swem-Oxidation (J. Org. Chem. 1976, 41, 957), durch Ley-Oxidation (Synthesis 1994, 639) oder durch eine TEMPO-katalysierte Oxidation (Tetrahedron Lett. 1992, 5029) durchgeführt werden.

Die so hergestellten Verbindungen der allgemeinen Formel VIII können dann gemäß dem in Schema 1 gezeigten Verfahren a) in die Endverbindungen der allgemeinen Formel I umgesetzt werden.

Die Herstellung von Verbindungen der allgemeinen Formel XV kann gemäß dem in Schema 4, in dem Ar, $R^2$ und p wie zuvor definiert sind, $R^{14}$ $-OS(O)_2CF_3$ und $R^{15}$ Wasserstoff oder Methyl bedeuten, gezeigten erfindungsgemäßen Verfahren d) ausgehend von Verbindungen der allgemeinen Formel XVIII erfolgen.

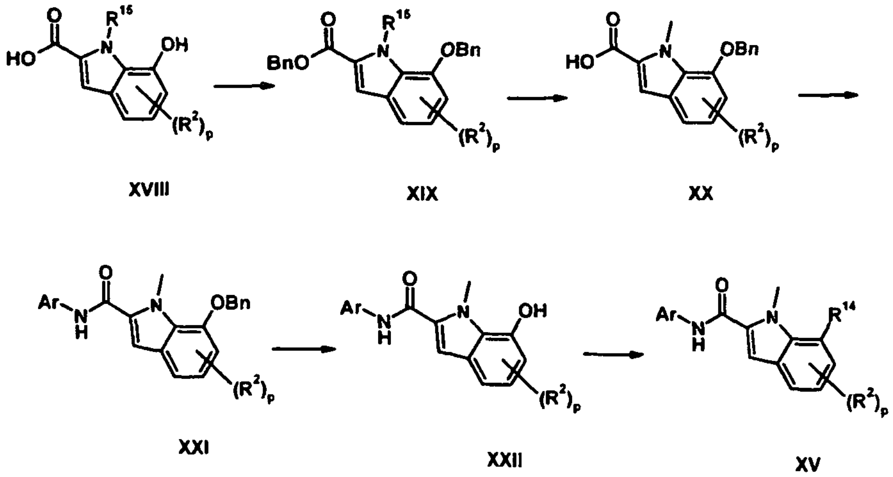

XVIII XIX XX

XXI XXII XV

Schema 4 (Verfahren d))

Dazu werden Verbindungen der allgemeinen Formel XVIII durch Umsetzung mit Benzylchlorid oder Benzylbromid in Anwesenheit einer eine Base wie beispielsweise Natriumhydrid, Kaliumhydrid, Kalium-tert.-butanolat, Natrium-tert.-butanolat, 1,4-Diazabicyclo[2.2.2]octan, 1,8-Diazabicyclo[5.4.0]undec-7-en, Triethylamin, N,N-Diisopropyl-N-ethylamin, Kaliumcarbonat oder Caesiumcarbonat in einem Lösungsmittel wie Tetrahydrofuran, 1,4-Dioxan, Acetonitril, N,N-Dimethylformamid, N-Methyl-pyrrolidon oder Dimethylsulfoxid bei Temperaturen zwischen -50°C und 120°C bevorzugt jedoch zwischen - 20°C und 80°C in Verbindungen der allgemeinen Formel XIX überführt.

Werden hierbei Verbindungen der allgemeinen Formel XVIII, in der $R^{15}$ Wasserstoff bedeutet, eingesetzt, so werden Verbindungen der allgemeinen Formel XIX, in der $R^{15}$ Wasserstoff bedeutet, erhalten. Diese können in Verbindungen der allgemeinen Formel XIX, in der $R^{15}$ Methyl bedeutet, überführt werden. Dazu wird mit einem Methylierungsmittel wie beispielsweise Iodmethan, Brommethan, Dimethylsulfat oder Dimethylcarbonat umgesetzt. Die Reaktion wird in Anwesenheit einer Base wie Natriumhydrid, Kaliumhydrid, Kalium-tert.-butanolat, Natrium-tert.-butanolat, 1,4-Diazabicyclo[2.2.2]octan, 1,8-Diazabicyclo[5.4.0]undec-7-en, Kaliumcarbonat oder Caesiumcarbonat in einem Lösungsmittel wie Tetrahydrofuran, 1,4-Dioxan, Acetonitril, N,N-Dimethylformamid oder N-Methyl-pyrrolidon bei Temperaturen zwischen -30°C und 150°C, bevorzugt jedoch zwischen 0°C und 100°C, durchgeführt.

Anschließend wird die Benzylestergruppe in den Verbindungen der allgemeinen Formel XIX gespalten. Diese Umsetzung erfolgt hydrolytisch in einem wässrigen Lösungsmittel, z.B. in Wasser, Methanol/Wasser, Isopropanol/Wasser, Essigsäure/Wasser, Tetrahydrofuran/Wasser oder 1,4-Dioxan/Wasser, vorzugsweise jedoch in Methanol/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid, vorzugsweise jedoch Natriumhydroxid, oder aprotisch, z.B. in Gegenwart von Iodtrimethylsilan, bei Temperaturen zwischen 0 und 120°C, vorzugsweise bei Temperaturen zwischen 10 und 100°C.

Die so erhaltenen Carbonsäuren der allgemeinen Formel XX werden dann mit einem Anilin zum Anilid der allgemeinen Formel XXI umgesetzt. Dazu wird die Carbonsäure in situ durch Zugabe von N,N'-Diisopropylcarbodiimid, N,N'-Dicyclohexylcarbodiimid, N-(3-Dimethylaminopropyl)-N'-ethyl-carbodiimid, O-(Benzotriazol-1-yl)-N,N,N',N'-tetramethyluronium-tetrafluoroborat (TBTU), O-(Benzotriazol-1-yl)-N,N,N',N'-tetramethyluronium-hexafluorophosphat (HBTU), O-(7-AzaBenzotriazol-1-yl)-N,N,N',N'-tetramethyluronium-hexafluorophosphat (HATU), (Benzotriazol-1-yloxy)-tris-(dimethylamino)-phosphonium-hexafluorophosphat (BOP), (Benzotriazol-1-yloxy)-tripyrrolidinophosphonium-hexafluorophosphat (PyBOP) oder n-Propylphosphonsäureanhydrid aktiviert und in einem dipolar aprotischen Lösungsmittel wie beispielsweise N,N-Dimethylformamid, Dimethylacetamid, Tetrahydrofuran, Acetonitril, N-Methylpyrrolidon oder Dimethylsulfoxid, vorzugsweise jedoch in N,N-Dimethyfformamid oder N-Methylpyrrolidon mit einem Anilin in Anwesenheit einer Base wie Triethylamin, N,N-Diisopropyl-N-ethyl-amin und gegebenenfalls eines Katalysators wie 4-N,N-Dimethylaminopyridin bei Temperaturen zwischen -20°C und 80°C, bevorzugt jedoch zwischen 0°C und 50°C, umgesetzt.

Alternativ wird die Carbonsäure zunächst in ein Säurechlorid überführt. Dazu wird die Carbonsäure mit Thionylchlorid, Phosphoroxychlorid oder Oxalylchlorid gegebenenfalls in einem Lösungsmittel wie Toluol, Benzol oder Dichlormethan bei Temperaturen zwischen 0°C und 120°C umgesetzt. Das so erhaltene Säurechlorid wird mit dem Anilin in einem Lösungsmittel wie Dichlormethan, Tetrahydrofuran, 1,4-Dioxan oder N,N-Dimethylformamid bei Temperaturen zwischen -30°C und 150°C, bevorzugt jedoch zwischen 0°C und 80°C und gegebenenfalls in Anwesenheit einer Base wie Triethylamin, N,N-Diisopropyl-N-ethyl-amin und gegebenenfalls in Anwesenheit eines Katalysators wie 4-N,N-Dimethylaminopyridin umgesetzt.

Desweiteren kann die Carbonsäure in ein Säureimidazolid überführt werden. Dazu wird sie mit Carbonyldiimidazol in einem Lösungsmittel wie Dichlormethan, Tetrahydrofuran oder 1,4-Dioxan bei Temperaturen zwischen 20°C und 100°C zur Reaktion gebracht. Das so erhaltene Säureimidazolid wird mit dem Anilin in einem Lösungsmittel wie Dichlormethan, Tetrahydrofuran, 1,4-Dioxan oder N,N-Dimethylformamid, bevorzugt jedoch Tetrahydrofuran, bei Temperaturen zwischen -30°C und 150°C, bevorzugt jedoch zwischen 0°C und 80°C, und gegebenenfalls in Anwesenheit einer Base wie Triethylamin, N,N-Diisopropyl-N-ethylamin und gegebenenfalls in Anwesenheit eines Katalysators wie 4-N,N-Dimethylaminopyridin umgesetzt.

Die Abspaltung des Benzylrestes in den Verbindungen der allgemeinen Formel XXI erfolgt vorteilhaft hydrogenolytisch, z.B. mit Wasserstoff in Gegenwart eines Katalysators wie Palladium auf Kohle oder Palladiumhydroxid auf Kohle in einem geeigneten Lösungsmittel wie Methanol, Ethanol, Tetrahydrofuran, 1,4-Dioxan, Essigsäureethylester oder Eisessig, gegebenenfalls unter Zusatz einer Säure wie Salzsäure bei Temperaturen zwischen 0 und 100°C, vorzugsweise jedoch bei Temperaturen zwischen 20 und 60°C, und bei einem Wasserstoffdruck von 1 bis 7 bar, vorzugsweise jedoch von 1 bis 3 bar, wobei Phenole der allgemeinen Formel XXII erhalten werden. Das Phenol der Formel XXII, in dem Ar 5-tert.-Butyl-3-methansulfonyl-2-methoxy-phenyl darstellt und $R^2$ Wasserstoff bedeutet, ist in US20040186114 beschrieben.

Danach werden die so erhaltenen Phenole der allgemeinen Formel XXII durch Umsetzung mit N-Phenyltrifluormethansulfonimid oder Trifluormethansulfonsäureanhydrid in Anwesenheit einer Base wie Natriumhydrid, Kaliumhydrid, Kalium-tert.-butanolat, Natrium-tert.-butanolat, Lithium-bis-(trimethylsilyl)-amid, Natrium-bis-(trimethylsilyl)-amid, Kalium-bis-(trimethylsilyl)-amid, 1,4-Diazabicyclo[2.2.2]octan, 1,8-Diazabicyclo[5.4.0]undec-7-en, Triethylamin, N,N-Diisopropyl-N-ethyl-amin, Pyridin, 2,6-Lutidin, 4-N,N-Dimethylaminopyridin, Kaliumcarbonat oder Caesiumcarbonat, vorzugsweise jedoch Triethylamin oder N,N-Diisopropyl-N-ethyl-amin, in einem Lösungsmittel wie Tetrahydrofuran, 1,4-Dioxan, Acetonitril, N,N-Dimethylformamid, N-Methyl-pyrrolidon oder Dimethylsulfoxid bei Temperaturen zwischen -50°C und 120°C, bevorzugt jedoch zwischen -20°C und 80°C, in Triflate der allgemeinen Formel XV überführt.

Die so hergestellten Verbindungen der allgemeinen Formel XV können dann gemäß dem in Schema 3 gezeigten Verfahren c) in die Verbindungen der allgemeinen Formel VIII umgesetzt werden, welche dann gemäß dem in Schema 1 gezeigten Verfahren a) in die Endverbindungen der allgemeinen Formel I überführt werden können.

Die Herstellung von Verbindungen der allgemeinen Formel XV kann gemäß dem in Schema 5, in dem Ar, $R^2$ und p wie zuvor definiert sind, $R^{14}$ Br oder I und $R^{15}$ Wasserstoff oder Methyl bedeuten, gezeigten erfindungsgemäßen Verfahren e) ausgehend von Verbindungen der allgemeinen Formel XXIII erfolgen.

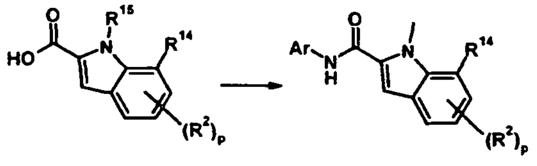

XXIII XV

Schema 5 (Verfahren e))

Verbindungen der allgemeinen Formel XXIII, in der $R^{15}$ Wasserstoff bedeutet, können in Verbindungen der allgemeinen Formel XXIII, in der $R^{15}$ Methyl bedeutet, überführt werden. Dazu wird mit einem Methylierungsmittel wie beispielsweise Iodmethan, Brommethan, Dimethylsulfat oder Dimethylcarbonat umgesetzt. Die Reaktion wird in Anwesenheit einer Base wie Natriumhydrid, Kaliumhydrid, Kalium-tert.-butanolat, Natrium-tert.-butanolat, 1,4-Diazabicyclo [2.2.2]octan, 1,8-Diazabicyclo[5.4.0]undec-7-en, Kaliumcarbonat oder Caesiumcarbonat in einem Lösungsmittel wie Tetrahydrofuran, 1,4-Dioxan, Acetonitril, N,N-Dimethylformamid oder N-Methyl-pyrrolidon bei Temperaturen zwischen -30°C und 150°C, bevorzugt jedoch zwischen 0°C und 100°C, durchgeführt. Der dabei erhaltene Methylester wird anschließend wieder gespalten. Diese Umsetzung erfolgt hydrolytisch in einem wässrigen Lösungsmittel, z.B. in Wasser, Methanol/Wasser, Isopropanol/Wasser, Essigsäure/Wasser, Tetrahydrofuran/Wasser oder 1,4-Dioxan/Wasser, vorzugsweise jedoch in Methanol/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid, vorzugsweise jedoch

Natriumhydroxid, oder aprotisch, z.B. in Gegenwart von Iodtrimethylsilan, bei Temperaturen zwischen 0 und 120°C, vorzugsweise bei Temperaturen zwischen 10 und 100°C.

Die Carbonsäuren der allgemeinen Formel XXIII in denen $R^{15}$ Methyl bedeutet, werden dann mit einem Anilin zum Anilid der allgemeinen Formel XV umgesetzt. Dazu wird die Carbonsäure in situ durch Zugabe von N,N'-Diisopropylcarbodiimid, N,N'-Dicyclohexylcarbodiimid, N-(3-Dimethylaminopropyl)-N'-ethyl-carbodiimid, O-(Benzotriazol-1-yl)-N,N,N',N'-tetramethyluronium-tetrafluoroborat (TBTU), O-(Benzotriazol-1-yl)-N,N,N',N'-tetramethyluronium-hexafluorophosphat (HBTU), O-(7-AzaBenzotriazol-1-yl)-N,N,N',N'-tetramethyluronium-hexafluorophosphat (HATU), (Benzotriazol-1-yloxy)-tris-(dimethylamino)-phosphonium-hexafluorophosphat (BOP), (Benzotriazol-1-yloxy)-tripyrrolidinophosphonium-hexafluorophosphat (PyBOP) oder n-Propylphosphonsäureanhydrid aktiviert und in einem dipolar aprotischen Lösungsmittel wie beispielsweise N,N-Dimethylformamid, Dimethylacetamid, Tetrahydrofuran, Acetonitril, N-Methylpyrrolidon oder Dimethylsulfoxid, vorzugsweise jedoch in N,N-Dimethylformamid oder N-Methylpyrrolidon, mit einem Anilin in Anwesenheit einer Base wie Triethylamin, N,N-Diisopropyl-N-ethyl-amin und gegebenenfalls eines Katalysators wie 4-N,N-Dimethylaminopyridin bei Temperaturen zwischen -20°C und 80°C, bevorzugt jedoch zwischen 0°C und 50°C, umgesetzt.

Alternativ wird die Carbonsäure zunächst in ein Säurechlorid überführt. Dazu wird die Carbonsäure mit Thionylchlorid, Phosphoroxychlorid oder Oxalylchlorid gegebenenfalls in einem Lösungsmittel wie Toluol, Benzol oder Dichlormethan bei Temperaturen zwischen 0°C und 120°C umgesetzt. Das so erhaltene Säurechlorid wird mit dem Anilin in einem Lösungsmittel wie Dichlormethan, Tetrahydrofuran, 1,4-Dioxan oder N,N-Dimethylformamid bei Temperaturen zwischen -30°C und 150°C, bevorzugt jedoch zwischen 0°C und 80°C und gegebenenfalls in Anwesenheit einer Base wie Triethylamin, N,N-Diisopropyl-N-ethyl-amin und gegebenenfalls in Anwesenheit eines Katalysators wie 4-N,N-Dimethylaminopyridin umgesetzt.

Desweiteren kann die Carbonsäure in ein Säureimidazolid überführt werden. Dazu wird sie mit Carbonyldiimidazol in einem Lösungsmittel wie Dichlormethan, Tetrahydrofuran oder 1,4-Dioxan bei Temperaturen zwischen 20°C und 100°C zur Reaktion gebracht. Das so erhaltene Säureimidazolid wird mit dem Anilin in einem Lösungsmittel wie Dichlormethan, Tetrahydrofuran, 1,4-Dioxan oder N,N-Dimethylformamid, bevorzugt jedoch Tetrahydrofuran, bei Temperaturen zwischen -30°C und 150°C, bevorzugt jedoch zwischen 0°C und 80°C, und gegebenenfalls in Anwesenheit einer Base wie Triethylamin, N,N-Diisopropyl-N-ethylamin und gegebenenfalls in Anwesenheit eines Katalysators wie 4-N,N-Dimethylaminopyridin umgesetzt.

Die so hergestellten Verbindungen der allgemeinen Formel XV können dann gemäß dem in Schema 3 gezeigten Verfahren c) in die Verbindungen der allgemeinen Formel VIII umgesetzt werden, welche dann gemäß dem in Schema 1 gezeigten Verfahren a) in die Endverbindungen der allgemeinen Formel I überführt werden können.

Die Herstellung von Verbindungen der allgemeinen Formel IX kann gemäß dem in Schema 6, in dem Ar, $R^2$, und p wie zuvor definiert sind, m 1 oder 2 bedeutet und $R^{14}$-$OS(O)_2CF_3$, Br oder I bedeuten, gezeigten erfindungsgemäßen Verfahren f) ausgehend von Verbindungen der allgemeinen Formel XV erfolgen.

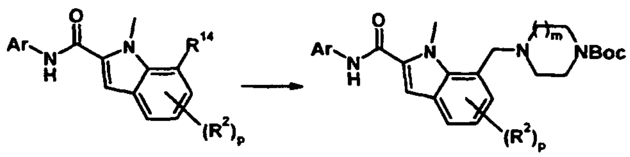

XV IX

Schema 6 (Verfahren f))

Dazu werden Verbindungen der allgemeinen Formel XV mit Kalium ((4-(tert.-butoxycarbonyl)-piperazin-1-yl)-methyl)-trifluoroborat (Herstellung: Org. Lett. 2007, 9, 1597-1600) oder ((4-(tert.-butoxycarbonyl)-homopiperazin-1-yl)-methyl)-trifluoroborat in Anwesenheit von Palladium-II-acetat sowie eines Liganden wie 2-Dicyclohexylphosphino-2',4',6'-triisopropyl-1,1'-biphenyl (XPhos), Tricyclohexylphosphin, Tri-tert.-butylphosphin, 1,3-Bis-(diphenylphosphino)-propan oder 1,1'-Bis-(diphenylphosphino)-ferrocen, vorzugsweise jedoch 2-Dicyclohexylphosphino-2',4',6'-triisopropyl-1,1'-biphenyl (XPhos), sowie einer Base wie Natriumcarbonat, Kaliumcarbonat, Cäsiumcarbonat, Triethylamin oder N,N-Diisopropyl-N-ethyl-amin, vorzugsweise jedoch Cäsiumcarbonat, in einem Lösungsmittel wie Tetrahydrofuran, 1,4-Dioxan, Cyclopentyl-methyl-ether, Acetonitril oder N,N-Dimethylformamid, vorzugsweise jedoch in Tetrahydrofuran oder Cyclopentyl-methyl-ether, gegebenenfalls unter Zusatz von Wasser, bei Temperaturen zwischen 50°C und 150°C, vorzugsweise jedoch zwischen 70°C und 120°C, zu den Verbindungen der allgemeinen Formel IX umgesetzt (siehe auch Org. Lett. 2007, 9, 1597-1600).

Die so hergestellten Verbindungen der allgemeinen Formel IX können dann gemäß dem in Schema 1 gezeigten Verfahren a) in die Endverbindungen der allgemeinen Formel I umgesetzt werden.

Die Herstellung von Verbindungen der allgemeinen Formel I kann auch gemäß dem in Schema 7, in dem Ar, T, $R^2$, p, m und $R^1$ wie zuvor definiert sind, A -C(H)< bedeutet, $R^{14}$-$OS(O)_2CF_3$, Br oder I darstellt, gezeigten erfindungsgemäßen Verfahren g) ausgehend von Verbindungen der allgemeinen Formel XV erfolgen.

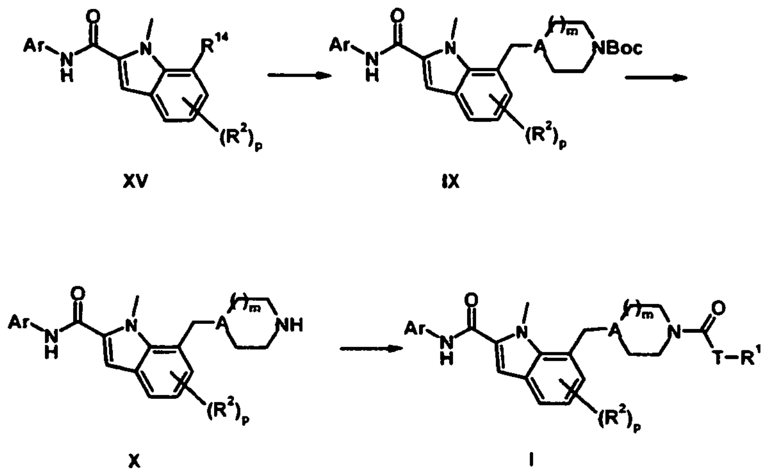

XV IX X I

**Schema 7 (Verfahren g))**

Dazu werden die Verbindungen der allgemeinen Formel XV mit 4-(9-borabicyclo[3.3.1]nonan-9-ylmethyl)-piperidin-1-carbonsäure tert.-butyl ester (wird erhalten aus der Umsetzung von 4-Methylen-piperidin-1-carbonsäure tert-butyl ester mit 9-Bora-bicyclo[3.3.1]nonan in Tetrahydrofuran unter Rückfluß) oder 4-(9-Bora-bicyclo[3.3.1]non-9-ylmethyl)-azepan-1-carbonsäure tert-butyl ester (wird erhalten aus der Umsetzung von 4-Methylen-azepan-1-carbonsäure tert-butyl ester mit 9-Bora-bicyclo[3.3.1]nonan in Tetrahydrofuran unter Rückfluß) in Anwesenheit eines Katalysators wie [1,1'-Bis(diphenylphosphino)ferrocen]-palladium-IIdichlorid-Dichlormethan-Komplex, Bis-tri-tert.-butylphosphin-palladium-(0), Bis-tri-cyclohexylphosphin-palladium-(0), [1,2-Bis(diphenylphosphino)ethan]palladium-II-dichlorid oder [1,3-Bis(diphenylphosphino)propan]palladium-II-dichlorid, vorzugsweise jedoch [1,1'-Bis(diphenylphosphino)ferrocen]-palladium-IIdichlorid-Dichlormethan-Komplex, sowie einer Base wie beispielsweise Natriumcarbonat, Kaliumcarbonat, Cäsiumcarbonat, Triethylamin oder N,N-Diisopropyl-N-ethyl-amin, vorzugsweise jedoch Kaliumcarbonat, in einem Lösungsmittel wie Tetrahydrofuran, 1,4-Dioxan, Acetonitril oder N,N-Dimethylformamid, gegebenenfalls unter Zusatz von Wasser, bei Temperaturen zwischen 30°C und 150°C, vorzugsweise jedoch zwischen 50°C und 120°C, zu den Verbindungen der allgemeinen Formel IX umgesetzt.

Die tert.-Butyloxycarbonylgruppe in Verbindungen der allgemeinen Formel IX wird durch Behandlung mit einer Säure wie Trifluoressigsäure oder Salzsäure oder durch Behandlung mit Iodtrimethylsilan gegebenenfalls unter Verwendung eines Lösungsmittels wie Methylenchlorid, 1,4-Dioxan, Methanol oder Diethylether abgespalten.

Die dabei erhaltenen Amine der allgemeinen Formel X werden dann zu Verbindungen der allgemeinen Formel I acyliert. Dabei können Acylreste eingeführt werden durch Umsetzung einer Verbindung der allgemeinen Formel X mit einem Acylierungsreagens wie beispielsweise einem Säurechlorid oder Säureanhydrid. Die Umsetzung kann in Anwesenheit einer Base wie Natriumhydroxid, Natriumhydrid, Natriumcarbonat, Kaliumcarbonat, Cäsiumcarbonat, Triethylamin oder N,N-Diisopropyl-N-ethyl-amin sowie in einem Lösungsmittel wie Dichlormethan, Tetrahydrofuran, 1,4-Dioxan oder N,N-Dimethylformamid bei Temperaturen zwischen -30°C und 120°C, bevorzugt jedoch zwischen 0°C und 80°C, erfolgen.

Alternativ kann die Umsetzung durch Acylierung mit einer Säure erfolgen. Dazu wird die Säure in situ durch Zugabe von N,N'-Diisopropylcarbodiimid, N,N'-Dicyclohexylcarbodiimid, N-(3-Dimethylaminopropyl)-N'-ethyl-carbodiimid, O-(Benzotriazol-1-yl)-N,N,N',N'-tetramethyluronium-tetrafluoroborat (TBTU), O-(Benzotriazol-1-yl)-N,N,N',N'-tetramethyluronium-hexafluorophosphat (HBTU), O-(7-AzaBenzotriazol-1-yl)-N,N,N',N'-tetramethyluronium-hexafluorophosphat (HATU), (Benzotriazol-1-yloxy)-tris-(dimethylamino)-phosphonium-hexafluorophosphat (BOP), (Benzotriazol-1-yloxy)-tripyrrolidinophosphonium-hexafluorophosphat (PyBOP) oder n-Propylphosphonsäureanhydrid aktiviert und in einem dipolar aprotischen Lösungsmittel wie beispielsweise N,N-Dimethylformamid, Dimethylacetamid, Tetrahydrofuran, Acetonitril, N-Methylpyrrolidon oder Dimethylsulfoxid, vorzugsweise jedoch in N,N-Dimethylformamid oder N-Methylpyrrolidon, mit einer Verbindung der allgemeinen Formel X in Anwesenheit einer Base wie Triethylamin oder N,N-Diisopropyl-N-ethyl-amin und gegebenenfalls eines Katalysators wie 4-N,N-Dimethylaminopyridin bei Temperaturen zwischen -20°C und 80°C, bevorzugt jedoch zwischen 0°C und 50°C, umgesetzt.

Aminocarbonylreste können eingeführt werden durch Umsetzung mit einem Isocyanat, gegebenenfalls in Anwesenheit einer Base wie beispielsweise Natriumcarbonat, Kaliumcarbonat, Cäsiumcarbonat, Triethylamin oder N,N-Diisopropyl-N-ethyl-amin in einem Lösungsmittel wie Dichlormethan, Tetrahydrofuran, 1,4-Dioxan oder N,N-Dimethylformamid bei Temperaturen zwischen -30°C und 150°C, bevorzugt jedoch zwischen 0°C und 100°C. Alternativ werden Aminocarbonylreste durch Umsetzung einer Verbindung der allgemeinen Formal X mit Phosgen, Diphosgen oder Triphosgen in einem Lösungsmittel wie Dichlormethan, Tetrahydrofuran oder 1,4-Dioxan in Anwesenheit einer Base wie beispielsweise Natriumcarbonat, Kaliumcarbonat, Cäsiumcarbonat, Triethylamin oder N,N-Diisopropyl-N-ethyl-amin, bevorzugt jedoch N,N-Diisopropyl-N-ethyl-amin und nachfolgende Behandlung mit einem Amin bei Temperaturen zwischen -20°C und 100°C, bevorzugt jedoch zwischen 0°C und 50°C, erhalten.

Die Herstellung von Verbindungen der allgemeinen Formel I kann auch gemäß dem in Schema 8, in dem Ar, A, $R^2$, p und m wie zuvor definiert sind, T $-CH_2-$ bedeutet, $R^1$ ein gegebenenfalls substituiertes cyclisches Amin darstellt, gezeigten erfindungsgemäßen Verfahren h) ausgehend von Verbindungen der allgemeinen Formel X erfolgen.

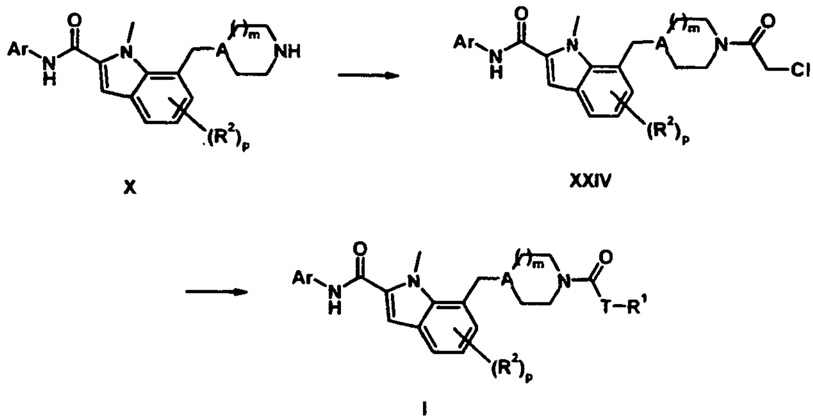

Schema 8 (Verfahren h))

Dazu werden die Verbindungen der allgemeinen Formel X mit Chloressigsäurechlorid in einem Lösungsmittel wie Dichlormethan, Tetrahydrofuran oder 1,4-Dioxan in Anwesenheit einer Base wie beispielsweise Triethylamin oder N,N-Diisopropyl-N-ethyl-amin und gegebenenfalls eines Katalysators wie 4-N,N-Dimethylaminopyridin bei Temperaturen zwischen -20°C und 80°C, bevorzugt jedoch zwischen 0°C und 50°C zu Verbindungen der allgemeinen Formel XXIV umgesetzt. Diese werden dann mit einem geeigneten cyclischen Amin in einem Lösungsmittel wie Dichlormethan, Tetrahydrofuran, 1,4-Dioxan oder N,N-Dimethylformamid, bevorzugt jedoch N,N-Dimethylformamid, in Anwesenheit einer Base wie beispielsweise Natriumcarbonat, Kaliumcarbonat, Cäsiumcarbonat, Triethylamin oder N,N-Diisopropyl-N-ethyl-amin, bevorzugt jedoch Kaliumcarbonat, bei Temperaturen zwischen - 30°C und 100°C, bevorzugt jedoch zwischen 0°C und 80°C, zu Verbindungen der allgemeinen Formel I umgesetzt.

Bei den vorstehend beschriebenen Umsetzungen können gegebenenfalls vorhandene reaktive Gruppen wie Carboxy-, Carbonyl-, Hydroxy-, Amino- oder Alkylaminogruppen während der Umsetzung durch übliche Schutzgruppen geschützt werden, welche nach der Umsetzung wieder abgespalten werden.

Beispielsweise kommt als Schutzrest für eine Carboxygruppe die Methyl-, Ethyl-, tert.-Butyl oder die Benzylgruppe in Betracht.

Beispielsweise kommt als Schutzrest für eine Carbonylgruppe eines Ketons oder Aldehyds ein Ketal bzw. Acetal, z.B. abgeleitet von Methanol, Glycol oder Propan-1,3-diol, in Betracht.

Beispielsweise kommen als Schutzrest für eine aliphatische Hydroxygruppe die Trimethylsilyl, tert-Butyldimethylsilyl-, Triisopropylsilyl-, Acetyl-, Pivaloyl, Benzoyl, Methyl, Allyl, Benzyl-, 4-Methoxybenzyl-, Trityl-, Methoxymethyl-, Ethoxymethyl-, 2-Trimethylsilylethoxymethyl- oder Tetrahydropyranylgruppe in Betracht.

Für eine phenolische OH-Gruppe eignen sich, neben den für die aliphatische Hydroxygruppe bereits genannten, Methylsulfonyl, Tosyl und Trifluormethylsulfonyl als Schutzgruppe.

Als Schutzreste für eine Amino- oder Alkylaminogruppe kommen beispielsweise die Formyl-, Acetyl-, Trifluoracetyl-, Ethoxycarbonyl-, tert.-Butoxycarbonyl-, Benzyloxycarbonyl-, Benzyl-, 4-Methoxybenzyl- oder 2,4-Dimethoxybenzylgruppe in Betracht.

Die Spaltung einer Carboxymethyl- oder Carboxyethyl-Einheit erfolgt beispielsweise hydrolytisch in einem wässrigen Lösungsmittel, z.B. in Wasser, Methanol/Wasser, Isopropanol/Wasser, Essigsäure/Wasser, Tetrahydrofuran/Wasser oder 1,4-Dioxan/Wasser, vorzugsweise jedoch in Methanol/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid vorzugsweise jedoch Natriumhydroxid, oder aprotisch, z.B. in Gegenwart von Iodtrimethylsilan, bei

Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt vorteilhaft hydrogenolytisch, z.B. mit Wasserstoff in Gegenwart eines Katalysators wie Palladium auf Kohle, Palladiumhydroxid oder Platinoxid in einem geeigneten Lösungsmittel wie Methanol, Ethanol, Essigsäureethylester oder Eisessig, gegebenenfalls unter Zusatz einer Säure wie Salzsäure bei Temperaturen zwischen 0 und 100°C, vorzugsweise jedoch zwischen 20 und 60°C, und bei einem Wasserstoffdruck von 1 bis 7 bar, vorzugsweise 3 bis 5 bar. Trimethylsilyliodid, Bortrichlorid oder Bortrifluorid in Gegenwart eines Abfangreagenzes, z.B. Anisol, Thioanisol oder Pentamethylbenzol, können ebenfalls zur Spaltung von Benzylethem einschließlich deren substituierten Derivaten verwendet werden. Die Abspaltung von elektronenreichen Benzylresten, wie z.B. 4-Methoxybenzyl, kann auch oxidativ mit z.B. 2,3-Dichlor-5,6-dicyan-1,4-benzochinon (DDQ) oder Cerammoniumnitrat (CAN), bevorzugt in alcoholischen oder wässrigen Lösungen zwischen 10 und 120 °C bewerkstelligt werden. Die 2,4-Dimethoxybenzylgruppe wird vorzugsweise in Trifluoressigsäure in Gegenwart eines Abfangreagenzes, z.B. Anisol, abgespalten.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Iodtrimethylsilan gegebenenfalls unter Verwendung eines Lösungsmittels wie Methylenchlorid, 1,4-Dioxan, Methanol oder Diethylether.

Die Abspaltung eines verwendeten Acetal- oder Ketal-Schutzrestes erfolgt bevorzugt in einem wässrigen Lösungsmittel, z.B. Wasser, Isopropanol/Wasser, Tetrahydrofuran/Wasser oder 1,4-Dioxan/Wasser, in Gegenwart einer Säure, z.B. Essigsäure, Trifluoressigsäure, Salzsäure oder Schwefelsäure, bei Temperaturen zwischen 0 und 120°C, vorzugsweise zwischen 10 und 100°C.

Eine Methylgruppe an einem tertiären Amin kann durch Behandlung mit 1-Chlorethylchloroformat abgespalten werden. HBr oder $BBr_3$ sind besonders zur Spaltung von Methylethern geeignet.

Ferner können die erhaltenen Verbindungen der allgemeinen Formel I, oder Zwischenprodukte der Synthese von Verbindungen der allgemeinen Formel I, wie bereits eingangs erwähnt, in ihre Enantiomeren und/oder Diastereomeren aufgetrennt werden. So können beispielsweise cis-/trans-Gemische in ihre cis- und trans-Isomere, und Verbindungen mit mindestens einem Stereozentrum in ihre Enantiomeren aufgetrennt werden.

So lassen sich beispielsweise die erhaltenen cis-/trans-Gemische durch Chromatographie in ihre cis- und trans-Isomeren, die die erhaltenen Verbindungen der allgemeinen Formel I, oder Zwischenprodukte der Synthese von Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I, oder Zwischenprodukte der Synthese von Verbindungen der allgemeinen Formel I, mit mindestens zwei asymmetrischen Kohlenstoffatomen, auf Grund ihrer physikalisch-chemischen Unterschiede nach an sich bekannten Methoden, z.B. durch Chromatographie und/oder fraktionierte Kristallisation, in ihre Diastereomeren auftrennen, die falls sie in racemischer Form anfallen, anschließend wie oben erwähnt in die Enantioneren aufgetrennt werden können.

Die Enantiomerentrennung erfolgt vorzugsweise durch Chromatographie an chiralen Phasen oder durch Umkristallisation aus einem optisch aktiven Lösungsmittel oder durch Umsetzen mit einer, mit der racemischen Verbindung Salze oder Derivate wie z. B. Ester oder Amide bildenden optische aktiven Substanz, insbesondere Säuren und ihre aktivierten Derivate oder Alkohole, und Trennen des auf diese Weise erhaltenen diastereomeren Salzgemisches oder Derivates, z. B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optische aktive Säuren sind z.B. die D- und L-Formen von Weinsäure oder Dibenzoylweinsäure, Di-O-p-toluoyl-weinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Des weiteren können die erhaltenen Verbindungen der allgemeinen Formel I, oder Zwischenprodukte der Synthese von Verbindungen der allgemeinen Formel I, in ihre Salze insbesondere für die pharmazeutische Anwendung in ihren physiologisch verträglichen Salze mit anorganischen oder organischen Säuren, überführt werden. Als Säuren kommen hierfür beispielsweise Salzsäure, Bromwasserstoffsäure, Schwefelsäure, Methansuffonsäure, Phosphorsäure, Fumarsäure, Bernsteinsäure, Milchsäure, Zitronensäure, Weinsäure oder Maleinsäure in Betracht.

Außerdem lassen sich die erhaltenen neuen Verbindungen der allgemeinen Formel I, oder Zwischenprodukte der Synthese von Verbindungen der allgemeinen Formel I, falls diese eine Carboxygruppe enthalten, gewünschtenfalls anschließend in ihre Salze mit anorganischen oder organischen Basen, insbesondere für die pharmazeutische Anwendung in ihren physiologisch verträglichen Salze, überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die als Ausgangsstoffe verwendeten Verbindungen der allgemeinen Formeln V, XI, XVIII und XXIII sind teilweise literaturbekannt oder können nach an sich literaturbekannten Verfahren sowie in Analogie zu den in den Beispielen beschriebenen Verfahren, gegebenenfalls unter zusätzlicher Einführung von Schutzresten, erhalten werden.

## Experimenteller Teil

### HPLC Methoden:

Methode 1: Säule: Merck Cromolith Speed ROD, RP18e, 50 x 4.6 mm; 1.5 ml/Minute; UV-Detektion: 230 nm / 254 nm; Eluent A: Wasser (0.1 % Ameisensäure), Eluent B: Acetonitril (0.1 % Ameisensäure)

| Gradient: | Time (min.) | % Eluent B |
|---|---|---|
| | 0.00 | 10 |
| | 4.50 | 90 |
| | 5.00 | 90 |
| | 5.50 | 10 |

Methode 2: Säule: Agilent Zorbax Bonus RP, 50 x 2.1 mm, 3.5 µm; 1.2 ml/Minute; UV-Detektion: 230 nm / 254 nm; Eluent A: Wasser (0.1 % Ameisensäure), Eluent B: Acetonitril (0.1 % Ameisensäure)

| Gradient: | Time (min.) | % Eluent B |
|---|---|---|
| | 0.00 | 10 |
| | 4.50 | 99 |
| | 5.00 | 99 |
| | 5.50 | 10 |

Methode 3: Präparative HPLC
Säule: XBridge Prep C18, 225 g Trennmaterial, 300 x 50 mm, 5 µm; 120 ml/Minute; UV-Detektion: 230 nm / 254 nm, Eluent A: Wasser (0.45 % konz. Ammoniak), Eluent B: Acetonitril

| Gradient: | Time (min.) | % Eluent B | Flussrate |
|---|---|---|---|
| | 0.00 | 10 | 60 ml/min. |
| | 2.00 | 10 | 60 ml/min. |
| | 3.00 | 10 | 120 ml/min. |
| | 3.50 | 10 | 120 ml/min. |
| | 22.00 | 100 | 120 ml/min. |
| | 25.50 | 100 | 120 ml/min. |
| | 26.50 | 10 | 120 ml/min. |
| | 30.00 | 10 | 120 ml/min. |

Methode 4: Präparative HPLC
Säule: Waters Symmetrie C18, 150 x 50 mm, 7 µm; 120 ml/Minute; Massengesteuerte Fraktionierung (MS: Thermo Finnigan Surveyor MSQ); Eluent A: Wasser (0.14 % Ameisensäure), Eluent B: Acetonitril

| Gradient: | Time (min.) | % Eluent B | Flussrate |
|---|---|---|---|
| | 0.00 | 10 | 120 ml/min. |
| | 2.00 | 10 | 120 ml/min. |
| | 9.00 | 95 | 120 ml/min. |
| | 11.50 | 95 | 120 ml/min. |
| | 12.00 | 5 | 120 ml/min. |
| | 14.00 | 5 | 120 ml/min. |

Methode 5: Säule: Waters Xbridge C18, 50 x 4,6 mm, 3,5 µm; 1,5 ml/Minute,
UV-Detektion: DAD 210-500 nm; Eluent A: Wasser (0.032 % Ammoniak),

Eluent B: Acetonitril

| Gradient: | Time (min.) | % Eluent B |
|---|---|---|
| | 0.00 | 0 |
| | 2.00 | 100 |
| | 3.00 | 100 |

Methode 6: Säule: Waters Xbridge C18, 30 x 4,6 mm, 2,5 μm; 1.2 ml/Minute; UV-Detektion: DAD 190-400 nm; Eluent A: Wasser (0.1 % Ammoniak), Eluent B: Acetonitril

| Gradient: | Time (min.) | % Eluent B |
|---|---|---|
| | 0.00 | 5 |
| | 0.15 | 5 |
| | 4.00 | 90 |
| | 4.20 | 90 |
| | 4.30 | 5 |
| | 5.00 | 5 |

Methode 7: Präparative HPLC
Säule: Microsorb 100 C18, 225 g Trennmaterial, 250 x 41,4 mm, 8 μm; 120 ml/Minute; UV-Detektion: 230 nm / 254 nm, Eluent A: Wasser (0.14 % Trifluoressigsäure), Eluent B: Acetonitril
Gradient: linearer Gradient (10 % Eluent B auf 100 % Eluent B innerhalb 30 Minuten.

**Herstellung der Ausgangsverbindungen**

Beispiel I

1-Methyl-1H-indol-7-carbaldehyd

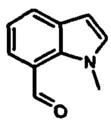

20 g 1H-Indol-7-carbaldehyd werden in 160 ml N,N-Dimethylformamid gelöst und unter Argon portionsweise mit 15,5 g Kalium-tert.-butylat versetzt. Nach beendeter Zugabe lässt man 20 Minuten nachrühren und tropft dann 8,7 ml Methyliodid zu. Anschließend lässt man 12 Stunden bei Raumtemperatur rühren und verteilt dann zwischen Wasser und Essigsäureethylester. Die wässrige Phase wird 2 mal mit Essigsäureethylester extrahiert und die vereinigten organischen Phasen werden mit gesättigter wässriger Natriumchloridlösung gewaschen. Nach Trocknen mit Magnesiumsulfat werden die Lösungsmittel im Vakuum entfernt. Der Rückstand wird aus Wasser ausgerührt. Der dabei erhaltene Feststoff wird abgesaugt und im Vakuum getrocknet.
Ausbeute: 20 g (91 % der Theorie)
Massenspektrum (ESI$^+$): m/z = 160 $[M+H]^+$

Beispiel II

(1-Methyl-1H-indol-7-yl)-methanol

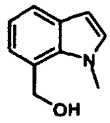

10,8 g 1-Methyl-1H-indol-7-carbaldehyd werden in 150 ml Ethanol gelöst, auf 0°C gekühlt und portionsweise mit 1,3 g Natriumborhydrid versetzt. Nach beendeter Zugabe wird das Kühlbad entfernt und 2 Stunden bei Raumtemperatur nachgerührt. Anschließend werden 68 ml 1 M Natronlauge zugegeben, 10 Minuten gerührt und dann der Großteil des Ethanols im Vakuum entfernt. Der Rückstand wird zwischen Wasser und Essigsäureethylester verteilt und die wässrige Phase wird 2 mal mit Essigsäureethylester extrahiert. Die vereinigten organischen Phasen werden mit gesättigter wässriger Natriumchloridlösung gewaschen und mit Magnesiumsulfat getrocknet. Danach werden die Lösungsmittel im Vakuum entfernt und der Rückstand im Vakuum getrocknet.
Ausbeute: 10,5 mg (96 % der Theorie)
HPLC (Methode 1): Retentionszeit = 2,67 min.
Massenspektrum ($ESI^+$): m/z = 162 $[M+H]^+$

Beispiel III

7-Hydroxymethyl-1-methyl-1H-indol-2-carbonsäure

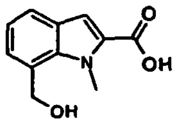

Unter Argon wird eine Lösung von 4 g (1-Methyl-1H-indol-7-yl)-methanol in 80 ml Diethylether zu 76 ml einer 1,6 M Lösung von n-Butyllithium in Hexan bei 0°C zugetropft, wobei die Temperatur 35°C nicht übersteigt. Anschließend wird 12 Stunden bei Raumtemperatur nachgerührt und die so erhaltene Lösung auf 30 g zerstoßenes Trockeneis in 100 ml Diethylether gegossen. Man lässt rühren bis sich das gesamte Trockeneis aufgelöst hat und beendet dann die Reaktion durch Zugabe von 40 ml Wasser. Die Phasen werden getrennt und die organische Phase wird 2 mal mit je 20 ml Wasser extrahiert. Die vereinigten wässrigen Phasen werden abgesaugt und das Filtrat mit konzentrierter Salzsäure auf pH 2 eingestellt. Der dabei ausfallende Feststoff wird abgesaugt und im Vakuum getrocknet.
Ausbeute: 1,93 mg (47 % der Theorie)
Massenspektrum ($ESI^-$): m/z = 204 $[M-H]^-$

Beispiel IV

7-Formyl-1-methyl-1H-indol-2-carbonsäure; Salz mit N-Methyl-morpholin

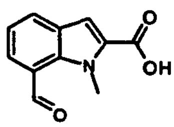

1,9 g 7-Hydroxymethyl-1-methyl-1H-indol-2-carbonsäure, 1,63 g N-Methyl-morpholin-N-oxid und 400 mg Molsieb (3Å) werden in 10 ml Dichlormethan gelöst und mit 260 mg Tetra-n-propyl-ammonium-perrhutenat versetzt. Man lässt 12 Stunden bei Raumtemperatur rühren und chromatographiert den Rückstand an Kieselgel mit Dichlormethan/Methanol (95:1 auf 80:20).
Ausbeute: 1,45 g (51 % der Theorie)
HPLC (Methode 2): Retentionszeit = 2,75 min.
Massenspektrum ($ESI^+$): m/z = 204 $[M+H]^+$

Analog Beispiel IV wird folgende Verbindung erhalten:

(1) 7-Formyl-1-methyl-1H-indol-2-carbonsäure-(5-tert-butyl-3-methansulfonylamino-2-methoxy-phenyl)-amid

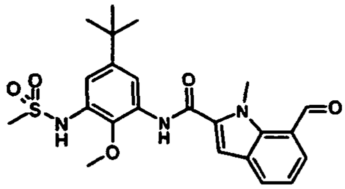

Massenspektrum (ESI$^+$): m/z = 458 [M+H]$^+$

Beispiel V

7-Formyl-1-methyl-1H-indol-2-carbonsäure (5-tert-butyl-3-methansulfonylamino-2-methoxy-phenyl)-amid

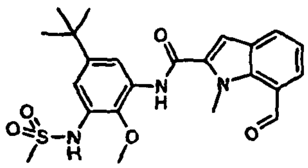

1,45 g 7-Formyl-1-methyl-1H-indol-2-carbonsäure (Salz mit N-Methyl-morpholin) werden in 8 ml N,N-Dimethylformamid gelöst und mit 2,72 g O-(7-Azabenzotriazol-1-yl)-N,N,N',N'-tetramethyluronium-hexafluorophosphat (HATU), 2,45 ml N,N-Diisopropyl-ethylamin (DIEA) sowie 324 mg 1-Hydroxy-7-azabenzotriazol (HOAT) versetzt. Man lässt 15 Minuten bei Raumtemperatur rühren und gibt dann 1,47 g N-(3-Amino-5-tert-butyl-2-methoxy-phenyl)-methansulfonamid Hydrochlorid zu. Die Reaktion wird 12 Stunden auf 50°C erhitzt und dann zwischen Wasser und Essigsäureethylester verteilt. Die organische Phase wird nacheinander mit 1 N Salzsäure, Wasser und gesättigter wässriger Natriumchloridlösung gewaschen und mit Magnesiumsulfat getrocknet. Nach Entfernen der Lösungsmittel im Vakuum wird der Rückstand an Kieselgel mit Cyclohexan/Essigsäureethylester (70:30 auf 20:80) chromatographiert.
Ausbeute: 1,43 g (66 % der Theorie)
HPLC (Methode 2): Retentionszeit = 3,37 min.
Massenspektrum (ESI$^+$): m/z = 458 [M+H]$^+$

Beispiel VI

4-[2-(5-tert-Butyl-3-methansulfonylamino-2-methoxy-phenylcarbamoyl)-1-methyl-1H-indol-7-ylmethyl]-piperazin-1-carbonsäure-tert-butyl ester

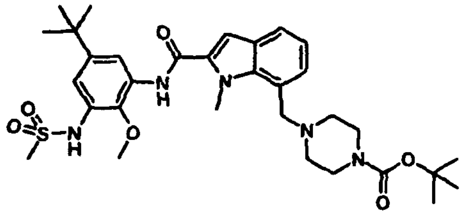

1,1 g 7-Formyl-1-methyl-1H-indol-2-carbonsäure (5-tert-butyl-3-methansulfonylamino-2-methoxy-phenyl)-amid werden in 50 ml 1,2-Dichlorethan gelöst und mit 2,63 ml Eisessig sowie einer Lösung von 4,48 g Piperazin-1-carbonsäure-tert-butyl ester in 20 ml 1,2-Dichlorethan versetzt. Man lässt 30 Minuten bei Raumtemperatur rühren und gibt dann 2,04 g Natriumtriacetoxyborhydrid portionsweise zu. Anschließend lässt man 4 Stunden rühren und beendet dann die Reaktion durch Zugabe von gesättigter wässriger Natriumhydrogencarbonatlösung. Man versetzt mit ausreichend Essigsäureethylester und trennt die Phasen. Die wässrige Phase wird 2 mal mit Essigsäureethylester extrahiert und die vereinigten organischen Pahsen werden mit gesättigter wässriger Natriumchloridlösung gewaschen. Nach Trocknen mit Magnesiumsulfat, werden die Lösungsmittel im Vakuum entfernt und der Rückstand an Kieselgel mit Cyclohexan/Essigsäureethylester (80:20 auf 20:80) chromatographiert.
Ausbeute: 1,13 g (75 % der Theorie)
$R_f$-Wert: 0,30 (Kieselgel, Cyclohexan/Essigsäureethylester 6:4)
Massenspektrum (ESI$^+$): m/z = 628 $[M+H]^+$

Analog Beispiel VI werden folgende Verbindungen erhalten:

(1) 4-[2-(5-tert-Butyl-3-methansulfonylamino-2-methoxy-phenylcarbamoyl)-1-methyl-1H-indol-7-ylmethyl]-[1,4]diazepan-1-carbonsäure-tert-butyl ester

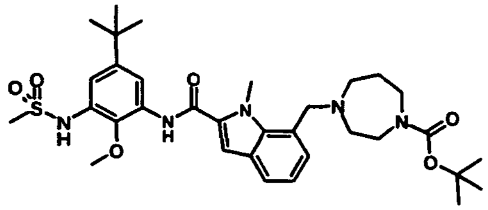

$R_f$-Wert: 0,50 (Kieselgel, Cyclohexan/Essigsäureethylester 1:1)
Massenspektrum (ESI$^+$): m/z = 642 $[M+H]^+$

(2) 1-[2-(5-tert-Butyl-3-methansulfonylamino-2-methoxy-phenylcarbamoyl)-1-methyl-1H-indol-7-ylmethyl]-piperidin-4-carbonsäure-tert-butyl ester

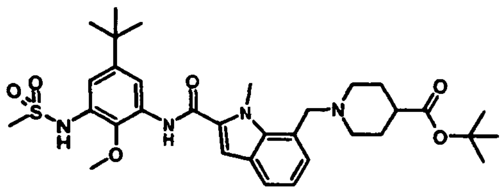

HPLC (Methode 1): Retentionszeit = 3,22 min.
Massenspektrum (ESI$^+$): m/z = 627 $[M+H]^+$

(3) (S)-1-Methyl-7-[4-(1-methyl-pyrrolidin-2-carbonyl)-piperazin-1-ylmethyl]-1H-indol-2-carbonsäure

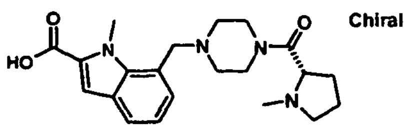

Als Reaktionspartner werden 7-Formyl-1-methyl-1H-indol-2-carbonsäure und (1-Methylpyrrolidin-2-yl)-piperazin-1-yl-methanon eingesetzt. Nach vollständiger Reaktion werden die Lösungsmittel im Vakuum entfernt und der Rückstand in wenig Dichlormethan/Methanol 80:20 aufgenommen. Die Lösung wird auf eine kurze Kieselgelschicht gegeben und durch Anlegen eines Unterdrucks in eine Vakuumsaugflasche gesaugt. Danach wird in mehreren kleinen Portionen Dichlormethan/Methanol/Wasser 1:1:0,1 zugegeben und wiederum durch Anlegen eines Unterdrucks die Lösung in eine Vakuumsaugflasche gesaugt. Die Produkt enthaltenden Fraktionen werden vereinigt und im Vakuum eingeengt.
HPLC (Methode 6): Retentionszeit = 1,99 min.
Massenspektrum (ESI$^+$): m/z = 385 $[M+H]^+$

Beispiel VII

1-Methyl-7-piperazin-1-ylmethyl-1H-indol-2-carbonsäure (5-tert-butyl-3-methansulfonylamino -2-methoxy-phenyl)-amid

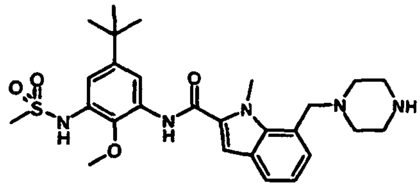

1,13 g 4-[2-(5-tert-Butyl-3-methansulfonylamino-2-methoxy-phenylcarbamoyl)-1-methyl-1H-indol-7-ylmethyl]-piperazin-1-carbonsäure-tert-butyl ester werden in 20 ml einer 5 M Lösung von HCl in Isopropanol gelöst und 12 Stunden bei Raumtemperatur gerührt. Anschließend wird mit gesättigter wässriger Natriumhydrogencarbonatlösung auf pH 9 eingestellt und 2 mal mit Essigsäureethylester extrahiert. Die vereinigten organischen Phasen werden mit Magnesiumsulfat getrocknet und die Lösungsmittel im Vakuum entfernt.
Ausbeute: 955 mg (101 % der Theorie)
HPLC (Methode 2): Retentionszeit = 1,88 min.
Massenspektrum (ESI$^+$): m/z = 528 $[M+H]^+$

Beispiel VIII

3-{4-[2-(5-tert-Butyl-3-methansulfonylamino-2-methoxy-phenylcarbamoyl)-1-methyl-1H-indol-7-ylmethyl]-piperazin-1-carbonyl}-azetidin-1-carbonsäure tert-butyl ester

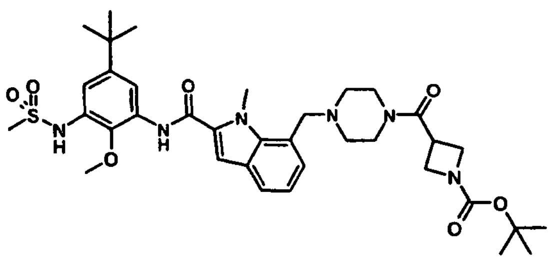

153 mg 1-(tert.-Butoxycarbonyl)-azetidin-3-carbonsäure werden in 3 ml N,N-Dimethylformamid gelöst und mit 244 mg O-(Benzotriazol-1-yl)-N,N,N',N'-tetramethyluronium-tetrafluoroborat (TBTU) sowie 245 µl N,N-Diisopropyl-ethylamin (DIEA) versetzt. Man lässt 20 Minuten nachrühren, gibt dann 150 mg 1-Methyl-7-piperazin-1-ylmethyl-1H-indol-2-carbonsäure (5-tert-butyl-3-methansulfonylamino-2-methoxy-phenyl)-amid hinzu und lässt 12 Stunden rühren. Anschließend wird zwischen Wasser und Essigsäureethylester verteilt und die wässrige Phase 2 mal mit Essigsäureethylester extrahiert. Die vereinigten organischen Phasen werden mit gesättigter wässriger Natriumchloridlösung gewaschen und mit Magnesiumsulfat getrocknet. Danach werden die Lösungsmittel im Vakuum entfernt und der Rückstand an Kieselgel mit Cyclohexan/Essigsäureethylester 70:30 auf 0:100 chromatographiert.
Ausbeute: 243 mg (90 % der Theorie)
$R_f$-Wert: 0,80 (Kieselgel, Essigsäureethylester)
Massenspektrum (ESI$^+$): m/z = 711 $[M+H]^+$

Analog Beispiel VIII werden folgende Verbindungen erhalten:

(1) (S)-3-{4-[2-(5-tert-Butyl-3-methansulfonylamino-2-methoxy-phenylcarbamoyl)-1-methyl-1H-indol-7-ylmethyl]-piperazin-1-carbonyl}-pyrrolidin-1-carbonsäure tert-butyl ester

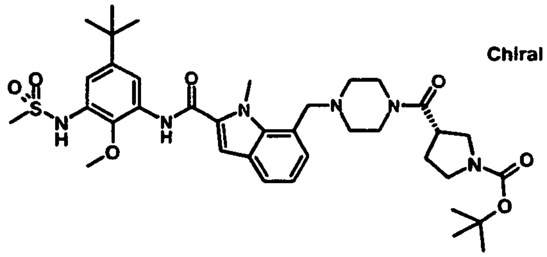

HPLC (Methode 1): Retentionszeit = 3,40 min.

(2) (S)-3-{4-[2-(5-tert-Butyl-3-methansulfonylamino-2-methoxy-phenylcarbamoyl)-1-methyl-1H-indol-7-ylmethyl]-[1,4]diazepan-1-carbonyl}-pyrrolidin-1-carbonsäure-tert-butyl ester

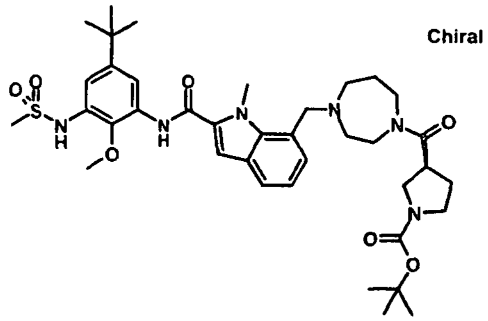

$R_f$-Wert: 0,70 (Kieselgel, Dichlormethan/Methanol/(Ammoniak in Methanol (gesättigt)) 95:5:0,1)

(3) (R)-3-{4-[2-(5-tert-Butyl-3-methansulfonylamino-2-methoxy-phenylcarbamoyl)-1-methyl-1H-indol-7-ylmethyl]-[1,4]diazepan-1-carbonyl}-pyrrolidin-1-carbonsäure-tert-butyl ester

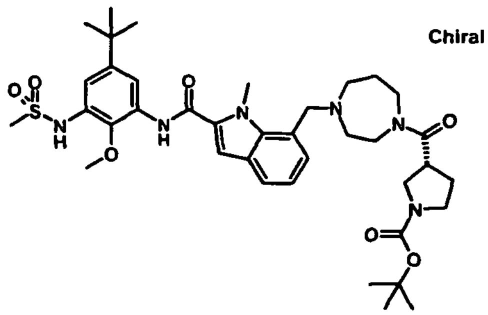

$R_f$-Wert: 0,73 (Kieselgel, Dichlormethan/Methanol/(Ammoniak in Methanol (gesättigt)) 95:5:0,1)

Beispiel IX

1-Methyl-azetidin-3-carbonsäure

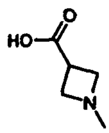

200 mg Azetidin-3-carbonsäure werden in 10 ml Methanol gelöst, mit 165 µl einer 37 %-igen Lösung von Formaldehyd in Wasser sowie 20 mg 10 % Palladium auf Kohle versetzt und bei 4 bar bis zum Ende der Wasserstoffaufnahme hydriert. Anschließend wird vom Katalysator abgesaugt, das Lösungsmittel im Vakuum entfernt, der Rückstand in Methanol und Toluol aufgenommen und die Lösungsmittel wieder im Vakuum entfernt.
Ausbeute: 214 mg (94 % der Theorie)
Massenspektrum ($ESI^+$): m/z = 116 $[M+H]^+$
Analog Beispiel IX werden folgende Verbindungen erhalten:

(1) (R)-1-Methyl-pyrrolidin-2-carbonsäure

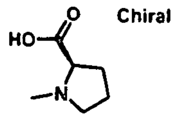

Massenspektrum ($ESI^+$): m/z = 130 $[M+H]^+$
(2) 1-Methyl-azetidin-2-carbonsäure

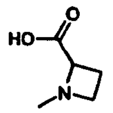

Massenspektrum ($ESI^+$): m/z = 116 $[M+H]^+$
(3) (2S,4R)-4-Hydroxy-1-methyl-pyrrolidin-2-carbonsäure

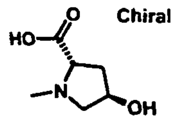

Massenspektrum ($ESI^+$): m/z = 146 $[M+H]^+$
(4) (2R,4R)-4-Methoxy-1-methyl-pyrrolidin-2-carbonsäure Hydrochlorid

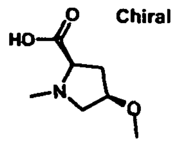

Massenspektrum ($ESI^+$): m/z = 160 $[M+H]^+$
(5) (2R,4R)-4-Hydroxy-1-methyl-pyrrolidin-2-carbonsäure

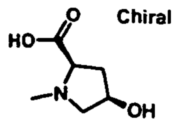

Massenspektrum ($ESI^+$): m/z = 146 $[M+H]^+$
(6) (2R,4S)-4-Hydroxy-1-methyl-pyrrolidin-2-carbonsäure

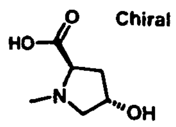

Massenspektrum (ESI$^+$): m/z = 146 [M+H]$^+$
(7) (2S,4S)-4-Hydroxy-1-methyl-pyrrolidin-2-carbonsäure

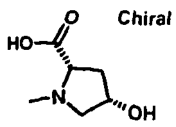

Massenspektrum (ESI$^+$): m/z = 146 [M+H]$^+$

Beispiel X

1-Methyl-7-piperidin-4-ylmethyl-1H-indol-2-carbonsäure (5-tert-butyl-3-methansulfonyl-amino-2-methoxy-phenyl)-amid

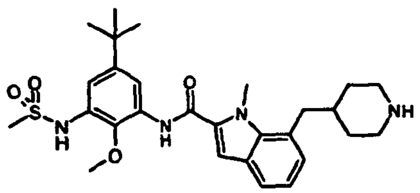

1,6 g 4-[2-(5-tert-Butyl-3-methansulfonylamino-2-methoxy-phenylcarbamoyl)-1-methyl-1H-indol-7-ylmethyl]-piperidin-1-carbonsäure-tert-butyl-ester werden in 10 ml Dichlormethan gelöst, mit 1,34 ml Trifluoressigsäure versetzt und 12 Stunden bei Raumtemperatur gerührt. Die Lösungsmittel werden im Vakuum entfernt, der Rückstand in Methanol aufgenommen und mit 1 N Natronlauge auf pH 12 eingestellt. Man lässt eine Stunde rühren, entfernt das Methanol im Vakuum und verteilt den Rückstand zwischen Wasser und Dichlormethan. Anschließend wird die wässrige Phase 2 mal mit Dichlormethan extrahiert. Die vereinigten organischen Phasen werden mit Magnesiumsulfat getrocknet und die Lösungsmittel werden im Vakuum entfernt.
Ausbeute: 1,6 g (119 % der Theorie)
HPLC (Methode 1): Retentionszeit = 2,83 min.

Beispiel XI

4-[2-(5-tert-Butyl-3-methansulfonylamino-2-methoxy-phenylcarbamoyl)-1-methyl-1H-indol-7-ylmethyl]-piperidin-1-carbonsäure tert-butyl ester

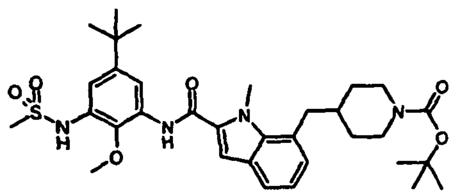

Unter Argon wird eine Mischung aus 620 mg 4-Methylen-piperidin-1-carbonsäure tert-butyl ester und 6,3 ml

einer 0,5 M Lösung von 9-Borabicyclo[3.3.1]nonan in Tetrahydrofuran für eine Stunde auf 80°C erhitzt. Die so hergestellte Lösung wird unter Argon zu einer Mischung aus 1,8 g Trifluoro-methansulfonsäure 2-(5-tert-butyl-3-methansulfonylamino-2-methoxy-phenylcarbamoyl)-1-methyl-1H-indol-7-yl ester, 3,5 g Kaliumcarbonat, und 253 mg [1,1'-Bis(diphenylphosphino)-ferrocen]dichloropalladium(II)-Komplex mit Dichlormethan (1:1) in 12 ml N,N-Dimethylformamid und 1,2 ml Wasser gegeben. Man erhitzt 3 Stunden auf 60°C, entfernt die Lösungsmittel im Vakuum, nimmt in Essigsäureethylester auf und filtriert die unlöslichen Bestandteile ab. Das Filtrat wird 2 mal mit Wasser und 1 mal mit gesättigter wässriger Natriumchloridlösung gewaschen. Nach Trocknen mit Magnesiumsulfat wird das Lösungsmittel im Vakuum entfernt und der Rückstand an Kieselgel mit Cyclohexan/Essigsäureethylester 80:20 auf 20:80 chromatographiert.
Ausbeute: 1,6 g (82 % der Theorie)
HPLC (Methode 1): Retentionszeit = 5,15 min.
Massenspektrum ($ESI^-$): m/z = 625 $[M-H]^-$

Analog Beispiel XI werden folgende Verbindungen erhalten:

(1) 4-[2-(5-tert-Butyl-3-methansulfonylamino-2-methoxy-phenylcarbamoyl)-1-methyl-1H-indol-7-ylmethyl]-azepan-1-carbonsäure-tert-butyl ester

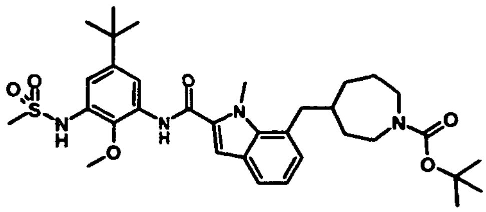

Es wird 7-Brom-1-methyl-1H-indol-2-carbonsäure-(5-tert-butyl-3-methansulfonylamino-2-methoxy-phenyl)-amid statt Trifluoro-methansulfonsäure 2-(5-tert-butyl-3-methansulfonylamino-2-methoxy-phenylcarbamoyl)-1-methyl-1H-indol-7-yl ester und 4-Methylen-azepan-1-carbonsäure-tert-butyl ester statt 4-Methylen-piperidin-1-carbonsäure tert-butyl ester verwendet.
$R_f$-Wert: 0,40 (Kieselgel, Dichlormethan/Methanol 98:2)
Massenspektrum ($ESI^-$): m/z = 639 $[M-H]^-$

(2) 4-[2-(5-tert-Butyl-3-methansulfonylamino-2-methoxy-phenylcarbamoyl)-1-methyl-1H-indol-7-ylmethyl]-piperidin-1-carbonsäure tert-butyl ester

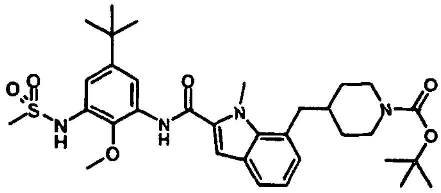

Es wird 7-Brom-1-methyl-1 H-indol-2-carbonsäure-(5-tert-butyl-3-methansulfonylamino-2-methoxy-phenyl)-amid statt Trifluoro-methansulfonsäure 2-(5-tert-butyl-3-methansulfonylamino-2-methoxy-phenylcarbamoyl)-1-methyl-1H-indol-7-yl ester eingesetzt. HPLC (Methode 1): Retentionszeit = 5,14 min.
Massenspektrum ($ESI^-$): m/z = 625 $[M-H]^-$

Beispiel XII

Trifluoro-methansulfonsäure 2-(5-tert-butyl-3-methansulfonylamino-2-methoxy-phenyl-carbamoyl)-1-methyl-1H-indol-7-yl ester

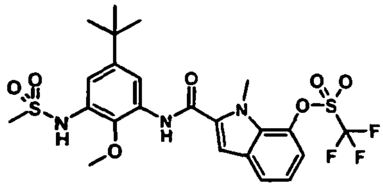

Unter Argon werden 1,75 g 7-Hydroxy-1-methyl-1H-indol-2-carbonsäure (5-tert-butyl-3-methansulfonylamino-2-methoxy-phenyl)-amid (Herstellung siehe US20040186114) in 30 ml Tetrahydrofuran gelöst, nacheinander mit 1,82 g N-Phenyltrifluormethansulfonimid sowie 1,4 ml Triethylamin versetzt und 12 Stunden auf 70°C erhitzt. Danach werden weitere 700 mg N-Phenyltrifluormethansulfonimid sowie 550 μl Triethylamin zugegeben und wiederum 12 Stunden auf 70°C erhitzt. Anschließend werden die Lösungsmittel im Vakuum entfernt und der Rückstand an Kieselgel mit Cyclohexan/Essigsäureethylester 80:20 auf 40:60 chromatographiert.
Ausbeute: 2 g (88 % der Theorie)
HPLC (Methode 1): Retentionszeit = 4,90 min.
Massenspektrum (ESI$^+$): m/z = 578 $[M+H]^+$

Beispiel XIII

(S)-1-Methyl-7-[4-(pyrrolidin-3-carbonyl)-piperazin-1-ylmethyl]-1H-indol-2-carbonsäure (5-tert-butyl-3-methansulfonylamino-2-methoxy-phenyl)-amid

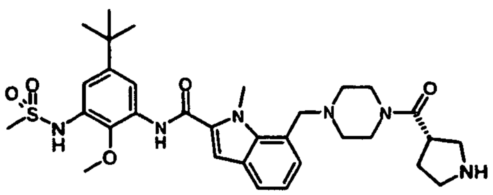

Chiral

220 mg (S)-3-{4-[2-(5-tert-Butyl-3-methansulfonylamino-2-methoxy-phenylcarbamoyl)-1-methyl-1H-indol-7-ylmethyl]-piperazin-1-carbonyl}-pyrrolidin-1-carbonsäure-tert-butyl-ester werden in 5 ml Dichlormethan gelöst, mit 231 μl Trifluoressigsäure versetzt und 12 Stunden bei Raumtemperatur gerührt. Danach werden die Lösungsmittel im Vakuum entfernt, zwischen gesättigter wässriger Natriumhydrogencarbonatlösung und Dichlormethan verteilt und 30 Minuten heftig gerührt. Die Phasen werden getrennt, die wässrige Phase 2 mal mit Dichlormethan extrahiert und die vereinigten organischen Phasen mit Magnesiumsulfat getrocknet. Anschließend werden die Lösungsmittel im Vakuum entfernt und das Rohprodukt an Kieselgel (Dichlormethan/(Methanol-konz. Ammoniak 9:1) 90:10 auf 80:20) chromatographiert.
Ausbeute: 104 mg (55 % der Theorie)
HPLC (Methode 1): Retentionszeit = 2,36 min.
Massenspektrum (ESI$^+$): m/z = 625 $[M+H]^+$

Analog Beispiel XIII werden folgende Verbindungen erhalten:

(1) 7-Azepan-4-ylmethyl-1-methyl-1H-indol-2-carbonsäure-(5-tert-butyl-3-methansulfonylamino-2-methoxy-phenyl)-amid

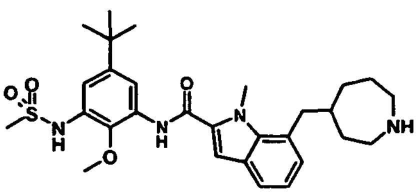

Es wird gesättigte Natriumcarbonatlösung statt gesättigter wässriger Natriumhydrogencarbonatlösung verwendet.
$R_f$-Wert: 0,20 (Kieselgel, Dichlormethan/Methanol/(Ammoniak in Methanol (gesättigt)) 90:10:0,1)
Massenspektrum (ESI$^+$): m/z = 541 $[M+H]^+$

(2) 7-[1,4]Diazepan-1-ylmethyl-1-methyl-1H-indol-2-carbonsäure-(5-tert-butyl-3-methansulfonylamino-2-methoxy-phenyl)-amid

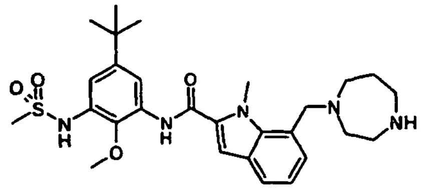

Es wird gesättigte Natriumcarbonatlösung statt gesättigter wässriger Natriumhydrogencarbonatlösung verwendet.
$R_f$-Wert: 0,10 (Kieselgel, Dichlormethan/Methanol/(Ammoniak in Methanol (gesättigt)) 95:5:0,1)
Massenspektrum (ESI$^+$): m/z = 542 $[M+H]^+$

(3) (S)-1-Methyl-7-[4-(pyrrolidin-3-carbonyl)-[1,4]diazepan-1-ylmethyl]-1H-indol-2-carbonsäure-(5-tert-butyl-3-methansulfonylamino-2-methoxy-phenyl)-amid

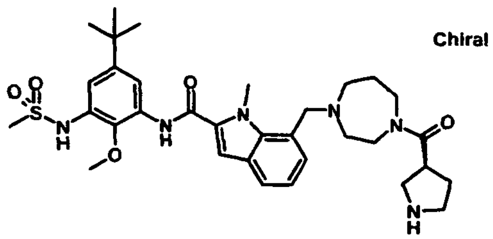

Es wird gesättigte Natriumcarbonatlösung statt gesättigter wässriger Natriumhydrogencarbonatlösung verwendet.
$R_f$-Wert: 0,10 (Kieselgel, Dichlormethan/Methanol/(Ammoniak in Methanol (gesättigt)) 95:5:0,1)

(4) (R)-1-Methyl-7-[4-(pyrrolidin-3-carbonyl)-[1,4]diazepan-1-ylmethyl]-1H-indol-2-carbonsäure-(5-tert-butyl-3-methansulfonylamino-2-methoxy-phenyl)-amid

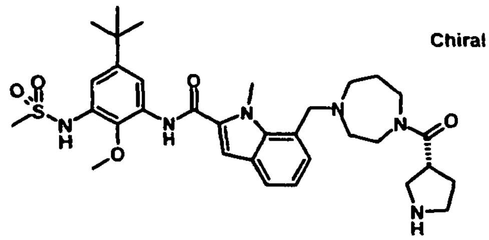

Es wird gesättigte Natriumcarbonatlösung statt gesättigter wässriger Natriumhydrogencarbonatlösung verwendet.
$R_f$-Wert: 0,10 (Kieselgel, Dichlormethan/Methanol/(Ammoniak in Methanol (gesättigt)) 95:5:0,1)

Beispiel XIV

(2R,4R)-4-Methoxy-pyrrolidin-2-carbonsäure Hydrochlorid

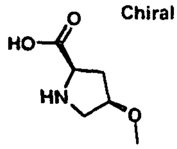

8,72 g (2R,4R)-4-Methoxy-pyrrolidin-1,2-dicarbonsäure 1-tert-butyl ester werden in 60 ml Tetrahydrofuran gelöst, tropfenweise mit 60 ml einer 6 M wässrigen Salzsäurelösung versetzt und 4 Stunden bei Raumtemperatur gerührt. Anschließend werden die Lösungsmittel im Vakuum entfernt und der Rückstand 2 mal in je 100 ml Acetonitril aufgenommen und dieses jeweils wieder im Vakuum entfernt.
Ausbeute: 6,91 g (107 % der Theorie)
Massenspektrum ($ESI^+$): m/z = 146 $[M+H]^+$

Beispiel XV

(2R,4R)-4-Methoxy-pyrrolidin-1,2-dicarbonsäure 1-tert-butyl ester

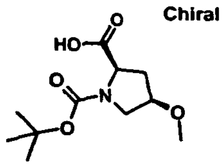

13,72 g (2R,4R)-4-Hydroxy-pyrrolidin-1,2-dicarbonsäure 1-tert-butyl ester werden in 150 ml N,N-Dimethylformamid gelöst, auf 0°C gekühlt und portionsweise mit 7,12 g einer 50%-igen Dispersion von Natriumhydrid in Paraffinöl versetzt. Man lässt 20 Minuten bei Raumtemperatur rühren und tropft dann 14,77 ml Methyliodid hinzu. Danach lässt man 18 Stunden Rühren, tropft dann weitere 1,47 ml Methyliodid zu und lässt weitere 6 Stunden Rühren. Anschließend wird mit Wasser verdünnt, die wässrige Phase 2 mal mit Essigsäureethylester extrahiert und die vereinigten organischen Phasen werden mit gesättigter wässriger Natriumchloridlösung gewaschen. Nach Trocknen mit Magnesiumsulfat werden die Lösungsmittel im Vakuum entfernt. Der Rückstand wird in 600 ml Methanol gelöst, mit 74 ml 4 M wässriger Natronlauge versetzt und 3 Tage bei Raumtemperatur gerührt. Nach Entfernen des Methanols im Vakuum wird der Rückstand mit Wasser verdünnt und mit gesättigter wässriger Kaliumhydrogensulfatlösung angesäuert. Es wird 3 mal mit Essigsäureethylester extrahiert, dann werden die vereinigten organischen Phasen mit Natriumsulfat getrocknet und anschließend die Lösungsmittel im Vakuum entfernt. Dann wird wieder in 500 ml Methanol aufgenommen, mit 74 ml 4 M wässriger Natronlauge versetzt und 12 Stunden gerührt. Man gibt weitere 74 ml 4 M wässrige Natronlaugen hinzu und erwärmt für 5 Stunden auf 50°C. Man entfernt etwa 200 ml Methanol im Vakuum und lässt weitere 12 Stunden Rühren. Anschließend wird das restliche Methanol im Vakuum entfernt, der Rückstand mit Wasser verdünnt und 1 mal mit Diethylether gewaschen. Die wässrige Phase wird mit gesättigter wässriger Kaliumhydrogensulfatlösung angesäuert und 4 mal mit Essigsäureethylester extrahiert. Die vereinigten organischen Phasen werden mit Natriumsulfat getrocknet und die Lösungsmittel werden im Vakuum entfernt. Der Rückstand wird durch präparative HPLC (Methode 4; 500 mg pro Injektion) gereinigt.
Ausbeute: 7,97 g (55 % der Theorie)
Massenspektrum ($ESI^-$): m/z = 244 $[M-H]^-$

Beispiel XVI

4-[2-(5-tert-Butyl-3-methansulfonylamino-2-methoxy-phenylcarbamoyl)-1-methyl-1H-indol-7-ylmethyl]-piperazin-1-carbonsäure-tert-butyl ester

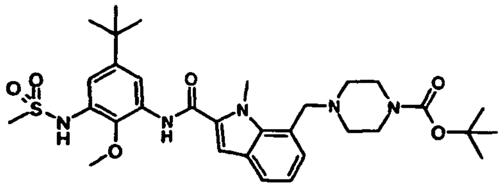

8,99 g 7-Brom-1-methyl-1H-indol-2-carbonsäure-(5-tert-butyl-3-methansulfonylamino-2-methoxy-phenyl)-amid und 5,95 g Kalium ((4-(tert.-butoxycarbonyl)-piperazin-1-yl)-methyl)-trifluoroborat (Herstellung: Org. Lett. 2007, 9, 1597-1600) werden in 150 ml Tetrahydrofuran und 15 ml Wasser gelöst, mit 17,3 g Cäsiumcarbonat versetzt und durch die erhaltene Lösung für 10 Minuten Argon geleitet. Dann werden 120 mg Palladium-II-acetat und 1,23 g 2-Dicyclohexylphosphino-2',4',6'-triisopropy,-1,1'-biphenyl, (XPhos) zugegeben und 12 Stunden auf 80°C erhitzt. Anschließend wird zwischen Wasser und Essigsäureethylester verteilt und die organische Phase mit gesättigter wässriger Natriumchloridlösung gewaschen. Nach Trocknen mit Magnesiumsulfat werden die Lösungsmittel im Vakuum entfernt. Das Rohprodukt wird direkt weiter umgesetzt.
Ausbeute: 13,5 g (97 % der Theorie)
HPLC (Methode 1): Retentionszeit = 3,44 min.
Massenspektrum (ESI$^+$): m/z = 628 [M+H]$^+$
Analog Beispiel XVI werden folgende Verbindungen erhalten:

(1) 4-[2-(5-Isobutyl-3-methansulfonylamino-2-methoxy-phenylcarbamoyl)-1-methyl-1H-indol-7-ylmethyl]-piperazin-1-carbonsäure-tert-butyl ester

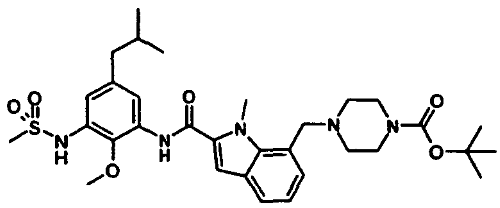

HPLC (Methode 1): Retentionszeit = 3,55 min.
Massenspektrum (ESI$^+$): m/z = 628 [M+H]$^+$

Beispiel XVII

7-Brom-1-methyl-1H-indol-2-carbonsäure-15-tert-butyl-3-methansulfonylamino-2-methoxy-phenyl)-amid

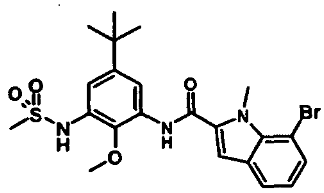

Unter Argon werden 6,15 g 7-Brom-1-methyl-1H-indol-2-carbonsäure in 60 ml Tetrahydrofuran gelöst, mit 16,8 Triethylamin sowie 19 ml einer 50 %-igen Lösung von n-Propylphosphonsäureanhydrid in Essigsäureethylester versetzt und 45 Minuten bei 0°C gerührt. Anschließend werden 6,59 g N-(3-Amino-5-tert-butyl-2-methoxy-phenyl)-methansulfonamid portionsweise zugegeben. Man läßt auf Raumtemperatur erwärmen und 2,5 Stunden nachrühren. Danach werden die Lösungsmittel im Vakuum entfernt und der Rückstand wird zwischen gesättigter wässriger Natriumhydrogencarbonatlösung und Essigsäureethylester verteilt. Dabei fällt ein Niederschlag aus, der abfiltriert und getrocknet wird (Feststoff 1; 6,29 g). Die Phasen werden getrennt und die wässrige Phase wird mit Essigsäureethylester extrahiert. Die vereinigten organischen Phasen werden mit Wasser und gesättigter wässriger Natriumchloridlösung gewaschen und anschließend mit Magnesiumsulfat getrocknet. Die Lösungsmittel werden im Vakuum entfernt und der Rückstand aus Ethanol ausgerührt. Der Niederschlag wird abfiltriert, mit wenig Ethanol gewaschen und getrocknet (Feststoff 2; 2,7 g).
Ausbeute: 8,99 g (73 % der Theorie) (Feststoff 1 + 2)
HPLC (Methode 1): Retentionszeit = 4,80 min.
Massenspektrum ($ESI^+$): m/z = 508 $[M+H]^+$

Analog Beispiel XVII werden folgende Verbindungen erhalten:

(1) (S)-4-(1-Methyl-pyrrolidin-2-carbonyl)-piperazin-1-carbonsäure-benzyl ester

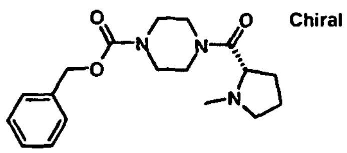

Als Reaktionspartner werden Piperazin-1-carbonsäure-benzyl ester und (S)-1-Methylpyrrolidin-2-carbonsäure verwendet. Nach wässriger Aufarbeitung wird das Rohprodukt durch Evaporation der organischen Phase erhalten und direkt weiter umgesetzt.
$R_f$-Wert: 0,50 (Kieselgel, Dichlormethan/Methanol/(Ammoniak in Methanol (gesättigt)) 90:10:0,1)
Massenspektrum ($ESI^+$): m/z = 332 $[M+H]^+$

(2) 7-Bromo-1-methyl-1H-indol-2-carbonsäure-(5-isobutyl-3-methansulfonylamino-2-methoxy-phenyl)-amid

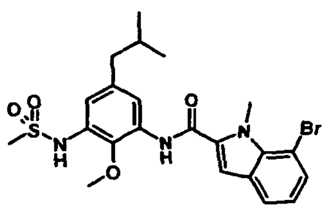

HPLC (Methode 1): Retentionszeit = 4,96 min.
Massenspektrum ($ESI^+$): m/z = 508 $[M+H]^+$

<u>Beispiel XVIII</u>

<u>7-Brom-1-methyl-1H-indol-2-carbonsäure</u>

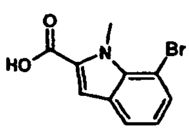

7,37 g 7-Brom-1H-indol-2-carbonsäure-ethyl ester werden 50 ml N,N-Dimethylformamid gelöst, auf 0°C gekühlt und mit 1,16 g Natriumhydrid (60 % in Mineralöl) versetzt. Man läßt 20 Minuten rühren und tropft dann 1,78 ml Methyliodid zu. Danach läßt man auf Raumtemperatur erwärmen und 12 Stunden rühren. Anschließend werden 80 ml Methanol und 27,5 ml 2 N Natronlauge zugegeben und 3 Stunden bei Raumtemperatur gerührt. Das Methanol wird im Vakuum entfernt, der Rückstand mit Wasser versetzt und der Niederschlag abgesaugt. Das Filtrat wird 2 mal mit Essigsäureethylester gewaschen und die wässrige Phase wird mit so viel 2 N Salzsäure versetzt, bis ein pH von 2 erreicht ist. Der ausgefallene Feststoff wird abgesaugt, mit Wasser nachgewaschen und getrocknet.
Ausbeute: 4,26 g (61 % der Theorie)
Massenspektrum ($ESI^-$): m/z = 252 $[M-H]^-$

Beispiel XIX

7-Brom-1H-indol-2-carbonsäure-ethyl ester

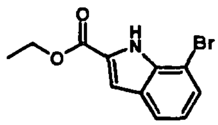

11,0 g 2-Bromphenylhydrazin und 550 mg p-Toluolsulfonsäure Monohydrat werden in 200 ml Toluol gelöst, mit 6,74 ml Brenztraubensäure-ethyl ester versetzt und 2 Stunden am Wasserabscheider zum Rückfluß erhitzt. Man läßt auf 40°C abkühlen und versetzt mit einer Lösung die durch lösen von 44,75 g p-Toluolsulfonsäure Monohydrat in 300 ml Toluol und zweistündiges Erhitzen zum Rückfluß am Wasserabscheider erhalten wird. Anschließend wird für 12 Stunden am Wasserabscheider zum Rückfluß erhitzt. Nach Abkühlen auf Raumtemperatur werden die Lösungsmittel im Vakuum entfernt, der Rückstand in Essigsäureethylester aufgenommen und nacheinander mit Wasser und gesättigter wässriger Natriumhydrogencarbonatlösung gewaschen. Nach Trocknen mit Magnesiumsulfat wird mit Aktivkohle versetzt, 15 Minuten gerührt und über Kieselgur abfiltriert. Die Prozedur der Zugabe von Aktivkohle, Rühren und Filtration wird noch 2 mal wiederholt. Die Lösungsmittel werden im Vakuum entfernt und der Rückstand in Petrolether/Dichlormethan 7:3 aufgenommen. Man gibt 30 g Kieselgel hinzu, läßt 10 Minuten Rühren und saugt dann über Kieselgur ab. Das abgesaugte Kieselgel wird mit Petrolether/Dichlormethan 7:3 nachgewaschen. Die Lösungsmittel werden im Vakuum entfernt.
Ausbeute: 7,37 g (47 % der Theorie)
HPLC (Methode 1): Retentionszeit = 3,82 min.
Massenspektrum ($ESI^+$): m/z = 268 $[M+H]^+$

Beispiel XX

2-Bromphenylhydrazin

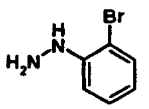

15 g 2-Bromphenylhydrazin Hydrochlorid werden in 400 ml Toluol suspendiert und mit 240 ml 1 N Natronlauge versetzt. Man läßt 1 Stunde rühren und trennt die Phasen. Die organische Phase wird mit Magnesiumsulfat getrocknet und die Lösungsmittel werden im Vakuum entfernt. Das so erhaltene Rohprodukt wird direkt weiter umgesetzt.
Ausbeute: 12,12 g (99 % der Theorie)
Massenspektrum ($ESI^+$): m/z = 187 $[M+H]^+$

Beispiel XXI

4-(2-{4-[2-(5-tert-Butyl-3-methansulfonylamino-2-methoxy-phenylcarbamoyl)-1-methyl-1H-indol-7-ylmethyl]piperazin-1-yl}-2-oxo-ethyl)-piperazin-1-carbonsäure-tert-butyl ester

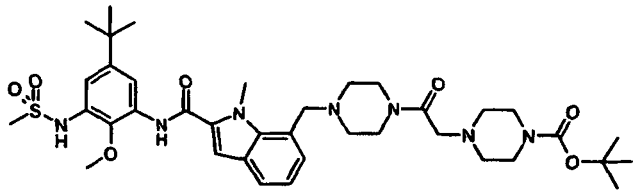

110 mg 4-Carboxymethyl-piperazin-1-carbonsäure-tert-butyl ester werden in 2 ml N,N-Dimethylformamid gelöst, mit 135 mg O-(Benzotriazol-1-yl)-N,N,N',N'-tetramethyluronium-tetrafluoroborat (TBTU) sowie 270 µl N,N-Diisopropyl-ethylamin (DIEA) versetzt und 20 Minuten gerührt. Anschließend werden 200 mg 1-Methyl-7-piperazin-1-ylmethyl-1H-indol-2-carbonsäure-(5-tert-butyl-3-methansulfonylamino-2-methoxy-phenyl)-amid Dihydrochlorid zugegeben, 12 Stunden bei Raumtemperatur gerührt und dann zwischen Wasser und Essigsäureethylester verteilt. Die organische Phase wird mit gesättigter wässriger Natriumchloridlösung gewaschen und mit Magnesiumsulfat getrocknet. Die Lösungsmittel werden im Vakuum entfernt und der Rückstand an Kieselgel (Dichlormethanl(Metahnollkonz. Ammoniak 9:1) 99:1 auf 90:10) chromatographiert.
Ausbeute: 160 mg (64 % der Theorie)
HPLC (Methode 1): Retentionszeit = 2,79 min.
Massenspektrum ($ESI^+$): m/z = 754 $[M+H]^+$

Beispiel XXII

1-Methyl-7-piverazin-1-ylmethyl-1H-indol-2-carbonsäure (5-tert-butyl-3-methansulfonylamino -2-methoxy-phenyl)-amid Dihydrochlorid

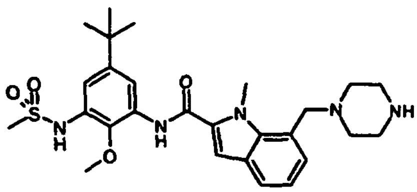

2,29 g 4-[2-(5-tert-Butyl-3-methansulfonylamino-2-methoxy-phenylcarbamoyl)-1-methyl-1H-indol-7-ylmethyl]-piperazin-1-carbonsäure-tert-butyl ester werden in 25 ml einer 5 M Lösung von HCl in Isopropanol gelöst und 12 Stunden bei Raumtemperatur gerührt. Anschließend werden die Lösungsmittel im Vakuum entfernt und der Rückstand 2 mal mit je 20 ml Methanol aufgenommen und dieses wieder im Vakuum entfernt.
Ausbeute: 2,03 g (105 % der Theorie)
Massenspektrum ($ESI^+$): m/z = 528 $[M+H]^+$

Beispiel XXIII

4-Methylen-azepan-1-carbonsäure-tert-butyl ester

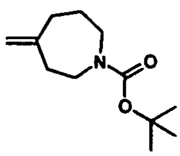

Unter Argon werden 12,56 g Methyltriphenylphosphoniumbromid in 100 ml Tetrahydrofuran gelöst, auf -14°C gekühlt und innerhalb von 45 Minuten eine 2 M Lösung von 17,58 ml n-Butyllithium in Hexan zugetropft. Man läßt 1 Stunde nachrühren und tropft dann eine Lösung von 5 g 4-Oxo-azepan-1-carbonsäure-tert-butyl ester in 20 ml Tetrahydrofuran zu. Danach läßt man auf Raumtemperatur erwärmen und 12 Stunden rühren. Anschließend wird zwischen Wasser und Hexan verteilt und die wässrige Phase wird 2 mal mit Hexan extrahiert. Die vereinigten organischen Phasen werden mit gesättigter wässriger Natriumchloridlösung gewaschen und mit Magnesiumsulfat getrocknet. Die Lösungsmittel werden im Vakuum entfernt und der Rückstand mit Petrolether/tert.-Butyl-methyl-ether verrührt. Der Feststoff wird abgesaugt und die Mutterlauge im Vakuum eingeengt. Der Rückstand wird an Kieselgel (Petrolether/ Essigsäureethylester 100:0, dann 95:5) chromatographiert.
Ausbeute: 4,2 g (85 % der Theorie)
$R_f$-Wert: 0,35 (Kieselgel, Petrolether/ Essigsäureethylester 95:5)
Massenspektrum (ESI$^+$): m/z = 212 [M+H]$^+$

Beispiel XXIV

7-Hydroxvmethyl-1-methyl-1H-indol-2-carbonsäure-(5-tert-butyl-3-methansulfonylamino-2-methoxy-phenyl)-amid

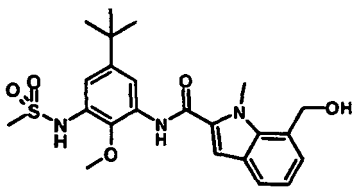

Unter Argon werden 14,7 g 2-(5-tert-Butyl-3-methansulfonylamino-2-methoxy-phenylcarbamoyl)-1-methyl-1H-indol-7-carbonsäure-methyl ester in 120 ml Tetrahydrofuran gelöst, auf 0°C gekühlt und tropfenweise mit 60 ml einer 1 M Lösung von Lithiumaluminiumhydrid in Tetrahydrofuran versetzt. Man läßt 2 Stunden rühren und beendet dann die Reaktion durch tropfenweise Zugabe von 30 ml 2 N Natronlauge. Danach läßt man 30 Minuten rühren, verdünnt mit Essigsäureethylester und filtriert über Kieselgur. Der Filterkuchen wird in 60 ml Tetrahydrofuran aufgenommen, 10 Minuten gerührt und wieder über Kieselgur abgesaugt. Die vereinigten organischen Phasen werden mit Magnesiumsulfat getrocknet und im Vakuum eingeengt. Der Rückstand wird aus Methanol ausgerührt. Der ausgefallene Feststoff wird abfiltriert und getrocknet.
Ausbeute: 8,2 g (59 % der Theorie)
Massenspektrum (ESI$^-$): m/z = 458 [M-H]$^-$

Beispiel XXV

2-(5-tert-Butyl-3-methansulfonylamino-2-methoxy-phenylcarbamoyl)-1-methyl-1H-indol-7-carbonsäure-methyl ester

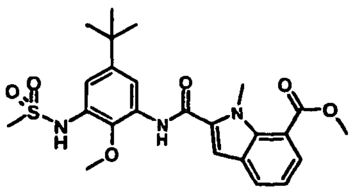

Durch eine Suspension von 18,8 g 7-Brom-1-methyl-1H-indol-2-carbonsäure-(5-tert-butyl-3-methansulfonyl-amino-2-methoxy-phenyl)-amid werden in 250 ml Methanol und 50 ml N,N-Dimethylformamid wird 10 Minuten Argon geleitet. Dann werden 7,2 ml Triethylamin und 2,3 g [1,1'-Bis(diphenylphosphino)ferrocen]-palladium-II-dichlorid-Dichlormethan-Komplex zugegeben und anschließend für 15 Stunden bei 80°C 2 bar Kohlenmonoxid aufgedrückt. Danach werden die Lösungsmittel im Vakuum entfernt, der Rückstand in Essigsäureethylester aufgenommen und mit 1 N Salzsäure, gesättigter wässriger Natriumhydrogencarbonatlösung und gesättigter wässriger Natriumchloridlösung gewaschen. Nach Trocknen mit Magnesiumsulfat wird das Lösungsmittel im Vakuum entfernt und der Rückstand aus Methanol ausgerührt. Der Feststoff wird abgesuagt, mit wenig Methanol gewaschen und getrocknet.
Ausbeute: 14,7 g (81 % der Theorie)
Massenspektrum (ESI$^+$): m/z = 488 $[M+H]^+$

Beispiel XXVI

N-(3-Amino-5-tert-butyl-2-methoxy-phenyl)-methansulfonamid

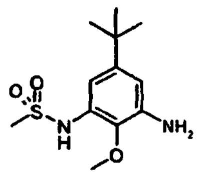

50 g N-(3-Amino-5-tert-butyl-2-methoxy-phenyl)-methansulfonamid Hydrochlorid (J. Med. Chem. 2007, 50 (17), 4016-26) werden in 200 ml Wasser und 600 ml Essigsäureethylester suspendiert und unter heftigem Rühren mit so viel 4 N Natronlauge versetzt bis ein pH-Wert von 11 erreicht ist. Anschließend werden die Phasen getrennt, die wässrige Phase wird mit Essigsäureethylester extrahiert und die vereinigten organischen Phasen werden mit Magnesiumsulfat getrocknet und im Vakuum eingeengt. Der so erhaltene Feststoff wird direkt weiter umgesetzt.
Ausbeute: 44 g (100 % der Theorie)

Beispiel XXVII

(S)-1-Methyl-7-[4-(1-methyl-pyrrolidin-2-carbonyl)-piperazin-1-ylmethyl]-1H-indol-2-carbonsäure-(3-amino-5-tert-butyl-2-methoxy-phenyl)-amid Trihvdrochlorid

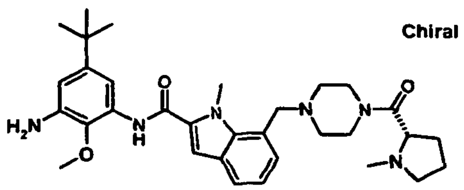

1,59 g [5-tert-Butyl-2-methoxy-3-({1-methyl-7-[4-(1-methyl-pyrrolidin-2-carbonyl)-piperazin-1-ylmethyl]-1H-indol-2-carbonyl}-amino)-phenyl]-carbaminsäure-tert-butyl ester werden in 15 ml Dichlormethan gelöst, mit 10 ml einer 5 M Lösung von Chlorwasserstoff in Isopropanol versetzt und 3 Stunden bei Raumtemperatur gerührt. Anschließend werden die Lösungsmittel im Vakuum entfernt. Das so erhaltene Produkt wird direkt weiter umgesetzt.
Ausbeute: 1,80mg (112 % der Theorie)
HPLC (Methode 1): Retentionszeit = 2,38 min.
Massenspektrum (ESI$^+$): m/z = 561 $[M+H]^+$

Analog Beispiel XXVII werden folgende Verbindungen erhalten:

(1) 1-Methyl-7-piperazin-1-ylmethyl-1H-indol-2-carbonsäure-(5-isobutyl-3-methansulfonylamino-2-methoxy-phenyl)-amid

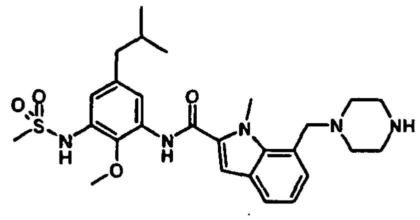

Es wird 4-[2-(5-Isobutyl-3-methansulfonylamino-2-methoxy-phenylcarbamoyl)-1-methyl-1H-indol-7-ylmethyl]-piperazin-1-carbonsäure-tert-butyl ester statt [5-tert-Buty1-2-methoxy-3-({1-methyl-7-[4-(1-methyl-pyrrolidin-2-carbonyl)-piperazin-1-ylmethyl)-1H-indol-2-carbonyl}-amino)-phenyl]-carbaminsäure-tert-butyl ester eingesetzt. Das Rohprodukt wird zwischen Dichlormethan und gesättigter wässriger Natriumhydrogencarbonatlösung verteilt. Die wässrige Phase wird mit Dichlormethan extrahiert. Die vereinigten organischen Phasen werden mit Magnesiumsulfat getrocknet und die Lösungsmittel werden im Vakuum entfernt. Das so erhaltene Produkt wird direkt weiter umgesetzt.
HPLC (Methode 1): Retentionszeit = 2,95 min.
Massenspektrum ($ESI^+$): m/z = 528 $[M+H]^+$

Beispiel XXVIII

1-[2-(5-tert-Butyl-3-methansulfonylamino-2-methoxy-phenylcarbamoyl)-1-methyl-1H-indol-7-ylmethyl]-piperidin-4-carbonsäure

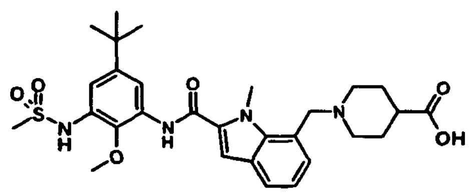

1,3 g 1-[2-(5-tert-Butyl-3-methansulfonylamino-2-methoxy-phenylcarbamoyl)-1-methyl-1H-indol-7-ylmethyl]-piperidin-4-carbonsäure-tert-butyl ester werden in 20 ml Dichlormethan gelöst, mit 1,04 ml einer 4 M Lösung von Chlorwasserstoff in 1,4-Dioxan versetzt und 12 Stunden gerührt. Es werden weitere 2 ml einer 4 M Lösung von Chlorwasserstoff in 1,4-Dioxan zugegeben und für weitere 24 Stunden gerührt. Die Lösungsmittel werden im Vakuum entfernt und der Rückstand wird 2 mal in Dichlormethan aufgenommen und dieses wieder im Vakuum entfernt. Das so erhaltene Produkt wird direkt weiter umgesetzt.
Ausbeute: 1,36 g (86 % der Theorie)
HPLC (Methode 1): Retentionszeit = 2,70 min.
Massenspektrum ($ESI^+$): m/z = 571 $[M+H]^+$

Beispiel XXIX

7-Formyl-1-methyl-1H-indol-2-carbonsäure

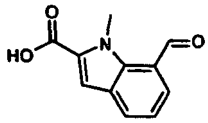

Unter Argon werden 4,14 g 7-Brom-1-methyl-1H-indol-2-carbonsäure in 36 ml Tetrahydrofuran und 5 ml 1,4-Dioxan gelöst, auf -10°C gekühlt und tropfenweise mit 37,6 ml einer 1,3 M Lösung von Isopropylmagnesiumchlorid-Lithiumchlorid-Komplex in Tetrahydrofuran versetzt. Man läßt langsam auf Raumtemperatur kommen und 12 Stunden rühren. Anschließend wird auf -10°C gekühlt und tropfenweise mit 6,62 ml N,N-Dimethylformamid versetzt. Man läßt 30 Minuten rühren und beendet dann die Reaktion durch Zugabe von Wasser. Die organischen Lösungsmittel werden zum Großteil im Vakuum entfernt und der pH-Wert des Rückstands durch Zugabe von 4 N Salzsäure auf 2 eingestellt. Der ausgefallene Feststoff wird abgesaugt, in Tetrahydrofuran aufgenommen und mit Magnesiumsulfat getrocknet. Das Lösungsmittel wird im Vakuum entfernt und der so erhaltene Feststoff wird direkt weiter umgesetzt.
Ausbeute: 1,95 g (59 % der Theorie)
HPLC (Methode 1): Retentionszeit = 2,80 min.
Massenspektrum (ESI$^+$): m/z = 204 $[M+H]^+$

Beispiel XXX

(S)-(1-Methyl-Pyrrolidin-2-yl)-piperazin-1-yl-methanon

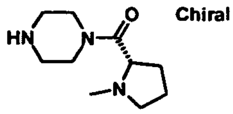

13,43 g (S)-4-(1-Methyl-pyrrolidin-2-carbonyl)-piperazin-1-carbonsäure-benzyl ester werden in 100 ml Methanol gelöst, mit 1,4 g 10 % Palladium auf Kohle versetzt und 6 Stunden bei Raumtemperatur und 3 bar Wasserstoffdruck hydriert. Anschließend wird vom Katalysator abfiltriert und die Lösungsmittel werden im Vakuum entfernt. Das so erhaltene Produkt wird direkt weiter umgesetzt.
Ausbeute: 7,95 g (99 % der Theorie)
$R_f$-Wert: 0,20 (Kieselgel, Dichlormethan/Methanol/(Ammoniak in Methanol (gesättigt)) 90:10:0,1)
Massenspektrum (ESI$^+$): m/z = 198 $[M+H]^+$

Beispiel XXXI

(S)-1-Methyl-7-[4-(1-methyl-pyrrolidin-2-carbonyl)-piperazin-1-ylmethyl]-1H-indol-2-carbonsäure-(5-tert-butyl-3-formyl-2-methoxy-phenyl)-amid

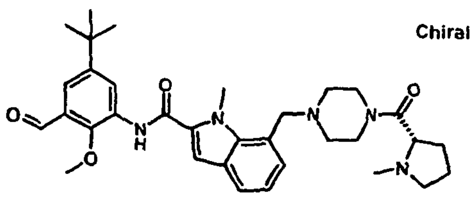

200 mg (S)-1-Methyl-7-[4-(1-methyl-pyrrolidin-2-carbonyl)-piperazin-1-ylmethyl]-1H-indol-2-carbonsäure-(5-tert-butyl-3-hydroxymethyl-2-methoxy-phenyl)-amid werden in 5 ml Dichlormethan-gelöst, auf 0°C gekühlt und mit 167 mg 1,1-Dihydro-1,1,1-triacetoxy-1,2-benziodoxol-3(1H)-on (Dess-Martin-Periodinan) versetzt. Man läßt 12 Stunden rühren, wobei auf Raumtemperatur erwärmt wird. Anschließend wird zwischen Dichlormethan und einer 10 %-igen wässrigen Kaliumcarbonatlösung verteilt. Dabei fällt ein Feststoff aus, der abfiltriert wird. Die flüssigen Phasen werden getrennt und die organische Phase wird mit Magnesiumsulfat getrocknet. Die Lösungsmittel werden dann im Vakuum entfernt.

Das so erhaltene Produkt wird direkt weiter umgesetzt.
Ausbeute: 220 mg (110 % der Theorie)
HPLC (Methode 1): Retentionszeit = 2,80 min.

Beispiel XXXII

(S)-1-Methyl-7-[4-(1-methyl-pyrrolidin-2-carbonyl)-piperazin-1-ylmethyl]-1H-indol-2-carbonsäure-(5-tert-butyl-3-hydroxymethyl-2-methoxy-phenyl)-amid

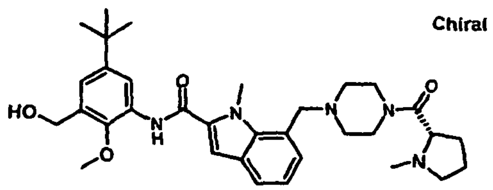

1,73 g (S)-1-Methyl-7-[4-(1-methyl-pyrrolidin-2-carbonyl)-piperazin-1-ylmethyl]-1H-indol-2-carbonsäure-(5-tert-butyl-2-methoxy-3-trimethylsilanyloxymethyl-phenyl)-amid werden in 15 ml Dichlormethan gelöst, mit 5,34 ml einer 5 M Lösung von Chlorwasserstoff in Isopropanol versetzt und 30 Minuten bei Raumtemperatur gerührt. Man verteilt zwischen Dichlormethan und gesättigter wässriger Natriumhydrogencarbonatlösung, extrahiert die wässrige Phase 2 mal mit Dichlormethan und wäscht die vereinigten organischen Phasen mit gesättigter wässriger Natriumchloridlösung. Nach Trocknen mit Magnesiumsulfat werden die Lösungsmittel im Vakuum entfernt und das so erhaltene Produkt direkt weiter umgesetzt.
Ausbeute: 1,67 g (109 % der Theorie)
Massenspektrum (ESI$^+$): m/z = 576 $[M+H]^+$

Beispiel XXXIII

(S)-1-Methyl-7-[4-(1-methyl-pyrrolidin-2-carbonyl)piperazin-1-ylmethyl]-1H-indol-2-carbonsäure-(5-tert-butyl-2-methoxy-3-trimethylsilanyloxymethyl-phenyl)-amid

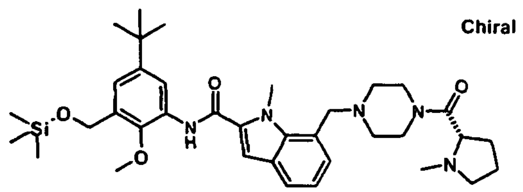

2 g (S)-1-Methyl-7-[4-(1-methyl-pyrrolidin-2-carbonyl)-piperazin-1-ylmethyl]-1H-indol-2-carbonsäure werden in 20 ml Acetonitril gelöst, mit 1,2 ml Oxalylchlorid sowie 10 µl N,N-Dimethylformamid versetzt und 2 Stunden gerührt. Dann werden weitere 450 µl Oxalylchlorid zugesetzt und eine weitere Stunde gerührt. Anschließend wird im Vakuum eingeengt, in Dichlormethan aufgenommen und dieses wieder im Vakuum entfernt. Der Rückstand wird in 20 ml 1,2-Dichlorethan aufgenommen, mit 2,85 ml Triethylamin sowie 1,17 g 5-tert-Butyl-2-methoxy-3-trimethylsilanyloxymethyl-phenylamin versetzt und 2 Stunden bei Raumtemperatur gerührt. Dann wird nacheinander mit Wasser und gesättigter wässriger Natriumchloridlösung gewaschen, mit Magnesiumsulfat getrocknet und die Lösungsmittel werden im Vakuum entfernt. Der Rückstand wird über Aluminiumoxid mit Dichlormethan/Essigsäureethylester filtriert.
Ausbeute: 1,73 g (64 % der Theorie)
HPLC (Methode 1): Retentionszeit = 2,43 min.
Massenspektrum (ESI$^+$): m/z = 648 $[M+H]^+$
Analog Beispiel XXXIII werden folgende Verbindungen erhalten:

(1) 5-tert-Butyl-2-methoxy-3-({1-methyl-7-[4-(1-methyl-pyrrolidin-2-carbonyl)-piperazin-1-ylmethyl]-1H-indol-2-carbonyl}-amino)-benzoesäure-methyl

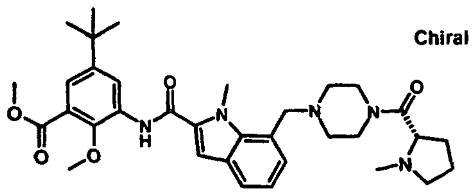

HPLC (Methode 1): Retentionszeit = 2,90 min.
Massenspektrum (ESI$^+$): m/z = 604 $[M+H]^+$

(2) (S)-[5-tert-Butyl-2-methoxy-3-({1-methyl-7-[4-(1-methyl-pyrrolidin-2-carbonyl)-piperazin-1-ylmethyl]-1H-indol-2-carbonyl}-amino)-phenyl]-carbaminsäure-tert-butyl ester

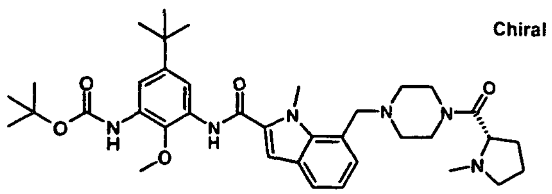

HPLC (Methode 1): Retentionszeit = 3,20 min.
Massenspektrum (ESI$^+$): m/z = 661 $[M+H]^+$

(3) (S)-1-Methyl-7-[4-(1-methyl-pyrrolidin-2-carbonyl)-piperazin-1-ylmethyl]-1H-indol-2-carbonsäure-(3-amino-2-methoxy-5-pentafluorethyl-phenyl)-amid

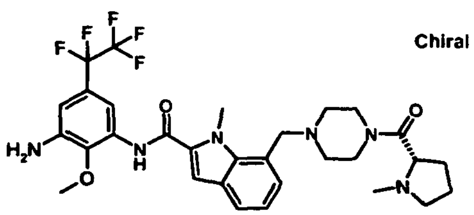

Das Rohprodukt wird an Kieselgel (Dichlormethan/(Methanol/Ammoniak in Methanol (gesättigt) 9:1) 99:1 auf 90:10) chromatographiert und das so erhaltene Produkt wird durch präparative HPLC (Methode 3) gereinigt.
HPLC (Methode 1): Retentionszeit = 2,76 min.

(4) (S)-1-Methyl-7-[4-(1-methyl-pyrrolidin-2-carbonyl)-piperazin-1-ylmethyl]-1H-indol-2-carbonsäure-(3-amino-2-methoxy-5-trifluormethyl-phenyl)-amid

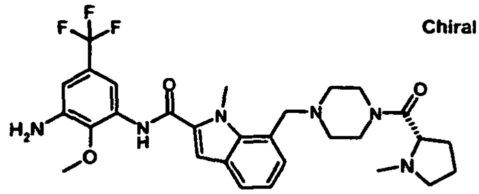

Das Rohprodukt wird an Kieselgel (Dichlormethan/(Methanol/Ammoniak in Methanol (gesättigt) 9:1) 99:1 auf 90:10) chromatographiert und das so erhaltene Produkt wird durch präparative HPLC (Methode 3) gereinigt.
HPLC (Methode 6): Retentionszeit = 4,00 min.
Massenspektrum (ESI$^+$): m/z = 573 [M+H]$^+$

Beispiel XXXIV

5-tert-Butyl-2-methoxy-3-({-methyl-7-[4-(1-methy)-pyrrolidin-2-carbonyl)-piperazin-1-ylmethyl]-1H-indol-2-carbonyl}-amino)-benzoesäure

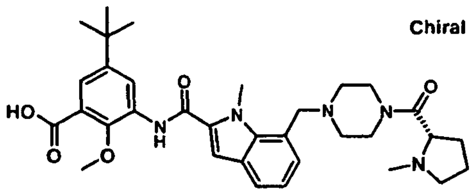

1,29 g 5-tert-Butyl-2-methoxy-3-({1-methyl-7-[4-(1-methyl-pyrrolidin-2-carbonyl)-piperazin-1-ylmethyl]-1H-indol-2-carbonyl}-amino)-benzoesäure-methyl ester werden in 25 ml Tetrahydrofuran gelöst, mit 4 ml 4 N Natronlauge versetzt und 2 Stunden auf 40°C erhitzt. Anschließend wird für 12 Stunden auf 60°C erhitzt, dann werden weitere 2 ml 4 N Natronlauge nachgegeben und wieder 12 Stunden auf 60°C erhitzt. Danach wird im Vakuum eingeengt, der Rückstand in Wasser aufgenommen und der pH-Wert durch Zugabe von 4 N Salzsäure auf 5 eingestellt. Es wird 5 mal mit Dichlormethan/Isopropanol 4:1 extrahiert und die vereinigten organischen Phasen werden mit Magnesiumsulfat getrocknet. Die Lösungsmittel werden im Vakuum entfernt und das so erhaltene Produkt wird direkt weiter umgesetzt.
Ausbeute: 1,3 g (103 % der Theorie)
HPLC (Methode 1): Retentionszeit = 2,48 min.
Massenspektrum (ESI$^+$): m/z = 590 [M+H]$^+$

Beispiel XXXV

3-Amino-5-tert-butyl-2-methoxy-benzoesäure-methyl ester

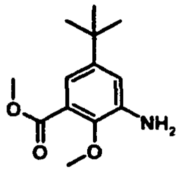

2,7 g 5-tert-Butyl-2-methoxy-3-nitro-benzoesäure-methyl ester werden 120 ml Methanol gelöst, mit 270 mg 10 % Palladium auf Kohle versetzt und 12 Stunden bei Raumtemperatur und 4 bar Wasserstoffdruck hydriert. Anschließend wird vom Katalysator abfiltriert, die Lösungsmittel werden im Vakuum entfernt und der so erhaltene Rückstand wird direkt weiter umgesetzt.
Ausbeute: 2,24 g (94 % der Theorie)
Massenspektrum (ESI$^+$): m/z = 238 [M+H]$^+$

Analog Beispiel XXXV werden folgende Verbindungen erhalten:

(1) 3-Amino-5-tert-butyl-2-methoxy-N,N-dimethyl-benzamid

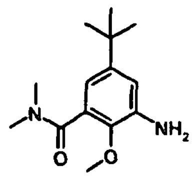

$R_f$-Wert 0,20 (Kieselgel, Petrolether/Essigsäureethylester 1:1)

(2) 5-tert-Butyl-2-methoxy-1,3-diamino-benzol

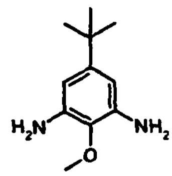

Massenspektrum (ESI$^+$): m/z = 195 $[M+H]^+$

(3) 5-sec-Butyl-2-methoxy-1,3-diamino-benzol

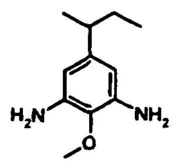

HPLC (Methode 1): Retentionszeit = 1,81 min. Massenspektrum (ESI$^+$): m/z = 195 $[M+H]^+$

(4) 5-Isopropyl-2-methoxy-1,3-diamino-benzol

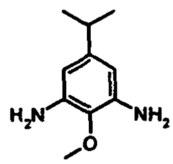

HPLC (Methode 1): Retentionszeit = 3,81 min.

(5) 2-Methoxy-5-pentafluorethyl-1,3-diamino-benzol

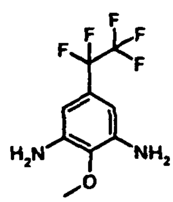

Es wird Raney-Nickel statt 10 % Palladium auf Kohle als Hydrierkatalysator eingesetzt. Massenspektrum (ESI$^+$): m/z = 257 $[M+H]^+$

(6) 2-Methoxy-5-trifluormethyl-1,3-diamino-benzol

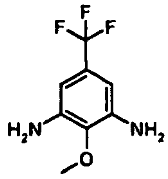

Es wird Raney-Nickel statt 10 % Palladium auf Kohle als Hydrierkatalysator eingesetzt. Das Rohprodukt wird direkt weiter umgesetzt.

(7) 5-tert-Butyl-3-methansulfonyl-2-methoxy-phenylamin

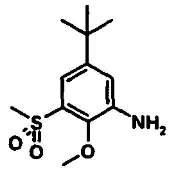

Das Rohprodukt wird aus n-Pentan ausgerührt.
$R_f$-Wert: 0,30 (Kieselgel, Petrolether/Essigsäureethylester 4:1)

(8) 5-tert-Butyl-3-methansulfonylmethyl-2-methoxy-phenylamin

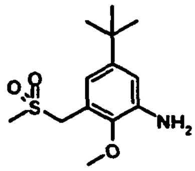

HPLC (Methode 1): Retentionszeit = 2,81 min.
Massenspektrum ($ESI^+$): m/z = 272 $[M+H]^+$

(9) (3-Amino-5-tert-butyl-2-methoxy-phenyl)-methanol

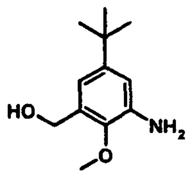

HPLC (Methode 1): Retentionszeit = 2,06 min.
Massenspektrum ($ESI^+$): m/z = 210 $[M+H]^+$

(10) 5-tert-Butyl-3-methansulfinylmethyl-2-methoxy-phenylamin

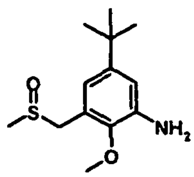

HPLC (Methode 1): Retentionszeit = 2,39 min.
Massenspektrum ($ESI^+$): m/z = 256 $[M+H]^+$

(11) 5-tert-Butyl-2-ethoxy-1,3-diamino-benzol

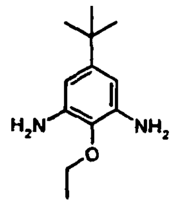

HPLC (Methode 1): Retentionszeit = 1,94 min.
Massenspektrum ($ESI^+$): m/z = 209 $[M+H]^+$

(12) 5-Isobutyl-2-methoxy- 1,3-diamino-benzol

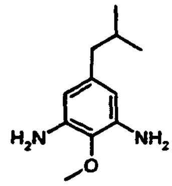

HPLC (Methode 1): Retentionszeit = 1,96 min.
Massenspektrum ($ESI^+$): m/z = 195 $[M+H]^+$

Beispiel XXXVI

5-tert-Butyl-2-methoxy-3-nitro-benzoesäure-methyl ester

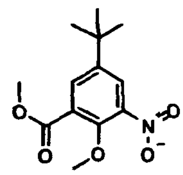

5,4 g 5-tert-Butyl-2-methoxy-3-nitro-benzoesäure werden in 50 ml Methanol gelöst, auf 0°C gekühlt und tropfenweise mit 2,32 ml Thionylchlorid versetzt. Nach beendeter Zugabe wird 12 Stunden auf 60°C erhitzt. Anschließend werden die Lösungsmittel im Vakuum entfernt, der Rückstand wird zwischen Wasser und Essigsäureethylester verteilt und die wässrige Phase wird 2 mal mit Essigsäureethylester extrahiert. Die vereinigten organischen Phasen werden mit Magnesiumsulfat getrocknet und die Lösungsmittel im Vakuum entfernt. Der so erhaltene Rückstand wird an Kieselgel (Cyclohexan/Essigsäureethylester (98:2 auf 60:40) chromatographiert.
Ausbeute: 2,69 g (47 % der Theorie)
HPLC (Methode 1): Retentionszeit = 4,30 min.
Massenspektrum ($ESI^+$): m/z = 268 $[M+H]^+$

Beispiel XXXVII

5-tert-Butyl-2-methoxy-3-nitro-benzoesäure

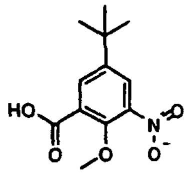

5 g 5-tert-Butyl-2-methoxy-benzoesäure werden in 15 ml Schwefelsäure gelöst, auf 0°C gekühlt und tropfenweise mit einer Lösung aus 2,3 ml Salpetersäure in 2,6 ml Schwefelsäure versetzt. Man läßt 1,5 Stunden bei 0°C und 1 Stunde bei Raumtemperatur nachrühren. Danach wird auf Eiswasser gegeben. Der Niederschlag wird abfiltriert, in Dichlormethan aufgenommen und mit Magnesiumsulfat getrocknet. Das Lösungsmittel wird im Vakuum entfernt und das so erhaltene Produkt wird direkt weiter umgesetzt.
Ausbeute: 5,4 g (89 % der Theorie)

Beispiel XXXVIII

5-tert-Butyl-2-methoxy-N,N-dimethyl-3-nitro-benzamid

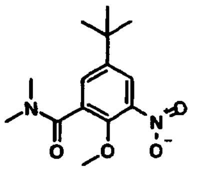

44 g 5-tert-Butyl-2-methoxy-3-nitro-benzoesäure werden in 400 ml Tetrahydrofuran gelöst, mit 31 g Carbonytdümidazol versetzt und 2 Stunden bei 50°C gerührt. Man kühlt auf Raumtemperatur ab, versetzt mit 173 ml einer 2 M Lösung von Dimethylamin in Tetrahydrofuran und läßt weitere 16 Stunden bei 50°C rühren. Anschließend wird mit Essigsäureethylester verdünnt und nacheinander mit 1 N Salzsäure, gesättigter wässriger Natriumhydrogencarbonatlösung und gesättigter wässriger Natriumchloridlösung gewaschen. Nach Trocknen mit Natriumsulfat werden die Lösungsmittel im Vakuum entfernt und der Rückstand wird an Kieselgel mit Chloroform chromatographiert.
Ausbeute: 40 g (82 % der Theorie)
$R_f$-Wert: 0,60 (Kieselgel, Petrolether/Essigsäureethylester 1:1)

Beispiel XXXIX

(3-Amino-5-tert-butyl-2-methoxy-phenyl)-carbamibsäure-tert-butyl ester

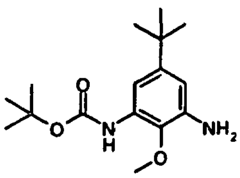

3,2 g 5-tert-Butyl-2-methoxy-1,3-diamino-benzol werden in 50 ml Acetonitril gelöst, mit 3,78 g Di-tert.-butyldicarbonat sowie 20 mg 4-Dimethylamino-pyridin versetzt und 12 Stunden bei Raumtemperatur gerührt. Die Lösungsmittel werden im Vakuum entfernt und der Rückstand wird an Kieselgel (Cyclohexan/Essigsäureethylester 95:5 auf 50:50) chromatographiert.
Ausbeute: 3,86 g (80 % der Theorie)
Massenspektrum (ESI$^+$): m/z = 295 [M+H]$^+$

Beispiel XL

5-tert-Butyl-2-methoxy-1,3-dinitro-benzol

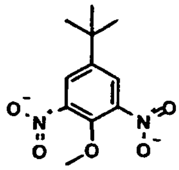

3 g 4-tert.-Butyl-anisol werden in 100 ml Acetonitril gelöst, auf -30°C gekühlt und portionsweise mit 6,1 g Nitronium-tetrafluoroborat versetzt. Man läßt innerhalb einer Stunde auf -15°C kommen, kühlt dann wieder auf -30°C ab, versetzt mit weiteren 1,0 g Nitronium-tetrafluoroborat und läßt 30 Minuten nachrühren. Dann wird zwischen gesättigter wässriger Natriumhydrogencarbonatlösung und Essigsäureethylester verteilt, die wässrige Phase wird 3 mal mit Essigsäureethylester extrahiert und die vereinigten organischen Phasen werden mit gesättigter wässriger Natriumchloridlösung gewaschen. Nach Trocknen mit Magnesiumsulfat werden die Lösungsmittel im Vakuum entfernt und der Rückstand wird aus Isopropanol ausgerührt. Der ausgefallene Feststoff wird abfiltriert und im Vakuum getrocknet.
Ausbeute: 3,75 g (81 % der Theorie)
HPLC (Methode 1): Retentionszeit = 4,36 min.

Analog Beispiel XL werden folgende Verbindungen erhalten:

(1) 5-sec-Butyl-2-methoxy-1,3-dinitro-benzol

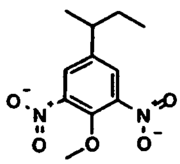

HPLC (Methode 1): Retentionszeit = 4,40 min.
Massenspektrum (EI): m/z = 254 $[M]^+$

(2) 5-Isopropyl-2-methoxy-1,3-dinitro-benzol

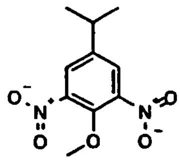

HPLC (Methode 1): Retentionszeit = 4,17 min.

(3) 2-Methoxy-1,3-dinitro-5-pentafluorethyl-benzol

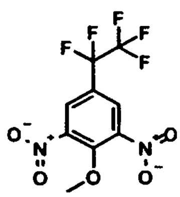

HPLC (Methode 1): Retentionszeit = 4,24 min.

(4) 2-Methoxy-1,3-dinitro-5-trifluormethyl-benzol

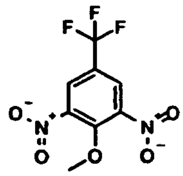

HPLC (Methode 1): Retentionszeit = 3,70 min.

(5) 5-tert-Butyl-2-hydroxy-3-nitro-benzaldehyd

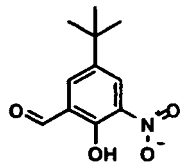

Massenspektrum (ESI$^-$): m/z = 222 $[M-H]^-$

(6) 4-tert-Butyl-2,6-dinitro-phenol

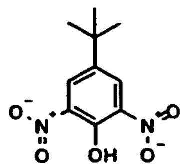

HPLC (Methode 1): Retentionszeit = 4,09 min.
Massenspektrum (ESI$^-$): m/z = 239 $[M-H]^-$

(7) 5-Isobutyl-2-methoxy-1,3-dinitro-benzol

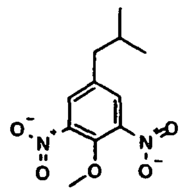

Das Rohprodukt wird direkt weiter umgesetzt.

Beispiel XLI

N-(3-Amino-5-sec-butyl-2-methoxy-phenyl)-methansulfonamid

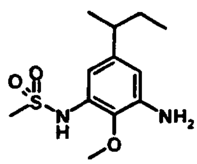

1,46 g 5-sec-Butyl-2-methoxy-1,3-diamino-benzol werden in 20 ml Dichlormethan gelöst, mit 1,21 ml Pyridin versetzt und auf 0°C gekühlt. Man tropft 600 µl Methansulfonsäurechlorid hinzu, läßt auf Raumtemperatur kommen und 2 Stunden nachrühren. Die Lösungsmittel werden im Vakuum entfernt und der Rückstand an Kieselgel (Cyclohexan/Essigsäureethylester 80:20 auf 20:80) chromatographiert.
Ausbeute: 1,23 g (60 % der Theorie)
HPLC (Methode 1): Retentionszeit = 3,02 min.
Massenspektrum ($ESI^+$): m/z = 273 $[M+H]^+$
Analog Beispiel XLI werden folgende Verbindungen erhalten:

(1) N-(3-Amino-5-isopropyl-2-methoxy-phenyl)-methansulfonamid

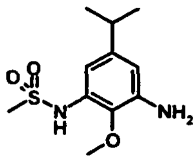

HPLC (Methode 1): Retentionszeit = 2,63 min.
Massenspektrum ($ESI^+$): m/z = 259 $[M+H]^+$

(2) N-(3-Amino-5-tert-butyl-2-ethoxy-phenyl)-methansulfonamid

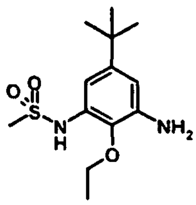

HPLC (Methode 1): Retentionszeit = 3,16 min.
Massenspektrum ($ESI^+$): m/z = 287 $[M+H]^+$

(3) N-(3-Amino-5-isobutyl-2-methoxy-phenyl)-methansulfonamid

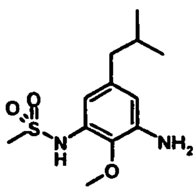

HPLC (Methode 6): Retentionszeit = 2,49 min.
Massenspektrum ($ESI^+$): m/z = 273 $[M+H]^+$

<u>Beispiel XLII</u>

<u>4-sec-Butyl-anisol</u>

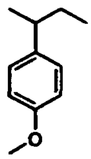

2 g 4-sec-Butyl-phenol werden in 15 ml N,N-Dimethylformamid gelöst, mit 2,2 g Kaliumcarbonat versetzt, 30 Minuten gerührt und dann mit 830 µl Methyliodid versetzt. Man läßt 12 Stunden rühren und gibt dann weitere 600 mg Kaliumcarbonat und 300 µl Methyliodid hinzu. Nach Rühren für 3 Stunden wird zwischen Wasser und Essigsäureethylester verteilt und die organische Phase wird mit gesättigter wässriger Natriumchloridlösung gewaschen. Man trocknet mit Magnesiumsulfat und entfernt die Lösungsmittel im Vakuum. Das so erhaltene Produkt wird direkt weiter umgesetzt.
Ausbeute: 2,14 g (98 % der Theorie)

Analog Beispiel XLII werden folgende Verbindungen erhalten:

(1) 4-Isopropyl-anisol

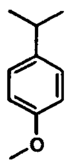

Massenspektrum (EI): m/z = 150 $[M]^+$

(2) 5-tert-Butyl-2-methoxy-3-nitro-benzaldehyd

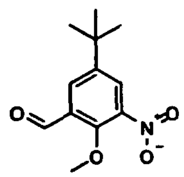

Massenspektrum (EI): m/z = 237 $[M]^+$

(3) 1-tert-Butyl-4-propoxy-benzol

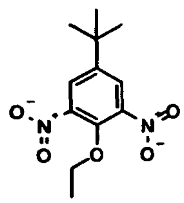

Es wird Ethyliodid statt Methyliodid eingesetzt.
HPLC (Methode 1): Retentionszeit = 4,58 min.
Massenspektrum (EI): m/z = 268 $[M]^+$

<u>Beispiel XLIII</u>

<u>4-pentafluorethyl-anisol</u>

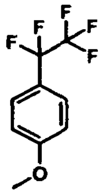

Unter Argon wird 1 g 4-lod-anisol in 7 ml N,N-Dimethylformamid gelöst, mit 1,2 g Kupfer-(I)-iodid, 300 mg Kaliumfluorid sowie 1,0 g Pentafluorethyl-trimethyl-silan versetzt. Man erhitzt 12 Stunden auf 80°C, gibt dann weitere 1,0 g Pentafluorethyl-trimethyl-silan und erhitzt wieder 12 Stunden auf 80°C. Man verdünnt mit einer 2 M wässrigen Ammoniaklösung, läßt 1 Stunde heftig rühren und filtriert den Feststoff ab. Das Filtrat wird 3 mal mit Essigsäureethylester extrahiert und die vereinigten organischen Phasen werden mit Magnesiumsulfat getrocknet. Die Lösungsmittel werden im Vakuum entfernt und der Rückstand in Petrolether/Essigsäureethylester 9:1 aufgenommen. Man filtriert über eine kurze Kieselgelschicht und wäscht mit Petrotether/Essigsäureethylester 9:1 nach. Die Lösungsmittel werden im Vakuum entfernt und das so erhaltene Produkt wird direkt weiter umgesetzt.
Ausbeute: 730 mg (76 % der Theorie)
HPLC (Methode 1): Retentionszeit = 4,42 min.

Beispiel XLIV

5-tert-Butyl-3-methansulfinyl-2-methoxy-phenylamin

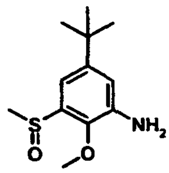

15,5 g 5-tert-Butyl-1-methansulfinyl-2-methoxy-3-nitro-benzol in 300 ml Essigsäureethylester werden 3,0 g 10 % Palladium-(II)-hydroxid auf Kohle gegeben und es wird für 16 Stunden bei 3 bar Wasserstoffdruck hydriert. Anschließend wird der Hydrierkatalysator über Kieselgur abfiltriert und mit 100 ml Essigsäureethylester nachgewaschen. Das Lösungsmittel wird im Vakuum entfernt und der Rückstand aus n-Pentan ausgerührt.
Ausbeute: 12,9 g (94 % der Theorie)
$R_f$-Wert: 0,30 (Kieselgel, Petrolether/Essigsäureethylester 1:1)

Beispiel XLV

5-tert-Butyl-1-methansulfinyl-2-methoxy-3-nitro-benzol

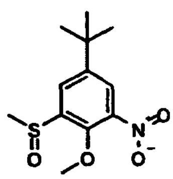

0,5 g 5-tert-Butyl-2-methoxy-1-methylsulfanyl-3-nitro-benzol werden in 15 ml 1,1,1,3,3,3-Hexafluorisopropanol gelöst, mit 1,1 ml einer 9 M Lösung von Wasserstoffperoxid in Wasser versetzt und 5 Stunden bei Raumtemperatur gerührt. Anschließend wird auf 0°C gekühlt und mit 10 %-iger wässriger Natriumthiosulfatlösung verdünnt. Man extrahiert 3 mal mit Essigsäureethylester und wäscht die vereinigten organischen Phasen mit gesättigter wässriger Natriumhydrogencarbonatlösung und gesättigter wässriger Natriumchloridlösung.
Nach Trocknen mit Natriumsulfat werden die Lösungsmittel im Vakuum entfernt und der Rückstand wird an

Kieselgel (Petrolether/Essigsäureethylester 95:5) chromatographiert.
Ausbeute: 350 mg (66 % der Theorie)
$R_f$-Wert: 0,20 (Kieselgel, Petrolether/Essigsäureethylester 9:1)

Analog Beispiel XLV werden folgende Verbindungen erhalten:

(1) 5-tert-Butyl-1-methansulfinylmethyl-2-methoxy-3-nitro-benzol

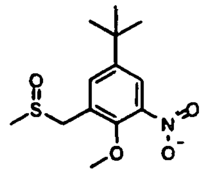

HPLC (Methode 1): Retentionszeit = 3,28 min.
Massenspektrum (ESI$^+$): m/z = 286 $[M+H]^+$

Beispiel XLVI

5-tert-Butyl-2-methoxy-1-methylsulfanyl-3-nitro-benzol

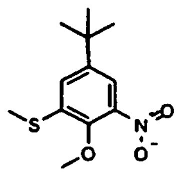

2 g 5-tert-Butyl-2-methoxy-3-nitro-phenylamin werden in 20 ml Acetonitril gelöst, nacheinander mit 3,6 ml Isopentylnitrit sowie 2,4 ml Dimethyldisulfid versetzt und 2 h zum Rückfluß erhitzt. Die Lösungsmittel werden im Vakuum entfernt und der Rückstand wird an Kieselgel (Petrolether/Essigsäureethylester 90:10) chromatographiert.
Ausbeute: 1,5 g (66 % der Theorie)
$R_f$-Wert: 0,70 (Kieselgel, Petrolether/Essigsäureethylester 9:1)

Beispiel XLVII

5-tert-Butyl-2-methoxy-3-nitro-phenylamin

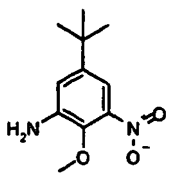

2 g 5-tert-Butyl-2-methoxy-1,3-dinitro-benzol werden in 20 ml Etahnol gelöst und mit 2 ml Wasser sowie 100 mg 10 % Palladium auf Kohle versetzt. Man erhitzt zum Rückfluß, tropft 1,9 ml 4-Methyl-1-cyclohexen hinzu und erhitzt für weitere 2 Stunden zum Rückfluß. Anschließend werden weitere 1,0 ml 4-Methyl-1-cyclohexen zugetropft und für weitere 16 Stunden zum Rückfluß erhitzt. Danach wird auf Raumtemperatur abgekühlt, die Lösungsmittel werden im Vakuum entfernt und der Rückstand wird in Essigsäureethylester aufgenommen. Man wäscht mit gesättigter wässriger Natriumhydrogencarbonatlösung, Wasser, gesättigter wässriger Natriumchloridlösung und trocknet mit Natriumsulfat. Das Lösungsmittel wird im Vakuum entfernt und das so erhaltene Produkt wird direkt weiter umgesetzt.
Ausbeute: 1,7 g (96 % der Theorie)
$R_f$-Wert: 0,20 (Kieselgel, Petrolether/Essigsäureethylester 9:1)

Beispiel XLVIII

5-tert-Butyl-1-methansulfonyl-2-methoxy-3-nitro-benzol

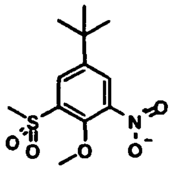

500 mg 5-tert-Butyl-2-methoxy-1 -methylsulfanyl-3-nitro-benzol werden in 10 ml Dichlormethan gelöst, auf 0°C gekühlt und mit 850 mg Metachlorperbenzoesäure versetzt. Man läßt auf Raumtemperatur kommen und 6 Sunden nachrühren. Anschließend wird mit Dichlormethan verdünnt und nacheinander mit gesättigter wässriger Natriumhydrogencarbonatlösung und gesättigter wässriger Natriumchloridlösung gewaschen. Nach Trocknen mit Natriumsulfat werden die Lösungsmittel im Vakuum entfernt und der Rückstand wird an Kieselgel (Petrolether/Essigsäureethylester 9:1) chromatographiert.
Ausbeute: 350 mg (62 % der Theorie)
$R_f$-Wert: 0,30 (Kieselgel, Petrolether/Essigsäureethylester 9:1)
Analog Beispiel XLVIII werden folgende Verbindungen erhalten:

(1) 5-tert-Butyl-1-methansulfonylmethyl-2-methoxy-3-nitro-benzol

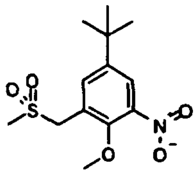

HPLC (Methode 1): Retentionszeit = 3,69 min.

Beispiel XLIX

5-tert-Butyl-2-methoxy-1-methylsulfanylmethyl-3-nitro-benzol

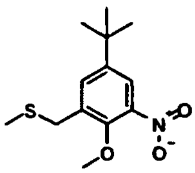

4,64 g Methansulfonsäure-5-tert-butyl-2-methoxy-3-nitro-benzyl ester werden in 50 ml 1,4-Dioxan gelöst, mit 950 mg Natriumthiomethanolat versetzt und 12 Stunden bei Raumtemperatur gerührt. Man gibt weitere 600 mg Natriumthiomethanolat hinzu und rührt 2 Stunden bei 40°C. Anschließend werden die Lösungsmittel im Vakuum entfernt und der Rückstand wird an Kieselgel (Cyclohexan/Essigsäureethylester 98:2 auf 60:40) chromatographiert.
Ausbeute: 2,91 g (87 % der Theorie)
HPLC (Methode 1): Retentionszeit = 4,74 min.

Beispiel L

Methansulfonsäure-5-tert-butyl-2-methoxy-3-nitro-benzyl ester

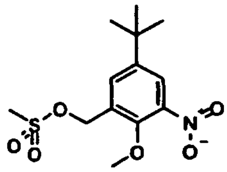

3,43 g (5-tert-Butyl-2-methoxy-3-nitro-phenyl)-methanol werden in 30 ml Dichlormethan gelöst, mit 2,39 ml Triethylamin versetzt und auf 0°C gekühlt. Man tropft 1,2 g Methansulfonsäurechlorid hinzu und läßt 2 Stunden nachrühren. Dann wird mit Dichlormethan verdünnt und wäscht 3 mal mit Wasser und 1 mal mit gesättigter wässriger Natriumchloridlösung. Nach Trocknen mit Magnesiumsulfat werden die Lösungsmittel im Vakuum entfernt und das so erhaltene Produkt wird direkt weiter umgesetzt.
Ausbeute: 4,67 g (87 % der Theorie)
Massenspektrum (ESI$^+$): m/z = 335 $[M+NH_4]^+$

Beispiel LI

5-tert-Butyl-2-methoxy-3-trimethylsilanyloxymethyl-phenylamin

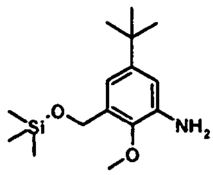

3,03 g (3-Amino-5-tert-butyl-2-methoxy-phenyl)-methanol und 2,4 g Imidazol werden in 20 ml Tetrahydrofuran gelöst und dazu 2,75 ml Trimethylsilylchlorid getropft. Man läßt 2 Stunden bei Raumtemperatur rühren, entfernt die Lösungsmittel im Vakuum und verteilt den Rückstand zwischen 10 %-iger wässriger Kaliumcarbonatlösung und Essigsäureethylester. Die wässrige Phase wird mit Essigsäureethylester extrahiert und die vereinigten organischen Phasen werden mit gesättigter Natriumchloridlkösung gewaschen. Nach Trocknen mit Magnesiumsulfat werden die Lösungsmittel im Vakuum entfernt und der Rückstand wird an Kieselgel (Cyclohexan/Essigsäureethylester 95:5 auf 50:50) chromatographiert.
Ausbeute: 1,96 g (48 % der Theorie)
Massenspektrum (ESI$^+$): m/z = 282 $[M+H]^+$

Beispiel LII

(5-tert-Butyl-2-methoxy-3-nitro-phenyl)-methanol

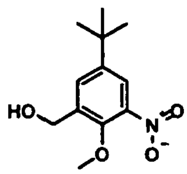

3,47 g 5-tert-Butyl-2-methoxy-3-nitro-benzaldehyd werden in 15 ml Dichlormethan und 15 ml Methanol gelöst,

auf 0°C gekühlt und mit 600 mg Natriumborhydrid versetzt. Man läßt auf Raumtemperatur kommen und 2 Stunden rühren. Dann werden die Lösungsmittel im Vakuum entfernt, der Rückstand wird in Wasser aufgenommen und mit 6 ml Essigsäure versetzt. Man läßt 5 Minuten heftig rühren und extrahiert dann 2 mal mit Essigsäureethylester. Die vereinigten organischen Phasen werden mit gesättigter wässriger Natriumchloridlösung gewaschen, mit Magnesiumsulfat getrocknet und die Lösungsmittel werden im Vakuum entfert. Das so erhaltene Produkt wird direkt weiter umgesetzt.
Ausbeute: 3,5 g (100 % der Theorie)

Beispiel LIII

1-Isobutyl-4-methoxy-benzol

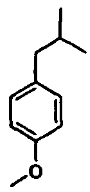

1,9 g 1-(4-Methoxy-phenyl)-2-methyl-propan-1-ol werden in 30 ml Dichlormethan gelöst, auf 0°C gekühlt und mit 3,37 ml Triethylsilan versetzt. Man läßt 10 Minuten rühren und gibt dann 1,62 ml Trifluoressigsäure hinzu. Innerhalb von 1 Stunde wird auf 10°C erwärmt, dann wird die Reaktion durch Zugabe von Wasser beendet und die Phasen werden getrennt. Die organische Phase wird mit gesättigter wässriger Natriumhydrogencarbonatlösung und gesättigter wässriger Natriumchloridlösung gewaschen und anschließend mit Magnesiumsulfat getrocknet. Die Lösungsmittel werden im Vakuum entfernt und der Rückstand wird an Kieselgel (Cyclohexan/Essigsäureethylester 95:5 auf 80:20) chromatographiert.
Ausbeute: 1,48 g (85 % der Theorie)
HPLC (Methode 1): Retentionszeit = 4,81 min.

Beispiel LIV

1-(4-Methoxy-phenyl)-2-methyl-propan-1-ol

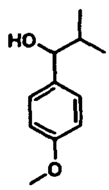

Zu 7,4 ml einer 2 M Lösung von Isopropylmagnesiumchlorid in Tetrahydrofuran werden bei 8°C eine Lösung von 1,5 ml 4-Methoxy-benzaldehyd in 10 ml Toluol getropft. Anschließend läßt man auf Raumtemperatur kommen und 1 Stunde nachrühren. Danach beendet man die Reaktion durch Zugabe von halbgesättigter wässriger Ammoniumchloridlösung. Die Phasen werden getrennt und die wässrige Phase wird mit Toluol extrahiert. Die vereinigten organischen Phasen werden mit halbgesättigter wässriger Ammoniumchloridlösung gewaschen und mit Magnesiumsulfat getrocknet. Die Lösungsmittel werden im Vakuum entfernt und das so erhaltene Produkt wird direkt weiter umgesetzt.
Ausbeute: 2,12 g (95 % der Theorie)
HPLC (Methode 1): Retentionszeit = 3,22 min.

**Herstellung der Endverbindungen**

Beispiel 1

1-Methyl-7-[4-(1-methyl-piperidin-4-carbonyl)-piperazin-1-ylmethyl]-1H-indol-2-carbonsäure (5-tert-butyl-3-methansulfonylamino-2-methoxy-phenyl)-amid

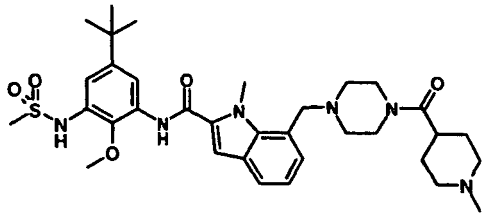

61 mg 1-Methyl-piperidin-4-carbonsäure werden in 1 ml N,N-Dimethylformamid gelöst und mit 137 mg O-(Benzotriazol-1-yl)-N,N,N',N'-tetramethyluronium-tetrafluoroborat (TBTU) sowie 138 μl N,N-Diisopropyl-ethylamin (DIEA) versetzt. Man lässt 20 Minuten nachrühren, gibt dann 150 mg 1-Methyl-7-piperazin-1-ylmethyl-1H-indol-2-carbonsäure (5-tert-butyl-3-methansuffonylamino-2-methoxy-phenyl)-amid hinzu und lässt 12 Stunden rühren. Anschließend wird zwischen Wasser und Essigsäureethylester verteilt und die wässrige Phase 2 mal mit Essigsäureethylester extrahiert. Die vereinigten organischen Phasen werden mit gesättigter wässriger Natriumchloridlösung gewaschen und mit Magnesiumsulfat getrocknet. Danach werden die Lösungsmittel im Vakuum entfernt und der Rückstand an Kieselgel mit Dichlormethan/(Methanol/Ammoniak 9:1) 99:1 auf 90:10 chromatographiert.
Ausbeute: 118 mg (64 % der Theorie)
HPLC (Methode 1): Retentionszeit = 2,38 min.
Massenspektrum (ESI$^+$): m/z = 653 [M+H]$^+$

Analog Beispiel 1 werden folgende Verbindungen erhalten:

(1) (S)-1-Methyl-7-[4-(1-methyl-pyrrolidin-2-carbonyl)-piperazin-1-ylmethyl]-1H-indol-2-carbonsäure (5-tert-butyl-3-methansuffonylamino-2-methoxy-phenyl)-amid

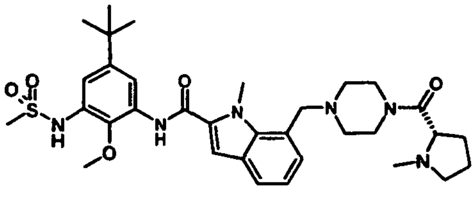

Chiral

HPLC (Methode 2): Retentionszeit = 1,54 min.
Massenspektrum (ESI$^+$): m/z = 639 [M+H]$^+$

(2) 1-Methyl-7-[4-(1-methyl-azetidin-3-carbonyl)-piperazin-1-ylmethyl)-1H-indol-2-carbonsäure (5-tert-butyl-3-methansulfonylamino-2-methoxy-phenyl)-amid

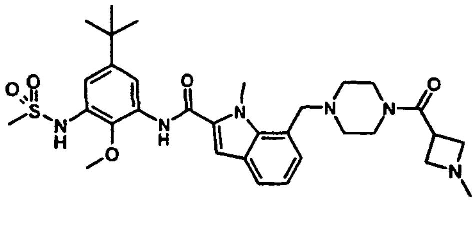

HPLC (Methode 1): Retentionszeit = 2,39 min.
Massenspektrum (ESI$^+$): m/z = 625 [M+H]$^+$

(3) (R)-1-Methyl-7-[4-(1-methyl-pyrrolidin-2-carbonyl)-piperazin-1-ylmethyl]-1H-indol-2-carbonsäure (5-tert-butyl-3-methansulfonylamino-2-methoxy-phenyl)-amid

Chiral

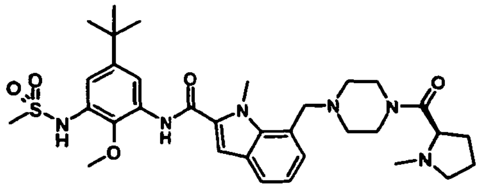

HPLC (Methode 1): Retentionszeit = 2,45 min.
Massenspektrum ($ESI^+$): m/z = 639 $[M+H]^+$

(4) (S)-1-Methyl-7-[4-(pyrrolidin-2-carbonyl)-piperazin-1-ylmethyl]-1H-indol-2-carbonsäure (5-tert-butyl-3-methansuffonylamino-2-methoxy-phenyl)-amid

Chiral

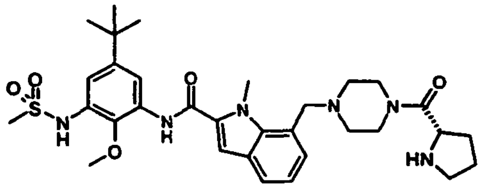

Massenspektrum ($ESI^+$): m/z = 625 $[M+H]^+$

(5) 1-Methyl-7-[4-(1-methyl-azetidin-2-carbonyl)-piperazin-1-ylmethyl]-1H-indol-2-carbonsäure (5-tert-butyl-3-methansuffonylamino-2-methoxy-phenyl)-amid

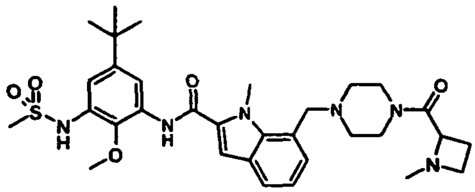

HPLC (Methode 1): Retentionszeit = 2,41 min.
Massenspektrum ($ESI^+$): m/z = 625 $[M+H]^+$

(6) (R)-1-Methyl-7-[1-(1-methyl-pyrrolidin-2-carbonyl)-piperidin-4-ylmethyl]-1H-indol-2-carbonsäure (5-tert-butyl-3-methansulfonylamino-2-methoxy-phenyl)-amid

Chiral

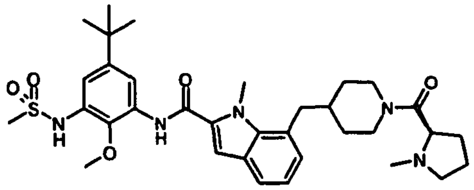

HPLC (Methode 1): Retentionszeit = 3,06 min.
Massenspektrum ($ESI^+$): m/z = 638 $[M+H]^+$

(7) (S)-1-Methyl-7-[1-(1-methyl-pyrrolidin-2-carbonyl)-piperidin-4-ylmethyl]-1H-indol-2-carbonsäure (5-tert-butyl-3-methansulfonylamino-2-methoxy-phenyl)-amid

Chiral

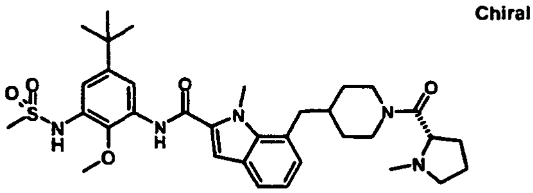

HPLC (Methode 1): Retentionszeit = 3,09 min.
Massenspektrum (ESI$^+$): m/z = 638 $[M+H]^+$

(8) 7-[4-((2S,4R)-4-Hydroxy-1-methyl-pyrrolidin-2-carbonyl)-piperazin-1-ylmethyl]-1-methyl-1H-indol-2-carbonsäure (5-tert-butyl-3-methansulfonylamino-2-methoxy-phenyl)-amid

Chiral

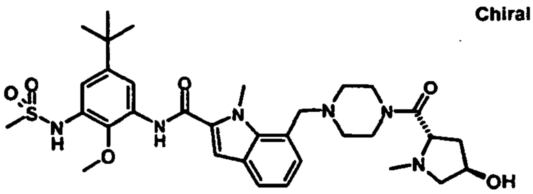

Massenspektrum (ESI$^+$): m/z = 655 $[M+H]^+$

(9) 7-[4-((2R,4R)-4-Methoxy-1-methyl-pyrrolidin-2-carbonyl)-piperazin-1-ylmethyl]-1-methyl-1H-indol-2-carbonsäure (5-tert-butyl-3-methansuffonylamino-2-methoxy-phenyl)-amid

Chiral

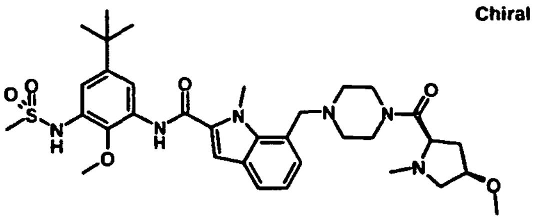

HPLC (Methode 1): Retentionszeit = 2,48 min.
Massenspektrum (ESI$^+$): m/z = 669 $[M+H]^+$

(10) 7-[4-((2R,4R)-4-Hydroxy-1-methyl-pyrrolidin-2-carbonyl)-piperazin-1-ylmethyl]-1-methyl-1H-indol-2-carbonsäure (5-tert-butyl-3-methansulfonylamino-2-methoxy-phenyl)-amid

Chiral

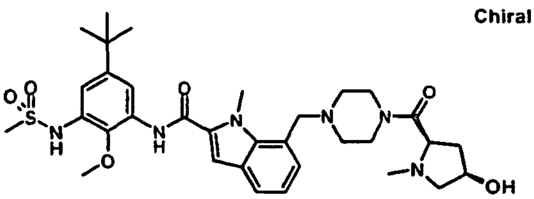

HPLC (Methode 1): Retentionszeit = 2,42 min.
Massenspektrum (ESI$^+$): m/z = 655 $[M+H]^+$

(11) 7-[4-((2R,4S)-4-Hydroxy-1-methyl-pyrrolidin-2-carbonyl)-piperazin-1-ylmethyl]-1-methyl-1H-indol-2-carbonsäure (5-tert-butyl-3-methansulfonylamino-2-methoxy-phenyl)-amid

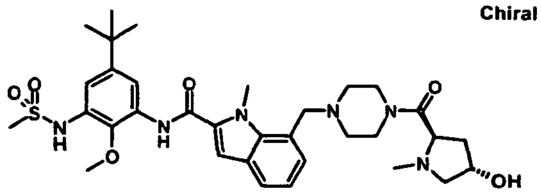

HPLC (Methode 1): Retentionszeit = 2,46 min.
Massenspektrum (ESI$^+$): m/z = 655 [M+H]$^+$

(12) 7-[4-((2S,4S)-4-Hydroxy-1-methyl-pyrrolidin-2-carbonyl)-piperazin-1-ylmethyl]-1-methyl-1H-indol-2-carbonsäure (5-tert-butyl-3-methansulfonylamino-2-methoxy-phenyl)-amid

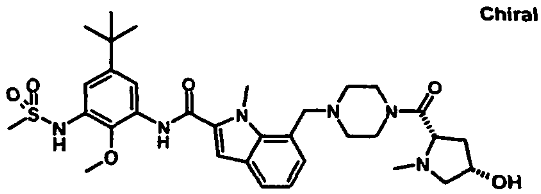

HPLC (Methode 1): Retentionszeit = 2,43 min.
Massenspektrum (ESI$^+$): m/z = 655 [M+H]$^+$

(13) 1-Methyl-7-[4-(2-pyrrolidin-1-yl-propionyl)-piperazin-1-ylmethyl]-1H-indol-2-carbonsäure-(5-tert-butyl-3-methansulfonylamino-2-methoxy-phenyl)-amid

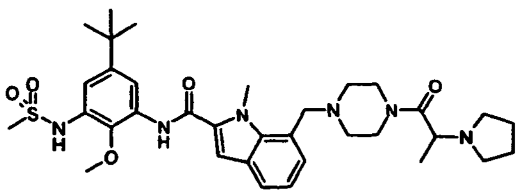

HPLC (Methode 1): Retentionszeit = 2,52 min.
Massenspektrum (ESI$^+$): m/z = 653 [M+H]$^+$

(14) 7-{4-[2-(4-Isopropyl-piperazin-1-yl)-acetyl]-piperazin-1-ylmethyl}-1-methyl-1H-indol-2-carbonsäure-(5-tert-butyl-3-methansulfonylamino-2-methoxy-phenyl)-amid

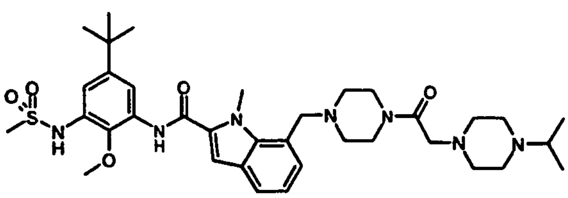

HPLC (Methode 1): Retentionszeit = 2,32 min.
Massenspektrum (ESI$^+$): m/z = 696 [M+H]$^+$

(15) (S)-7-{4-[2-(3-Hydroxy-pyrrolidin-1-yl)-acetyl]-piperazin-1-ylmethyl}-1-methyl-1H-indol-2-carbonsäure-(5-tert-butyl-3-methansulfonylamino-2-methoxy-phenyl)-amid

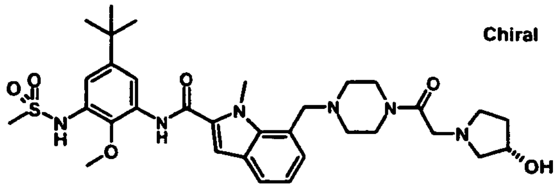

HPLC (Methode 1): Retentionszeit = 2,41 min.
Massenspektrum ($ESI^+$): m/z = 655 $[M+H]^+$

(16) 1-Methyl-7-[4-(2-pyrrolidin-1-yl-acetyl)-piperazin-1-ylmethyl]-1H-indol-2-carbonsäure-(5-tert-butyl-3-methansuffonylamino-2-methoxy-phenyl)-amid

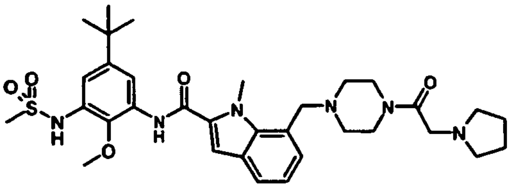

HPLC (Methode 1): Retentionszeit = 2,47 min.
Massenspektrum ($ESI^+$): m/z = 639 $[M+H]^+$

(17) 1-Methyl-7-{4-[2-(4-methyl-piperazin-1-yl)-acetyl]-piperazin-1-ylmethyl}-1H-indol-2-carbonsäure-(5-tert-butyl-3-methansulfonylamino-2-methoxy-phenyl)-amid

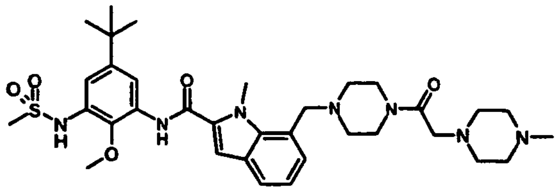

HPLC (Methode 1): Retentionszeit = 2,32 min.
Massenspektrum ($ESI^+$): m/z = 668 $[M+H]^+$

(18) 1-Methyl-7-[1-((2S)-1-methyl-pyrrolidin-2-carbonyl)-azepan-4-ylmethyl]-1H-indol-2-carbonsäure-(5-tert-butyl-3-methansulfonylamino-2-methoxy-phenyl)-amid

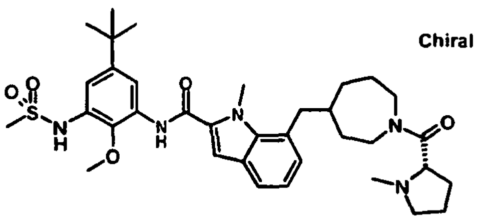

$R_f$-Wert: 0,70 (Kieselgel, Dichlormethan/Methanol/(Ammoniak in Methanol (gesättigt)) 90:10:0,1)
Massenspektrum ($ESI^+$): m/z = 652 $[M+H]^+$

(19) 1-Methyl-7-[1-(1-methyl-piperidin-4-carbonyl)-azepan-4-ylmethyl]-1H-indol-2-carbonsäure-(5-tert-butyl-3-methansulfonylamino-2-methoxy-phenyl)-amid

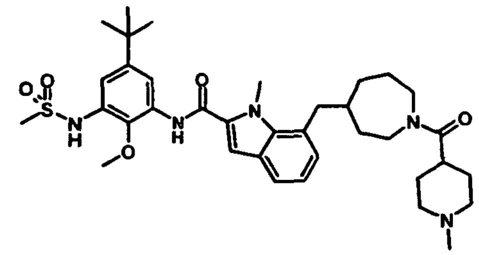

$R_f$-Wert: 0,53 (Kieselgel, Dichlormethan/Methanol/(Ammoniak in Methanol (gesättigt)) 90:10:0,1)
Massenspektrum ($ESI^+$): m/z = 666 $[M+H]^+$

(20) 1-Methyl-7-[1-((2R)-1-methyl-pyrrolidin-2-carbonyl)-azepan-4-ylmethyl]-1H-indol-2-carbonsäure-(5-tert-butyl-3-methansulfonylamino-2-methoxy-phenyl)-amid

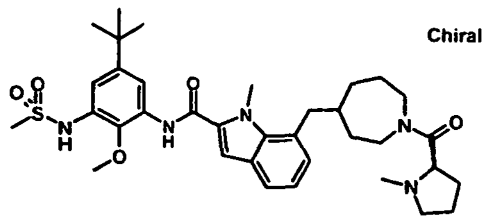


$R_f$-Wert: 0,70 (Kieselgel, Dichlormethan/Methanol/(Ammoniak in Methanol (gesättigt)) 90:10:0,1)
Massenspektrum ($ESI^+$): m/z = 652 $[M+H]^+$

(21) 1-Methyl-7-[4-((2R)-1-methyl-pyrrolidin-2-carbonyl)-[1,4]diazepan-1-ylmethyl]-1H-indol-2-carbonsäure-(5-tert-butyl-3-methansulfonylamino-2-methoxy-phenyl)-amid

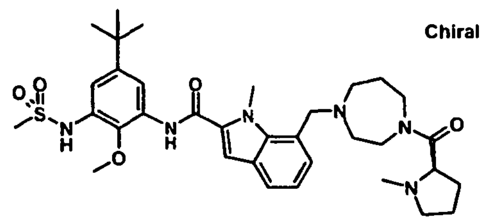


$R_f$-Wert: 0,76 (Kieselgel, Dichlormethan/Methanol/(Ammoniak in Methanol (gesättigt)) 90:10:0,1)
Massenspektrum ($ESI^+$): m/z = 653 $[M+H]^+$

(22) 1-Methyl-7-[4-(4-methyl-piperazin-1-carbonyl)-piperidin-1-ylmethyl]-1H-indol-2-carbonsäure-(5-tert-butyl-3-methansulfonylamino-2-methoxy-phenyl)-amid

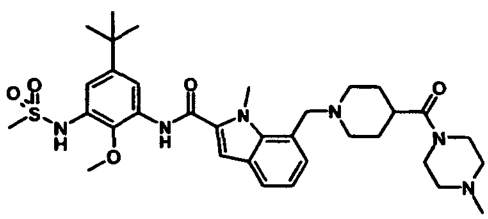

Als Reaktionspartner werden 1-[2-(5-tert-Butyl-3-methansulfonylamino-2-methoxy-phenylcarbamoyl)-1-methyl-1H-indol-7-ylmethyl]-piperidin-4-carbonsäure und 1-Methylpiperazin verwendet. Es wird Tetrahydrofuran statt N,N-Dimethylformamid eingesetzt. HPLC (Methode 1): Retentionszeit = 2,27 min.

Massenspektrum (ESI$^+$): m/z = 653 $[M+H]^+$

(23) (S)-7-[4-(3-Dimethylamino-pyrrolidin-1-carbonyl)-piperidin-1-ylmethyl]-1-methyl-1H-indol-2-carbonsäure-(5-tert-butyl-3-methansulfonylamino-2-methoxy-phenyl)-amid

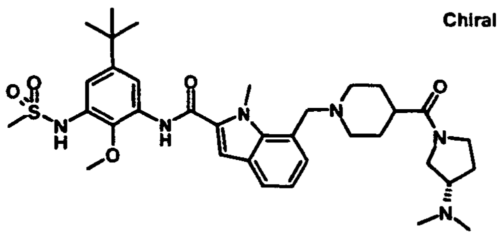

Als Reaktionspartner werden 1-[2-(5-tert-Butyl-3-methansulfonylamino-2-methoxy-phenylcarbamoyl)-1-methyl-1H-indol-7-ylmethyl]-piperidin-4-carbonsäure und (S)-3-Dimethylamino-pyrrolidin verwendet.
HPLC (Methode 1): Retentionszeit = 2,30 min.
Massenspektrum (ESI$^+$): m/z = 667 $[M+H]^+$

(24) (R)-7-[4-(3-Dimethylamino-pyrrolidin-1-carbonyl)-piperidin-1-ylmethyl]-1-methyl-1H-indol-2-carbonsäure-(5-tert-butyl-3-methansulfonylamino-2-methoxy-phenyl)-amid

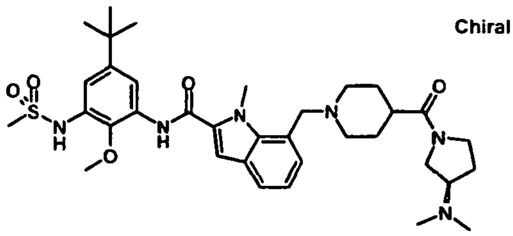

Als Reaktionspartner werden 1-[2-(5-tert-Butyl-3-methansulfonylamino-2-methoxy-phenylcarbamoyl)-1-methyl-1H-indol-7-ylmethyl]-piperidin-4-carbonsäure und (R)-3-Dimethylamino-pyrrolidin verwendet.
HPLC (Methode 1): Retentionszeit = 2,28 min.
Massenspektrum (ESI$^+$): m/z = 667 $[M+H]^+$

(25) (S)-1-Methyl-7-[4-(1-methyl-pyrrolidin-2-carbonyl)-piperazin-1-ylmethyl]-1H-indol-2-carbonsäure-(3-morpholin-4-yl-phenyl)-amid

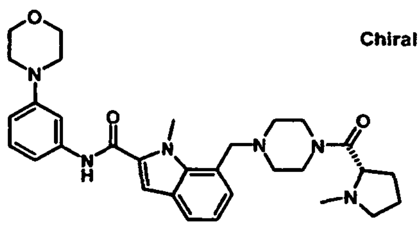

Als Reaktionspartner werden 1-Methyl-7-[4-(1-methyl-pyrrolidin-2-carbonyl)-piperazin-1-ylmethyl]-1H-indol-2-carbonsäure und 3-Morpholin-4-yl-phenylamin verwendet. Es wird O-(7-AzaBenzotriazol-1-yl)-N,N,N',N'-tetramethyluronium-hexafluorophosphat (HATU) satt O-(Benzotriazol-1-yl)-N,N,N',N'-tetramethyluronium-tetrafluoroborat (TBTU) eingesetzt.
HPLC (Methode 5): Retentionszeit = 2,23 min.
Massenspektrum (ESI$^+$): m/z = 545 $[M+H]^+$

Beispiel 2

Chiral

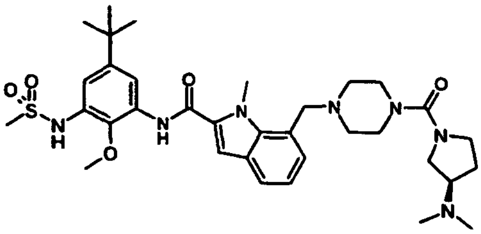

(R)-7-[4-(3-Dimethylamino-pyrrolidin-1-carbonyl)-piperazin-1-ylmethyl]-1-methyl-1H-indol-2-carbonsäure (5-tert-butyl-3-methansulfonylamino-2-methoxy-phenyl)-amid

120 µl einer 20 %-igen Lösung von Phosgen in Toluol werden in 2 ml Tetrahydrofuran gelöst, auf 0°C gekühlt und nacheinander 173 µl N,N-Diisopropyl-ethylamin (DIEA) sowie einer Lösung von 100 mg 1-Methyl-7-piperazin-1-ylmethyl-1H-indol-2-carbonsäure (5-tert-butyl-3-methansulfonylamino-2-methoxy-phenyl)-amid in 1 ml Tetrahydrofuran zugetropft. Man lässt 1 Stunde bei 0°C rühren und tropft dann eine Lösung von 26 µl (R)-Dimethyl-pyrrolidin-3-ylamin in 1 ml Tetrahydrofuran zu. Man lässt auf Raumtemperatur erwärmen und 1 Stunde nachrühren. Anschließend wird zwischen Wasser und Essigsäureethylester verteilt. Die wässrige Phase wird mit Essigsäureethylester extrahiert und die vereinigten organischen Phasen werden mit gesättigter wässriger Natriumchloridlösung gewaschen. Die Lösungsmittel werden im Vakuum entfernt und der Rückstand an Kieselgel mit Dichlormethan/Methanol 100:0 auf 90:10 chromatographiert.
Ausbeute: 44 mg (35 % der Theorie)
Massenspektrum (ESI$^+$): m/z = 668 $[M+H]^+$

Analog Beispiel 2 werden folgende Verbindungen erhalten:

(1) (S)-7-[4-(3-Dimethylamino-pyrrolidin-1-carbonyl)-piperazin-1-ylmethyl]-1-methyl-1H-indol-2-carbonsäure (5-tert-butyl-3-methansulfonylamino-2-methoxy-phenyl)-amid

Chiral

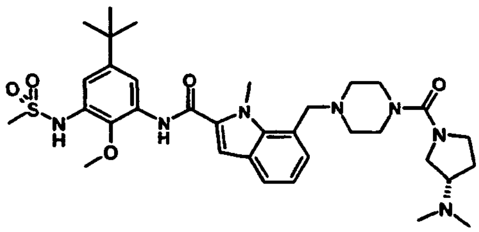

$R_f$-Wert: 0,50 (Kieselgel, Dichlormethan/Methanol 9:1)
Massenspektrum (ESI$^+$): m/z = 668 $[M+H]^+$

Beispiel 3

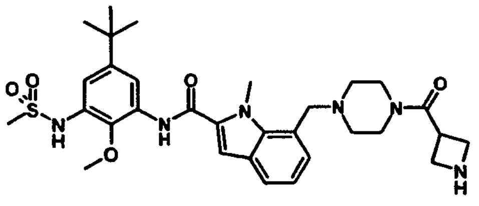

7-[4-(Azetidin-3-carbonyl)-piperazin-1-ylmethyl]-1-methyl-1H-indol-2-carbonsäure (5-tert-butyl-3-methansulfonylamino-2-methoxy-phenyl)-amid

243 mg 3-{4-[2-(5-tert-Butyl-3-methansulfonylamino-2-methoxy-phenylcarbamoyl)-1-methyl-1H-indol-7-ylmethyl]-piperazin-1-carbonyl}-azetidin-1-carbonsäure tert-butyl ester werden in 3 ml Dichlormethan gelöst, auf 0°C gekühlt und mit 261 µl Trifluoressigsäure versetzt. Man lässt auf Raumtemperatur kommen und 12 Stunden rühren. Anschließend werden weitere 1,5 ml Trifluoressigsäure zugegeben und für 2 Stunden gerührt. Danach wird mit Dichlormethan verdünnt und mit 1 N Natronlauge auf pH 9 eingestellt. Man gibt gesättigte Natriumchloridlösung sowie gesättigte Kaliumcarbonatlösung hinzu und trennt die Phasen. Die wässrige Phase wird 2 mal mit Dichlormethan extrahiert. Die vereinigten organischen Phasen werden mit Magnesiumsulfat getrocknet und die Lösungsmittel werden im Vakuum entfernt.
Ausbeute: 83 mg (40 % der Theorie)
Massenspektrum ($ESI^+$): m/z = 611 $[M+H]^+$

Beispiel 4

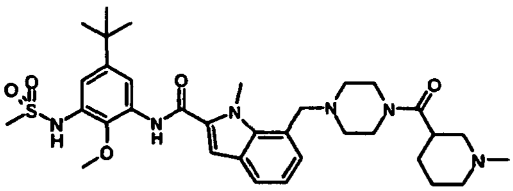

1-Methyl-7-[4-(1-methyl-piperidin-3-carbonyl)-piperazin-1-ylmethyl]-1H-indol-2-carbonsäure (5-tert-butyl-3-methansulfonylamino-2-methoxy-phenyl)-amid

61 mg 1-Methyl-piperidin-3-carbonsäure werden in 1 ml Tetrahydrofuran gelöst, mit 69 mg Carbonyldiimidazol versetzt und 20 Minuten bei 50°C gerührt. Man lässt auf Raumtemperatur abkühlen, gibt 150 mg 1-Methyl-7-piperazin-1-ylmethyl-1H-indol-2-carbonsäure (5-tert-butyl-3-methansulfonylamino-2-methoxy-phenyl)-amid zu und lässt dann 12 Stunden bei Raumtemperatur rühren. Anschließend werden weitere 122 mg 1-Methylpiperidin-3-carbonsäure in 2 ml Tetrahydrofuran mit 138 mg Carbonyldiimidazol versetzt, 20 Minuten bei 50°C gerührt und diese Lösung zum Reaktionsgemisch zugegeben. Man lässt 2 Stunden bei 50°C rühren, verteilt dann zwischen Wasser und Essigsäureethylester, wäscht die organische Phase mit gesättigter wässriger Natriumchloridlösung, trocknet mit Magnesiumsulfat und entfernt die Lösungsmittel im Vakuum. Der Rückstand wird an Kieselgel mit Dichlormethan/(Methanol/Ammoniak 9:1) 99:1 auf 90:10 chromatographiert. Das so erhaltene Produkt durch präparative HPLC gereinigt (HPLC Methode 3).
Ausbeute: 102 mg (55 % der Theorie)
HPLC (Methode 1): Retentionszeit = 2,40 min.
Massenspektrum ($ESI^+$): m/z = 653 $[M+H]^+$

Beispiel 5

(S)-1-Methyl-7-[4-(1-methyl-pyrrolidin-3-carbonyl)-piperazin-1-ylmethyl]-1H-indol-2-carbonsäure (5-tert-butyl-3-methansulfonylamino-2-methoxy-phenyl)-amid

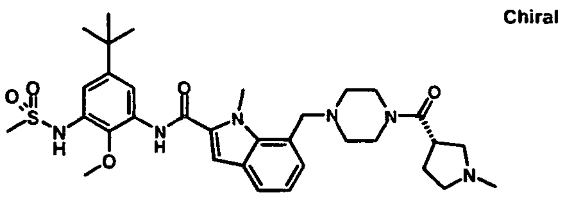

100 mg (S)-1-Methyl-7-[4-(pyrrolidin-3-carbonyl)-piperazin-1-ylmethyl]-1H-indol-2-carbonsäure (5-tert-butyl-3-methansulfonylamino-2-methoxy-phenyl)-amid werden in 10 ml Methanol gelöst, mit 14 µl einer 37 %-igen Lösung von Formaldehyd in Wasser sowie 20 mg Palladium auf Kohle (10 %) versetzt und 4 Stunden bei 4 bar hydriert. Der Katalysator wird abfiltert, die Lösungsmittel im Vakuum entfernt und der Rückstand an Aluminiumoxid (Dichlormethan/Methanol 95: 5 auf 80:20) chromatographiert.
Ausbeute: 81 mg (79 % der Theorie)

HPLC (Methode 1): Retentionszeit = 2,38 min.
Massenspektrum (ESI$^+$): m/z = 639 [M+H]$^+$

Analog Beispiel 5 werden folgende Verbindungen erhalten:

(1) (S)-1-Methyl-7-[4-(1-methyl-pyrrolidin-3-carbonyl)-[1,4]diazepan-1-ylmethyl]-1H-indol-2-carbonsäure-(5-tert-butyl-3-methansulfonylamino-2-methoxy-phenyl)-amid

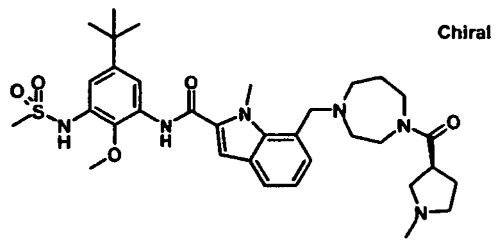

$R_f$-Wert: 0,27 (Kieselgel, Dichlormethan/Methanol/(Ammoniak in Methanol (gesättigt)) 95:5:0,1)
Massenspektrum (ESI$^+$): m/z = 653 [M+H]$^+$

(2) (R)-1-Methyl-7-[4-(1-methyl-pyrrolidin-3-carbonyl)-[1,4]diazepan-1-ylmethyl]-1H-indol-2-carbonsäure-(5-tert-butyl-3-methansulfonylamino-2-methoxy-phenyl)-amid

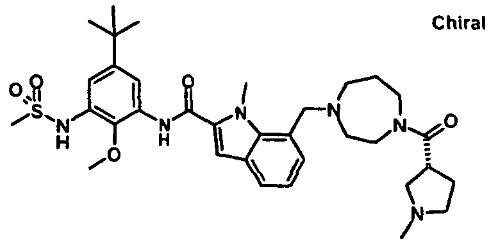

$R_f$-Wert: 0,30 (Kieselgel, Dichlormethan/Methanol/(Ammoniak in Methanol (gesättigt)) 95:5:0,1)
Massenspektrum (ESI$^+$): m/z = 653 [M+H]$^+$

Beispiel 6

7-{4-[2-(4-Dimethylamino-piperidin-1-yl)-acetyl]-piperazin-1-ylmethyl}-1-methyl-1H-indol-2-carbonsäure-(5-tert-butyl-3-methansulfonylamino-2-methoxy-phenyl)-amid

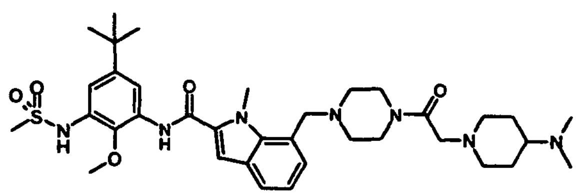

160 mg 1-Methyl-7-piperazin-1-ylmethyl-1H-indol-2-carbonsäure (5-tert-butyl-3-methansulfonylamino-2-methoxy-phenyl)-amid werden in 5 ml Dichlormethan gelöst, mit 100 μL N,N-Diisopropyl-ethylamin (DIEA) versetzt und auf 0°C gekühlt. Danach werden 30 μl Chloressigsäurechlorid zugetropft, auf Raumtemperatur erwärmt und 1 Stunde nachgerührt. Das Lösungsmittel wird im Vakuum entfernt und der Rückstand in 2 ml N,N-Dimethylformamid aufgenommen. Dann werden nacheinander 65 mg Kaliumcarbonat und 55 μl 4-Dimethylamino-piperidin zugegeben. Man läßt 12 Stunden bei Raumtemperatur rühren, verteilt zwischen Wasser und Essigsäureethylester und wäscht die organische Phase mit gesättigter wässriger Natriumchloridlösung. Nach Trocknen mit Magnesiumsulfat werden die Lösungsmittel im Vakuum entfernt und der Rückstand an Kieselgel (Dichlormethan/(Methanol/konz. Ammoniak 9:1) 99:1 auf 85:15)

chromatographiert.
Ausbeute: 90 mg (43 % der Theorie)
HPLC (Methode 1): Retentionszeit = 2,13 min.
Massenspektrum (ESI+): m/z = 696 [M+H]+

Analog Beispiel 6 werden folgende Verbindungen erhalten:

(1) (R)-7-{4-[2-(3-Hydroxy-pyrrolidin-1-yl)-acetyl]-piperazin-1-ylmethyl}-1-methyl-1H-indol-2-carbonsäure-(5-tert-butyl-3-methansulfonylamino-2-methoxy-phenyl)-amid

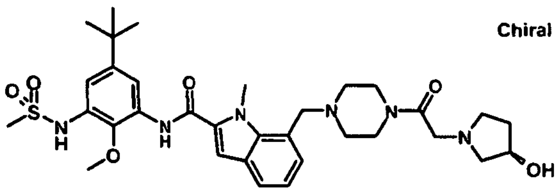

HPLC (Methode 1): Retentionszeit = 2,34 min.
Massenspektrum (ESI+): m/z = 655 [M+H]+

(2) (R)-7-{4-[2-(3-Dimethylamino-pyrrolidin-1-yl)-acetyl]-piperazin-1-ylmethyl}-1-methyl-1H-indol-2-carbonsäure-(5-tert-butyl-3-methansulfonylamino-2-methoxy-phenyl)-amid

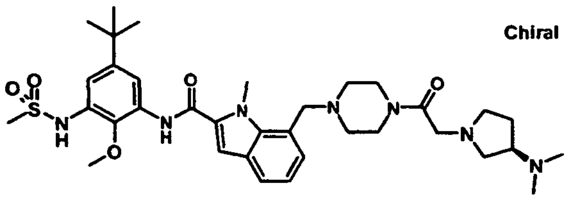

HPLC (Methode 1): Retentionszeit = 2,18 min.
Massenspektrum (ESI+): m/z = 682 [M+H]+

(3) (S)-7-{4-[2-(3-Dimethylamino-pyrrolidin-1-yl)-acetyl]-piperazin-1-ylmethyl}-1-methyl-1H-indol-2-carbonsäure-(5-tert-butyl-3-methansulfonylamino-2-methoxy-phenyl)-amid

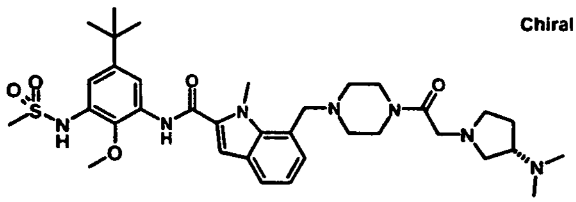

HPLC (Methode 1): Retentionszeit = 2,17 min.
Massenspektrum (ESI+): m/z = 682 [M+H]+

Beispiel 7

1-Methyl-7-[4-(2-piperazin-1-yl-acetyl)-piperazin-1-ylmethyl]-1H-indol-2-carbonsäure-(5-tert-butyl-3-methansulfonylamino-2-methoxy-phenyl)-amid

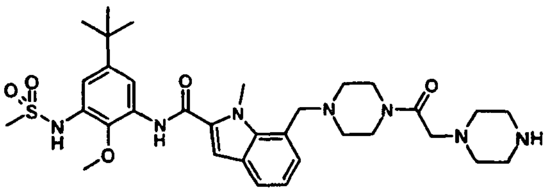

160 mg 4-(2-{4-[2-(5-tert-Butyl-3-methansulfonylamino-2-methoxy-phenylcarbamoyl)-1-methyl-1H-indol-7-yl-methyl]-piperazin-1-yl}-2-oxo-ethyl)-piperazin-1-carbonsäure-tert-butyl ester werden in 5 ml Dichlormethan gelöst, mit 1 ml einer 5 M Lösung von Chlorwasserstoff in Isopropanol versetzt und 12 Stunden bei Raumtemperatur gerührt. Dann werden die Lösungsmittel im Vakuum entfernt und der Rückstand wird zwischen gesättigter wässriger Natriumhydrogencarbonatlösung und Essigsäureethylester verteilt. Die organische Phase wird mit gesättigter wässriger Natriumchloridlösung gewaschen und anschließend mit Magnesiumsulfat getrocknet. Das Lösungsmittel wird im Vakuum entfernt und der Rückstand an Aluminiumoxid (Dichlormethan/Methanol 100:0 auf 95:5) chromatographiert.
Ausbeute: 80 mg (58 % der Theorie)
HPLC (Methode 1): Retentionszeit = 2,26 min.
Massenspektrum (ESI$^+$): m/z = 654 $[M+H]^+$

Beispiel 8

1-Methyl-7-[4-((2S)-1-methyl-pyrrolidin-2-carbonyl)-[1,4]diazepan-1-ylmethyl]-1H-indol-2-carbonsäure-(5-tert-butyl-3-methansulfonylamino-2-methoxy-phenyl)-amid

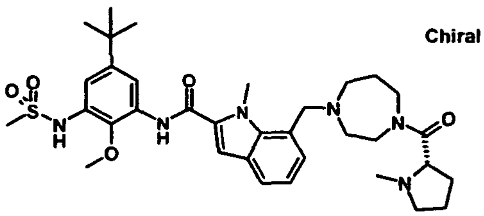

Unter Stickstoff werden 90 mg 7-[1,4]Diazepan-1-ylmethyl-l-methyl-1H-indol-2-carbonsäure-(5-tert-butyl-3-methansulfonylamino-2-methoxy-phenyl)-amid und 28 mg (S)-1-Methylpyrrolidin-2-carbonsäure in 2 ml Tetrahydrofuran gelöst. Nacheinander werden 140 µl Triethylamin und 160 µl einer 50 %-igen Lösung von n-Propylphosphonsäureanhydrid in Essigsäureethylester zugegeben und 48 Stunden bei Raumtemperatur gerührt. Danach wird mit Essigsäureethylester verdünnt und mit gesättigter wässriger Natriumhydrogencarbonatlösung und gesättigter wässriger Natriumchloridlösung gewaschen. Nach Trocknen mit Magnesiumsulfat werden die Lösungsmittel im Vakuum entfernt und der Rückstand an Kieselgel (Dichlormethan/Methanol/(Ammoniak in Methanol (gesättigt)) 97:3:0 auf 93:6:1) chromatographiert. Das so erhaltene Produkt wird in wenig Diethylether gelöst und durch Zugabe von n-Hexan auskristallisiert. Der Feststoff wird abgesaugt und getrocknet.
Ausbeute: 38 mg (35 % der Theorie)
$R_f$-Wert: 0,76 (Kieselgel, Dichlormethan/Methanol/(Ammoniak in Methanol (gesättigt)) 90:10:0,1)
Massenspektrum (ESI$^+$): m/z = 653 $[M+H]^+$

Analog Beispiel 8 werden folgende Verbindungen erhalten:

(1) 1-Methyl-7-(4-(1-methyl-piperidin-4-carbonyl)-[1,4]diazepan-1-ylmethyl]-1H-indol-2-carbonsäure-(5-tert-butyl-3-methansulfonylamino-2-methoxy-phenyl)-amid

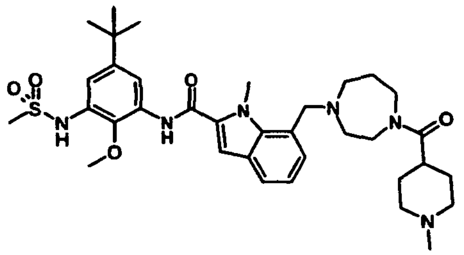

$R_f$-Wert: 0,60 (Kieselgel, Dichlormethan/Methanol/(Ammoniak in Methanol (gesättigt)) 90:10:0,1)
Massenspektrum ($ESI^+$): m/z = 667 $[M+H]^+$

(2) (S)-1-Methyl-7-[4-(1-methyl-pyrrolidin-2-carbonyl)-piperazin-1-ylmethyl]-1H-indol-2-carbonsäure-(5-isobutyl-3-methansulfonylamino-2-methoxy-phenyl)-amid

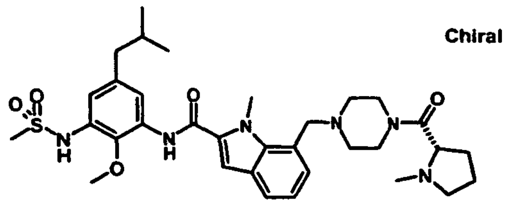

HPLC (Methode 1): Retentionszeit = 2,65 min.
Massenspektrum ($ESI^+$): m/z = 639 $[M+H]^+$

Beispiel 9

(S)-1-Methyl-7-[4-(1-methyl-pyrrolidin-2-carbonyl)-piperazin-1-ylmethyl]-1H-indol-2-carbonsäure-[5-tert-butyl-2-methoxy-3-(4-methyl-piperazin-1-ylmethyl)-phenyl]-amid

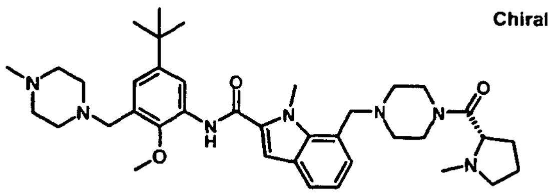

160 mg (S)-1-Methyl-7-[4-(1-methyl-pyrrolidin-2-carbonyl)-piperazin-1-ylmethyl]-1H-indol-2-carbonsäure-(5-tert-butyl-3-formyl-2-methoxy-phenyl)-amid werden in 2 ml 1,2-Dichlorethan gelöst, nacheinander mit 40 µl Essigsäure sowie 45 µl 1-Methylpiperazin versetzt und 1 Stunde bei Raumtemperatur gerührt. Anschließend gibt man 70 mg Natriumtriacetoxyborhydrid hinzu und läßt 5 Stunden nachrühren. Dann wird zwischen Dichlormethan und gesättigter wässriger Natriumhydrogencarbonatlösung verteilt und die organische Phase wird mit gesättigter wässriger Natriumchloridlösung gewaschen. Nach Trocknen mit Magnesiumsulfat werden die Lösungsmittel im Vakuum entfernt und der Rückstand wird durch präparative HPLC (Methode 7) gereinigt.
Ausbeute: 45 mg (31 % der Theorie)
HPLC (Methode 1): Retentionszeit = 2,01 min.
Massenspektrum ($ESI^+$): m/z = 658 $[M+H]^+$

Analog Beispiel 9 werden folgende Verbindungen erhalten:

(1) (S)-1-Methyl-7-[4-(1-methyl-pyrrolidin-2-carbonyl)-piperazin-1-ylmethyl]-1H-indol-2-carbonsäure-{5-tert-butyl-3-[(diisopropylamino)-methyl]-2-methoxy-phenyl}-amid

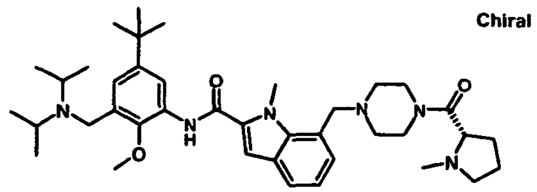

HPLC (Methode 1): Retentionszeit = 2,18 min.
Massenspektrum (ESI$^+$): m/z = 659 $[M+H]^+$

(2) (S)-1-Methyl-7-[4-(1-methyl-pyrrolidin-2-carbonyl)-piperazin-1-ylmethyl]-1H-indol-2-carbonsäure-(5-tert-butyl-3-diethylaminomethyl-2-methoxy-phenyl)-amid

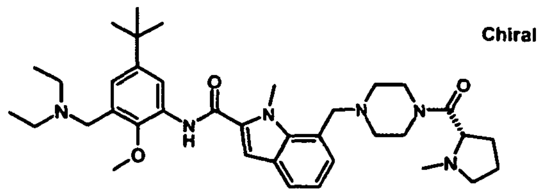

HPLC (Methode 1): Retentionszeit = 2,06 min.
Massenspektrum (ESI$^+$): m/z = 631 $[M+H]^+$

(3) 1-Methyl-7-[4-((2S)-1-methyl-pyrrolidin-2-carbonyl)-piperazin-1-ylmethyl]-1H-indol-2-carbonsäure-[5-tert-butyl-3-(3-dimethylamino-piperidin-1-ylmethyl)-2-methoxy-phenyl]-amid

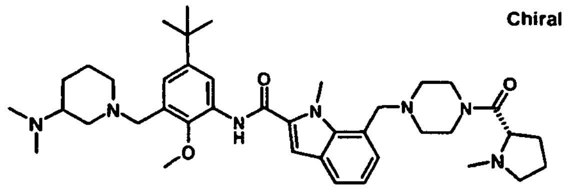

HPLC (Methode 1): Retentionszeit = 1,80 min.
Massenspektrum (ESI$^+$): m/z = 686 $[M+H]^+$

(4) 1-Methyl-7-[4-((2S)-1-methyl-pyrrolidin-2-carbonyl)-piperazin-1-ylmethyl]-1H-indol-2-carbonsäure-[5-tert-butyl-3-(3-diethylamino-pyrrolidin-1-ylmethyl)-2-methoxy-phenyl]-amid

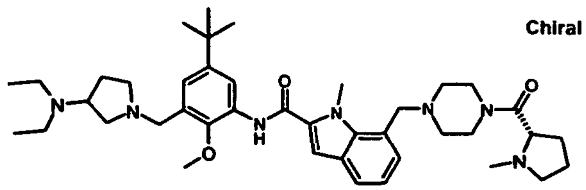

HPLC (Methode 1): Retentionszeit = 1,81 min.
Massenspektrum (ESI$^+$): m/z = 700 $[M+H]^+$

(5) (S)-1-Methyl-7-[4-(1-methyl-pyrrolidin-2-carbonyl)-piperazin-1-ylmethyl]-1H-indol-2-carbonsäure-[5-tert-butyl-3-(4-dimethylamino-piperidin-1-ylmethyl)-2-methoxy-phenyl]-amid

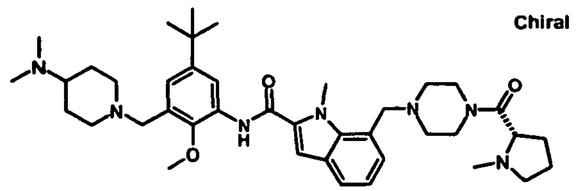

HPLC (Methode 1): Retentionszeit = 1,76 min.
Massenspektrum (ESI$^+$): m/z = 686 $[M+H]^+$

(6) (S)-1-Methyl-7 -[4-(1-methyl-pyrrolidin-2-carbonyl)-piperazin-1-ylmethyl]-1H-indol-2-carbonsäure-[5-tert-butyl-3-(4-diethylamino-piperidin-1-ylmethyl)-2-methoxy-phenyl]-amid

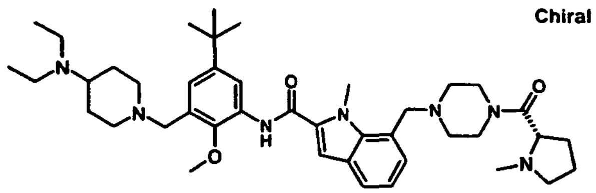

HPLC (Methode 1): Retentionszeit = 1,79 min.
Massenspektrum (ESI$^+$): m/z = 714 $[M+H]^+$

(7) (S)-1-Methyl-7-[4-(1-methyl-pyrrolidin-2-carbonyl)-piperazin-1-ylmethyl]-1H-indol-2-carbonsäure-[5-tert-butyl-3-(4-isopropyl-piperazin-1-ylmethyl)-2-methoxy-phenyl]-amid

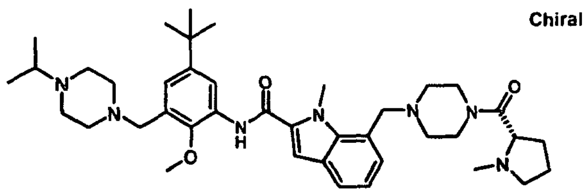

HPLC (Methode 1): Retentionszeit = 2,06 min.
Massenspektrum (ESI$^+$): m/z = 686 $[M+H]^+$

Beispiel 10

(S)-1-Methyl-7-[4-(1-methyl-pyrrolidin-2-carbonyl)-piperazin-1-ylmethyl]-1H-indol-2-carbonsäure-(5-tert-butyl-3-cyclopropylcarbamoyl-2-methoxy-phenyl)-amid

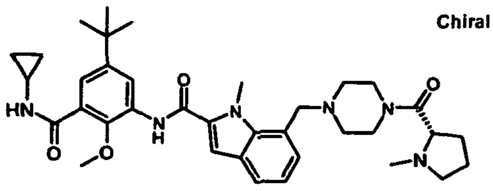

150 mg 5-tert-Butyl-2-methoxy-3-({1-methyl-7-[4-(1-methyl-pyrrolidin-2-carbonyl)-piperazin-1-ylmethyl]-1H-indol-2-carbonyl}-amino)-benzoesäure werden in 2 ml N,N-Dimethylformamid gelöst und mit 105 mg O-(Benzotriazol-1-yl)-N,N,N',N'-tetramethyluronium-tetrafluoroborat (TBTU) sowie 120 µl N,N-Diisopropyl-ethylamin (DIEA) versetzt. Man lässt 20 Minuten nachrühren, gibt dann 21 µl Cyclopropylamin hinzu und lässt 12 Stunden rühren. Es werden weitere

21 µl Cyclopropylamin hinzugegeben und 30 Minuten gerührt. Anschließend werden die Lösungsmittel im Vakuum entfernt und der Rückstand wird durch präparative HPLC (Methode 3) gereinigt.
Ausbeute: 97 mg (61 % der Theorie)
HPLC (Methode 1): Retentionszeit = 2,52 min.
Massenspektrum (ESI$^+$): m/z = 629 $[M+H]^+$

Analog Beispiel 10 werden folgende Verbindungen erhalten:

(1) (S)-1-Methyl-7-[4-(1-methyl-pyrrolidin-2-carbonyl)-piperazin-1-ylmethyl]-1H-indol-2-carbonsäure-(5-tert-butyl-3-isopropylcarbamoyl-2-methoxy-phenyl)-amid

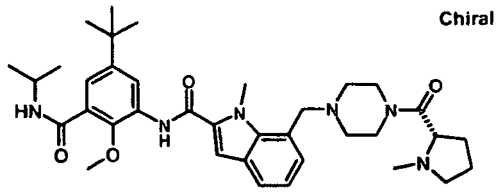

HPLC (Methode 1): Retentionszeit = 2,66 min.
Massenspektrum (ESI$^+$): m/z = 631 $[M+H]^+$

(2) (S)-1-Methyl-7-[4-(1-methyl-pyrrolidin-2-carbonyl)-piperazin-1-ylmethyl]-1H-indol-2-carbonsäure-(5-tert-butyl-3-ethylcarbamoyl-2-methoxy-phenyl)-amid

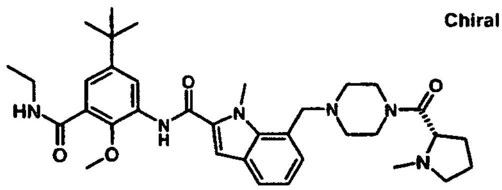

HPLC (Methode 6): Retentionszeit = 4,00 min.
Massenspektrum (ESI$^+$): m/z = 617 $[M+H]^+$

(3) (S)-1-Methyl-7-[4-(1-methyl-pyrrolidin-2-carbonyl)-piperazin-1-ylmethyl]-1H-indol-2-carbonsäure-(5-tert-butyl-2-methoxy-3-methylcarbamoyl-phenyl)-amid

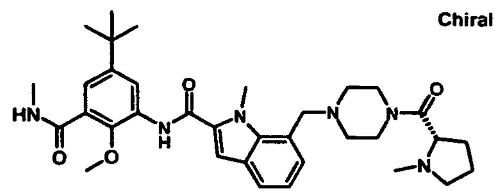

HPLC (Methode 6): Retentionszeit = 3,81 min.
Massenspektrum (ESI$^+$): m/z = 603 $[M+H]^+$

(4) (S)-1-Methyl-7-[4-(1-methyl-pyrrolidin-2-carbonyl)-piperazin-1-ylmethyl]-1H-indol-2-carbonsäure-(5-tert-butyl-3-carbamoyl-2-methoxy-phenyl)-amid

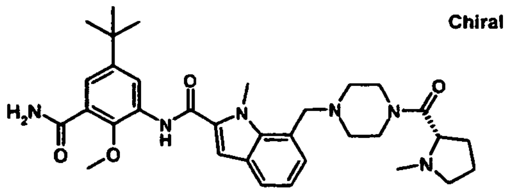

Chiral

HPLC (Methode 1): Retentionszeit = 2,33 min.
Massenspektrum (ESI$^+$): m/z = 589 [M+H]$^+$

Beispiel 11

(S)-1-Methyl-7-[4-(1-methyl-pyrrolidin-2-carbonyl)-piperazin-1-ylmethyl]-1H-indol-2-carbonsäure-(5-tert-butyl-3-dimethylcarbamoyl-2-methoxy-phenyl)-amid

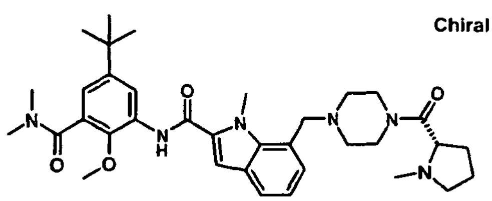

Chiral

150 mg (S)-1-Methyl-7-[4-(1-methyl-pyrrolidin-2-carbonyl)-piperazin-1-ylmethyl]-1H-indol-2-carbonsäure werden in 2 ml Acetonitril gelöst, mit 113 µl Oxalylchlorid sowie 10 µl N,N-Dimethylformamid versetzt und 1 Stunden gerührt. Anschließend wird im Vakuum eingeengt, in Dichlormethan aufgenommen und dieses wieder im Vakuum entfernt. Der Rückstand wird in 2 ml 1,2-Dichlorethan aufgenommen, mit 130 µl Triethylamin sowie 78 mg 3-Amino-5-tert-butyl-2-methoxy-N,N-dimethyl-benzamid versetzt und 2 Stunden bei Raumtemperatur gerührt. Dann wird zwischen Wasser und Dichlormethan verteilt, die wässrige Phase wird 2 mal mit Dichlormethan extrahiert und die vereinigten organischen Phasen werden mit Magnesiumsulfat getrocknet. Die Lösungsmittel werden im Vakuum entfernt und der Rückstand wird an Kieselgel (Dichlormethan/(Methanol/Ammoniak in Methanol (gesättigt) 9:1) 99:1 auf 90:10) chromatographiert.
Ausbeute: 115 mg (60 % der Theorie)
HPLC (Methode 1): Retentionszeit = 2,50 min.
Massenspektrum (ESI$^+$): m/z = 617 [M+H]$^+$

Analog Beispiel 11 werden folgende Verbindungen erhalten:

(1) 1-Methyl-7-[4-((2S)-1-methyl-pyrrolidin-2-carbonyl)-piperazin-1-ylmethyl]-1H-indol-2-carbonsäure-(5-sec-butyl-3-methansulfonylamino-2-methoxy-phenyl)-amid

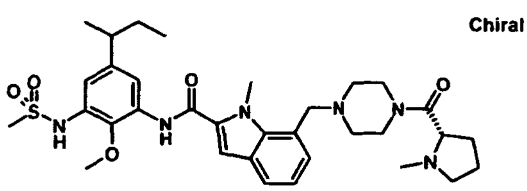

Chiral

Das Rohprodukt wird durch präparative HPLC (Methode 3) gereinigt.
HPLC (Methode 1): Retentionszeit = 2,74 min.
Massenspektrum (ESI$^+$): m/z = 639 [M+H]$^+$

(2) (S)-1-Methyl-7-[4-(1-methyl-pyrrolidin-2-carbonyl)-piperazin-1-ylmethyl]-1H-indol-2-carbonsäure-(5-isopropyl-3-methansulfonylamino-2-methoxy-phenyl)-amid

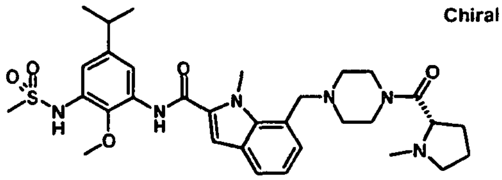

Chiral

Das Rohprodukt wird durch präparative HPLC (Methode 3) gereinigt.
HPLC (Methode 1): Retentionszeit = 2,44 min.
Massenspektrum ($ESI^+$): m/z = 625 $[M+H]^+$

(3) 1-Methyl-7-[4-((2S)-1-methyl-pyrrolidin-2-carbonyl)-piperazin-1-ylmethyl]-1H-indol-2-carbonsäure-(5-tert-butyl-3-methansulfinyl-2-methoxy-phenyl)-amid

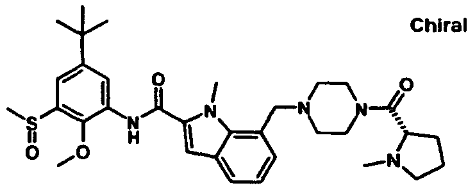

Chiral

Das Produkt wird durch präparative HPLC (Methode 7) gereinigt. Die Produktfraktionen werden vereinigt, das Acetonitril wird im Vakuum entfernt und die wässrige Phase wird mit gesättigter wässriger Natriumhydrogencarbonatlösung verdünnt. Man extrahiert 3 mal mit Dichlormethan, trocknet mit Magnesiumsulfat und entfernt die Lösungsmittel im Vakuum.
HPLC (Methode 1): Retentionszeit = 2,33 min.
Massenspektrum ($ESI^+$): m/z = 608 $[M+H]^+$

Die beiden Diastereomeren werden über präparative HPLC nach folgender Methode getrennt:

Säule: 2 mal Daicel ASH; 250 x 10 mm; 10 ml/Minute; UV-Detektion 220 nm; Eluent: $CO_2$/(0,2 % Diethylamin in Isopropanol) 60:40 (isokratisch)
Diastereomer1:

Retentionszeit: 14,2 Minuten
Massenspektrum ($ESI^+$): m/z = 608 $[M+H]^+$

Diastereomer2:

Retentionszeit: 21,8 Minuten
Massenspektrum ($ESI^+$): m/z = 608 $[M+H]^+$

(4) (S)-1-Methyl-7-[4-(1-methyl-pyrrolidin-2-carbonyl)-piperazin-1-ylmethyl]-1H-indol-2-carbonsäure-(5-tert-butyl-3-methansulfonyl-2-methoxy-phenyl)-amid

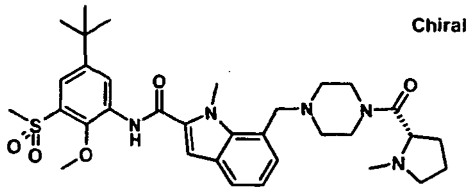

Chiral

HPLC (Methode 1): Retentionszeit = 2,58 min.
Massenspektrum ($ESI^+$): m/z = 624 $[M+H]^+$

(5) (S)-1-Methyl-7-[4-(1-methyl-pyrrolidin-2-carbonyl)-piperazin-1-ylmethyl]-1H-indol-2-carbonsäure-(5-tert-butyl-3-methansulfonylmethyl-2-methoxy-phenyl)-amid

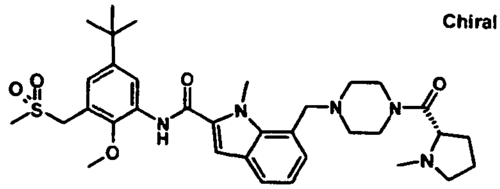

HPLC (Methode 1): Retentionszeit = 2,51 min.
Massenspektrum (ESI$^+$): m/z = 638 [M+H]$^+$

(6) 1-Methyl-7-[4-((2S)-1-methyl-pyrrolidin-2-carbonyl)-piperazin-1-ylmethyl]-1H-indol-2-carbonsäure-(5-tert-butyl-3-methansulfinylmethyl-2-methoxy-phenyl)-amid

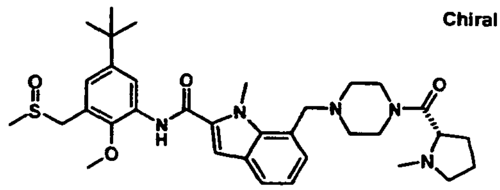

HPLC (Methode 1): Retentionszeit = 2,38 min.
Massenspektrum (ESI$^+$): m/z = 622 [M+H]$^+$

(7) (S)-1-Methyl-7-[4-(1-methyl-pyrrolidin-2-carbonyl)-piperazin-1-ylmethyl]-1H-indol-2-carbonsäure-(5-tert-butyl-3-methansulfonylamino-2-ethoxy-phenyl)-amid

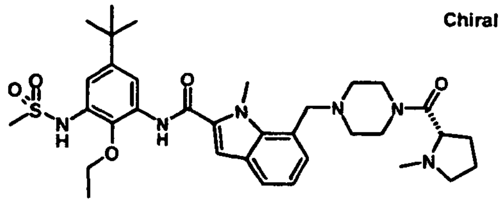

HPLC (Methode 1): Retentionszeit = 2,67 min.
Massenspektrum (ESI$^+$): m/z = 653 [M+H]$^+$

<u>Beispiel 12</u>

<u>(S)-1-Methyl-7-[4-(1-methyl-pyrrolidin-2-carbonyl)-piperazin-1-ylmethyl]-1H-indol-2-carbonsäure-(5-tert-butyl-2-methoxy-3-propionylamino-phenyl)-amid</u>

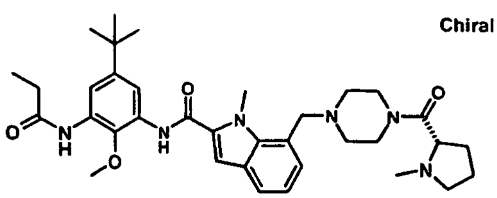

150 mg 1-Methyl-7-[4-(1-methyl-pyrrolidin-2-carbonyl)-piperazin-1-ylmethyl]-1H-indol-2-carbonsäure-(3-amino-5-tert-butyl-2-methoxy-phenyl)-amid Trihydrochlorid werden in 2 ml Dichlormethan gelöst und nacheinander mit 160 µl Triethylamin sowie 25 µl Propionylchlorid versetzt. Man läßt 2 Stunden bei Raumtemperatur rühren, entfernt die Lösungsmittel im Vakuum und reinigt den Rückstand durch präparative HPLC (Methode 3).
Ausbeute: 54 mg (39 % der Theorie)
HPLC (Methode 1): Retentionszeit = 2,51 min.
Massenspektrum (ESI$^+$): m/z = 617 [M+H]$^+$

Analog Beispiel 12 werden folgende Verbindungen erhalten:

(1) (S)-1-Methyl-7-[4-(1-methyl-pyrrolidin-2-carbonyl)-piperazin-1-ylmethyl]-1H-indol-2-carbonsäure-(5-tert-butyl-3-butyrylamino-2-methoxy-phenyl)-amid

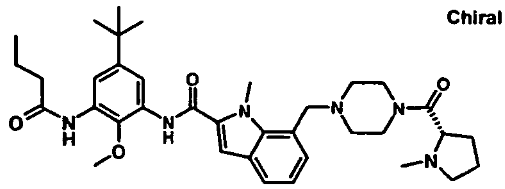

HPLC (Methode 6): Retentionszeit = 4,22 min.
Massenspektrum (ESI$^+$): m/z = 631 [M+H]$^+$

(2) (S)-1-Methyl-7-[4-(1-methyl-pyrrolidin-2-carbonyl)-piperazin-1-ylmethyl]-1H-indol-2-carbonsäure-(3-acetylamino-5-tert-butyl-2-methoxy-phenyl)-amid

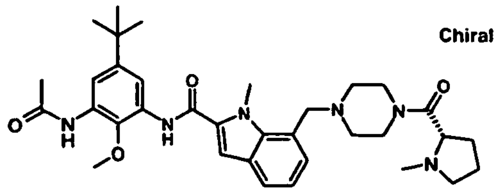

HPLC (Methode 1): Retentionszeit = 2,42 min.
Massenspektrum (ESI$^+$): m/z = 603 [M+H]$^+$

(3) (S)-1-Methyl-7-[4-(1-methyl-pyrrolidin-2-carbonyl)-piperazin-1-ylmethyl]-1H-indol-2-carbonsäure-(5-tert-butyl-3-isobutyrylamino-2-methoxy-phenyl)-amid

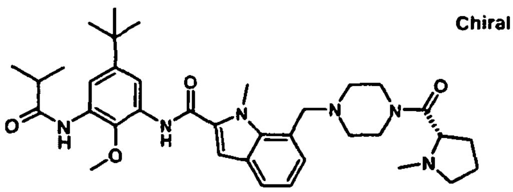

HPLC (Methode 1): Retentionszeit = 2,69 min.
Massenspektrum (ESI$^+$): m/z = 631 [M+H]$^+$

(4) (S)-1-Methyl-7-[4-(1-methyl-pyrrolidin-2-carbonyl)-piperazin-1-ylmethyl]-1H-indol-2-carbonsäure-[5-tert-butyl-3-(cyclopropancarbonyl-amino)-2-methoxy-phenyl]-amid

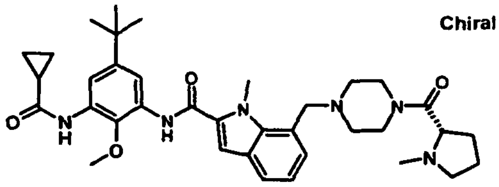

HPLC (Methode 1): Retentionszeit = 2,65 min.
Massenspektrum (ESI$^+$): m/z = 629 [M+H]$^+$

Beispiel 13

(S)-1-Methyl-7-[4-(1-methyl-pyrrolidin-2-carbonyl)-piperazin-1-ylmethyl]-1H-indol-2-carbonsäure-[5-tert-butyl-2-methoxy-3-(propan-1-sulfonylamino)-phenyl]-amid

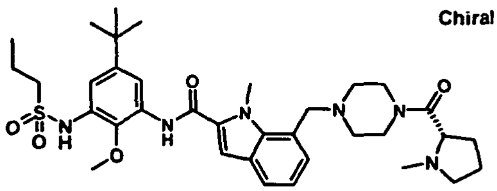

200 mg 1-Methyl-7-[4-(1-methyl-pyrrolidin-2-carbonyl)-piperazin-1-ylmethyl]-1H-indol-2-carbonsäure-(3-amino-5-tert-butyl-2-methoxy-phenyl)-amid Trihydrochlorid werden in 2 ml Dichlormethan gelöst und nacheinander mit 210 µl Triethylamin, 80 µl Propylsulfonsäurechlorid und 70 µl Pyridin versetzt. Man läßt 2 Stunden bei Raumtemperatur rühren, gibt weitere 80 µl Propylsulfonsäurechlorid hinzu und läßt weitere 12 Stunden rühren. Die Lösungsmittel werden im Vakuum entfernt und der Rückstand durch präparative HPLC (Methode 3) gereinigt.
Ausbeute: 110 mg (55 % der Theorie)
HPLC (Methode 1): Retentionszeit = 2,79 min.
Massenspektrum (ESI$^+$): m/z = 667 [M+H]$^+$

Analog Beispiel 13 werden folgende Verbindungen erhalten:

(1) (S)-1-Methyl-7-[4-(1-methyl-pyrrolidin-2-carbonyl)-piperazin-1-ylmethyl]-1H-indol-2-carbonsäure-(5-tert-butyl-3-cyclopropansulfonylamino-2-methoxy-phenyl)-amid

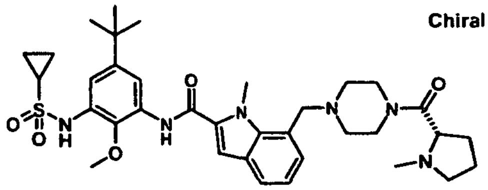

HPLC (Methode 1): Retentionszeit = 2,69 min.
Massenspektrum (ESI$^+$): m/z = 665 [M+H]$^+$

Beispiel 14

(S)-1-Methyl-7-[4-(1-methyl-pyrrolidin-2-carbonyl)-piperazin-1-ylmethyl]-1H-indol-2-carbonsäure-(3-methansulfonylamino-2-methoxy-5-pentafluoroethyl-phenyl)-amid

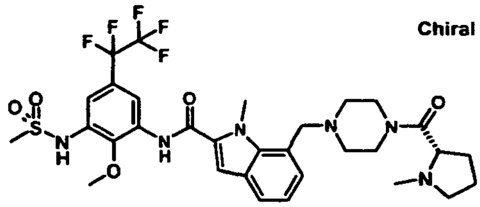

70 mg (S)-1-Methyl-7-[4-(1-methyl-pyrrolidin-2-carbonyl)-piperazin-1-ylmethyl]-1H-indol-2-carbonsäure-(3-amino-2-methoxy-5-pentafluorethyl-phenyl)-amid werden in 2 ml Dichlormethan gelöst, mit 36 µl Triethylamin versetzt und zu dieser Mischung werden 20 µl Methansulfonsäurechlorid getropft. Man läßt 2 Stunden rühren, gibt weitere 20 µl Methansulfonsäurechlorid sowie 36 µl Triethylamin hinzu und läßt dann 1,5 Stunden nachrühren. Anschließend werden 2 ml Methanol sowie 600 µl 2 N Natronlauge zugegeben. Man läßt 2 Stunden rühren, entfernt die Lösungsmittel im Vakuum und chromatographiert den Rückstand an Kieselgel (Dichlormethan/(Methanol/Ammoniak in Methanol (gesättigt) 9:1)99:1 auf 85:15).
Ausbeute: 48 mg (61 % der Theorie)
HPLC (Methode 6): Retentionszeit = 2,75 min.
Massenspektrum (ESI$^+$): m/z = 701 [M+H]$^+$

Analog Beispiel 14 werden folgende Verbindungen erhalten:

(1) (S)-1-Methyl-7 -[4-(1-methyl-pyrrolidin-2-carbonyl)-piperazin-1-ylmethyl]-1H-indol-2-carbonsäure-(3-methansulfonylamino-2-methoxy-5-trifluoromethyl-phenyl)-amid

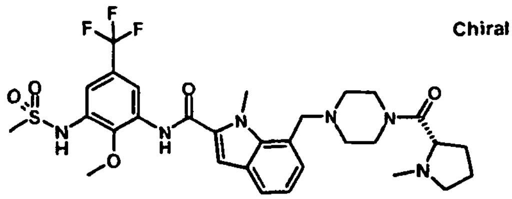

HPLC (Methode 6): Retentionszeit = 2,70 min.
Massenspektrum (ESI$^+$): m/z = 651 [M+H]$^+$

Beispiel 15

(S)-1-Methyl-7-[4-(1-methyl-pyrrolidin-2-carbonyl)-piperazin-1-ylmethyl]-1H-indol-2-carbonsäure-(5-tert-butyl-3-hydroxymethyl-2-methoxy-phenyl)-amid

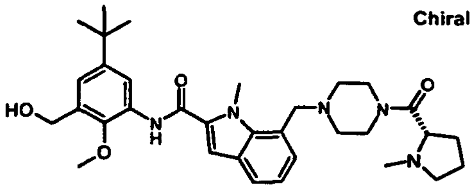

270 mg (S)-1-Methyl-7-[4-(1-methyl-pyrrolidin-2-carbonyl)-piperazin-1-ylmethyl]-1H-indol-2-carbonsäure-(5-tert-butyl-2-methoxy-3-trimethylsilanyloxymethyl-phenyl)-amid werden in 5 ml Dichlormethan gelöst, mit 833 µl einer 5 M Lösung von Chlorwasserstoff in Isopropanol versetzt und 30 Minuten bei Raumtemperatur gerührt. Man verteilt zwischen Dichlormethan und gesättigter wässriger Natriumhydrogencarbonatlösung, extrahiert die wässrige Phase 2 mal mit Dichlormethan und wäscht die vereinigten organischen Phasen mit gesättigter wässriger Natriumchloridlösung. Nach Trocknen mit Magnesiumsulfat werden die Lösungsmittel im Vakuum entfernt und das so erhaltene Produkt wird durch präparative HPLC (Methode 3) gereinigt.
Ausbeute: 75 mg (31 % der Theorie)
HPLC (Methode 6): Retentionszeit = 4,09 min.

Massenspektrum ($ESI^+$): m/z = 576 $[M+H]^+$

Nachfolgend werden Beispiele zu Darreichungsformen beschrieben, worin die Angabe "Wirksubstanz" eine oder mehrere erfindungsgemäße Verbindungen, einschließlich deren Salze bedeutet. Im Falle einer der beschriebenen Kombinationen mit einem oder mehreren weiteren Wirksubstanzen umfasst der Begriff "Wirkstoff' auch die weiteren Wirksubstanzen.

Beispiel A

Dragees mit 75 mg Wirksubstanz

Zusammensetzung:

1 Dragéekern enthält:

| | |
|---|---|
| Wirksubstanz | 75.0 mg |
| Calciumphosphat | 93.0 mg |
| Maisstärke | 35.5 mg |
| Polyvinylpyrrolidon | 10.0 mg |
| Hydroxypropylmethylcellulose | 15.0 mg |
| Magnesiumstearat | 1.5 mg |
| | 230.0 mg |

Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1.5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Dragéekerne werden mit einem Film überzogen, der im wesentlichen aus Hydroxypropylmethylcellulose besteht. Die fertigen Filmdragees werden mit Bienenwachs geglänzt.

Dragéegewicht: 245 mg.

Beispiel B

Tabletten mit 100 mg Wirksubstanz

Zusammensetzung:

1 Tablette enthält:

| | |
|---|---|
| Wirksubstanz | 100.0 mg |
| Milchzucker | 80.0 mg |
| Maisstärke | 34.0 mg |
| Polyvinylpyrrolidon | 4.0 mg |
| Magnesiumstearat | 2.0 mg |
| | 220.0 mg |

Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2.0 mm-Maschenweite) und Trocknen im Hordentrockenschrank bei 50°C wird erneut gesiebt (1.5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

Beispiel C

Tabletten mit 150 mg Wirksubstanz

Zusammensetzung:

1 Tablette enthält:

| | |
|---|---|
| Wirksubstanz | 150.0 mg |
| Milchzucker pulv. | 89.0 mg |
| Maisstärke | 40.0 mg |
| Kolloide Kieselgelsäure | 10.0 mg |
| Polyvinylpyrrolidon | 10.0 mg |
| Magnesiumstearat | 1.0 mg |
| | 300.0 mg |

Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1.5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

Beispiel D

Hartgelatine-Kapseln mit 150 mg Wirksubstanz

Zusammensetzung:

1 Kapsel enthält:

| | | |
|---|---|---|
| Wirkstoff | | 150.0 mg |
| Maisstärke getr. | ca. | 180.0 mg |
| Milchzucker pulv. | ca. | 87.0 mg |
| Magnesiumstearat | | 3.0 mg |
| ca. | | 420.0 mg |

Herstellung:

Der Wirkstoff wird mit den Hilfsstoffen vermengt, durch ein Sieb von 0.75 mm-Maschenweite gegeben und in einem geeigneten Gerät homogen gemischt. Die Endmischung wird in Hartgelatine-Kapseln der Größe 1 abgefüllt.

Kapselfüllung: ca. 320 mg
Kapselhülle: Hartgelatine-Kapsel Größe 1.

Beispiel E

Suppositorien mit 150 mg Wirksubstanz

Zusammensetzung:

1 Zäpfchen enthält:

| | |
|---|---|
| Wirkstoff | 150.0 mg |
| Polyäthylenglykol 1500 | 550.0 mg |
| Polyäthylenglykol 6000 | 460.0 mg |
| Polyoxyäthylensorbitanmonostearat | 840.0 mg |
| | 2000.0 mg |

Herstellung:

Nach dem Aufschmelzen der Suppositorienmasse wird der Wirkstoff darin homogen verteilt und die Schmelze in vorgekühlte Formen gegossen.

Beispiel F

Suspension mit 50 mg Wirksubstanz

Zusammensetzung:

100 ml Suspension enthalten:

| | |
|---|---|
| Wirkstoff | 1.00 g |
| Carboxymethylcellulose-Na-Salz | 0.10 g |
| p-Hydroxybenzoesäuremethylester | 0.05 g |
| p-Hydroxybenzoesäurepropylester | 0.01 g |
| Rohrzucker | 10.00 g |
| Glycerin | 5.00 g |
| Sorbitlösung 70%ig | 20.00 g |
| Aroma | 0.30 g |
| Wasser dest. ad 100.00 ml | |

Herstellung:

Destilliertes Wasser wird auf 70°C erhitzt. Hierin wird unter Rühren p-Hydroxybenzoesäuremethylester und -propylester sowie Glycerin und

Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

Beispiel G

Ampullen mit 10 mg Wirksubstanz

Zusammensetzung:

| | |
|---|---|
| Wirkstoff | 10.0 mg |
| 0.01 n Salzsäure s.q. | |
| Aqua bidest ad | 2.0 ml |

Herstellung:

Die Wirksubstanz wird in der erforderlichen Menge 0.01 n HCl gelöst, mit Kochsalz isotonisch gestellt, sterilfiltriert und in 2 ml Ampullen abgefüllt.

Beispiel H

Ampullen mit 50 mg Wirksubstanz

Zusammensetzung:

| | |
|---|---|
| Wirkstoff | 50.0 mg |
| 0.01 n Salzsäure s.q. | |
| Aqua bidest | ad 10.0 ml |

Herstellung:

Die Wirksubstanz wird in der erforderlichen Menge 0.01 n HCl gelöst, mit Kochsalz isotonisch gestellt, sterilfiltriert und in 10 ml Ampullen abgefüllt.

Beispiel I

Kapseln zur Pulverinhalation mit 5 mg Wirksubstanz
1 Kapsel enthält:

| | |
|---|---|
| Wirksubstanz | 5.0 mg |
| Lactose für Inhalationszwecke | 15.0 mg |
| | 20.0 mg |

Herstellung:

Die Wirksubstanz wird mit Lactose für Inhalationszwecke gemischt. Die Mischung wird auf einer Kapselmaschine in Kapseln (Gewicht der Leerkapsel ca. 50 mg) abgefüllt.
Kapselgewicht: 70.0 mg
Kapselgröße: 3

Beispiel J

Inhalationslösung für Handvernebler mit 2.5 mg Wirksubstanz
1 Hub enthält:

| | | |
|---|---|---|
| Wirksubstanz | 2.500 mg | |
| Benzalkoniumchlorid | 0.001 mg | |
| 1 N-Salzsäure q.s. | | |
| Ethanol/Wasser (50/50) | ad | 15.000 mg |

Herstellung:

Die Wirksubstanz und Benzalkoniumchlorid werden in Ethanol/Wasser (50/50) gelöst. Der pH-Wert der Lösung wird mit 1N-Salzsäure eingestellt. Die eingestellte Lösung wird filtriert und in für den Handvernebler geeignete Behälter (Kartuschen) abgefüllt.
Füllmasse des Behälters: 4.5 g

## Patentansprüche

**1.** Verbindungen der allgerneinen Formel **I**

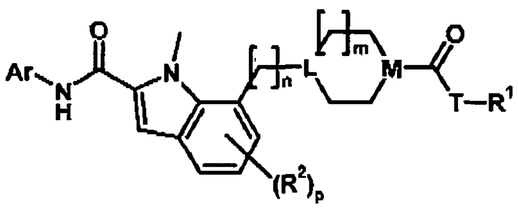

**I**

in der

Ar einen Substituenten der Formel (II), (III) oder (IV)

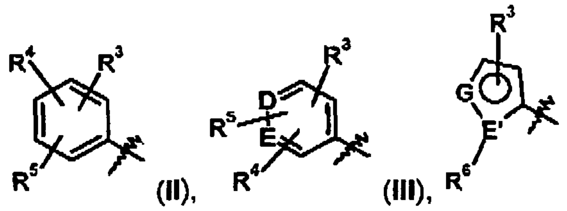

**(II)**, **(III)**, **(IV)**

bedeutet
worin $R^3$ $C_{1-6}$-Alkyl, $C_{3-6}$-Cycloalkyl, $C_{1-4}$-Alkoxy, $C_{1-2}$-Perfluoroalkyl, 3-Methyl-oxetan-3-yl, $C_{1-2}$-Perfluoroalkoxy, Morpholinyl bedeutet,
worin der Cycloalkyl-Rest gegebenenfalls mit $C_{1-3}$-Alkyl substituiert sein kann,
worin $R^4$ H, $C_{1-4}$-Alkyl, $C_{1-4}$-Alkoxy bedeutet,
worin $R^5$ H, $C_{1-5}$-Alkyl-sulfonyl-amino, $C_{3-6}$-Cycloalkyl-sulfonyl-amino, ($C_{1-5}$-Alkyl-sulfonyl)-(methyl)-amino, ($C_{3-6}$-Cycloalkyl-sulfonyl)-(methyl)-amino, $C_{1-5}$-Alkyl-carbonyl-amino, $C_{3-6}$-Cycloalkyl-carbonyl-amino, ($C_{1-5}$-Alkyl-carbonyl)-(methyl)-amino, ($C_{3-6}$-Cycloalkyl-carbonyl)-(methyl)-amino, Aminocarbonyl, $C_{1-5}$-Alkyl-amino-carbonyl, $C_{3-6}$-Cycloalkyl-amino-carbonyl, Di-($C_{1-3}$-Alkyl)-amino-carbonyl, Di-($C_{1-3}$-Alkyl)-amino-$C_{1-2}$-alkyl, Di-($C_{1-3}$-Alkyl)-amino-pyrrolidin-1-yl-$C_{1-2}$-alkyl, 4-$C_{1-5}$-Alkyl-piperazin-1-yl-$C_{1-2}$-alkyl, 4-Di-($C_{1-3}$-Alkyl)-amino-piperidin-1-yl-$C_{1-2}$-alkyl, 3-Di-($C_{1-3}$-Alkyl)-amino-piperidin-1-yl-$C_{1-2}$-alkyl, $C_{1-5}$-Alkyl-sulfinyl, $C_{3-6}$-Cycloalkyl-sulfinyl, $C_{1-5}$-Alkyl-sulfonyl, $C_{3-6}$-Cycloalkyl-sulfonyl, Hydroxy-$C_{1-2}$-alkyl, $C_{1-5}$-Alkyl-sulfinyl-methyl, $C_{1-5}$-Alkyl-sulfonyl-methyl bedeutet,
worin $R^6$ $C_{1-3}$-Alkyl oder Phenyl bedeutet,
wobei der $C_{1-3}$-Alkyl-Rest gegebenenfalls mit Hydroxy oder Di-($C_{1-3}$-Alkyl)-amino substituiert sein kann und wobei der Phenyl-Rest gegebenenfalls mit Fluor oder $C_{1-3}$-Alkyl substituiert sein kann.
worin D oder E Stickstoff bedeuten,
worin G und E' unabhängig voneinander Stickstoff oder Sauerstoff bedeuten,
worin $R^6$ nur dann an E' gebunden ist, wenn E' Stickstoff bedeutet,
$R^1$ ein $C_{4-6}$-Cycloalkyl-System bedeutet, welches 1 bis 2-Stickstoffatome enthält, welches gegebenenfalls 1- bis 2-mal mit $R^7$ substituiert sein kann,
worin $R^7$ $C_{1-3}$-Alkyl, Hydroxy, $C_{1-3}$-Alkoxy, Amino, $C_{1-3}$-Alkyl-amino, Di-($C_{1-3}$-Alkyl)-amino sein kann.
$R^2$ Wasserstoff, Halogen oder $C_{1-4}$-Alkyl bedeutet,
L -C(H)< oder -N< bedeutet,

M -C(H)< oder -N< bedeutet,
T eine Bindung oder $C_{1-4}$-Alkylen bedeutet, wobei der $C_{1-4}$-Alkylen Rest mit $C_{1-2}$-Alkyl substituiert sein kann,
m 0, 1, 2 oder 3 bedeutet,
n 1, 2 oder 3 bedeutet,
p 0, 1, 2 oder 3 bedeutet,

wobei, soweit nichts anderes erwähnt wurde, die vorstehend erwähnten Alkylgruppen geradkettig oder verzweigt sein können,
deren Tautomere, deren Stereoisomere, deren Gemische und deren Salze, insbesondere deren physiologisch verträgliche Salze.

**2.** Verbindungen der allgemeinen Formel (I) nach Anspruch 1,
worin
Ar ein Substituent nach der allgemeinen Formel (II') ist

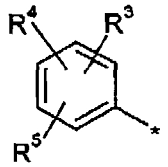
(II')

worin $R^3$ $-C_1$-$C_6$-Alkyl, $-C_3$-$C_6$-Cycloalkyl, $-CF_3$, $-CF_2CF_3$ oder

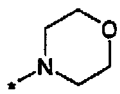

bedeutet,
wobei der $-C_3$-$C_6$-Cycloalkyl-Rest mit $-C_1$-$C_3$-Alkyl substituiert sein kann, worin $R^4$ -H, $-C_1$-$C_4$-Alkyl oder $-O-C_1$-$C_4$-Alkyl bedeutet,
worin $R^5$ ausgewählt ist aus
$-NH-S(O)_2-C_1$-$C_4$-Alkyl;
$-NH-S(O)_2-C_3$-$C_5$-Cycloalkyl;
$-N(CH_3)-S(O)_2-C_1$-$C_4$-Alkyl;
$-N(CH_3)-S(O)_2-C_3$-$C_5$-Cycloalkyl;
$-NH-C(O)-C_1$-$C_4$-Alkyl;
$-NH-C(O)-C_3$-$C_5$-Cycloalkyl;
$-N(CH_3)-C(O)-C_1$-$C_4$-Alkyl;
$-N(CH_3)-C(O)-C_3$-$C_5$-Cycloalkyl;
$-C(O)-NH_2$;
$-C(O)-NH-C_1$-$C_4$-Alkyl;
$-C(O)-N(Di-C_1$-$C_4$-Alkyl);
$-C(O)-NH-C_3$-$C_5$-Cycloalkyl;
$-C(O)-N(C_1$-$C_4$-Alkyl)($C_3$-$C_5$-Cycloalkyl);
$-S(O)-C_1$-$C_4$-Alkyl;
$-S(O)-C_3$-$C_5$-Cycloalkyl;
$-S(O)_2-C_1$-$C_4$-Alkyl;
$-S(O)_2-C_3$-$C_5$-Cycloalkyl;
$-C_1$-$C_4$-Alkyl-OH
$-CH_2-S(O)-C_1$-$C_4$-Alkyl;
$-CH_2-S(O)-C_3$-$C_5$-Cycloalkyl;
$-CH_2-S(O)_2-C_1$-$C_4$-Alkyl;
$-CH_2-S(O)_2-C_3$-$C_5$-Cycloalkyl;
$-C_1$-$C_4$-Alkylen-$N(R^8,R^{8'})$;
worin $R^1$ $-C_4$-$C_8$-Cycloalkyl bedeutet, welches 1 bis 2 Stickstoffatome im Ring enthält,

worin $R^1$ gegebenenfalls substituiert ist mit einer Gruppe ausgewählt aus $-C_1-C_4$-Alkyl, -OH, $-O-C_1-C_4$-Alkyl, und $-N(R^9,R^{9'})$,
worin $R^2$ ausgewählt ist aus -H, -Halogen und $C_1-C_4$-Alkyl,
worin jeweils $R^8$, $R^{8'}$, $R^9$ und $R^{9'}$ unabhängig voneinander ausgewählt sind aus -H und $-C_1-C_5$-Alkyl,
worin die beiden Reste $R^8$ und $R^{8'}$ zusammen mit dem Stickstoff, an den sie gebunden sind, ein 4 bis 6-gliedriges Ringsystem bilden können, welches mit -OH oder $-N(R^{10},R^{10'})$ substituiert sein kann,
worin jeweils $R^{10}$ und $R^{10'}$ unabhängig voneinander ausgewählt sind aus -H und $-C_1-C_5$-Alkyl,
worin L und M unabhängig voneinander ausgewählt sind aus -CH< und -N<,
worin T ausgewählt ist aus einer Bindung und $-C_1-C_3$-Alkylen,
wobei der $C_{1-3}$-Alkylen Rest mit Methyl substituiert sein kann,
worin m 0-3 sein kann,
worin n 1-3 sein kann,
worin p 0-3 sein kann,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

**3.** Verbindungen der allgemeinen Formel (I) nach einem der vorstehenden Ansprüche,
worin
Ar ein Substituent nach der allgemeinen Formel (II''') ist

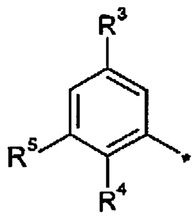

(II''')

worin $R^3$ $-C(CH_3)_3$, $-CH(CH_3)(C_2H_5)$, $-i-C_3H_7$, $-CH_2-i-C_3H_7$, $-CF_3$ oder $-CF_2CF_3$ bedeutet,
worin $R^4$ $-O-CH_3$ oder$-O-C_2H_5$ bedeutet,
worin $R^5$ ausgewählt ist aus $-NH-S(O)_2-CH_3$, $-NH-S(O)_2-n-C_3H_7$, $-NH-S(O)_2-c-C_3H_5$, $-NH-C(O)-CH_3$, $-NH-C(O)-C_2H_5$, $-NH-C(O)-n-C_3H_7$, $-NH-C(O)-i-C_3H_7$, $-NH-C(O)-c-C_3H_5$, $-C(O)-NH_2$, $-C(O)-NH-CH_3$, $-C(O)-NH-C_2H_5$, $-C(O)-NH-i-C_3H_7$, $-C(O)-NH-c-C_3H_5$,- $C(O)-N(CH_3)_2$,$-CH_2-N(CH_3)_2$, , $-CH_2-N(C_2H_5)_2$, ,$-CH_2-N(i-C_3H_7)_2$,

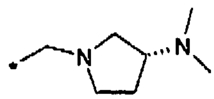,

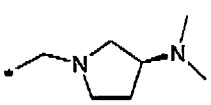, 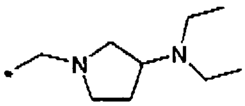, 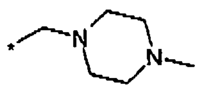, 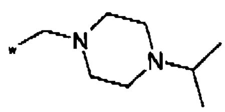,

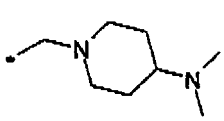, 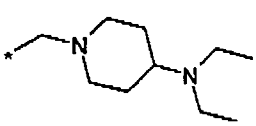, 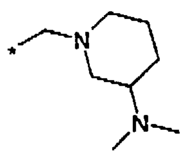,

$-S(O)-CH_3$, $-S(O)_2-CH_3$, $-CH_2-OH$, $-CH_2-S(O)-CH_3$ oder $-CH_2-S(O)_2-CH_3$,
worin $R^1$ ausgewählt ist aus,

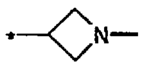, 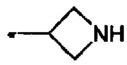, 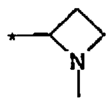, 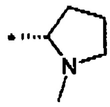, 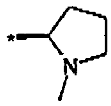,

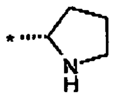, 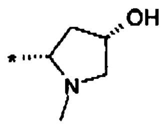, 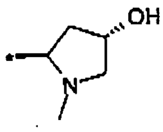, 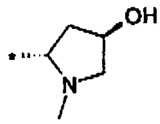, 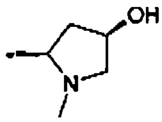, 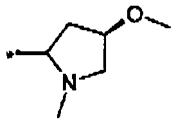,

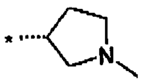, 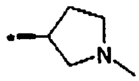, 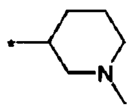, 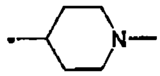, 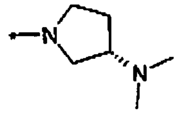, 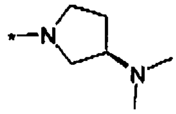,

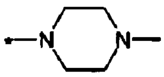, 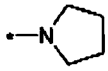, 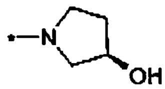, 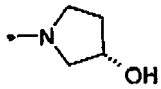, 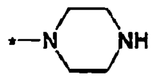, 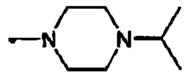,

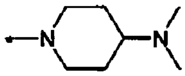,

worin $R^2$ -H bedeutet,
worin L ausgewählt ist aus -CH< und -N<,
worin M -N< bedeutet,
worin T eine Bindung bedeutet,
worin m 1 bedeutet,
worin n 1 bedeutet,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

**4.** Verbindungen der allgemeinen Formel (I) nach einem der vorhergehenden Ansprüche, worin Ar ein Substituent nach der allgemeinen Formel (II') ist

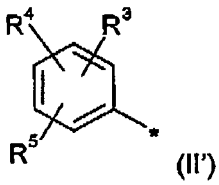

**(II')**

worin $R^3$ $-C_1-C_6$-Alkyl, $-C_3-C_5$-Cycloalkyl, $-CF_3$, $-CF_2CF_3$ oder

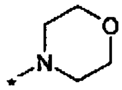

bedeutet,
wobei der $-C_3-C_6$-Cycloalkyl-Rest mit $-C_1-C_3$-Alkyl substituiert sein kann,
worin $R^4$ -H, $-C_1-C_4$-Alkyl oder $-O-C_1-C_4$-Alkyl bedeutet,

worin $R^5$ ausgewählt ist aus
-NH-$S(O)_2$-$C_1$-$C_4$-Alkyl;
-NH-$S(O)_2$-$C_3$-$C_5$-Cycloalkyl;
-N($CH_3$)-$S(O)_2$-$C_1$-$C_4$-Alkyl;
-N($CH_3$)-$S(O)_2$-$C_3$-$C_5$-Cycloalkyl;
-NH-C(O)-$C_1$-$C_4$-Alkyl;
-NH-C(O)-$C_3$-$C_5$-Cycloalkyl;
-N($CH_3$)-C(O)-$C_1$-$C_4$-Alkyl;
-N($CH_3$)-C(O)-$C_3$-$C_5$-Cycloalkyl;
-C(O)-$NH_2$;
-C(O)-NH-$C_1$-$C_4$-Alkyl;
-C(O)-N(Di-$C_1$-$C_4$-Alkyl);
-C(O)-NH-$C_3$-$C_5$-Cycloalkyl;
-C(O)-N($C_1$-$C_4$-Alkyl)($C_3$-$C_5$-Cycloalkyl);
-S(O)-$C_1$-$C_4$-Alkyl;
-S(O)-$C_3$-$C_5$-Cycloalkyl;
-$S(O)_2$-$C_1$-$C_4$-Alkyl;
-$S(O)_2$-$C_3$-$C_5$-Cycloalkyl;
-$C_1$-$C_4$-Alkyl-OH
-$CH_2$-S(O)-$C_1$-$C_4$-Alkyl;
-$CH_2$-S(O)-$C_3$-$C_5$-Cycloalkyl;
-$CH_2$-$S(O)_2$-$C_1$-$C_4$-Alkyl;
-$CH_2$-$S(O)_2$-$C_3$-$C_5$-Cycloalkyl;
-$C_1$-$C_4$-Alkylen-N($R^8$,$R^{8'}$);
worin jeweils $R^8$ und $R^{8'}$ unabhängig voneinander ausgewählt sind aus -H und -$C_1$-$C_5$-Alkyl,
worin die beiden Reste $R^8$ und $R^{8'}$ zusammen mit dem Stickstoff, an den sie gebunden sind, ein 4 bis 6-gliedriges Ringsystem bilden können, welches mit -OH oder -N($R^{10}$,$R^{10'}$) substituiert sein kann,
worin jeweils $R^{10}$ und $R^{10'}$ unabhängig voneinander ausgewählt sind aus -H und -$C_1$-$C_5$-Alkyl,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

**5.** Verbindungen der allgemeinen Formel (I) nach einem der vorstehenden Ansprüche, worin $R^1$ ein $C_{4-6}$-Cycloalkyl-System bedeutet, welches 1 bis 2-Stickstoffatome enthält, welches gegebenenfalls 1- bis 2-mal mit $R^7$ substituiert sein kann, worin $R^7$ $C_{1-3}$-Alkyl, Hydroxy, $C_{1-3}$-Alkoxy, Amino, $C_{1-3}$-Alkyl-amino, Di-($C_{1-3}$-Alkyl)-amino sein kann,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

**6.** Verbindungen der allgemeinen Formel (I) nach einem der vorstehenden Ansprüche,
worin $R^1$
Azetidin-2-yl, Azetidin-3-yl, 1-Methyl-azetidin-2-yl, 1-Methyl-azetidin-3-yl, 1-Ethyl-azetidin-2-yl, 1-Ethyl-azetidin-3-yl, Pyrrolidin-9-yl, Pyrrolidin-2-yl, Pyrrolidin-3-yl, 3-Hydroxy-pyrrolidin-1-yl, 3-Amino-pyrrolidin-1-yl, 3-Methylamino-pyrroüdin-1-yl, 3-Dimethylamino-pyrrolidin-1-yl, 3-Ethylamino-pyrrolidin-1-yl, 3-Diethylamino-pyrrolidin-1-yl, 1-Methyl-pyrrolidin-2-yl, 1-Methyl-pyrrolidin-3-yl, 1-Ethyl-pyrrofidin-2-yl, 1-Ethyl-pyrrolidin-3-yl, 1-Methyl-4-hydroxy-pyrrolidin-2-yl, 1-Methyl-4-methoxy-pyrrolidin-2-yl, Piperidin-1-yl, Piperidin-2-yl, Piperidin-3-yl, Piperidin-4-yl, 4-Amino-piperidin-1-yl, 4-Methylamino-piperidin-1-yl, 4-Dimethylamino-piperidin-1-yl, 4-Ethylamino-piperidin-1-yl, 4-Diethylamino-piperidin-1-yl, 1-Methyl-piperidin-2-yl, 1-Methyl-piperidin-3-yl, 1-Methyl-piperidin-4-yl, 1-Ethyl-piperidin-2-yl, 1-Ethyl-piperidin-3-yl, 1-Ethyl-piperidin-4-yl, Piperazin-1-yl, 4-Methyl-piperazin-1-yl, 4-Ethyl-piperazin-1-yl, 4-Isopropyl-piperazin-1-yl bedeutet,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

**7.** Verbindungen der allgemeinen Formel (I) nach einem der Ansprüche 1-4, worin
$R^1$ ausgewählt ist aus -$C_4$-$C_8$-Cycloalkyl, welches 1 bis 2 Stickstoffatome im Ring enthält,
worin $R^1$ gegebenenfalls substituiert ist mit einer Gruppe ausgewählt aus -$C_1$-$C_4$-Alkyl, -OH, -O-$C_1$-$C_4$-Alkyl, und -N($R^9$,$R^{9'}$),
wobei $R^9$, $R^{9'}$ die vorstehend genannten Bedeutungen haben,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

**8.** Verbindungen der allgemeinen Formel (I) nach einem der Ansprüche 1-4, worin $R^1$ ausgewählt ist aus

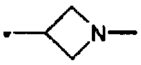, 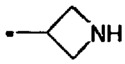, 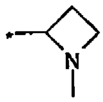, 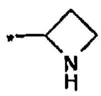, 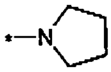, 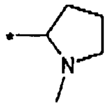,

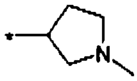, 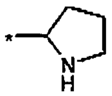, 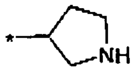, 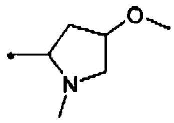, 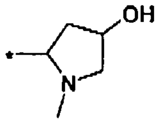, 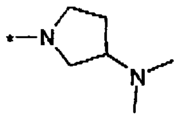,

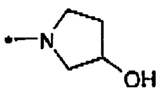, 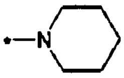, 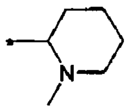, 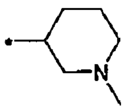, 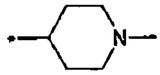, 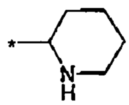, 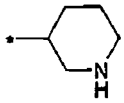,

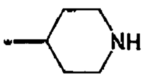, 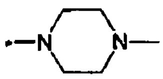, 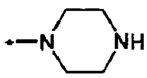, 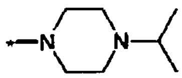,

gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

**9.** Verbindungen der allgemeinen Formel (I) nach einem der vorstehenden Ansprüche,
worin $R^2$ ausgewählt ist aus -H, -F, -Cl und $-CH_3$,
und worin p ausgewählt ist aus 0, 1, 2 oder 3
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

**10.** Verbindungen der allgemeinen Formel (I) nach einem der vorstehenden Ansprüche,
worin L und M unabhängig voneinander ausgewählt sind aus -CH< and -N<,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

**11.** Verbindungen der allgemeinen Formel (I) nach einem der vorstehenden Ansprüche, worin T ausgewählt ist aus einer Bindung und $-C_1-C_3$-Alkylen, wobei der $-C_1-C_3$-Alkylen Rest mit Methyl substituiert sein kann,
gegebenenfalls in Form ihrer individuellen optischen Isomere, Mischungen dieser Isomere, Racemate, gegebenenfalls in Form ihrer Säureadditionssalze mit pharmakologisch akzeptablen Säuren.

**12.** Verbindungen der allgemeinen Formel (I) nach einem der vorstehenden Ansprüche zur Behandlung von Erkrankungen ausgewählt aus Asthma oder COPD.

**13.** Verwendung von Verbindungen der allgemeinen Formel (I) nach einem der vorstehenden Ansprüche zur Herstellung eines Medikamentes zur Behandlung von Erkrankungen ausgewählt aus Asthma oder COPD.

**14.** Verbindungen der allgemeinen Formel (I) nach Anspruch 1, ausgewählt unter
1-Methyl-7-[4-(1-methyl-piperidin-4-carbonyl)-piperazin-1-ylmethyl]-1H-indol-2-carbonsäure (5-tert-butyl-3-methansulfonylamino-2-methoxy-phenyl)-amid,
(S)-1-Methyl-7-[4-(1-methyl-pyrrolidin-2-carbonyl)-piperazin-1-ylmethyl]-1H-indol-2-carbonsäure (5-tert-butyl-3-methansulfonylamino-2-methoxy-phenyl)-amid,
1-Methyl-7-[4-(1-methyl-azetidin-3-carbonyl)-piperazin-1-ylmethyl]-1H-indol-2-carbonsäure (5-tert-butyl-3-methansulfonylamino-2-methoxy-phenyl)-amid,
(R)-1-Methyl-7-[4-(1-methyl-pyrrolidin-2-carbonyl)-piperazin-1-ylmethyl]-1H-indol-2-carbonsäure (5-tert-butyl-3-methansulfonylamino-2-methoxy-phenyl)-amid,
(S)-1-Methyl-7-[4-(pyrrolidin-2-carbonyl)-piperazin-1-ylmethyl]-1H-indol-2-carbonsäure (5-tert-butyl-3-methansulfonylamino-2-methoxy-phenyl)-amid,
1-Methyl-7-[4-(1-methyl-azetidin-2-carbonyl)-piperazin-1-ylmethyl]-1H-indol-2-carbonsäure (5-tert-butyl-3-methansulfonylamino-2-methoxy-phenyl)-amid,
(R)-1-Methyl-7-[1-(1-methyl-pyrrolidin-2-carbonyl)-piperidin-4-ylmethyl]-1H-indol-2-carbonsäure (5-tert-butyl-3-methansulfonylamino-2-methoxy-phenyl)-amid,
(S)-1-Methyl-7-[1-(1-methyl-pyrrolidin-2-carbonyl)-piperidin-4-ylmethyl]-1H-indol-2-carbonsäure (5-tert-butyl-3-methansulfonylamino-2-methoxy-phenyl)-amid,
7-[4-((2S,4R)-4-Hydroxy-1-methyl-pyrrolidin-2-carbonyl)-piperazin-1-ylmethyl]-1-methyl-1H-indol-2-carbonsäure (5-tert-butyl-3-methansulfonylamino-2-methoxy-phenyl)-amid,
7-[4-((2R,4R)-4-Methoxy-1-methyl-pyrrolidin-2-carbonyl)-piperazin-1-ylmethyl]-1-methyl-1H-indol-2-carbonsäure (5-tert-butyl-3-methansulfonylamino-2-methoxy-phenyl)-amid,
7-[4-((2R,4R)-4-Hydroxy-1-methyl-pyrrolidin-2-carbonyl)-piperazin-1-ylmethyl]-1-methyl-1H-indol-2-carbonsäure (5-tert-butyl-3-methansulfonylamino-2-methoxy-phenyl)-amid,
7-[4-((2R,4S)-4-Hydroxy-1-methyl-pyrrrolidin-2-carbonyl)-piperazin-1-ylmethyl]-1-methyl-1H-indol-2-carbonsäure (5-tert-butyl-3-methansulfonylamino-2-methoxy-phenyl)-amid,
7-[4-((2S,4S)-4-Hydroxy-1-methyl-pyrrolidin-2-carbonyl)-piperazin-1-ylmethyl]-1-methyl-1H-indol-2-carbonsäure (5-tert-butyl-3-methansulfonylamino-2-methoxy-phenyl)-amid,
1-Methyl-7-[4-(2-pyrrolidin-1-yl-propionyl)-piperazin-1-ylmethyl]-1H-indo1-2-carbonsäure-(5-tert-butyl-3-methansulfonylamino-2-methoxy-phenyl)-amid,
7-{4-[2-(4-Isopropyl-piperazin-1-yl)-acetyl]-piperazin-1-ylmethyl}-1-methyl-1H-indol-2-carbonsäure-(5-tert-butyl-3-methansulfonylamino-2-methoxy-phenyl)-amid,
(S)-7-{4-[2-(3-Hydroxy-pyrrolidin-1-yl)-acetyl]-piperazin-1-ylmethyl}-1-methyl-1H-indol-2-carbonsäure-(5-tert-butyl-3-methansulfonylamino-2-methoxy-phenyl)-amid,
1-Methyl-7-[4-(2-pyrrolidin-1-yl-acetyl)-piperazin-1-ylmethyl]-1H-indol-2-carbonsäure-(5-tert-butyl-3-methansulfonylamino-2-methoxy-phenyl)-amid,
1-Methyl-7-{4-[2-(4-methyl-piperazin-1-yl)-acetyl]-piperazin-1-ylmethyl}-1H-indol-2-carbonsäure-(5-tert-butyl-3-methansulfonylamino-2-methoxy-phenyl)-amid,
1-Methyl-7-[1-((2S)-1-methyl-pyrrolidin-2-carbonyl)-azepan-4-ylmethyl]-1H-indol-2-carbonsäure-(5-tert-butyl-3-methansulfonylamino-2-methoxy-phenyl)-amid,
1-Methyl-7-[1-(1-methyl-piperidin-4-carbonyl)-azepan-4-ylmethyl]-1H-indol-2-carbonsäure-(5-tert-butyl-3-methansulfonylamino-2-methoxy-phenyl)-amid,
1-Methyl-7-[1-((2R)-1-methyl-pyrrolidin-2-carbonyl)-azepan-4-ylmethyl]-1H-indol-2-carbonsäure-(5-tert-butyl-3-methansulfonylamino-2-methoxy-phenyl)-amid,
1-Methyl-7-[4-((2R)-1-methyl-pyrrolidin-2-carbonyl)-[1,4]diazepan-1-ylmethyl]-1H-indol-2-carbonsäure-(5-tert-butyl-3-methansulfonylamino-2-methoxy-phenyl)-amid,
1-Methyl-7-[4-(4-methyl-piperazin-1-carbonyl)-piperidin-1-ylmethyl]-1H-indol-2-carbonsäure-(5-tert-butyl-3-methansulfonylamino-2-methoxy-phenyl)-amid,
(S)-7-[4-(3-Dimethylamino-pyrrolidin-1-carbonyl)-piperidin-1-ylmethyl]-1-methyl-1H-indol-2-carbonsäure-(5-tert-butyl-3-methansulfonylamino-2-methoxy-phenyl)-amid,
(R)-7-[4-(3-Dimethylamino-pyrrolidin-1-carbonyl)-piperidin-1-ylmethyl]-1-methyl-1H-indol-2-carbonsäure-(5-tert-butyl-3-methansulfonylamino-2-methoxy-phenyl)-amid,
(S)-1-Methyl-7-[4-(1-methyl-pyrrolidin-2-carbonyl)-piperazin-1-ylmethyl]-1H-indol-2-carbonsäure-(3-morpholin-4-yl-phenyl)-amid,
(R)-7-[4-(3-Dimethylamino-pyrrolidin-1-carbonyl)-piperazin-1-ylmethyl]-1-methyl-1H-indol-2-carbonsäure (5-tert-butyl-3-methansulfonylamino-2-methoxy-phenyl)-amid,
(S)-7-[4-(3-Dimethylamino-pyrrolidin-1-carbonyl)-piperazin-1-ylmethyl]-1-methyl-1H-indol-2-carbonsäure (5-tert-butyl-3-methansulfonylamino-2-methoxy-phenyl)-amid,
7-[4-(Azetidin-3-carbonyl)-piperazin-1-ylmethyl]-1-methyl-1H-indol-2-carbonsäure (5-tert-butyl-3-methansulfonylamino-2-methoxy-phenyl)-amid,
1-Methyl-7-[4-(1-methyl-piperidin-3-carbonyl)-piperazin-1-ylmethyl]-1H-indol-2-carbonsäure (5-tert-butyl-3-methansultonylamino-2-methoxy-phenyl)-amid,
(S)-1-Methyl-7-[4-(1-methyl-pyrrolidin-3-carbonyl)-piperazin-1-ylmethyl]-1H-indol-2-carbonsäure (5-tert-butyl-3-methansulfonylamino-2-methoxy-phenyl)-amid,
(S)-1-Methyl-7-[4-(1-methyl-pyrrolidin-3-carbonyl)-[1,4]diazepan-1-ylmethyl]-1H-indol-2-carbonsäure-(5-tert-butyl-3-methansulfonylamino-2-methoxy-phenyl)-amid,
(R)-1-Methyl-7-[4-(1-methyl-pyrrolidin-3-carbonyl)-[1,4]diazepan-1-ylmethyl]-1H-indol-2-carbonsäure-(5-tert-butyl-3-methansulfonylamino-2-methoxy-phenyl)-amid,
7-{4-[2-(4-Dimethylamino-piperidin-1-yl)-acetyl]-piperazin-1-ylmethy}-1-methyl-1H-indol-2-carbonsäure-(5-tert-butyl-3-methansulfonylamino-2-methoxy-phenyl)-amid,
(R)-7-{4-[2-(3-Hydroxy-pyrrolidin-1-yl)-acetyl]-piperazin-1-ylmethyl}-1-methyl-1H-indol-2-carbonsäure-(5-tert-butyl-3-methansulfonylamino-2-methoxy-phenyl)-amid,
(R)-7-{4-[2-(3-Dimethylamino-pyrrolidin-1-yl)-acetyl]-piperazin-1-ylmethyl}-1-methyl-1H-indol-2-carbonsäure-(6-tert-butyl-3-methansulfonylamino-2-methoxy-phenyl)-amid,
(8)-7-{4-[2-(3-Dimethylamino-pyrrolidin-1-yl)-acetyl]-piperazin-1-ylmethyl}-1-methyl-1H-indol-2-carbonsäure-(6-tert-butyl-3-methansulfonylamino-2-methoxy-phenyl)-amid,
1-Methyl-7-[4-(2-piperazin-1-yl-acetyl)-piperazin-1-ylmethyl]-1H-indol-2-carbonsäure-(5-tert-butyl-3-methansulfonylamino-2-methoxy-phenyl)-amid
1-Methyl-7-[4-((2S)-1-methyl-pyrrolidin-2-carbonyl)-11,4]diazepan-1-ylmethyl]-1H-indol-2-carbonsäure-(5-tert-butyl-3-methansulfonylamino-2-methoxy-phenyl)-amid,
1-Methyl-7-[4-(1-methyl-piperidin-4-carbonyl)-[1,4]diazepan-1-ylmethyl]-1H-indol-2-carbonsäure-(5-tert-butyl-3-methansulfonylamino-2-methoxy-phenyl)-amid,
(S)-1-Methyl-7-[4-(1-methyl-pyrrolidin-2-carbonyl)-piperazin-1-ylmethyl]-1H-indol-2-carbonsäure-(5-isobutyl-3-methansulfonylamino-2-methoxy-phenyl)-amid,
(S)-1-Methyl-7-[4-(1-methyl-pyrrolidin-2-carbonyl)-piperazin-1-ylmethyl]-1H-indol-2-carbonsäure-[5-tert-butyl-2-methoxy-3-(4-methyl-piperazin-1-ylmethyl)-phenyl]-amid,
(S)-1-Methyl-7-[4-(1-methyl-pyrrolidin-2-carbonyl)-piperazin-1-ylmethyl]-1H-indol-2-carbonsäure-{5-tert-butyl-3-[(diisopropylamino)-methyl]-2-methoxy-phenyl}-amid
(S)-1-Methyl-7-[4-(1-methyl-pyrrolidin-2-carbonyl)-piperazin-1-ylmethyl]-1H-indol-2-carbonsäure-(5-tert-butyl-3-diethylaminomethyl-2-methoxy-phenyl)-amid,
1-Methyl-7-[4-((2S)-1-methyl-pyrrolidin-2-carbonyl)-piperazin-1-ylmethyl]-1H-indol-2-carbonsäure-[5-tert-butyl-3-(dimethylamino-piperidin-1-ylmethyl)-2-methoxy-phenyl]-amid,
1-Methyl-7-[4-((2S)-1-methyl-pyrrolidin-2-carbonyl)-piperazin-1-ylmethyl]-1H-indol-2-carbonsäure-[5-tert-butyl-3-(3-diethylamino-pyrrolidin-1-ylmethyl)-2-methoxy-phenyl]-amid,
(S)-1-Methyl-7-[4-(1-methyl-pyrrolidin-2-carbonyl)-piperazin-1-ylmethyl]-1H-indol-2-carbonsäure-[5-tert-butyl-3-(4-dimethylamino-piperidin-1-ylmethyl)-2-methoxy-phenyl]-amid,
(S)-1-Methyl-7-[4-(1-methyl-pyrrolidin-2-carbonyl)-piperazin-1-ylmethyl]-1H-indol-2-carbonsäure-[5-tert-butyl-3-(4-diethylamino-piperidin-1-ylmethyl)-2-methoxy-phenyl]-amid,
(S)-1-Methyl-7-[4-(1-methyl-pyrrolidin-2-carbonyl)-piperazin-1-ylmethyl]-1H-indol-2-carbonsäure-[5-tert-butyl-3-(4-isopropyl-piperazin-1-ylmethyl)-2-methoxy-phenyl]-amid,
(S)-1-Methyl-7-[4-(1-methyl-pyrrolidin-2-carbonyl)-piperazin-1-ylmethyl]-1H-indol-2-carbonsäure-(5-tert-butyl-3-cyclopropylcarbamoyl-2-methoxy-phenyl)-amid,
(S)-1-Methyl-7-[4-(1-methyl-pyrrolidin-2-carbonyl)-piperazin-1-ylmethyl]-1H-indol-2-carbonsäure-(5-tert-butyl-3-isopropylcarbamoyl-2-methoxy-phenyl)-amid,
(S)-1-Methyl-7-[4-(1-methyl-pyrrolidin-2-carbonyl)-piperazin-1-ylmethyl]-1H-indol-2-carbonsäure-(5-tert-butyl-3-ethylcarbamoyl-2-methoxy-phenyl)-amid
(S)-1-Methyl-7-[4-(1-methyl-pyrrolidin-2-carbonyl)-piperazin-1-ylmethyl]-1H-indol-2-carbonsäure-(5-tert-butyl-2-methoxy-3-methylcarbamoyl-phenyl)-amid,
(S)-1-Methyl-7-[4-(1-methyl-pyrrolidin-2-carbonyl)-piperazin-1-ylmethyl]-1H-indol-2-carbonsäure-(5-tert-butyl-3-carbamoyl-2-methoxy-phenyl)-amid,
(S)-1-Methyl-7-[4-(1-methyl-pyrrolidin-2-carbonyl)-piperazin-1-ylmethyl]-1H-indol-2-carbonsäure-(5-tert-butyl-3-dimethylcarbamoyl-2-methoxy-phenyl)-amid,
1-Methyl-7-[4-((2S)-1-methyl-pyrrolidin-2-carbonyl)-piperazin-1-ylmethyl]-1H-indol-2-carbonsäure-(5-sec-butyl-3-methansulfonylamino-2-methoxy-phenyl)-amid,
(S)-1-Methyl-7-[4-(1-methyl-pyrrolidin-2-carbonyl)-piperazin-1-ylmethyl]-1H-indol-2-carbonsäure-(5-isopropyl-3-methansulfonylamino-2-methoxy-phenyl)-amid,
1-Methyl-7-[4-((2S)-1-methyl-pyrrolidin-2-carbonyl)-piperazin-1-ylmethyl]-1H-indol-2-carbonsäure-(5-tert-butyl-3-methansulfinyl-2-methoxy-phenyl)-amid,
(S)-1-Methyl-7-[4-(1-methyl-pyrrolidin-2-carbonyl)-piperazin-1-ylmethyl]-1H-indol-2-carbonsäure-(5-tert-butyl-3-methansulfonyl-2-methoxy-phenyl)-amid,
(S)-1-Methyl-7-[4-(1-methyl-pyrrolidin-2-carbonyl)-piperazin-1-ylmethyl]-1H-indol-2-carbonsäure-(5-tert-butyl-3-methansulfonylmethyl-2-methoxy-phenyl)-amid,
1-Methyl-7-[4-((2S)-1-methyl-pyrrolidin-2-carbonyl)-piperazin-1-ylmethyl]-1H-indol-2-carbonsäure-(5-tert-butyl-3-methansulfinylmethyl-2-methoxy-phenyl)-amid,
(S)-1-Methyl-7-[4-(1-methyl-pyrrolidin-2-carbonyl)-piperazin-1-ylmethyl]-1H-indol-2-carbonsäure-(5-tert-butyl-3-methansulfonylamino-2-ethoxy-phenyl)-amid,
(S)-1-Methyl-7-[4-(1-methyl-pyrrolidin-2-carbonyl)-piperazin-1-ylmethyl]-1H-indol-2-carbonsäure-(5-tert-butyl-2-methoxy-3-propionylamino-phenyl)-amid,
(S)-1-Methyl-7-[4-(1-methyl-pyrrolidin-2-carbonyl)-piperazin-1-ylmethyl]-1H-indol-2-carbonsäure-(5-tert-butyl-3-butyrylamino-2-methoxy-phenyl)-amid,
(S)-1-Methyl-7-[4-(1-methyl-pyrrolidin-2-carbonyl)-piperazin-1-ylmethyl]-1H-indol-2-carbonsäure-(3-acetylamino-5-tert-butyl-2-methoxy-phenyl)-amid,
(S)-1-Methyl-7-[4-(1-methyl-pyrrolidin-2-carbonyl)-piperazin-1-ylmethyl]-1H-indol-2-carbonsäure-(5-tert-butyl-3-isobutyrylamino-2-methoxy-phenyl)-amid,
(S)-1-Methyl-7-[4-(1-methyl-pyrrolidin-2-carbonyl)-piperazin-1-ylmethyl]-1H-indol-2-carbonsäure-[5-tert-butyl-3-(cyclopropancarbonyl-amino)-2-methoxy-phenyl]-amid,
(S)-1-Methyl-7-[4-(1-methyl-pyrrolidin-2-carbonyl)-piperazin-1-ylmethyl]-1H-indol-2-carbonsäure-[5-tert-butyl-2-methoxy-3-(propan-1-sulfonylamino)-phenyl]-amid,
(S)-1-Methyl-7-[4-(1-methyl-pyrrolidin-2-carbonyl)-piperazin-1-ylmethyl]-1H-indol-2-carbonsäure-(5-tert-butyl-3-cyclopropansulfonylamino-2-methoxy-phenyl)-amid,
(S)-1-Methyl-7-[4-(1-methyl-pyrrolidin-2-carbonyl)-piperazin-1-ylmethyl]-1H-indol-2-carbonsäure-(3-methansulfonylamino-2-methoxy-5-pentafluoroethyl-phenyl)-amid,
(S)-1-Methyl-7-[4-(1-methyl-pyrrolidin-2-carbonyl)-piperazin-1-ylmethyl]-1H-indol-2-carbonsäure-(3-methansulfonylamino-2-methoxy-5-trifluoromethyl-phenyl)-amid,
(S)-1-Methyl-7-[4-(1-methyl-pyrrolidin-2-carbonyl)-piperazin-1-ylmethyl]-1H-indol-2-carbonsäure-(5-tert-butyl-3-hydroxymethyl-2-methoxy-phenyl)-amid,
deren Tautomere, deren Stereoisomere und deren Gemische.

**15.** Physiologisch verträgliche Salze der Verbindungen nach einem oder mehreren der Ansprüche 1 bis 14 mit anorganischen oder organischen Säuren.

**16.** Verwendung einer Verbindung nach einem oder mehreren der Ansprüche 1 bis 14 oder eines physiologisch verträglichen Salzes gemäß Anspruch 15 zur Herstellung eines Arzneimittels.

**17.** Arzneimittel, enthaltend eine Verbindung nach einem oder mehreren der Ansprüche 1 bis 14 oder ein physiologisch verträgliches Salz gemäß Anspruch 15 neben gegebenenfalls einem oder mehreren inerten Trägerstoffen, Konservierungs- und/oder Verdünnungsmitteln.

**18.** Verwendung mindestens einer Verbindung nach einem oder mehreren der Ansprüche 1 bis 14 oder eines physiologisch verträglichen Salzes gemäß Anspruch 15, zur Herstellung eines Arzneimittels, das zur Behandlung oder Prophylaxe von Atemwegserkrankungen geeignet ist.

**19.** Verwendung nach Anspruch 18, **dadurch gekennzeichnet, dass** es sich bei der Atemwegserkrankung um COPD oder Asthma handelt.

**20.** Verfahren zur Herstellung eines Arzneimittels gemäß Anspruch 17, **dadurch gekennzeichnet, dass** auf nichtchemischem Wege eine Verbindung nach mindestens einem der Ansprüche 1 bis 14 oder ein physiologisch verträgliches Salz gemäß Anspruch 15 in einen oder mehrere inerte Trägerstoffe, Konservierungs- und/oder Verdünnungsmittel eingearbeitet wird.

## Claims

**1.** Compounds of general formula I

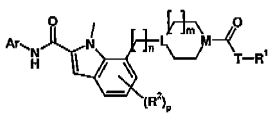

I

wherein

Ar denotes a substituent of formula (II), (III) or (IV)

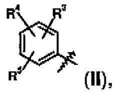 (II), 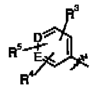 (III), 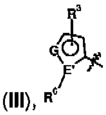 (IV)

wherein $R^3$ denotes $C_{1-6}$-alkyl, $C_{3-6}$-cycloalkyl, $C_{1-4}$-alkoxy, $C_{1-2}$-perfluoroalkyl, 3-methyl-oxetan-3-yl, $C_{1-2}$-perfluoroalkoxy, morpholinyl,
wherein the cycloalkyl group may optionally be substituted by $C_{1-3}$-alkyl,
wherein $R^4$ denotes H, $C_{1-4}$-alkyl, $C_{1-4}$-alkoxy,
wherein $R^5$ denotes H, $C_{1-5}$-alkyl-sulphonyl-amino, $C_{3-6}$-cycloalkyl-sulphonyl-amino, ($C_{1-5}$-alkyl-sulphonyl)-(methyl)-amino, ($C_{3-6}$-cycloalkyl-sulphonyl)-(methyl)-amino, $C_{1-5}$-alkyl-carbonyl-amino, $C_{3-6}$-cycloalkyl-carbonyl-amino, ($C_{1-5}$-alkyl-carbonyl)-(methyl)-amino, ($C_{3-6}$-cycloalkyl-carbonyl)-(methyl)-amino, aminocarbonyl, $C_{1-5}$-alkyl-amino-carbonyl, $C_{3-6}$-cycloalkyl-amino-carbonyl, di-($C_{1-3}$-alkyl)-amino-carbonyl, di-($C_{1-3}$-alkyl)-amino-$C_{1-2}$-alkyl, di-($_{1-3}$-alkyl)-amino-pyrrolidin-1-yl-$C_{1-2}$-alkyl, 4-$C_{1-5}$-alkyl-piperazin-1-yl-$C_{1-2}$-alkyl, 4-di-($C_{1-3}$-alkyl)-amino-piperidin-1-yl-$C_{1-2}$-alkyl, 3-di-($C_{1-3}$-alkyl)-amino-piperidin-1-yl-$C_{1-2}$-alkyl, $C_{1-5}$-alkyl-sulphinyl, $C_{3-6}$-cycloalkyl-sulphinyl, $C_{1-5}$-alkyl-sulphonyl, $C_{3-6}$-cycloalkyl-sulphonyl, hydroxy-$C_{1-2}$-alkyl, $C_{1-5}$-alkyl-sulphinyl-methyl, $C_{1-5}$-alkyl-sulphonyl-methyl,
wherein $R^6$ denotes $C_{1-3}$-alkyl or phenyl,
wherein the $C_{1-3}$-alkyl group may optionally be substituted by hydroxy or di-($C_{1-3}$-alkyl)-amino and
wherein the phenyl group may optionally be substituted by fluorine or $C_{1-3}$-alkyl.
wherein D or E represents nitrogen,
wherein G and E' independently of one another represent nitrogen or oxygen,wherein $R^6$ is only bound to E' if E' denotes nitrogen,
$R^1$ denotes a $C_{4-6}$-cycloalkyl system, which contains 1 to 2 nitrogen atoms that may optionally be 1- to 2-substituted by $R^7$,
wherein $R^7$ may be $C_{1-3}$-alkyl, hydroxy, $C_{1-3}$-alkoxy, amino, $C_{1-3}$-alkyl-amino, di-($C_{1-3}$-alkyl)-amino,
$R^2$ denotes hydrogen, halogen or $C_{1-4}$-alkyl,
L denotes-C(H)< or -N<,
M denotes-C(H)< or-N<,
T denotes a bond or $C_{1-4}$-alkylene,
wherein the $C_{1-4}$-alkylene group may be substituted by $C_{1-2}$-alkyl,
m denotes 0, 1, 2 or 3,
n denotes 1, 2 or 3,
p denotes 0, 1, 2 or 3,

wherein, unless stated otherwise, the above-mentioned alkyl groups may be straight-chain or branched,
the tautomers, the stereoisomers, the mixtures and the salts thereof, particularly the physiologically acceptable salts thereof.

**2.** Compounds of general formula (I) according to claim 1,
wherein
Ar is a substituent according to general formula (II')

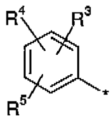 (II')

wherein $R^3$ denotes $-C_1$-$C_6$-alkyl, $-C_3$-$C_6$-cycloalkyl, $-CF_3$, $-CF_2CF_3$ or

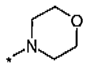,

wherein the $-C_3-C_6$-cycloalkyl group may be substituted by $-C_1-C_3$-alkyl, wherein $R^4$ denotes -H, $-C_1-C_4$-alkyl or $-O-C_1-C_4$-alkyl,
wherein $R^5$ is selected from
$-NH-S(O)_2-C_1-C_4$-alkyl;
$-NH-S(O)_2-C_3-C_5$-cycloalkyl;
$-N(CH_3)-S(O)_2-C_1-C_4$-alkyl;
$-N(CH_3)-S(O)_2-C_3-C_5$-cycloalkyl;
$-NH-C(O)-C_1-C_4$-alkyl;
$-NH-C(O)-C_3-C_5$-cycloalkyl;
$-N(CH_3)-C(O)-C_1-C_4$-alkyl;
$-N(CH_3)-C(O)-C_3-C_5$-cycloalkyl;
$-C(O)-NH_2$;
$-C(O)-NH-C_1-C_4$-alkyl;
$-C(O)-N(di-C_1-C_4$-alkyl) ;
$-C(O)-NH-C_3-C_5$-cycloalkyl;
$-C(O)-N(C_1-C_4$-alkyl)$(C_3-C_5$-cycloalkyl);
$-S(O)-C_1-C_4$-alkyl;
$-S(O)-C_3-C_5$-cycloalkyl;
$-S(O)_2-C_1-C_4$-alkyl;
$-S(O)_2-C_3-C_5$-cycloalkyl;
$-C_1-C_4$-alkyl-OH
$-CH_2-S(O)-C_1-C_4$-alkyl;
$-CH_2-S(O)-C_3-C_5$-cycloalkyl;
$-CH_2-S(O)_2-C_1-C_4$-alkyl;
$-CH_2-S(O)_2-C_3-C_5$-cycloalkyl;
$-C_1-C_4$-alkylene-$N(R^8,R^{8'})$;
wherein $R^1$ denotes $-C_4-C_6$-cycloalkyl, which contains 1 to 2 nitrogen atoms in the ring,
wherein $R^1$ is optionally substituted by a group selected from $-C_1-C_4$-alkyl, -OH, $-O-C_1-C_4$-alkyl, and $-N(R^9,R^{9'})$,
wherein $R^2$ is selected from -H, -halogen and $C_1-C_4$-alkyl,
wherein in each case $R^8$, $R^{8'}$, $R^9$ and $R^{9'}$ independently of one another are selected from -H and $-C_1-C_5$-alkyl,
wherein the two groups $R^8$ and $R^{8'}$ together with the nitrogen to which they are bound may form a 4- to 6-membered ring system, which may be substituted by -OH or-$N(R^{10},R^{10'})$,
wherein in each case $R^{10}$ and $R^{10'}$ independently of one another are selected from -H and $-C_1-C_5$-alkyl,
wherein L and M independently of one another are selected from -CH< and -N<,
wherein T is selected from a bond and $-C_1-C_3$-alkylene,
wherein the $C_{1-3}$-alkylene group may be substituted by methyl,
wherein m may be 0-3,
wherein n may be 1-3,
wherein p may be 0-3,
optionally in the form of the individual optical isomers thereof, mixtures of these isomers, racemates, optionally in the form of the acid addition salts thereof with pharmacologically acceptable acids.

**3.** Compounds of general formula (I) according to one of the preceding claims, wherein
Ar is a substituent according to general formula (II''')

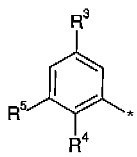
(II''')

wherein $R^3$ denotes $-C(CH_3)_3$, $-CH(CH_3)(C_2H_5)$, $-i-C_3H_7$, $-CH_2-i-C_3H_7$, $-CF_3$ or $-CF_2CF_3$,
wherein $R^4$ denotes $-O-CH_3$ or $-O-C_2H_5$,

wherein $R^5$ is selected from $-NH-S(O)_2-CH_3$, $-NH-S(O)_2-n-C_3H_7$, $-NH-S(O)_2-c-C_3H_5$, $-NH-C(O)-CH_3$, $-NH-C(O)-C_2H_5$, $-NH-C(O)-n-C_3H_7$, $-NH-C(O)-i-C_3H_7$, $-NH-C(O)-c-C_3H_5$, $-C(O)-NH_2$, $-C(O)-NH-CH_3$, $-O(O)-NH-C_2H_5$, $-C(O)-NH-i-C_8H_7$, $-C(O)-NH-c-C_3H_5$, $-C(O)-N(CH_3)_2$,$-CH_2-N(CH_3)_2$, ,$-CH_2-N(C_2H_5)_5$, ,$-CH_2-N(i-C_3H_7)_2$,

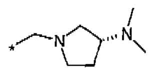, 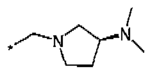, 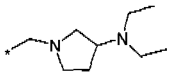, 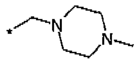,

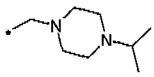, 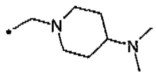, 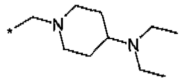, 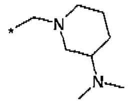, -

$S(O)-CH_3$, $-S(O)_2-CH_3$,
$-CH_2-OH$, $-CH_2-S(O)-CH_3$ or $-CH_2-S(O)_2-CH_3$,
wherein $R^1$ is selected from

, 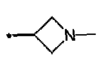, 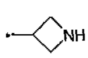, 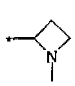, 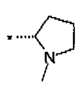, 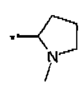, 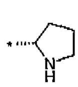, 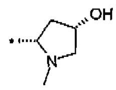,

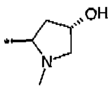, 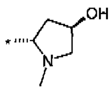, 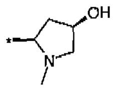, 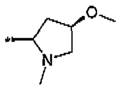, 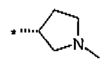, 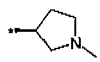,

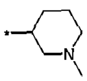, 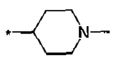, 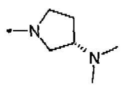, 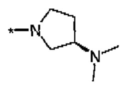, 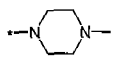, 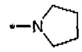,

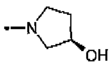, 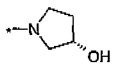, 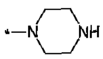, 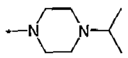, 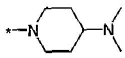,

wherein $R^2$ denotes -H,
wherein L is selected from -CH< and -N<,
wherein M denotes -N<,
wherein T denotes a bond,
wherein m denotes 1,
wherein n denotes 1,
optionally in the form of the individual optical isomers thereof, mixtures of these isomers, racemates, optionally in the form of the acid addition salts thereof with pharmacologically acceptable acids.

**4.** Compounds of general formula (I) according to one of the preceding claims, wherein Ar is a substituent according to general formula (II')

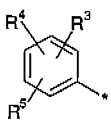

(II')

wherein $R^3$ denotes $-C_1-C_8$-alkyl, $-C_3-C_6$-cycloalkyl, $-CF_3$, $-CF_2CF_3$ or

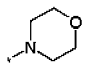

,

wherein the $-C_3-C_8$-cycloalkyl group may be substituted by $-C_1-C_3$-alkyl,
wherein $R^4$ denotes -H, $-C_1-C_4$-alkyl or $-O-C_1-C_4$-alkyl,
wherein $R^5$ is selected from
$-NH-S(O)_2-C_1-C_4$-alkyl;
$-NH-S(O)_2-C_3-C_5$-cycloalkyl;
$-N(CH_3)-S(O)_2-C_1-C_4$-alkyl ;
$-N(CH_3)-S(O)_2-C_3-C_5$-cycloalkyl;
$-NH-C(O)-C_1-C_4$-alkyl;
$-NH-C(O)-C_3-C_5$-cycloalkyl;
$-N(CH_3)-C(O)-C_1-C_4$-alkyl;
$-N(CH_3)-C(O)-C_3-C_5$-cycloalkyl;
$-C(O)-NH_2$;
$-C(O)-NH-C_1-C_4$-alkyl;
-C(O)-N(di-$C_1-C_4$-alkyl) ;
$-C(O)-NH-C_3-C_5$-cycloalkyl;
-C(O)-N($C_1-C_4$-alkyl)($C_3-C_5$-cycloalkyl);
$-S(O)-C_1-C_4$-alkyl;
$-S(O)-C_3-C_5$-cycloalkyl;
$-S(O)_2-C_1-C_4$-alkyl;
$-S(O)_2-C_3-C_5$-cycloalkyl;
$-C_1-C_{47}$-alkyl-OH
$-CH_2-S(O)-C_1-C_4$-alkyl;
$-CH_2-S(O)-C_3-C_5$-cycloalkyl;
$-CH_2-S(O)_2-C_1-C_4$-alkyl;
$-CH_2-S(O)_2-C_3-C_5$-cycloalkyl;
$-C_1-C_4$-alkylene-N($R^8$,$R^{8'}$);
wherein $R^8$ and $R^{8'}$ are each selected independently of one another from -H and $-C_1-C_5$-alkyl,
wherein the two groups $R^8$ and $R^{8'}$ together with the nitrogen to which they are bound may form a 4- to 6-membered ring system, which may be substituted by-OH or -N($R^{10}$,$R^{10'}$),
wherein $R^{10}$ and $R^{10'}$ are each selected independently of one another from -H and-$C_1-C_5$-alkyl,
optionally in the form of the individual optical isomers thereof, mixtures of these isomers, racemates, optionally in the form of the acid addition salts thereof with pharmacologically acceptable acids.

**5.** Compounds of general formula (I) according to one of the preceding claims,
wherein $R^1$ denotes a $C_{4-6}$-cycloalkyl system, which contains 1 to 2 nitrogen atoms that may optionally be 1- to 2-substituted by $R^7$, wherein $R^7$ may be $C_{1-3}$-alkyl, hydroxy, $C_{1-3}$-alkoxy, amino, $C_{1-3}$-alkyl-amino, di-($C_{1-3}$-alkyl)-amino, optionally in the form of the individual optical isomers thereof, mixtures of these isomers, racemates, optionally in the form of the acid addition salts thereof with pharmacologically acceptable acids.

**6.** Compounds of general formula (I) according to one of the preceding claims, wherein
R’ denotes azetidin-2-yl, azetidin-3-yl, 1-methyl-azetidin-2-yl, 1-methyl-azetidin-3-yl, 1-ethyl-azetidin-2-yl, 1-ethyl-azetidin-3-yl, pyrrolidin-1-yl, pyrrolidin-2-yl, pyrrolidin-3-yl, 3-hydroxy-pyrrolidin-1-yl, 3-amino-pyrrolidin-1-yl, 3-methylamino-pyrrolidin-1-yl, 3-dimethylamino-pyrrolidin-1-yl, 3-ethylamino-pyrrolidin-1-yl, 3-diethylamino-pyrrolidin-1-yl, 1-methyl-pyrrolidin-2-yl, 1-methyl-pyrrolidin-3-yl, 1-ethyl-pyrrolidin-2-yl, 1-ethyl-pyrrolidin-3-yl, 1-methyl-4-hydroxy-pyrrolidin-2-yl, 1-methyl-4-methoxy-pyrrolidin-2-yl, piperidin-1-yl, piperidin-2-yl, piperidin-3-yl, piperidin-4-yl, 4-amino-piperidin-1-yl, 4-methylamino-piperidin-1-yl, 4-dimethylamino-piperidin-1-yl, 4-ethylamino-piperidin-1-yl, 4-diethylamino-piperidin-1-yl, 1-methyl-piperidin-2-yl, 1-methyl-piperidin-3-yl, 1-methyl-piperidin-4-yl, 1-ethyl-piperidin-2-yl, 1-ethyl-piperidin-3-yl, 1-ethyl-piperidin-4-yl, piperazin-1-yl, 4-methyl-piperazin-1-yl, 4-ethyl-piperazin-1-yl, 4-isopropyl-piperazin-1-yl,
optionally in the form of the individual optical isomers thereof, mixtures of these isomers, racemates, optionally in the form of the acid addition salts thereof with pharmacologically acceptable acids.

**7.** Compounds of general formula (I) according to one of claims 1 to 4, wherein

$R^1$ is selected from $-C_4-C_6$-cycloalkyl, which contains 1 to 2 nitrogen atoms in the ring, wherein $R^1$ is optionally substituted by a group selected from $-C_1-C_4$-alkyl, -OH, $-O-C_1-C_4$-alkyl, and $-N(R^9,R^{9'})$, wherein $R^9$, $R^{9'}$ have the meanings given above, optionally in the form of the individual optical isomers thereof, mixtures of these isomers, racemates, optionally in the form of the acid addition salts thereof with pharmacologically acceptable acids.

**8.** Compounds of general formula (I) according to one of claims 1-4, wherein R' is selected from

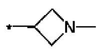, 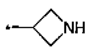, 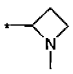, 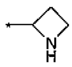, 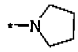, 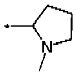, 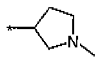,

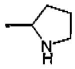, 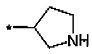, 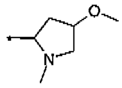, 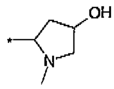, 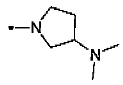, 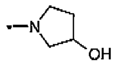,

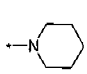, 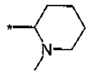, 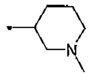, 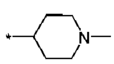, 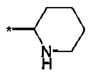, 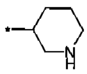,

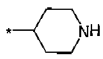, 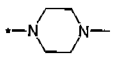, 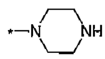, 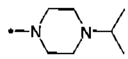,

optionally in the form of the individual optical isomers thereof, mixtures of these isomers, racemates, optionally in the form of the acid addition salts thereof with pharmacologically acceptable acids.

**9.** Compounds of general formula (I) according to one of the preceding claims, wherein $R^2$ is selected from -H, -F, -Cl and $-CH_3$, and wherein p is selected from 0, 1, 2 or 3 optionally in the form of the individual optical isomers thereof, mixtures of these isomers, racemates, optionally in the form of the acid addition salts thereof with pharmacologically acceptable acids.

**10.** Compounds of general formula (I) according to one of the preceding claims, wherein L and M independently of one another are selected from -CH< and -N<, optionally in the form of the individual optical isomers thereof, mixtures of these isomers, racemates, optionally in the form of the acid addition salts thereof with pharmacologically acceptable acids.

**11.** Compounds of general formula (I) according to one of the preceding claims, wherein T is selected from a bond and $-C_1-C_3$-alkylene, where the $-C_1-C_3$-alkylene group may be substituted by methyl, optionally in the form of the individual optical isomers thereof, mixtures of these isomers, racemates, optionally in the form of the acid addition salts thereof with pharmacologically acceptable acids.

**12.** Compounds of general formula (I) according to one of the preceding claims, for the treatment of diseases selected from asthma or COPD.

**13.** Use of compounds of general formula (I) according to one of the preceding claims for preparing a medicament for the treatment of diseases selected from asthma or COPD.

**14.** Compounds of general formula (I) according to claim 1, selected from
1-Methyl-7-[4-(1-methyl-piperidine-4-carbonyl)-piperazin-1-ylmethyl]-1H-indole-2-carboxylic acid (5-tert-butyl-3-methanesulphonylamino-2-methoxy-phenyl)-amide,
(S)-1-methyl-7-[4-(1-methyl-pyrrolidine-2-carbonyl)-piperazin-1-ylmethyl]-1H-indole-2-carboxylic acid (5-tert-butyl-3-methanesulphonylamino-2-mothoxy-phenyl)-amide,
1-methyl-7-[4-(1-methyl-azetidine-3-carbonyl)-piperazin-1-ylmethyl]-1H-indole-2-carboxylic acid (5-tert-butyl-3-methanesulphonylamino-2-methoxy-phenyl)-amide,
(R)-1-methyl-7-[4-(1-methyl-pyrrolidine-2-carbonyl)-piperazin-1-ylmethyl]-1H-indole-2-carboxylic acid (5-tert-butyl-3-methanesulphonylamino-2-methoxy-phenyl)-amide,
(S)-1-methyl-7-[4-(pyrrolidine-2-carbonyl)-piperazin-1-ylmethyl]-1H-indole-2-carboxylic acid (5-tert-butyl-3-methanesulphonylamino-2-methoxy-phenyl)-amide,
1-methyl-7-[4-(1-methyl-azetidine-2-carbonyl)-piperazin-1-ylmethyl]-1H-indole-2-carboxylic acid (5-tert-butyl-3-methanesulphonylamino-2-methoxy-phenyl)-amide,
(R)-1-methyl-7-[1-(1-methyl-pyrrolidine-2-carbonyl)-piperidin-4-ylmethyl]-1H-indole-2-carboxylic acid (5-tert-butyl-3-methanesulphonylamino-2-methoxy-phenyl)-amide,
(S)-1-methyl-7-[1-(1-methyl-pyrrolidine-2-carbonyl)-piperidin-4-ylmethyl]-1H-indole-2-carboxylic acid (5-tert-butyl-3-methanesulphonylamino-2-methoxy-phenyl)-amide,
7-[4-((25,4R)-4-hydroxy-1-methyl-pyrrolidine-2-carbonyl)-piperazin-1-ylmethyl]-1-methyl-1H-indole-2-carboxylic acid (5-tert-butyl-3-methanesulphonylamino-2-methoxyphenyl)-amide,
7-[4-((2R,4R)-4-methoxy-1-methyl-pyrrolidine-2-carbonyl)-piperazin-1-ylmethyl]-1-methyl-1H-indole-2-carboxylic acid (5-tert-butyl-3-methanesulphonylamino-2-methoxy phenyl)-amide,
7-[4-((2R,4R)-4-hydroxy-1-methyl-pyrrolidine-2-carbonyl)-piperazin-1-ylmethyl]-1-methyl-1H-indole-2-carboxylic acid (5-tert-butyl-3-methanesulphonylamino-2-methoxy phenyl)-amide,
7-[4-((2R,4S)-4-hydroxy-1-methyl-pyrrolidine-2-carbonyl)-piperazin-1-ylmethyl]-1-methyl-1H-indole-2-carboxylic acid (5-tert-butyl-3-methanesulphonylamino-2-methoxy phenyl)-amide,
7-[4-((2S,4S)-4-hydroxy-1-methyl-pyrrolidine-2-carbonyl)-piperazin-1-ylmethyl]-1-methyl-1H-indole-2-carboxylic acid (5-tert-butyl-3-methanesulphonylamino-2-methoxy phenyl)-amide,
1-methyl-7-[4-(2-pyrrolidin-1-yl-propionyl)-piperazin-1-ylmethyl]-1H-indole-2-carboxylic acid-(5-tert-butyl-3-methanesulphonylamino-2-methoxy-phenyl)-amide,
7-{4-[2-(4-isopropyl-piperazin-1-yl)-acetyl]-piperazin-1-ylmethyl}-1-methyl-1H-indole-2-carboxylic acid-(5-tert-butyl-3-methanesulphonylamino-2-methoxy-phenyl)-amide,
(S)-7-{4-[2-(3-hydroxy-pyrrolidin-1-yl)-acetyl]-piperazin-1-ylmethyl}-1-methyl-1H-indole-2-carboxylic acid-(5-tert-butyl-3-methanesulphonylamino-2-methoxy-phenyl)-amide,
1-methyl-7-[4-(2-pyrrolidin-1-yl-acetyl)-piperazin-1-ylmethyl]-1H-indole-2-carboxylic acid-(5-tert-butyl-3-methanesulphonylamino-2-methoxy-phenyl)-amide,
1-methyl-7-{4-[2-(4-methyl-piperazin-1-yl)-acetyl]-piperazin-1-ylmethyl}-1H-indole-2-carboxylic acid-(5-tert-butyl-3-methanesulphonylamino-2-methoxy-phenyl)-amide,
1-methyl-7-[1-((28)-1-methyl-pyrrolidine-2-carbonyl)-azepan-4-ylmethyl]-1H-indole-2-carboxylic acid-(5-tert-butyl-3-methanesulphonylamino-2-methoxy-phenyl)-amide,
1-methyl-7-[1-(1-methyl-piperidine-4-carbonyl)-azepan-4-ylmethyl]-1H-indole-2-carboxylic acid-(5-tert-butyl-3-methanesulphonylamino-2-methoxy-phenyl)-amide,
1-methyl-7-[1-((2R)-1-methyl-pyrrolidine-2-carbonyl)-azepan-4-ylmethyl]-1H-indole-2-carboxylic acid-(5-tert-butyl-3-methanesulphonylamino-2-methoxy-phenyl)-amide,
1-methyl-7-[4-((2R)-1-methyl-pyrrolidine-2-carbonyl)-[1,4]diazepan-1-ylmethyl]-1H-indole-2-carboxylic acid-(5-tert-butyl-3-methanesulphonylamino-2-methoxy-phenyl)-amide,
1-methyl-7-[4-(4-methyl-piperazine-1-carbonyl)-piperidin-1-ylmethyl]-1H-indole-2-carboxylic acid-(5-tert-butyl-3-methanesulphonylamino-2-methoxy-phenyl)-amide,
(S)-7-[4-(3-dimethylamino-pyrrolidine-1-carbonyl)-piperidin-1-ylmethyl]-1-methyl-1H-indole-2-carboxylic acid-(5-tert-butyl-3-methanesulphonylamino-2-methoxy-phenyl)-amide,
(R)-7-[4-(3-dimethylamino-pyrrolidine-1-carbonyl)-piperidin-1-ylmethyl]-1-methyl-1H-indole-2-carboxylic acid-(5-tert-butyl-3-methanesulphonylamino-2-methoxy-phenyl)-amide,
(S)-1-methyl-7-[4-(1-methyl-pyrrolidine-2-carbonyl)-piperazin-1-ylmethyl]-1H-indole-2-carboxylic acid-(3-morpholin-4-yl-phenyl)-amide,
(R)-7-[4-(3-dimethylamino-pyrrolidine-1-carbonyl)-piperazin-1-ylmethyl]-1-methyl-1H-indole-2-carboxylic acid (5-tert-butyl-3-methanesulphonylamino-2-methoxy-phenyl)-amide,
(S)-7-[4-(3-dimethylamino-pyrrolidine-1-carbonyl)-piperazin-1-ylmethyl]-1-methyl-1H-indole-2-carboxylic acid (5-tert-butyl-3-methanesulphonylamino-2-methoxy-phenyl)-amide,
7-[4-(azetidine-3-carbonyl)-piperazin-1-ylmethyl]-1-methyl-1H-indole-2-carboxylic acid (5-tert-butyl-3-methanesulphonylamino-2-methoxy-phenyl)-amide,
1-methyl-7-[4-(1-methyl-piperidine-3-carbonyl)-piperazin-1-ylmethyl]-1H-indole-2-carboxylic acid (5-tert-butyl-3-methanesulphonylamino-2-methoxy-phenyl)-amide,
(S)-1-methyl-7-[4-(1-methyl-pyrrolidine-3-carbonyl)-piperazin-1-ylmethyl]-1H-indole-2-carboxylic acid (5-tert-butyl-3-methanesulphonylamino-2-methoxy-phenyl)-amide,
(S)-1-methyl-7-[4-(1-methyl-pyrrolidine-3-carbonyl)-[1,4]diazepan-1-ylmethyl]-1H-indole-2-carboxylic acid-(5-tert-butyl-3-methanesulphonylamino-2-methoxy-phenyl)-amide,
(R)-1-methyl-7-[4-(1-methyl-pyrrolidine-3-carbonyl)-[1,4]diazepan-1-ylmethyl]-1H-indole-2-carboxylic acid-(5-tert-butyl-3-methanesulphonylamino-2-methoxy-phenyl)-amide,
7-{4-[2-(4-dimethylamino-piperidin-1-yl)-acetyl]-piperazin-1-ylmethyl}-1-methyl-1H-indole-2-carboxylic acid-(5-tert-butyl-3-methanesulphonylamino-2-methoxy-phenyl)-amide,
(R)-7-{4-[2-(3-hydroxy-pyrrolidin-1-yl)-acetyl]-piperazin-1-ylmethyl}-1-methyl-1H-indole-2-carboxylic acid-(5-tert-butyl-3-methanesulphonylamino-2-methoxy-phenyl)-amide,
(R)-7-{4-[2-(3-dimethylamino-pyrrolidin-1-yl)-acetyl]-piperazin-1-ylmethyl}-1-methyl-1H-indole-2-carboxylic acid-(5-tert-butyl-3-methanesulphonylamino-2-methoxy-phenyl)-amide,
(S)-7-{4-[2-(3-dimethylamino-pyrrolidin-1-yl)-acetyl]-piperazin-1-ylmethyl}-1-methyl-1H-indole-2-carboxylic acid-(5-tert-butyl-3-methanesulphonylamino-2-methoxy-phenyl)-amide,
1-methyl-7-[4-(2-piperazin-1-yl-acetyl)-piperazin-1-ylmethyl]-1H-indole-2-carboxylic acid-(5-tert-butyl-3-methanesulphonylamino-2-methoxy-phenyl)-amide
1-methyl-7-[4-((2S)-1-methyl-pyrrolidine-2-carbonyl)-[1,4]diazepan-1-ylmethyl]-1H-indole-2-carboxylic acid-(5-tert-butyl-3-methanesulphonylamino-2-methoxy-phenyl)-amide,
1-methyl-7-[4-(1-methyl-piperidine-4-carbonyl)-[1,4]diazepan-1-ylmethyl]-1H-indole-2-carboxylic acid-(5-tert-butyl-3-methanesulphonylamino-2-methoxy-phenyl)-amide,
(S)-1-methyl-7-[4-(1-methyl-pyrrolidine-2-carbonyl)-piperazin-1-ylmethyl]-1H-indole-2-carboxylic acid-(5-isobutyl-3-methanesulphonylamino-2-methoxy-phenyl)-amide,
(S)-1-methyl-7-[4-(1-methyl-pyrrolidine-2-carbonyl)-piperazin-1-ylmethyl]-1H-indole-2-carboxylic acid-[5-tert-butyl-2-methoxy-3-(4-methyl-piperazin-1-ylmethyl)-phenyl]-amide,
(S)-1-methyl-7-[4-(1-methyl-pyrrolidine-2-carbonyl)-piperazin-1-ylmethyl]-1H-indole-2-carboxylic acid-{5-tert-butyl-3-[(diisopropylamino)-methyl]-2-methoxy-phenyl}-amide
(S)-1-methyl-7-[4-(1-methyl-pyrrolidine-2-carbonyl)-piperazin-1-ylmethyl]-1H-indole-2-carboxylic acid-(5-tert-butyl-3-diethylaminomethyl-2-methoxy-phenyl)-amide,
1-methyl-7-[4-((2S)-1-methyl-pyrrolidine-2-carbonyl)-piperazin-1-ylmethyl]-1H-indole-2-carboxylic acid-[5-tert-butyl-3-(3-dimethylamino-piperidin-1-ylmethyl)-2-methoxy-phenyl]-amide,
1-methyl-7-[4-((2S)-1-methyl-pyrrolidine-2-carbonyl)-piperazin-1-ylmethyl]-1H-indole-2-carboxylic acid-[5-tert-butyl-3-(3-diethylamino-pyrrolidin-1-ylmethyl)-2-methoxy-phenyl]-amide,
(S)-1-methyl-7-[4-(1-methyl-pyrrolidine-2-carbonyl)-piperazin-1-ylmethyl]-1H-indole-2-carboxylic acid-[5-tert-butyl-3-(4-dimethylamino-piperidin-1-ylmethyl)-2-methoxy-phenyl]-amide,
(S)-1-methyl-7-[4-(1-methyl-pyrrolidine-2-carbonyl)-piperazin-1-ylmethyl]-1H-indole-2-carboxylic acid-[5-tert-butyl-3-(4-diethylamino-piperidin-1-ylmethyl)-2-methoxy-phenyl]-amide,
(S)-1-methyl-7-[4-(1-methyl-pyrrolidine-2-carbonyl)-piperazin-1-ylmethyl]-1H-indole-2-carboxylic acid-[5-tert-butyl-3-(4-isopropyl-piperazin-1-ylmethyl)-2-methoxy-phenyl]-amide,
(S)-1-methyl-7-[4-(1-methyl-pyrrolidine-2-carbonyl)-piperazin-1-ylmethyl]-1H-indole-2-carboxylic acid-(5-tert-butyl-3-cyclopropylcarbamoyl-2-methoxy-phenyl)-amide,
(S)-1-methyl-7-[4-(1-methyl-pyrrolidine-2-carbonyl)-piperazin-1-ylmethyl]-1H-indole-2-carboxylic acid-(5-tert-butyl-3-isopropylcarbamoyl-2-methoxy-phenyl)-amide,
(S)-1-methyl-7-[4-(1-methyl-pyrrolidine-2-carbonyl)-piperazin-1-ylmethyl]-1H-indole-2-carboxylic acid-(5-tert-butyl-3-ethylcarbamoyl-2-methoxy-phenyl)-amide
(S)-1-methyl-7-[4-(1-methyl-pyrrolidine-2-carbonyl)-piperazin-1-ylmethyl]-1H-indole-2-carboxylic acid-(5-tert-butyl-2-methoxy-3-methylcarbamoyl-phenyl)-amide,
(S)-1-methyl-7-[4-(1-methyl-pyrrolidine-2-carbonyl)-piperazin-1-ylmethyl]-1H-indole-2-carboxylic acid-(5-tert-butyl-3-carbamoyl-2-methoxy-phenyl)-amide,
(S)-1-methyl-7-[4-(1-methyl-pyrrolidine-2-carbonyl)-piperazin-1-ylmethyl]-1H-indole-2-carboxylic acid-(5-tert-butyl-3-dimethylcarbamoyl-2-methoxy-phenyl)-amide,
1-methyl-7-[4-((2S)-1-methyl-pyrrolidine-2-carbonyl)-piperazin-1-ylmethyl]-1H-indole-2-carboxylic acid-(5-sec-butyl-3-methanesulphonylamino-2-methoxy-phenyl)-amide,
(S)-1-methyl-7-[4-(1-methyl-pyrrolidine-2-carbonyl)-piperazin-1-ylmethyl]-1H-indole-2-carboxylic acid-(5-isopropyl-3-methanesulphonylamino-2-methoxy-phenyl)-amide,
1-methyl-7-[4-((2S)-1-methyl-pyrrolidine-2-carbonyl)-piperazin-1-ylmethyl]-1H-indole-2-carboxylic acid-(5-tert-butyl-3-methanesulphinyl-2-methoxy-phenyl)-amide,
(S)-1-methyl-7-[4-(1-methyl-pyrrolidine-2-carbonyl)-piperazin-1-ylmethyl]-1H-indole-2-carboxylic acid-(5-tert-butyl-3-methanesulphonyl-2-methoxy-phenyl)-amide,
(S)-1-methyl-7-[4-(1-methyl-pyrrolidine-2-carbonyl)-piperazin-1-ylmethyl]-1H-indole-2-carboxylic acid-(5-tert-butyl-3-methanesulphonylmethyl-2-methoxy-phenyl)-amide,
1-methyl-7-[4-((2S)-1-methyl-pyrrolidine-2-carbonyl)-piperazin-1-ylmethyl]-1H-indole-2-carboxylic acid-(5-tert-butyl-3-methanesulphinylmethyl-2-methoxy-phenyl)-amide,
(S)-1-methyl-7-[4-(1-methyl-pyrrolidine-2-carbonyl)-piperazin-1-ylmethyl]-1H-indole-2-carboxylic acid-(5-tert-butyl-3-methanesulphonylamino-2-ethoxy-phenyl)-amide,
(S)-1-methyl-7-[4-(1-methyl-pyrrolidine-2-carbonyl)-piperazin-1-ylmethyl]-1H-indole-2-carboxylic acid-(5-tert-butyl-2-methoxy-3-propionylamino-phenyl)-amide,
(S)-1-methyl-7-[4-(1-methyl-pyrrolidine-2-carbonyl)-piperazin-1-ylmethyl]-1H-indole-2-carboxylic acid-(5-tert-butyl-3-butyrylamino-2-methoxy-phenyl)-amide,
(S)-1-methyl-7-[4-(1-methyl-pyrrolidine-2-carbonyl)-piperazin-1-ylmethyl]-1H-indole-2-carboxylic acid-(3-acetylamino-5-tert-butyl-2-methoxy-phenyl)-amide,
(S)-1-methyl-7-[4-(1-methyl-pyrrolidine-2-carbonyl)-piperazin-1-ylmethyl]-1H-indole-2-carboxylic acid-(5-tert-butyl-3-isobutyrylamino-2-methoxy-phenyl)-amide,
(S)-1-methyl-7-[4-(1-methyl-pyrrolidine-2-carbonyl)-piperazin-1-ylmethyl]-1H-indole-2-carboxylic acid-[5-tert-butyl-3-(cyclopropanecarbonyl-amino)-2-methoxy-phenyl]-amide,
(S)-1-methyl-7-[4-(1-methyl-pyrrolidine-2-carbonyl)-piperazin-1-ylmethyl]-1H-indole-2-carboxylic acid-[5-tert-butyl-2-methoxy-3-(propane-1-sulphonylamino)-phenyl]-amide,
(S)-1-methyl-7-[4-(1-methyl-pyrrolidine-2-carbonyl)-piperazin-1-ylmethyl]-1H-indole-2-carboxylic acid-(5-tert-butyl-3-cyclopropanesulphonylamino-2-methoxy-phenyl)-amide,
(S)-1-methyl-7-[4-(1-methyl-pyrrolidine-2-carbonyl)-piperazin-1-ylmethyl]-1H-indole-2-carboxylic acid-(3-methanesulphonylamino-2-methoxy-5-pentafluoroethyl-phenyl)-amide,
(S)-1-methyl-7-[4-(1-methyl-pyrrolidine-2-carbonyl)-piperazin-1-ylmethyl]-1H-indole-2-carboxylic acid-(3-methanesulphonylamino-2-methoxy-5-trifluoromethyl-phenyl)-amide,
(S)-1-methyl-7-[4-(1-methyl-pyrrolidine-2-carbonyl)-piperazin-1-ylmethyl]-1H-indole-2-carboxylic acid-(5-tert-butyl-3-hydroxymethyl-2-methoxy-phenyl)-amide,
the tautomers, the stereoisomers and the mixtures thereof.

**15.** Physiologically acceptable salts of the compounds according to one or more of claims 1 to 14 with inorganic or organic acids.

**16.** Use of a compound according to one or more of claims 1 to 14 or a physiologically acceptable salt according to claim 15 as a medicament.

**17.** Medicament, containing a compound according to one or more of claims 1 to 14 or a physiologically acceptable salt according to claim 15 optionally together with one or more inert carriers, preservatives and/or diluents.

**18.** Use of at least one compound according to one or more of claims 1 to 14 or of a physiologically acceptable salt according to claim 15, for preparing a medicament that is suitable for the treatment or prevention of respiratory complaints.

**19.** Use according to claim 18, **characterised in that** the respiratory complaint is COPD or asthma.

**20.** Method for preparing a medicament according to claim 17, **characterised in that** a compound according to at least one of claims 1 to 14 or a physiologically acceptable salt according to claim 15 is incorporated by a non-chemical method in one or more inert carriers, preservatives and/or diluents.

## Revendications

**1.** Composés de formule générale I :

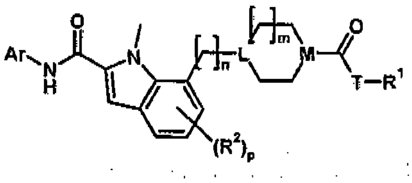

I

dans laquelle

Ar représente un substituant de formule (II), (III) ou (IV) :

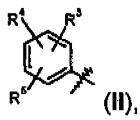 (II), 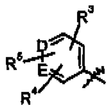 (III), 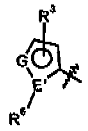 (IV)

- où $R^3$ représente alkyle en $C_{1-5}$, cycloalkyle en $C_{3-6}$, alcoxy en $C_{1-4}$, perfluoroalkyle en $C_{1-2}$, 3-méthyloxétan-3-yle, perfluoroalcoxy en $C_{1-2}$, morpholinyle,
- -où le radical cycloalkyle peut être le cas échéant substitué par alkyle en $C_{1-3}$,
- où $R^4$ représente H, alkyle en $C_{1-4}$, alcoxy en $C_{1-4}$,
- où $R^5$ représente H, (alkyl en $C_{1-5}$)sulfonylamino, (cycloalkyl en $C_{3-6}$)sulfonylamino, ((alkyl en $C_{1-5}$)-sulfonyl)(méthyl)amino, ((cycloalkyl en $C_{3-6}$)sulfonyl)-(méthyl)amino, (alkyl en $C_{1-5}$)carbonylamino, (cycloalkyl en $C_{3-6}$) carbonylamino, ((alkyl en $C_{1-5}$)carbonyl)(méthyl)-amino, ((cycloalkyl en $C_{3-6}$)-carbonyl)(méthyl)amino, aminocarbonyle, (alkyl en $C_{1-5}$)-aminocarbonyle, (cycloalkyl en $C_{3-6}$)aminocarbonyle, di (alkyl en $C_{1-3}$)aminocarbonyle, di(alkyl en $C_{1-3}$)-aminoalkyle en $C_{1-2}$, di-(alkyl en $C_{1-3}$)aminopyrrolidin-1-ylalkyle en $C_{1-2}$, 4-(alkyl en $C_{1-5}$) pipérazin-1-ylalkyle en $C_{1-2}$, 4-di(alkyl en $C_{1-3}$)aminopipéridin-1-ylalkyle en $C_{1-2}$, 3-di(alkyl en $C_{1-3}$)aminopipéridin-1-ylalkyle en $C_{1-2}$, (alkyl en $C_{1-5}$)-sulfinyle, (cycloalkyl en $C_{3-6}$)sulfinyle, (alkyl en $C_{1-5}$)sulfonyle, (cycloalkyl en $C_{3-6}$)sulfonyle, hydroxyalkyle en $C_{1-2}$, (alkyl en $C_{1-5}$)sulfinylméthyle, (alkyl en $C_{1-5}$)sulfonylméthyle,
- où $R^6$ représente alkyle en $C_{1-3}$ ou phényle,

  - - où le radical alkyle en $C_{1-3}$ peut être le cas échéant substitué par hydroxyle ou di(alkyl en $C_{1-3}$)-amino, et
  - - où le radical phényle peut être le cas échéant substitué par fluor ou alkyle en $C_{1-3}$,

- où D ou E représentent azote,
- où G et E' représentent indépendamment l'un de l'autre, azote ou oxygène,
- où $R^6$ n'est alors lié qu'à $E^1$, quand $E^1$ représente l'azote,

$R^1$ représente un système cycloalkyle en $C_{4-6}$, qui contient 1 à 2 atomes d'azote, qui peut être le cas échéant substitué 1 à 2 fois par $R^7$,
- où $R^7$ peut être alkyle en $C_{1-3}$, hydroxyle, alcoxy en $C_{1-3}$, amino, (alkyl en $C_{1-3}$)amino, di(alkyl en $C_{1-3}$)-amino,

$R^2$ représente hydrogène, halogène ou alkyle en $C_{1-4}$,
L représente -C(H)< où -N<,
M représente -C(H)< ou -N<,
T représente une liaison ou alkylène en $C_{1-4}$,
- où le radical alkylène en $C_{1-4}$ peut être substitué par alkyle en $C_{1-2}$,

m représente 0, 1, 2 ou 3,
n représente 1, 2 ou 3,
p représente 0, 1, 2 ou 3,
où, sauf indication contraire, les radicaux alkyle mentionnés peuvent être linéaires ou ramifiés,
leurs tautomères, leurs stéréoisomères, leurs mélanges et leurs sels, en particulier leurs sels physiologiquement compatibles.

**2.** Composés de la formule générale (I) selon la revendication 1,
où

- Ar est un substituant de la formule générale (II') :

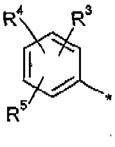 (II')

- où $R^3$ représente alkyle en $C_{1-8}$, cycloalkyle en $C_{3-5}$, $-CF_3$, $-CF_2CF_3$ ou

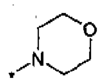 ,

- - où le radical cycloalkyle en $C_{3-6}$ peut être substitué par alkyle en $C_{1-3}$,
- où $R^4$ représente H, alkyle en $C_{1-4}$ ou -O-alkyl en $C_{1-4}$,
- où $R^5$ est choisi parmi
- -NH-$S(O)_2$-alkyl en $C_{1-4}$,
- -NH-$S(O)_2$-cycloalkyl en $C_{3-5}$,
- -$N(CH_3)$-$S(O)_2$-alkyl en $C_{1-4}$,
- -$N(CH_3)$-$S(O)_2$-cycloalkyl en $C_{3-5}$,
- -NH-C(O)-alkyl en $C_{1-4}$,
- -NH-C(O)-cycloalkyl en $C_{3-5}$,
- -$N(CH_3)$-C(O)-alkyl en $C_{1-4}$,
- -$N(CH_3)$-C(O)-cycloalkyl en $C_{3-5}$,
- -C(O)-$NH_2$,
- -C(O)-NH-(alkyl en $C_{1-4}$),
- -C(O)-N-di(alkyl en $C_{1-4}$),
- -C(O)-NH-cycloalkyl en $C_{3-5}$,
- -C(O)-N(alkyl en $C_{1-4}$) (cycloalkyl en $C_{3-5}$),
- -S(O)-alkyl en $C_{1-4}$,
- -S(O)-cycloalkyl en $C_{3-5}$,
- -$S(O)_2$-alkyl en $C_{1-4}$,
- -$S(O)_2$-cycloalkyl en $C_{3-5}$,
- -alkyle en $C_{1-5}$-OH,
- -$CH_2$-S(O)-alkyl en $C_{1-4}$,
- -$CH_2$-S(O)-cycloalkyl en $C_{3-5}$,
- -$CH_2$-$S(O)_2$-alkyl en $C_{1-4}$,
- -$CH_2$-$S(O)_2$-cycloalkyl en $C_{3-5}$,
- -alkylène en $C_{1-4}$-$N(R^8,R^8)$ ;

où $R^1$ représente cycloalkyle en $C_{4-6}$, qui contient 1 à 2 atomes d'azote dans le cycle,
- où $R^1$ est le cas échéant substitué par un radical choisi parmi alkyle en $C_{1-4}$, -OH, -O-alkyl en $C_{1-4}$, et $N(R^9,R^{9'})$,

où $R^2$ est choisi parmi H, halogène et alkyle en $C_{1-4}$,
où chaque $R^8$, $R^{8'}$, $R^9$, $R^{9'}$ est choisi indépendamment les uns des autres parmi H et alkyle en $C_{1-5}$,
où les deux radicaux $R^8$ et $R^{8'}$ avec l'atome d'azote auquel ils sont liés, peuvent former un système cyclique ayant 4 à 6 membres, qui peut être substitué par -OH ou -$N(R^{10}, R^{10'})$,
où chaque $R^{10}$ et $R^{10'}$ est choisi indépendamment l'un de l'autre parmi H et alkyle en $C_{1-5}$,
où L et M sont choisis indépendamment l'un de l'autre, parmi -C(H)< et -N<,
où T est choisi parmi une liaison et alkylène en $C_{1-3}$,

- - où le radical alkylène en $C_{1-3}$ peut être substitué par méthyle,

où m peut être 0-3,
où n peut être 1-3,
où p peut être 0-3,
le cas échéant sous forme de leurs isomères optiques individuels, de mélanges de ces isomères, de racémiques, le cas échéant sous forme de leur sels d'addition d'acide avec des acides pharmaceutiquement acceptables.

**3.** Composés de la formule générale (I) selon l'une des revendications précédentes, où Ar est un substituant de la formule générale (II''') :

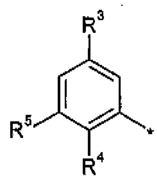

(II''')

- où $R^3$ représente $-C(CH_3)_3$, $-CH(CH_3)(C_2H_5)$, $-i-C_3H_7$, $-CH_2-i-C_3H_7$, $-CF_3$ ou $-CF_2CF_3$,
- où $R^4$ représente $-O-CH_3$ ou $-O-C_2H_5$,
- où $R^5$ est choisi parmi $-NH-S(O)_2-CH_3$, $-NH-S(O)_2-n-C_3H_7$, $-NH-S(O)_2-C-C_3H_5$, $-NH-C(O)-CH_3$, $-NH-C(O)-C_2H_5$, $-NH-C(O)-n-C_3H_7$, $-NH-C(O)-i-C_3H_7$, $-NH-C(O)-C-C_3H_5$, $-C(O)-NH_2$, $-C(O)-NH-CH_3$, $-C(O)-NH-C_2H_5$, $-C(O)-NH-i-C_3H_7$, $-C(O)-NH-C-C_3H_5$, $-C(O)-N(CH_3)_2$, $-CH_2-N(CH_3)_2$, $-CH_2-N(C_2H_5)_2$, $-CH_2-N(i-C_3H_7)_2$,

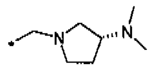,

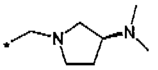, 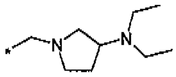, 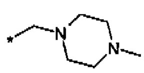, 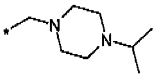,

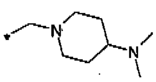, 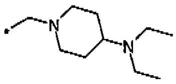, 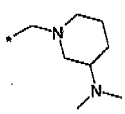,

$-S(O)-CH_3$, $-S(O)_2-CH_3$, $-CH_2-OH$, $-CH_2-S(O)-CH_3$, ou $-CH_2-S(O)_2-CH_3$, où $R^1$ est choisi parmi

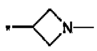, 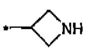, 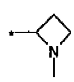, 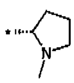, 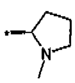,

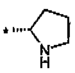, 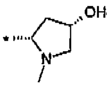, 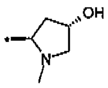, 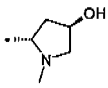, 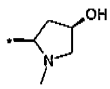, 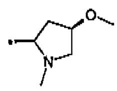,

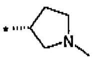, 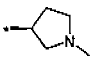, 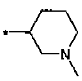, 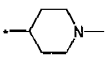, 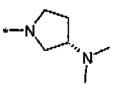, 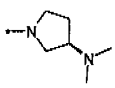,

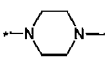, 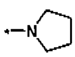, 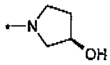, 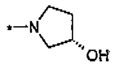, 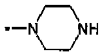, 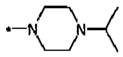,

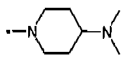,

où $R^2$ représente H,
où L est choisi parmi -CH< et -N<,
où M représente -N<,
où T représente une liaison,

où m représente ,1,
où n représente
le cas échéant sous forme de leurs isomères optiques individuels, de mélanges de ces isomères, de racémiques, le cas échéant sous forme de leur sels d'addition d'acide avec des acides pharmaceutiquement acceptables.

**4.** Composés de la formule générale (I) selon l'une des revendications précédentes, où
Ar est un substituant de la formule générale (II') :

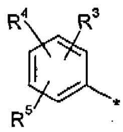

(II')

- où $R^3$ représente alkyle en $C_{1-8}$, cycloalkyle en $C_{3-8}$, $-CF_3$, $-CF_2CF_3$ ou

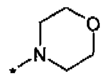

,

- - où le radical cycloalkyle en $C_{3-8}$ peut être substitué par alkyle en $C_{1-3}$,

- où $R^4$ représente H, alkyle en $C_{1-4}$ ou -O-alkyl en $C_{1-4}$,
- où $R^5$ est choisi parmi
- $-NH-S(O)_2$-alkyl en $C_{1-4}$,
- $-NH-S(O)_2$-cycloalkyl en $C_{3-5}$,
- $-N(CH_3)-S(O)_2$-alkyl en $C_{1-4}$,
- $-N(CH_3)-S(O)_2$-cycloalkyl en $C_{3-5}$,
- -NH-C(O)-alkyl en $C_{1-4}$,
- -NH-C(O)-cycloalkyl en $C_{3-5}$,
- $-N(CH_3)$-C(O)-alkyl en $C_{1-4}$,
- $-N(CH_3)$-C(O)-cycloalkyl en $C_{3-5}$,
- $-C(O)-NH_2$,
- -C(O)-NH-(alkyl en $C_{1-4}$),
- -C(O)-N-di(alkyl en $C_{1-4}$),
- -C (O)-NH-cycloalkyl en $C_{3-5}$,
- -C(O)-N(alkyl en $C_{1-4}$) (cycloalkyl en $C_{3-5}$),
- -S(O)-alkyl en $C_{1-4}$,
- -S(O)-cycloalkyl en $C_{3-5}$,
- $-S(O)_2$-alkyl en $C_{1-4}$,
- $-S(O)_2$-cycloalkyl en $C_{3-5}$,
- -alkyle en $C_{1-4}$-OH,
- $-CH_2$-S(O)-alkyl en $C_{1-4}$,
- $-CH_2$-S(O)-cycloalkyl en $C_{3-5}$.
- $-CH_2-S(O)_2$-alkyl en $C_{1-4}$,
- $-CH_2-S(O)_2$-cycloalkyl en $C_{3-5}$,
- -alkylène en $C_{1-4}$-N($R^8$,$R^{8'}$) ;

où chaque $R^8$ et $R^{8'}$ est choisi indépendamment l'un de l'autre parmi H et alkyle en $C_{1-5}$.
où les deux radicaux $R^8$ et $R^{8'}$ avec l'atome d'azote auquel ils sont liés, peuvent former un système cyclique ayant 4 à 6 membres, qui peut être substitué par -OH ou -N ($R^{10}$, $R^{10'}$),
où chaque $R^{10}$ et $R^{10'}$ est choisi indépendamment l'un de l'autre parmi H et alkyle en $C_{1-5}$,
le cas échéant sous forme de leurs isomères optiques individuels, de mélanges de ces isomères, de racémiques, le cas échéant sous forme de leur sels d'addition d'acide avec des acides pharmaceutiquement acceptables.

**5.** Composés de la formule générale (I) selon l'une des revendications précédentes,
où $R^1$ représente un système cycloalkyle en $C_{4-6}$, qui contient 1 à 2 atomes d'azote, qui peut être le cas échéant

substitué 1 à 2 fois par $R^7$, où $R^7$ peut être alkyle en $C_{1-3}$, hydroxyle, alcoxy en $C_{1-3}$, amino, (alkyl en $C_{1-3}$)amino, di (alkyl en $C_{1-3}$)amino,
le cas échéant sous forme de leurs isomères optiques individuels, de mélanges de ces isomères, de racémiques, le cas échéant sous forme de leur sels d'addition d'acide avec des acides pharmaceutiquement acceptables.

**6.** composés de la formule générale (I) selon l'une des revendications précédentes, où $R^1$ représente azétidin-2-yle, azétidin-3-yle, 1-méthylazétidin-2-yle, 1-méthylazétidin-3-yle, 1-éthylazétidin-2-yle, 1-éthylazétidin-3-yle, pyrrolidin-1-yle, pyrrolidin-2-yle, pyrrolidin-3-yle, 3-hydroxypyrrolidin-1-yle, 3-aminopyrrolidin-1-yle, 3-méthylaminopyrrolidin-1-yle, 3-diméthylaminopyrrolidin-1-yle, 3-éthylaminopyrxolidin-1-yle, 3-diéthylaminopyrrolidin-1-yle, 1-méthylpyrrolidin-2-yle, 1-méthylpyrrolidin-3-yle, 1-éthylpyrrolidin-2-yle, 1-éthylpyrrolidin-3-yle, 1-méthyl-4-hydroxypyrrolidin--2-yle, 1-méthyl-4-méthoxypyrrolidin-2-yle, pipéridin-1-yle, pipéridin-2-yle, pipéridin-3-yle, pipéridin-4-yle, 4-aminopipéridin-1-yle, 4-méthylaminopipéridin-1-yle, d-diméthylaminopipéridin-1-yle, 4-éthylaminopipéridin-1-yle, 4-diéthylaminopipéridin-1-yle, 1-méthylpipéridin-2-yle, 1-méthylpipéridin-3-yle, 1-méthylpipéridin-4-yle, 1-éthylpipéridin-2-yle, 1-éthylpipéridin-3-yle, 1-éthylpipéridin-4-yle, pipérazin-1-yle, 4-méthylpipérazin-1-yle, 4-éthylpipérazin-1-yle, 4-isopropylpipérazin-1-yle,
le cas échéant sous forme de leurs isomères optiques individuels, de mélanges de ces isomères, de racémiques, le cas échéant sous forme de leur sels d'addition d'acide avec des acides pharmaceutiquement acceptables.

**7.** Composés de la formule générale (I) selon l'une des revendications 1-4, où $R^1$ représente un système cycloalkyle en $C_{4-6}$, qui contient 1 à 2 atomes d'azote, où $R^1$ est le cas échéant substitué par un radical choisi parmi alkyle en $C_{1-4}$, -OH, -O-alkyl en $C_{1-4}$, et $-N(R^9,R^{9'})$ ,
où $R^9$, $R^{9'}$ ont les significations données ci-dessus,
le cas échéant sous forme de leurs isomères optiques individuels, de mélanges de ces isomères, de racémiques, le cas échéant sous forme de leur sels d'addition d'acide avec des acides pharmaceutiquement acceptables.

**8.** Composés de la formule générale (I) selon l'une des revendications 1-4, où $R^1$ est choisi parmi

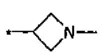 , 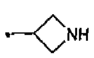 , 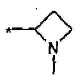 , 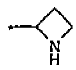 , 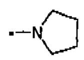 , 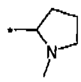 ,

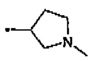 , 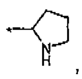 , 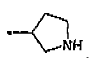 , 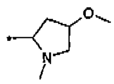 , 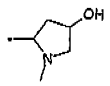 , 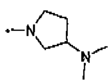 ,

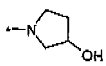 , 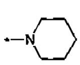 , 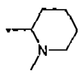 , 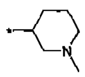 , 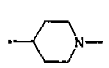 , 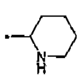 , 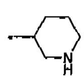 ,

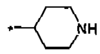 , 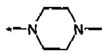 , 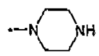 , 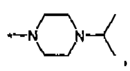 ,

le cas échéant sous forme de leurs isomères optiques individuels, de mélanges de ces isomères, de racémiques, le cas échéant sous forme de leur sels d'addition d'acide avec des acides pharmaceutiquement acceptables.

**9.** Composés de la formule générale (I) selon l'une des revendications précédentes, où $R^1$ est choisi parmi -H, -F, -Cl et $-CH_3$, et où p est choisi parmi 0, 1, 2 ou 3,
le cas échéant sous forme de leurs isomères optiques individuels, de mélanges de ces isomères, de racémiques, le cas échéant sous forme de leur sels d'addition d'acide avec des acides pharmaceutiquement acceptables.

**10.** Composés de la formule générale (I) selon l'une des revendications précédentes, où L et M sont choisis indépendamment l'un de l'autre, parmi -CH< et -N<, le cas échéant sous forme de leurs isomères optiques individuels, de mélanges de ces isomères, de racémiques, le cas échéant sous forme de leur sels d'addition d'acide avec des acides pharmaceutiquement acceptables.

**11.** Composés de la formule générale (I) selon l'une des revendications précédentes, où T est choisi parmi une liaison et alkylène en $C_{1-3}$, où le radical alkylène en $C_{1-3}$ peut être substitué par méthyle,
le cas échéant sous forme de leurs isomères optiques individuels, de mélanges de ces isomères, de racémiques,
le cas échéant sous forme de leur sels d'addition d'acide avec des acides pharmaceutiquement acceptables.

**12.** Composés de la formule générale (I) selon l'une des revendications précédentes, pour le traitement de maladies sélectionnées parmi l'asthme ou la broncho-pneumopathie chronique obstructive (COPD).

**13.** Utilisation des composés de la formule générale (I) selon l'une des revendications précédentes, pour la préparation d'un médicament pour le traitement de maladies sélectionnées parmi l'asthme ou la broncho-pneumopathie chronique obstructive (COPD).

**14.** Composés de la formule générale (I) selon la revendication 1, choisis parmi
le (5-t-butyl-3-méthanesulfonylamino-2-méthoxyphényl)amide de l'acide 1-méthyl-7-[4-(1-méthylpipéridin-4-carbonyl)pipérazin-1-ylméthyl]-1H-indol-2-carboxylique,
le (S)-(5-t-butyl-3-méthanesulfonylamino-2-méthoxyphényl)amide de l'acide 1-méthyl-7-[4-(1-méthylpyrrolidin-2-carbonyl)pipérazin-1-ylméthyl]-1H-indol-2-carboxylique,
le (5-t-butyl-3-méthanesulfonylamino-2-méthoxyphényl)amide de l'acide 1-méthyl-7-[4-(1-méthylazétidin-3-carbonyl)pipérazin-1-ylméthyl]-1H-indol-2-carboxylique,
le (R)-(5-t-butyl-3-méthanesulfonylamino-2-méthoxyphényl)amide de l'acide 1-méthyl-7-[4-(1-néthylpyrrolidin-2-carbonyl)pipérazin-1-ylméthyl]-1H-indol-2-carboxylique,
le (S)-(5-t-butyl-3-méthanesulfonylamino-2-méthoxyphényl)amide de l'acide 1-méthyl-7-[4-(pyrrolidin-2-carbonyl) pipérazin-1-ylméthyl]-1H-indol-2-carboxylique,
le (5-t-butyl-3-méthanesulfonylamino-2-méthoxyphényl)amide de l'acide 1-méthyl-7-[4-(1-méthylazétidin-2-carbonyl)pipérazin-1-ylméthyl]-1H-indol-2-carboxylique,
le (R)-(5-t-butyl-3-méthanesulfonylamino-2-méthoxyphényl)amide de l'acide 1-méthyl-7-[1-(1-méthylpyrrolidin-2-carbonyl)pipéridin-4-ylméthyl]-1H-indol-2-carboxylique,
le (S)-(5-t-butyl-3-méthanesulfonylamino-2-méthoxyphényl)amide de l'acide 1-méthyl-7-[1-(1-méthylpyrralidin-2-carbonyl)pipéridin-4-ylméthyl]-1H-indol-2-carboxylique,
le (5-t-butyl-3-méthanesulfonylamino-2-méthoxyphényl)amide de l'acide 7-[4-((2S,4R)-4-hydroxy-1-méthylpyrrolidin-2-carbonyl)pipérazin-1-ylméthyl]-1-méthyl-1H-indol-2-carboxylique,
le (5-t-butyl-3-méthanesulfonylamino-2-méthoxyphényl)amide de l'acide 7-[4-((2R,4R)-4-méthoxy-1-méthylpyrrolidin-2-carbonyl)pipérazin-1-ylméthyl]-1-méthyl-1H-indol-2-carboxylique,
le (5-t-butyl-3-méthanesulfonylamino-2-méthoxyphényl)amide de l'acide 7-[4-((2R,4R)-4-hydroxy-1-méthylpyrrolidin-2-carbanyl)pipérazin-1-ylméthyl]-1-méthyl-1H-indol-2-carboxylique,
le (5-t-butyl-3-méthanesulfonylamino-2-méthoxyphényl)amide de l'acide 7-[A-((2R,4S)-4-hydroxy-1-méthylpyrrolidin-2-carbonyl)pipérazin-1-ylméthyl]-1-méthyl-1H-indol-2-carboxylique,
le (5-t-butyl-3-méthanesulfonylamino-2-méthoxyphényl)amide de l'acide 7-[4-((2S,4S)-4-hydroxy-1-méthylpyrrolidin-2-carbonyl)pipérazin-1-ylméthyl]-1-méthyl-1H-indol-2-carboxylique,
le (5-t-butyl-3-méthanesulfonylamino-2-méthoxyphényl)amide de l'acide 1-méthyl-7-[4-(2-pyrrolidin-1-ylpropionyl) pipérazin-1-ylméthyl]-1H-indol-2-carboxylique,
le (5-t-butyl-3-méthanesulfonylamino-2-méthoxyphényl)amide de l'acide 7-[4-2-(4-isopropylpipérazin-1-yl)acétyl]piperazin-1-ylméthyl]-1-méthyl-1H-indol-2-carboxylique,
le (S)-(5-t-butyl-3-méthanesulfonylamino-2-méthoxyphényl)amide de l'acide 7-{4-[2-(3-hydroxypyrrolidin-1-yl)acétyl]pipérazin-1-ylméthyl}-1-méthyl-1H-indol-2-carboxylique,
le (5-t-butyl-3-méthanesulfonylamino-2-méthoxyphényl)amide de l'acide 1-méthyl-7-[4-(2-pyrrolidin-1-ylacétyl)pipérazin-1-ylméthyl]-1H-indol-2-carboxylique,
le (5-t-butyl-3-méthanesulfonylamino-2-méthoxyphényl)amide de l'acide 1-méthyl-7-{4-[2-(4-méthylpipérazin-1-yl) acétyl]pipérazin-1-ylméthyl}-1H-indol-2-carboxylique,
le (5-t-butyl-3-méthanesulfonylamino-2-méthox-y-phényl)amide de l'acide 1-méthyl-7-[1-((2S)-1-méthylpyrrolidin-2-carbonyl)azépan-4-ylmethyl]-1H-indol-2-carboxylique,
le (5-t-butyl-3-méthanesulfonylamino-2-méthoxyphényl)amide de l'acide 1-méthyl-7-[1-(1-méthylpipéridin-4-carbonyl)azépan-4-ylméthyl]-1H-indol-2-carboxylique,
le (5-t-butyl-3-méthanesulfonylamino-2-méthoxyphényl)amide de l'acide 1-méthyl-7-[1-((2R)-1-méthylpyrrolidin-2-carbonyl)azépan-4-ylmethyl]-1H-indol-2-carboxylique,
le (5-t-butyl-3-méthanesulfonylamino-2-méthoxyphényl)amide de l'acide 1-méthyl-7-[4-((2R)-1-méthylpyrrolidin-2-carbonyl)-[1,4]-diazépan-1-ylméthyl]-1H-indol-2-carboxylique,
le (5-t-butyl-3-méthanesulfonylamino-2-méthoxyphényl)amide de l'acide 1-méthyl-7-[4-(4-méthylpipérazin-1-carbonyl)pipéridin-1-ylméthyl]-1H-indol-2-carboxylique,
le (S)-(5-t-butyl-3-méthanesulfonylamino-2-méthoxyphényl)amide de l'acide 7-[4-(3-diméthylaminopyrrolidin-1-carbonyl)pipéridin-1-ylméthyl]-1-méthyl-1H-indol-2-carboxylique,
le (R)-(5-t-butyl-3-méthanesulfonylamino-2-méthoxyphényl)amide de l'acide 7-[4-(3-diméthylaminopyrrolidin-1-carbanyl)pipéridin-1-ylméthyl]-1-méthyl-1H-indol-2-carboxylique,
le (3-morpholin-4-ylphényl)amide de l'acide (S)-1-méthyl-7-[4-(1-méthylpyrrolidin-2-carbonyl)pipérazin-1-ylméthyl]-1H-indol-2-carboxylique,
le (R)-(5-t-butyl-3-méthanesulfonylamino-2-méthoxyphényl)amide de l'acide 7-[4-(3-diméthylaminopyrrolidin-1-carbonyl)pipérazin-1-ylméthyl]-1-méthyl-1H-indol-2-carboxylique,
le (S)-(5-t-butyl-3-méthanesulfonylamino-2-méthoxyphényl)amide de l'acide 7-[4-(3-diméthylaminopyrrolidin-1-carbonyl)pipérazin-1-ylméthyl]-1-méthyl-1H-indol-2-carboxylique,
le (5-t-butyl-3-méthanesulfonylamino-2-méthoxyphényl)amide de l'acide 7-[4-(azétidin-3-carbonyl)pipérazin-1-ylméthyl]-1-méthyl-1H-indol-2-carboxylique,
le (5-t-butyl-3-méthanesulfonylamino-2-méthoxyphényl)amide de l'acide 1-méthyl-7-[4-(1-méthylpipéridin-3-carbonyl)pipérazin-1-ylméthyl]-1H-indol-2-carboxylique,
le (S)-(5-t-butyl-3-méthanesulfonylamino-2-méthoxyphényl)amide de l'acide 1-méthyl-7-[4-(1-méthylpyrrolidin-3-carbonyl)pipérazin-1-ylméthyl]-1H-indol-2-carboxylique,
le (S)-(5-t-butyl-3-méthanesulfonylamino-2-méthoxyphényl)amide de l'acide 1-méthyl-7-[4-(1-méthylpyrrolidin-3-carbonyl)-[1,4]diazépan-1-ylméthyl]-1H-indol-2-carboxylique,
le (R)-(5-t-butyl-3-méthanesulfonylamino-2-méthoxyphényl)amide de l'acide 1-méthyl-7-[4-(1-méthylpyrrolidin-3-carbonyl)-[1,4]diazépan-1-ylméthyl]-1H-indol-2-carboxylique,
le (5-t-butyl-3-méthanesulfanylamino-2-méthoxyphényl)amide de l'acide 7-{4-[2-(4-diméthylaminopipéridin-1-yl)acétyl]pipérazin-1-ylméthyl}-1-méthyl-1H-indol-2-carboxylique,
le (R)-(5-t-butyl-3-méthanesulfonylamino-2-méthoxyphényl)amide de l'acide 7-{4-[2-(3-hydroxypyrrolidin-1-yl)acétyl]pipérazin-1-ylméthyl}-1-méthyl-1H-indol-2-carboxylique,
le (R)-(5-t-butyl-3-méthanesulfonylamino-2-méthoxyphényl)amide de l'acide 7-{4-[2-(3-diméthylaminopyrrolidin-1-yl)acétyl]pipérazin-1-ylméthyl}-1-méthyl-1H-indol-2-carboxylique,
le (S)-(5-t-butyl-3-méthanesulfanylamino-2-méthoxyphényl)amide de l'acide 7-{4-[2-(3-diméthylaminopyrrolidin-1-yl)acétyl]pipérazin-1-ylméthyl}-1-méthyl-1H-indol-2-carboxylique,
le (5-t-butyl-3-méthanesulfonylamino-2-méthoxyphényl)amide de l'acide 1-méthyl7-[4-(2-pipérazin-1-ylacétyl)pipérazin-1-ylméthyl]-1H-indol-2-carboxylique,
le (5-t-butyl-3-méthanesulfonylamino-2-méthoxyphényl)amide de l'acide 1-méthyl-7-[4-((2S)-1-méthylpyrrolidin-2-carbonyl)-[1,4]diazépan-1-ylméthyl]-1H-indol-2-carboxylique,
le (5-t-butyl-3-méthanesulfonylamino-2-méthoxyphényl)amide de l'acide 1-méthyl-7-[4-(1-méthylpipéridin-4-carbanyl)-[1,4]diazépan-1-ylméthyl]-1H-indol-2-carboxylique,
le (S)-(5-isobutyl-3-méthanesulfonylamino-2-méthoxyphényl)amide de l'acide 1-méthyl-7-[4-(1-méthylpyrrolidin-2-carbonyl)pipérazin-1-ylméthyl]-1H-indol-2-carboxylique,
le (S)-[5-t-butyl-2-méthoxy-3-(4-méthylpipérazin-1-ylméthyl)phényl]amide de l'acide 1-méthyl-7-[4-(1-méthylpyrrolidin-2-carbonyl)pipérazin-1-ylméthyl]-1H-indol-2-carboxylique,
le (S)-{5-t-butyl-3-[(diisopropylamino)méthyl]-2-méthoxyphényl]amide de l'acide 1-méthyl-7-[4-(1-méthylpyrrolidin-2-carbonyl)pipérazin-1-ylméthyl]-1H-indol-2-carboxylique,
le (5)-(5-t-butyl-3-diéthylaminométhyl-2-méthoxyphényl)amide de l'acide 1-méthyl-7-[4-(1-méthylpyrrolidin-2-carbonyl)pipérazin-1-ylméthyl]-1H-indol-2-carboxylique,
le [5-t-butyl-3-(3-diméthylaminopipéridin-1-ylméthyl)-2-méthoxyphényl]amide de l'acide 1-méthyl-7-[4-((2S)-1-méthylpyrrolidin-2-carbonyl)pipérazin-1-ylméthyl]-1H-indol-2-carboxylique,
le [5-t-butyl-2-méthoxy-3-(3-diéthylaminopyrrolidin-1-ylméthyl)-2-méthoxyphényl]amide de l'acide 1-méthyl-7-14-((2S)-1-méthylpyrrolidin-2-carbonyl)-pipérazin-1-ylméthyl]-1H-indol-2-carboxylique,
le (S)-[5-t-butyl-3-(4-diméthylaminopipéridin-1-ylméthyl)-2-méthoxyphényl]amide de l'acide 1-méthyl-7-[4-(1-méthylpyrrolidin-2-carbonyl)pipérazin-1-ylméthyl]-1H-indol-2-carboxylique,
le (S)-[5-t-butyl-3-(4-diéthylaminopipéridin-1-ylméthyl)-2-méthoxyphényl]amide de l'acide 1-méthyl-7-[4-(1-méthylpyrrolidin-2-carbonyl)pipérazin-1-ylméthyl]-1H-indol-2-carboxylique,
le (S)-[5-t-butyl-3-(4-isopropylpipérazin-1-ylméthyl)-2-méthoxyphényl]amide de l'acide 1-méthyl-7-[4-(1-méthylpyrrolidin-2-carbonyl)pipérazin-1-ylméthyl]-1H-indol-2-carboxylique,
le (S)-(5-t-butyl-3-cyclopropylcarbamoyl-2-méthoxyphényl)amide de l'acide 1-méthyl-7-[4-(1-méthyl-pyrrolidin-2-carbonyl)pipérazin-1-ylméthyl]-1H-indol-2-carboxylique,
le (S)-(5-t-butyl-3-isopropylcarbamoyl-2-méthoxyphényl)amide de l'acide 1-methyl-7-[4-(1-méthylpyrrolidin-2-carbonyl)pipérazin-1-ylméthyl]-1H-indol-2-carboxylique,
le (S)-(5-t-butyl-3-éthylcarbamoyl-2-méthoxyphényl)amide de l'acide 1-méthyl-7-[4-(1-méthylpyrrolidin-2-carbonyl)

pipérazin-1-ylméthyl]-1H-indol-2-carboxylique,
le (S)-(5-t-butyl-2-méthoxy-3-méthylcarbamoylphényl)amide de l'acide 1-méthyl-7-[4-(1-méthylpyrrolidin-2-carbonyl)pipérazin-1-ylméthyl]-1H-indol-2-carboxylique,
le (S)-(5-t-butyl-3-carbamoyl-2-méthoxyphényl)-amide de l'acide 1-méthyl-7-14-(1-méthylpyrrolidin-2-carbonyl)pipérazin-1-ylméthyl]-1H-indol-2-carboxylique,
le (S)-(5-t-butyl-3-diméthylcarbamoyl-2-méthoxyphényl)amide de l'acide 1-méthyl-7-[4-(1-méthylpyrrolidin-2-carbonyl)pipérazin-1-ylméthyl]-1H-indol-2-carboxylique,
le (5-s-butyl-3-méthanesulfonylamino-2-méthoxyphényl)amide de l'acide 1-méthyl-7-[4-((2S)-1-méthylpyrrolidin-2-carbonyl)pipérazin-1-ylméthyl]-1H-indol-2-carboxylique,
le (S)-(5-isopropyl-3-méthanesulfonylamino-2-méthoxyphényl)amide de l'acide 1-méthyl-7-[4-(1-méthylpyrrolidin-2-carbonyl)pipérazin-1-ylméthyl]-1H-indol-2-carboxylique,
le (5-t-butyl-3-méthanesulfinylamino-2-méthoxyphényl)amide de l'acide 1-méthyl-7-[4-((2S)-1-méthylpyrrolidin-2-carbonyl)pipérazin-1-ylméthyl]-1H-indol-2-carboxylique,
le (S)-(5-t-butyl-3-méthanesulfonyl-2-méthoxyphényl)amide de l'acide 1-méthyl-7-[4-(1-méthylpyrrolidin-2-carbonyl)pipérazin-1-ylméthyl]-1H-indol-2-carboxylique,
le (S)-(5-t-butyl-3-méthanesulfonylméthyl-2-méthoxyphényl)amide de l'acide 1-méthyl-7-[4-(1-méthylpyrrolidin-2-carbonyl)pipérazin-1-ylméthyl]-1H-indol-2-carboxylique,
le (5-t-butyl-3-méthanesulfinylméthyl-2-méthoxyphényl)amide de l'acide 1-méthyl-7-[4-((2S)-1-méthylpyrrolidin-2-carbonyl)pipérazin-1-ylméthyl]-1H-indol-2-carboxylique,
le (S)-(5-t-butyl-3-méthanesulfonylamino-2-éthoxyphényl)amide de l'acide 1-méthyl-7-[4-(1-méthylpyrrolidin-2-carbonyl)pipérazin-1-ylméthyl]-1H-indol-2-carboxylique,
le (S)-(5-t-butyl-2-méthoxy-3-propionylaminophényl)amide de l'acide 1-méthyl-7-[4-(1-méthylpyrrolidin-2-carbonyl) pipérazin-1-ylméthyl]-1H-indol-2-carboxylique,
le (S)-(5-t-butyl-3-butyrylamino-2-méthoxyphényl)-amide de l'acide 1-méthyl-7-[4-(1-méthylpyrrolidin-2-carbonyl)pipérazin-1-ylméthyl]-1H-indol-2-carboxylique,
le (S)-(3-acétylamine-5-t-butyl-2-méthoxyphényl)-amide de l'acide 1-méthyl-7-[4-(1-méthylpyrrolidin-2-carbonyl)pipérazin-1-ylméthyl]-1H-indol-2-carboxylique,
le (S)-(5-t-butyl-3-isobutyrylamine-2-méthoxyphényl)amide de l'acide 1-méthyl-7-[4-(1-méthylpyrrolidin-2-carbonyl) pipérazin-1-ylméthyl]-1H-indol-2-carboxylique,
le (S)-(5-t-butyl-3-(cyclopropancarbonylamino)-2-méthoxyphényl)amide de l'acide 1-méthyl-7-[4-(1-méthylpyrrolidin-2-carbonyl)pipérazin-1-ylméthyl]-1H-indol-2-carboxylique,
le (S)-[5-t-butyl-2-méthoxy-3-(propan-1-ylsulfonylamino)phényl]amide de l'acide 1-méthyl-7-[4-(1-méthylpyrrolidin-2-carbonyl)pipérazin-1-ylméthyl]-1H-indol-2-carboxylique,
le (S)-(5-t-butyl-3-cyclopropansulfonylamino-2-méthoxyphényl)amide de l'acide 1-méthyl-7-[4-(1-méthylpyrrolidin-2-carbonyl)pipérazin-1-ylméthyl]-1H-indol-2-carboxylique,
le (S)-(3-méthanesulfonylamino-2-méthoxy-5-pentafluoroéthylphényl)amide de l'acide 1-méthyl-7-[4-(1-méthylpyrralidin-2-carbonyl)pipérazin-1-ylméthyl]-1H-indol-2-carboxylique,
le (S)-(3-méthanesulfonylamino-2-métho-xy-5-trifluorométhylphényl)amide de l'acide 1-méthyl-7-[4-(1-méthylpyrrolidin-2-carbonyl)pipérazin-1-ylméthyl]-1H-indol-2-carboxylique,
le (S)-(5-t-butyl-3-hydroxyméthyl-2-méthoxyphényl)amide de l'acide 1-methyl-7-[4-(1-méthylpyrrolidin-2-carbonyl) pipérazin-1-ylméthyl]-1H-indol-2-carboxylique,
leurs tautomères, leurs stéréoisomères et leurs mélanges.

**15.** Sels physiologiquement compatibles des composés selon l'une ou plusieurs des revendications 1 à 14 avec des acides minéraux ou organiques.

**16.** Utilisation d'un composé selon l'une ou plusieurs des revendications 1 à 14 ou d'un sel physiologiquement compatible selon la revendication 15, comme agent pharmaceutique.

**17.** Agent pharmaceutique, contenant un composé selon l'une ou plusieurs des revendications 1 à 14 ou un sel physiologiquement compatible selon la revendication 15, le cas échéant avec un ou plusieurs supports inertes, agents de conservation et/ou diluants.

**18.** Utilisation d'au moins un composé selon l'une ou plusieurs des revendications 1 à 14 ou d'un sel physiologiquement compatible selon la revendication 15, pour la préparation d'un agent pharmaceutique, qui est approprié pour le traitement ou la prophylaxie de maladies des voies respiratoires.

**19.** Utilisation selon la revendication 18, **caractérisée en ce que** la maladie des voies respiratoires consiste en la

broncho-pneumopathie chronique obstructive (COPD) ou l'asthme.

**20.** Procédé d'un agent pharmaceutique selon la revendication 17, **caractérisé en ce que** l'on traite par une voie non chimique, un composé selon l'une ou plusieurs des revendications 1 à 14 ou un sel physiologiquement compatible selon la revendication 15, dans un ou plusieurs supports inertes, agents de conservation et/ou diluants.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2003087085 A
- WO 2005016918 A
- WO 2005108387 A
- WO 2006026235 A
- US 20040186114 A


### In der Beschreibung aufgeführte Nicht-Patentliteratur


- **P. J. Bames.** *Journal of Allergy and Clinical Immunology,* 2007, vol. 119 (5), 1055-1062
- **M. F. Fitzgerlad ; J. C. Fox.** *Drug Discovery Today,* 2007, vol. 12 (11/12), 479-486
- *J. Org. Chem.,* 2007, vol. 72, 2978-2987
- *J. Chem. Soc.,* 1983, vol. 48, 4156
- *J. Org. Chem.,* 1976, vol. 41, 957
- *Synthesis,* 1994, 639
- *Tetrahedron Lett.,* 1992, 5029
- *Org. Lett.,* 2007, vol. 9, 1597-1600
- **Allinger N. L. ; Eliel E. L.** Topics in Stereochemistry. Wiley Interscience, 1971, vol. 6
- *J. Med. Chem.,* 2007, vol. 50 (17), 4016-26